# EUROPEAN PATENT SPECIFICATION

(11) **EP 2 635 707 B1**
(45) Date of publication and mention of the grant of the patent: **27.03.2019**
(21) Application number: 11837763.9
(22) Date of filing: 07.11.2011
(51) Int. Cl.: C12Q 1/68, A61K 31/7088, A61K 48/00, A61P 13/12, C12N 5/071, C12N 5/10, C12N 15/09, G01N 33/53

(54) **METHOD OF EXAMINING POLYCYSTIC KIDNEY DISEASE AND METHOD OF SCREENING FOR THERAPEUTIC AGENT OF THE DISEASE**
VERFAHREN ZUR UNTERSUCHUNG DER POLYZYSTISCHEN NIERENERKRANKUNG UND VERFAHREN ZUM SCREENING AUF EIN KRANKHEITSBEHANDLUNGSMITTEL
PROCÉDÉ D'EXAMEN DE MALADIE RÉNALE POLYKYSTIQUE ET PROCÉDÉ DE CRIBLAGE D'UN AGENT THÉRAPEUTIQUE DE LA MALADIE

(30) Priority: 01.09.2011 US 201161530269 P; 05.11.2010 US 410757 P
(43) Date of publication of application: 11.09.2013
(73) Proprietor: Kyoto University, Kyoto-shi, Kyoto 606-8501 (JP)
(72) Inventor: OSAFUNE, Kenji, Kyoto-shi Kyoto 606-8501 (JP); TOYODA, Taro, Kyoto-shi Kyoto 606-8501 (JP); SHIOTA, Fumihiko, Kyoto-shi Kyoto 606-8501 (JP); NAKAO, Kazuwa, Kyoto-shi Kyoto 606-8501 (JP); SONE, Masakatsu, Kyoto-shi Kyoto 606-8501 (JP); TAURA, Daisuke, Kyoto-shi Kyoto 606-8501 (JP)
(74) Representative: D Young & Co LLP
(86) International application number: PCT/JP2011/006203
(87) International publication number: WO 2012/060109

(56) References cited:
- JP-A- 2001 520 502
- JP-A- 2004 313 194
- JP-A- 2006 288 265
- NAKAMURA T ET AL: "ELEVATION OF SERUM LEVELS OF METALLOPROTEINASE-1, TISSUE INHIBITOR OF METALLOPROTEINASE-1 AND TYPE IV COLLAGEN, AND PLASMA LEVELS OF METALLOPROTEINASE-9 IN POLYCYSTIC KIDNEY DISEASE", AMERICAN JOURNAL OF NEPHROLOGY, S. KARGER AG, CH, vol. 20, no. 1, 1 January 2000 (2000-01-01), pages 32-36, XP008011473, ISSN: 0250-8095, DOI: 10.1159/000013552
- HUSSON H ET AL.: "New insights into ADPKD molecular pathways using combination of SAGE and microarray technologies", GENOMICS, vol. 84, 2004, pages 497-510, XP002721494,
- JIANG X ET AL.: "Inhibition of MMP-1 expression by antisense RNA decreases invasiveness of human chondrosarcoma", JOURNAL OF ORTHOPAEDIC RESEARCH, vol. 21, no. 6, November 2003 (2003-11), pages 1063-1070, XP002721495,
- JAE EUN LEE ET AL: "THE GENE EXPRESSION PROFILE OF CYST EPITHELIAL CELLS IN AUTOSOMAL DOMINANT POLYCYSTIC KIDNEY DISEASE PATIENTS", JOURNAL OF BIOCHEMISTRY AND MOLECULAR BIOLOGY, KOREAN SOCIETY FOR BIOCHEMISTRY AND MOLECULAR BIOLOGY, KR, vol. 37, no. 5, 30 September 2004 (2004-09-30), pages 612-617, XP008061324, ISSN: 1225-8687
- SONG X ET AL.: "Systems biology of autosomal dominant polycystic kidney disease (ADPKD): computional identification of gene expression pathways and integrated regulatory networks", HUMAN MOLECULAR GENETICS, vol. 18, no. 13, 2009, pages 2328-2343, XP002721496,
- OSAFUNE, K. ET AL.: 'Establishment of ''polycystic kidney disease''-specific iPS cells and studies aiming at understanding pathological mechanism' NIHON JINZO GAKKAISHI vol. 52, no. 3, May 2010, pages 302, 02 - 04-27, XP008168232
- NAKAHATA, T. ET AL.: 'Future medical care with the use of iPS cells' NIHON SYUJUTSU KANGO GAKKAISHI vol. 6, no. 2, August 2010, page 142, XP008168990
- OSAFUNE, K. ET AL.: 'Modeling for polycystic Kidney disease with patient-dirived pluripotent stem cells' NIHON SAISEI IRYOU GAKKAI ZASSHI vol. 8, no. 3, 2009, pages 38 - 42, XP008168233
- WANG, D. ET AL.: 'Endothelial dysfunction and reduced nitric oxide in resistance arteries in autosomal-dominant polycystic kidney disease' KIDNEY INTERNATIONAL vol. 64, 2003, pages 1381 - 1388, XP055107413
- IKEDA, H. ET AL.: 'Pathological investigation on cerebral artery circle in the autopsy case of Polycystic kidney' BRAIN & NERVE vol. 39, no. 10, 1987, pages 909 - 913, XP008168210
- TSUNODA, T.: 'Large-scale Analysis 01 Gene Expression and Polymorphisms for Disease Diagnostics Based on Genome-wide Data' NIHON SEITAI IKOUGAKU vol. 44, no. 3, 2006, pages 410 - 415, XP008168211
- YAMASHITA, J. ET AL.: 'Vascular cell differentiation from ES and iPS cells' NIHON SAISEI IRYOU GAKKAI ZASSHI vol. 8, no. 1, 2009, pages 28 - 33, XP008168991
- NARAZAKI, G. ET AL.: 'Directed and systematic differentiation of cardiovascular cells from mouse induced pluripotent stem cells' CIRCULATION vol. 118, 2008, pages 498 - 506, XP055067463
- YAMASHITA, J. ET AL.: 'Flkl-positive cells derived from embryonic stem cells serve as vascular progenitors' NATURE vol. 408, no. 2, 2000, pages 92 - 96, XP001146921
- ABOUALAIWI, W. A. ET AL.: 'Ciliary Polycystin-2 is a mechanosensitive calcium channnel involved in nitric oxide signaling cascades' CIRCULATION vol. 104, 2009, pages 860 - 869, XP055107406
- OSAFUNE, K.: 'Production of in-vitro disease model with the use of iPS cells from patients with intractable kidney disease' KOUSEI ROUDOU KAGAKU KENKYUUHI HOJOKIN vol. H21ST, December 2010, pages 27 - 28, XP008169436
- OSAFUNE, K. ET AL.: 'Kidney regeneration and disease modeling using iPS cell technology' IGAKU NO AYUMI vol. 236, no. 13, March 2011, pages 1191 - 1192, XP008168231
- Andreas D. Kistler ET AL: "Identification of a unique urinary biomarker profile in patients with autosomal dominant polycystic kidney disease", KIDNEY INTERNATIONAL, vol. 76, no. 1, 1 July 2009 (2009-07-01), pages 89-96, XP55319783, LONDON, GB ISSN: 0085-2538, DOI: 10.1038/ki.2009.93
- Esther Meijer ET AL: "Association of Urinary Biomarkers With Disease Severity in Patients With Autosomal Dominant Polycystic Kidney Disease: A Cross-sectional Analysis", AMERICAN JOURNAL OF KIDNEY DISEASES., vol. 56, no. 5, 1 November 2010 (2010-11-01), pages 883-895, XP55319763, US ISSN: 0272-6386, DOI: 10.1053/j.ajkd.2010.06.023

### SEQUENCE LISTING

<110> Kyoto University
<120> Method of Examining Polycystic Kidney Disease and Method of Screening for Therapeutic Agent of the Disease
<130> PH-4536-PCT
<150> US 61/410,757
   <151> 2010-11-05
<150> US 61/530,269
   <151> 2011-09-01
<160> 107
<170> PatentIn version 3.1
<210> 1
   <211> 3434
   <212> DNA
   <213> Homo sapiens
<220>
   <221> CDS
   <222> (719)..(2338)
   <223>
<400> 1
   gaggtacacg aggagttact ggaaacggcg cttctgtgga ggagccgggg gggatgggga      60
   gtagagggag ggggccctgt tgcctcagcg ccccgaggtc gtggagcggc agcagctgca     120
   gccggagcag caccagcaac agcaacagcg agcgggacgg agttaggacc gctcggagcg     180
   cacaggtctc gagggtgttg gtgccagaag aaaagaatga ttgatgggaa acagacaccg     240
   ggctatagac actcatcctt ttgcttcaga tactgatatc tcagcctgct tgagcatccc     300
   ttgtgagctg tgaacattga ggatcactca gggttatcgg atgtacaacg ggagagccat     360
   cgctttgcta aattattatc tgcaattgga catcttttac aaaaaccaaa ctagacctga     420
   gtctaataga tatgttctaa gacaaagaaa aagctgcaag ttgttaacgc ctaacacaca     480
   agtatgttag gcttccacca aagtcctcaa tatacctgaa tacgcacaat atcttaactc     540
   ttcatatttg gttttgggat ctgctttgag gtcccatctt catttaaaaa aaaatacaga     600
   gacctaccta cccgtacgca tacatacata tgtgtatata tatgtaaact agacaaagat     660
   cgcagatcat aaagcaagct ctgctttagt ttccaagaag attacaaaga atttagag       718
   atg tat ttg tca aga ttc ctg tcg att cat gcc ctt tgg gtt acg gtg       766
   Met Tyr Leu Ser Arg Phe Leu Ser Ile His Ala Leu Trp Val Thr Val
   1               5                   10                  15
   tcc tca gtg atg cag ccc tac cct ttg gtt tgg gga cat tat gat ttg       814
   Ser Ser Val Met Gln Pro Tyr Pro Leu Val Trp Gly His Tyr Asp Leu
               20                  25                  30
   tgt aag act cag att tac acg gaa gaa ggg aaa gtt tgg gat tac atg       862
   Cys Lys Thr Gln Ile Tyr Thr Glu Glu Gly Lys Val Trp Asp Tyr Met
           35                  40                  45
   gcc tgc cag ccg gaa tcc acg gac atg aca aaa tat ctg aaa gtg aaa      910
   Ala Cys Gln Pro Glu Ser Thr Asp Met Thr Lys Tyr Leu Lys Val Lys
       50                  55                  60
   ctc gat cct ccg gat att acc tgt gga gac cct cct gag acg ttc tgt      958
   Leu Asp Pro Pro Asp Ile Thr Cys Gly Asp Pro Pro Glu Thr Phe Cys
   65                  70                  75                  80
   gca atg ggc aat ccc tac atg tgc aat aat gag tgt gat gcg agt acc     1006
   Ala Met Gly Asn Pro Tyr Met Cys Asn Asn Glu Cys Asp Ala Ser Thr
                   85                  90                  95
   cct gag ctg gca cac ccc cct gag ctg atg ttt gat ttt gaa gga aga     1054
   Pro Glu Leu Ala His Pro Pro Glu Leu Met Phe Asp Phe Glu Gly Arg
               100                 105                 110
   cat ccc tcc aca ttt tgg cag tct gcc act tgg aag gag tat ccc aag     1102
   His Pro Ser Thr Phe Trp Gln Ser Ala Thr Trp Lys Glu Tyr Pro Lys
           115                 120                 125
   cct ctc cag gtt aac atc act ctg tct tgg agc aaa acc att gag cta     1150
   Pro Leu Gln Val Asn Ile Thr Leu Ser Trp Ser Lys Thr Ile Glu Leu
       130                 135                 140
   aca gac aac ata gtt att acc ttt gaa tct ggg cgt cca gac caa atg     1198
   Thr Asp Asn Ile Val Ile Thr Phe Glu Ser Gly Arg Pro Asp Gln Met
   145                 150                 155                 160
   atc ctg gag aag tct ctc gat tat gga cga aca tgg cag ccc tat cag     1246
   Ile Leu Glu Lys Ser Leu Asp Tyr Gly Arg Thr Trp Gln Pro Tyr Gln
                   165                 170                 175
   tat tat gcc aca gac tgc tta gat gct ttt cac atg gat cct aaa tcc     1294
   Tyr Tyr Ala Thr Asp Cys Leu Asp Ala Phe His Met Asp Pro Lys Ser
               180                 185                 190
   gtg aag gat tta tca cag cat acg gtc tta gaa atc att tgc aca gaa     1342
   Val Lys Asp Leu Ser Gln His Thr Val Leu Glu Ile Ile Cys Thr Glu
           195                 200                 205
   gag tac tca aca ggg tat aca aca aat agc aaa ata atc cac ttt gaa     1390
   Glu Tyr Ser Thr Gly Tyr Thr Thr Asn Ser Lys Ile Ile His Phe Glu
       210                 215                 220
   atc aaa gac agg ttc gcg ttt ttt gct gga cct cgc cta cgc aat atg     1438
   Ile Lys Asp Arg Phe Ala Phe Phe Ala Gly Pro Arg Leu Arg Asn Met
   225                 230                 235                 240
   gct tcc ctc tac gga cag ctg gat aca acc aag aaa ctc aga gat ttc     1486
   Ala Ser Leu Tyr Gly Gln Leu Asp Thr Thr Lys Lys Leu Arg Asp Phe
                   245                 250                 255
   ttt aca gtc aca gac ctg agg ata agg ctg tta aga cca gcc gtt ggg     1534
   Phe Thr Val Thr Asp Leu Arg Ile Arg Leu Leu Arg Pro Ala Val Gly
               260                 265                 270
   gaa ata ttt gta gat gag cta cac ttg gca cgc tac ttt tac gcg atc     1582
   Glu Ile Phe Val Asp Glu Leu His Leu Ala Arg Tyr Phe Tyr Ala Ile
           275                 280                 285
   tca gac ata aag gtg cga gga agg tgc aag tgt aat ctc cat gcc act      1630
   Ser Asp Ile Lys Val Arg Gly Arg Cys Lys Cys Asn Leu His Ala Thr
       290                 295                 300
   gta tgt gtg tat gac aac agc aaa ttg aca tgc gaa tgt gag cac aac      1678
   Val Cys Val Tyr Asp Asn Ser Lys Leu Thr Cys Glu Cys Glu His Asn
   305                 310                 315                 320
   act aca ggt cca gac tgt ggg aaa tgc aag aag aat tat cag ggc cga      1726
   Thr Thr Gly Pro Asp Cys Gly Lys Cys Lys Lys Asn Tyr Gln Gly Arg
                   325                 330                 335
   cct tgg agt cca ggc tcc tat ctc ccc atc ccc aaa ggc act gca aat      1774
   Pro Trp Ser Pro Gly Ser Tyr Leu Pro Ile Pro Lys Gly Thr Ala Asn
               340                 345                 350
   acc tgt atc ccc agt att tcc agt att ggt aat tgt gaa tgc ttc ggc      1822
   Thr Cys Ile Pro Ser Ile Ser Ser Ile Gly Asn Cys Glu Cys Phe Gly
           355                 360                 365
   cac tcc aat cga tgc agt tat atc gat ctg cta aat aca gtc att tgc      1870
   His Ser Asn Arg Cys Ser Tyr Ile Asp Leu Leu Asn Thr Val Ile Cys
       370                 375                 380
   gtg agc tgt aaa cac aac act aga ggg cag cac tgt gag tta tgc agg      1918
   Val Ser Cys Lys His Asn Thr Arg Gly Gln His Cys Glu Leu Cys Arg
   385                 390                 395                 400
   ctg ggc tac ttc aga aat gct tct gca caa ctg gac gat gag aat gtg      1966
   Leu Gly Tyr Phe Arg Asn Ala Ser Ala Gln Leu Asp Asp Glu Asn Val
                   405                 410                 415
   tgc ata gag tgt tat tgt aac cct ttg ggc tca atc cat gat cgt tgt      2014
   Cys Ile Glu Cys Tyr Cys Asn Pro Leu Gly Ser Ile His Asp Arg Cys
               420                 425                 430
   aat ggc tca gga ttt tgt gag tgt aag act gga aca aca ggg cct aag      2062
   Asn Gly Ser Gly Phe Cys Glu Cys Lys Thr Gly Thr Thr Gly Pro Lys
           435                 440                 445
   tgt gat gag tgt ctg ccg gga aat tcc tgg cac tac ggc tgt caa ccg      2110
   Cys Asp Glu Cys Leu Pro Gly Asn Ser Trp His Tyr Gly Cys Gln Pro
       450                 455                 460
   aat gtc tgc gac aac gag ctc ctg cac tgc cag aac gga ggg acg tgc      2158
   Asn Val Cys Asp Asn Glu Leu Leu His Cys Gln Asn Gly Gly Thr Cys
   465                 470                 475                 480
   cac aac aac gtg cgc tgc ctg tgc ccg gcc gca tac acg ggc atc ctc      2206
   His Asn Asn Val Arg Cys Leu Cys Pro Ala Ala Tyr Thr Gly Ile Leu
                   485                 490                 495
   tgc gag aag ctg cgg tgc gag gag gct ggc agc tgc ggc tcc gac tct      2254
   Cys Glu Lys Leu Arg Cys Glu Glu Ala Gly Ser Cys Gly Ser Asp Ser
               500                 505                 510
   ggc cag ggc gcg ccc ccg cac ggc tcc cca gcg ctg ctg ctg ctg acc      2302
   Gly Gln Gly Ala Pro Pro His Gly Ser Pro Ala Leu Leu Leu Leu Thr
           515                 520                 525
   acg ctg ctg gga acc gcc agc ccc ctg gtg ttc tag gtgtcacctc           2348
   Thr Leu Leu Gly Thr Ala Ser Pro Leu Val Phe
       530                 535
   cagccacacc ggacgggcct gtgccgtggg gaagcagaca caacccaaac atttgctact   2408
   aacataggaa acacacacat acagacaccc ccactcagac agtgtacaaa ctaagaaggc   2468
   ctaactgaac taagccatat ttatcacccg tggacagcac atccgagtca agactgttaa   2528
   tttctgactc cagaggagtt ggcagctgtt gatattatca ctgcaaatca cattgccagc   2588
   tgcagagcat attgtggatt ggaaaggctg cgacagcccc ccaaacagga aagacaaaaa   2648
   acaaacaaat caaccgacct aaaaacattg gctactctag cgtggtgcgc cctagtacga   2708
   ctccgcccag tgtgtggacc aaccaaatag cattctttgc tgtcaggtgc attgtgggca   2768
   taaggaaatc tgttacaagc tgccatattg gcctgcttcc gtccctgaat cccttccaac   2828
   ctgtgcttta gtgaacgttg ctctgtaacc cttgttggtt gaaagatttc tttgtctgat   2888
   gttagtgatg cacatgtgta acagccccct ctaaaagcgc aagccagtca tacccctgta   2948
   tatcttagca gcactgagtc cagtgcgagc acacacccac tatacaagag tggctatagg   3008
   aaaaaagaaa gtgtatctat ccttttgtat tcaaatgaag ttatttttct tgaactactg   3068
   taatatgtag attttttgta ttattgccaa tttgtgttac cagacaatct gttaatgtat   3128
   ctaattcgaa tcagcaaaga ctgacatttt attttgtcct ctttcgttct gttttgtttc   3188
   actgtgcaga gatttctctg taagggcaac gaacgtgctg gcatcaaaga atatcagttt   3248
   acatatataa caagtgtaat aagattccac caaaggacat tctaaatgtt ttcttgttgc   3308
   tttaacactg gaagatttaa agaataaaaa ctcctgcata aacaaaaaaa aaaaaaaaaa   3368
   aaaaaaaaaa aaaaaaaaaa aaaaaaaaaa aaaaaaaaaa aaaaaaaaaa aaaaaaaaaa   3428
   aaaaaa                                                              3434
<210> 2
   <211> 539
   <212> PRT
   <213> Homo sapiens
<400> 2
   Met Tyr Leu Ser Arg Phe Leu Ser Ile His Ala Leu Trp Val Thr Val
   1               5                   10                  15
   Ser Ser Val Met Gln Pro Tyr Pro Leu Val Trp Gly His Tyr Asp Leu
               20                  25                  30
   Cys Lys Thr Gln Ile Tyr Thr Glu Glu Gly Lys Val Trp Asp Tyr Met
           35                  40                  45
   Ala Cys Gln Pro Glu Ser Thr Asp Met Thr Lys Tyr Leu Lys Val Lys
       50                  55                  60
   Leu Asp Pro Pro Asp Ile Thr Cys Gly Asp Pro Pro Glu Thr Phe Cys
   65                  70                  75                  80
   Ala Met Gly Asn Pro Tyr Met Cys Asn Asn Glu Cys Asp Ala Ser Thr
                   85                  90                  95
   Pro Glu Leu Ala His Pro Pro Glu Leu Met Phe Asp Phe Glu Gly Arg
               100                 105                 110
   His Pro Ser Thr Phe Trp Gln Ser Ala Thr Trp Lys Glu Tyr Pro Lys
           115                 120                 125
   Pro Leu Gln Val Asn Ile Thr Leu Ser Trp Ser Lys Thr Ile Glu Leu
       130                 135                 140
   Thr Asp Asn Ile Val Ile Thr Phe Glu Ser Gly Arg Pro Asp Gln Met
   145                 150                 155                 160
   Ile Leu Glu Lys Ser Leu Asp Tyr Gly Arg Thr Trp Gln Pro Tyr Gln
                   165                 170                 175
   Tyr Tyr Ala Thr Asp Cys Leu Asp Ala Phe His Met Asp Pro Lys Ser
               180                 185                 190
   Val Lys Asp Leu Ser Gln His Thr Val Leu Glu Ile Ile Cys Thr Glu
           195                 200                 205
   Glu Tyr Ser Thr Gly Tyr Thr Thr Asn Ser Lys Ile Ile His Phe Glu
       210                 215                 220
   Ile Lys Asp Arg Phe Ala Phe Phe Ala Gly Pro Arg Leu Arg Asn Met
   225                 230                 235                 240
   Ala Ser Leu Tyr Gly Gln Leu Asp Thr Thr Lys Lys Leu Arg Asp Phe
                   245                 250                 255
   Phe Thr Val Thr Asp Leu Arg Ile Arg Leu Leu Arg Pro Ala Val Gly
               260                 265                 270
   Glu Ile Phe Val Asp Glu Leu His Leu Ala Arg Tyr Phe Tyr Ala Ile
           275                 280                 285
   Ser Asp Ile Lys Val Arg Gly Arg Cys Lys Cys Asn Leu His Ala Thr
       290                 295                 300
   Val Cys Val Tyr Asp Asn Ser Lys Leu Thr Cys Glu Cys Glu His Asn
   305                 310                 315                 320
   Thr Thr Gly Pro Asp Cys Gly Lys Cys Lys Lys Asn Tyr Gln Gly Arg
                   325                 330                 335
   Pro Trp Ser Pro Gly Ser Tyr Leu Pro Ile Pro Lys Gly Thr Ala Asn
               340                 345                 350
   Thr Cys Ile Pro Ser Ile Ser Ser Ile Gly Asn Cys Glu Cys Phe Gly
           355                 360                 365
   His Ser Asn Arg Cys Ser Tyr Ile Asp Leu Leu Asn Thr Val Ile Cys
       370                 375                 380
   Val Ser Cys Lys His Asn Thr Arg Gly Gln His Cys Glu Leu Cys Arg
   385                 390                 395                 400
   Leu Gly Tyr Phe Arg Asn Ala Ser Ala Gln Leu Asp Asp Glu Asn Val
                   405                 410                 415
   Cys Ile Glu Cys Tyr Cys Asn Pro Leu Gly Ser Ile His Asp Arg Cys
               420                 425                 430
   Asn Gly Ser Gly Phe Cys Glu Cys Lys Thr Gly Thr Thr Gly Pro Lys
           435                 440                 445
   Cys Asp Glu Cys Leu Pro Gly Asn Ser Trp His Tyr Gly Cys Gln Pro
       450                 455                 460
   Asn Val Cys Asp Asn Glu Leu Leu His Cys Gln Asn Gly Gly Thr Cys
   465                 470                 475                 480
   His Asn Asn Val Arg Cys Leu Cys Pro Ala Ala Tyr Thr Gly Ile Leu
                   485                 490                 495
   Cys Glu Lys Leu Arg Cys Glu Glu Ala Gly Ser Cys Gly Ser Asp Ser
               500                 505                 510
   Gly Gln Gly Ala Pro Pro His Gly Ser Pro Ala Leu Leu Leu Leu Thr
           515                 520                 525
   Thr Leu Leu Gly Thr Ala Ser Pro Leu Val Phe
       530                 535
<210> 3
   <211> 3219
   <212> DNA
   <213> Homo sapiens
<220>
   <221> CDS
   <222> (119)..(2458)
   <223>
<400> 3
   agactctcag gttgatgcag tgttccctcc cacaactctg acatgtatat aaattctgag      60
   ctctccaaag cccactgcca gttctcttcg gggactaact gcaacggaga gactcaag       118
   atg att ccc ttt tta ccc atg ttt tct cta cta ttg ctg ctt att gtt       166
   Met Ile Pro Phe Leu Pro Met Phe Ser Leu Leu Leu Leu Leu Ile Val
   1               5                   10                  15
   aac cct ata aac gcc aac aat cat tat gac aag atc ttg gct cat agt       214
   Asn Pro Ile Asn Ala Asn Asn His Tyr Asp Lys Ile Leu Ala His Ser
               20                  25                  30
   cgt atc agg ggt cgg gac caa ggc cca aat gtc tgt gcc ctt caa cag       262
   Arg Ile Arg Gly Arg Asp Gln Gly Pro Asn Val Cys Ala Leu Gln Gln
           35                  40                  45
   att ttg ggc acc aaa aag aaa tac ttc agc act tgt aag aac tgg tat       310
   Ile Leu Gly Thr Lys Lys Lys Tyr Phe Ser Thr Cys Lys Asn Trp Tyr
       50                  55                  60
   aaa aag tcc atc tgt gga cag aaa acg act gtg tta tat gaa tgt tgc       358
   Lys Lys Ser Ile Cys Gly Gln Lys Thr Thr Val Leu Tyr Glu Cys Cys
   65                  70                  75                  80
   cct ggt tat atg aga atg gaa gga atg aaa ggc tgc cca gca gtt ttg       406
   Pro Gly Tyr Met Arg Met Glu Gly Met Lys Gly Cys Pro Ala Val Leu
                   85                  90                  95
   ccc att gac cat gtt tat ggc act ctg ggc atc gtg gga gcc acc aca       454
   Pro Ile Asp His Val Tyr Gly Thr Leu Gly Ile Val Gly Ala Thr Thr
               100                 105                 110
   acg cag cgc tat tct gac gcc tca aaa ctg agg gag gag atc gag gga       502
   Thr Gln Arg Tyr Ser Asp Ala Ser Lys Leu Arg Glu Glu Ile Glu Gly
           115                 120                 125
   aag gga tcc ttc act tac ttt gca ccg agt aat gag gct tgg gac aac       550
   Lys Gly Ser Phe Thr Tyr Phe Ala Pro Ser Asn Glu Ala Trp Asp Asn
       130                 135                 140
   ttg gat tct gat atc cgt aga ggt ttg gag agc aac gtg aat gtt gaa       598
   Leu Asp Ser Asp Ile Arg Arg Gly Leu Glu Ser Asn Val Asn Val Glu
   145                 150                 155                 160
   tta ctg aat gct tta cat agt cac atg att aat aag aga atg ttg acc       646
   Leu Leu Asn Ala Leu His Ser His Met Ile Asn Lys Arg Met Leu Thr
                   165                 170                 175
   aag gac tta aaa aat ggc atg att att cct tca atg tat aac aat ttg       694
   Lys Asp Leu Lys Asn Gly Met Ile Ile Pro Ser Met Tyr Asn Asn Leu
               180                 185                 190
   ggg ctt ttc att aac cat tat cct aat ggg gtt gtc act gtt aat tgt       742
   Gly Leu Phe Ile Asn His Tyr Pro Asn Gly Val Val Thr Val Asn Cys
           195                 200                 205
   gct cga atc atc cat ggg aac cag att gca aca aat ggt gtt gtc cat       790
   Ala Arg Ile Ile His Gly Asn Gln Ile Ala Thr Asn Gly Val Val His
       210                 215                 220
   gtc att gac cgt gtg ctt aca caa att ggt acc tca att caa gac ttc        838
   Val Ile Asp Arg Val Leu Thr Gln Ile Gly Thr Ser Ile Gln Asp Phe
   225                 230                 235                 240
   att gaa gca gaa gat gac ctt tca tct ttt aga gca gct gcc atc aca        886
   Ile Glu Ala Glu Asp Asp Leu Ser Ser Phe Arg Ala Ala Ala Ile Thr
                   245                 250                 255
   tcg gac ata ttg gag gcc ctt gga aga gac ggt cac ttc aca ctc ttt        934
   Ser Asp Ile Leu Glu Ala Leu Gly Arg Asp Gly His Phe Thr Leu Phe
               260                 265                 270
   gct ccc acc aat gag gct ttt gag aaa ctt cca cga ggt gtc cta gaa        982
   Ala Pro Thr Asn Glu Ala Phe Glu Lys Leu Pro Arg Gly Val Leu Glu
           275                 280                 285
   agg atc atg gga gac aaa gtg gct tcc gaa gct ctt atg aag tac cac       1030
   Arg Ile Met Gly Asp Lys Val Ala Ser Glu Ala Leu Met Lys Tyr His
       290                 295                 300
   atc tta aat act ctc cag tgt tct gag tct att atg gga gga gca gtc       1078
   Ile Leu Asn Thr Leu Gln Cys Ser Glu Ser Ile Met Gly Gly Ala Val
   305                 310                 315                 320
   ttt gag acg ctg gaa gga aat aca att gag ata gga tgt gac ggt gac       1126
   Phe Glu Thr Leu Glu Gly Asn Thr Ile Glu Ile Gly Cys Asp Gly Asp
                   325                 330                 335
   agt ata aca gta aat gga atc aaa atg gtg aac aaa aag gat att gtg       1174
   Ser Ile Thr Val Asn Gly Ile Lys Met Val Asn Lys Lys Asp Ile Val
               340                 345                 350
   aca aat aat ggt gtg atc cat ttg att gat cag gtc cta att cct gat       1222
   Thr Asn Asn Gly Val Ile His Leu Ile Asp Gln Val Leu Ile Pro Asp
           355                 360                 365
   tct gcc aaa caa gtt att gag ctg gct gga aaa cag caa acc acc ttc       1270
   Ser Ala Lys Gln Val Ile Glu Leu Ala Gly Lys Gln Gln Thr Thr Phe
       370                 375                 380
   acg gat ctt gtg gcc caa tta ggc ttg gca tct gct ctg agg cca gat       1318
   Thr Asp Leu Val Ala Gln Leu Gly Leu Ala Ser Ala Leu Arg Pro Asp
   385                 390                 395                 400
   gga gaa tac act ttg ctg gca cct gtg aat aat gca ttt tct gat gat       1366
   Gly Glu Tyr Thr Leu Leu Ala Pro Val Asn Asn Ala Phe Ser Asp Asp
                   405                 410                 415
   act ctc agc atg gat cag cgc ctc ctt aaa tta att ctg cag aat cac       1414
   Thr Leu Ser Met Asp Gln Arg Leu Leu Lys Leu Ile Leu Gln Asn His
               420                 425                 430
   ata ttg aaa gta aaa gtt ggc ctt aat gag ctt tac aac ggg caa ata       1462
   Ile Leu Lys Val Lys Val Gly Leu Asn Glu Leu Tyr Asn Gly Gln Ile
           435                 440                 445
   ctg gaa acc atc gga ggc aaa cag ctc aga gtc ttc gta tat cgt aca       1510
   Leu Glu Thr Ile Gly Gly Lys Gln Leu Arg Val Phe Val Tyr Arg Thr
       450                 455                 460
   gct gtc tgc att gaa aat tca tgc atg gag aaa ggg agt aag caa ggg       1558
   Ala Val Cys Ile Glu Asn Ser Cys Met Glu Lys Gly Ser Lys Gln Gly
   465                 470                 475                 480
   aga aac ggt gcg att cac ata ttc cgc gag atc atc aag cca gca gag      1606
   Arg Asn Gly Ala Ile His Ile Phe Arg Glu Ile Ile Lys Pro Ala Glu
                   485                 490                 495
   aaa tcc ctc cat gaa aag tta aaa caa gat aag cgc ttt agc acc ttc      1654
   Lys Ser Leu His Glu Lys Leu Lys Gln Asp Lys Arg Phe Ser Thr Phe
               500                 505                 510
   ctc agc cta ctt gaa gct gca gac ttg aaa gag ctc ctg aca caa cct      1702
   Leu Ser Leu Leu Glu Ala Ala Asp Leu Lys Glu Leu Leu Thr Gln Pro
           515                 520                 525
   gga gac tgg aca tta ttt gtg cca acc aat gat gct ttt aag gga atg      1750
   Gly Asp Trp Thr Leu Phe Val Pro Thr Asn Asp Ala Phe Lys Gly Met
       530                 535                 540
   act agt gaa gaa aaa gaa att ctg ata cgg gac aaa aat gct ctt caa      1798
   Thr Ser Glu Glu Lys Glu Ile Leu Ile Arg Asp Lys Asn Ala Leu Gln
   545                 550                 555                 560
   aac atc att ctt tat cac ctg aca cca gga gtt ttc att gga aaa gga      1846
   Asn Ile Ile Leu Tyr His Leu Thr Pro Gly Val Phe Ile Gly Lys Gly
                   565                 570                 575
   ttt gaa cct ggt gtt act aac att tta aag acc aca caa gga agc aaa      1894
   Phe Glu Pro Gly Val Thr Asn Ile Leu Lys Thr Thr Gln Gly Ser Lys
               580                 585                 590
   atc ttt ctg aaa gaa gta aat gat aca ctt ctg gtg aat gaa ttg aaa      1942
   Ile Phe Leu Lys Glu Val Asn Asp Thr Leu Leu Val Asn Glu Leu Lys
           595                 600                 605
   tca aaa gaa tct gac atc atg aca aca aat ggt gta att cat gtt gta      1990
   Ser Lys Glu Ser Asp Ile Met Thr Thr Asn Gly Val Ile His Val Val
       610                 615                 620
   gat aaa ctc ctc tat cca gca gac aca cct gtt gga aat gat caa ctg      2038
   Asp Lys Leu Leu Tyr Pro Ala Asp Thr Pro Val Gly Asn Asp Gln Leu
   625                 630                 635                 640
   ctg gaa ata ctt aat aaa tta atc aaa tac atc caa att aag ttt gtt      2086
   Leu Glu Ile Leu Asn Lys Leu Ile Lys Tyr Ile Gln Ile Lys Phe Val
                   645                 650                 655
   cgt ggt agc acc ttc aaa gaa atc ccc gtg act gtc tat aag cca att      2134
   Arg Gly Ser Thr Phe Lys Glu Ile Pro Val Thr Val Tyr Lys Pro Ile
               660                 665                 670
   att aaa aaa tac acc aaa atc att gat gga gtg cct gtg gaa ata act      2182
   Ile Lys Lys Tyr Thr Lys Ile Ile Asp Gly Val Pro Val Glu Ile Thr
           675                 680                 685
   gaa aaa gag aca cga gaa gaa cga atc att aca ggt cct gaa ata aaa      2230
   Glu Lys Glu Thr Arg Glu Glu Arg Ile Ile Thr Gly Pro Glu Ile Lys
       690                 695                 700
   tac act agg att tct act gga ggt gga gaa aca gaa gaa act ctg aag      2278
   Tyr Thr Arg Ile Ser Thr Gly Gly Gly Glu Thr Glu Glu Thr Leu Lys
   705                 710                 715                 720
   aaa ttg tta caa gaa gag gtc acc aag gtc acc aaa ttc att gaa ggt      2326
   Lys Leu Leu Gln Glu Glu Val Thr Lys Val Thr Lys Phe Ile Glu Gly
                   725                 730                 735
   ggt gat ggt cat tta ttt gaa gat gaa gaa att aaa aga ctg ctt cag      2374
   Gly Asp Gly His Leu Phe Glu Asp Glu Glu Ile Lys Arg Leu Leu Gln
               740                 745                 750
   gga gac aca ccc gtg agg aag ttg caa gcc aac aaa aaa gtt caa gga      2422
   Gly Asp Thr Pro Val Arg Lys Leu Gln Ala Asn Lys Lys Val Gln Gly
           755                 760                 765
   tct aga aga cga tta agg gaa ggt cgt tct cag tga aaatccaaaa           2468
   Ser Arg Arg Arg Leu Arg Glu Gly Arg Ser Gln
       770                 775
   accagaaaaa aatgtttata caaccctaag tcaataacct gaccttagaa aattgtgaga   2528
   gccaagttga cttcaggaac tgaaacatca gcacaaagaa gcaatcatca aataattctg   2588
   aacacaaatt taatattttt ttttctgaat gagaaacatg agggaaattg tggagttagc   2648
   ctcctgtggt aaaggaattg aagaaaatat aacaccttac accctttttc atcttgacat    2708
   taaaagttct ggctaacttt ggaatccatt agagaaaaat ccttgtcacc agattcatta   2768
   caattcaaat cgaagagttg tgaactgtta tcccattgaa aagaccgagc cttgtatgta   2828
   tgttatggat acataaaatg cacgcaagcc attatctctc catgggaagc taagttataa   2888
   aaataggtgc ttggtgtaca aaacttttta tatcaaaagg ctttgcacat ttctatatga   2948
   gtgggtttac tggtaaatta tgttattttt tacaactaat tttgtactct cagaatgttt   3008
   gtcatatgct tcttgcaatg catatttttt aatctcaaac gtttcaataa aaccattttt   3068
   cagatataaa gagaattact tcaaattgag taattcagaa aaactcaaga tttaagttaa   3128
   aaagtggttt ggacttggga acaggacttt atacctcttt tactgtaaca agtactcatt   3188
   aaaggaaatt gaatgaaatt aaaaaaaaaa a                                   3219
<210> 4
   <211> 779
   <212> PRT
   <213> Homo sapiens
<400> 4
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Ile Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Asp Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
           515                 520                 525
   Gly Asp Trp Thr Leu Phe Val Pro Thr Asn Asp Ala Phe Lys Gly Met
       530                 535                 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
               580                 585                 590
   Ile Phe Leu Lys Glu Val Asn Asp Thr Leu Leu Val Asn Glu Leu Lys
           595                 600                 605
   Ser Lys Glu Ser Asp Ile Met Thr Thr Asn Gly Val Ile His Val Val
       610                 615                 620
   Asp Lys Leu Leu Tyr Pro Ala Asp Thr Pro Val Gly Asn Asp Gln Leu
   625                 630                 635                 640
   Leu Glu Ile Leu Asn Lys Leu Ile Lys Tyr Ile Gln Ile Lys Phe Val
                   645                 650                 655
   Arg Gly Ser Thr Phe Lys Glu Ile Pro Val Thr Val Tyr Lys Pro Ile
               660                 665                 670
   Ile Lys Lys Tyr Thr Lys Ile Ile Asp Gly Val Pro Val Glu Ile Thr
           675                 680                 685
   Glu Lys Glu Thr Arg Glu Glu Arg Ile Ile Thr Gly Pro Glu Ile Lys
       690                 695                 700
   Tyr Thr Arg Ile Ser Thr Gly Gly Gly Glu Thr Glu Glu Thr Leu Lys
   705                 710                 715                 720
   Lys Leu Leu Gln Glu Glu Val Thr Lys Val Thr Lys Phe Ile Glu Gly
                   725                 730                 735
   Gly Asp Gly His Leu Phe Glu Asp Glu Glu Ile Lys Arg Leu Leu Gln
               740                 745                 750
   Gly Asp Thr Pro Val Arg Lys Leu Gln Ala Asn Lys Lys Val Gln Gly
           755                 760                 765
   Ser Arg Arg Arg Leu Arg Glu Gly Arg Ser Gln
       770                 775
<210> 5
   <211> 7616
   <212> DNA
   <213> Homo sapiens
<220>
   <221> CDS
   <222> (363)..(6968)
   <223>
<400> 5
attacagagg aaggagctcg ctatataagc cagccaaagt tggctgcacc ggccacagcc      60
   tgcctactgt cacccgcctc tcccgcgcgc agatacacgc ccccgcctcc gtgggcacaa      120
   aggcagcgct gctggggaac tcgggggaac gcgcacgtgg gaaccgccgc agctccacac     180
   tccaggtact tcttccaagg acctaggtct ctcgcccatc ggaaagaaaa taattctttc     240
   aagaagatca gggacaactg atttgaagtc tactctgtgc ttctaaatcc ccaattctgc     300
   tgaaagtgag ataccctaga gccctagagc cccagcagca cccagccaaa cccacctcca     360
   cc atg ggg gcc atg act cag ctg ttg gca ggt gtc ttt ctt gct ttc        407
   ctt gcc ctc gct acc gaa ggt ggg gtc ctc aag aaa gtc atc cgg cac       455
   aag cga cag agt ggg gtg aac gcc acc ctg cca gaa gag aac cag cca       503
   gtg gtg ttt aac cac gtt tac aac atc aag ctg cca gtg gga tcc cag       551
   tgt tcg gtg gat ctg gag tca gcc agt ggg gag aaa gac ctg gca ccg       599
   cct tca gag ccc agc gaa agc ttt cag gag cac aca gtg gat ggg gaa       647
   aac cag att gtc ttc aca cat cgc atc aac atc ccc cgc cgg gcc tgt       695
   ggc tgt gcc gca gcc cct gat gtt aag gag ctg ctg agc aga ctg gag       743
   gag ctg gag aac ctg gtg tct tcc ctg agg gag caa tgt act gca gga       791
   gca ggc tgc tgt ctc cag cct gcc aca ggc cgc ttg gac acc agg ccc       839
   ttc tgt agc ggt cgg ggc aac ttc agc act gaa gga tgt ggc tgt gtc       887
   tgc gaa cct ggc tgg aaa ggc ccc aac tgc tct gag ccc gaa tgt cca       935
   ggc aac tgt cac ctt cga ggc cgg tgc att gat ggg cag tgc atc tgt      983
   gac gac ggc ttc acg ggc gag gac tgc agc cag ctg gct tgc ccc agc     1031
   gac tgc aat gac cag ggc aag tgc gta aat gga gtc tgc atc tgt ttc     1079
   gaa ggc tac gcc ggg gct gac tgc agc cgt gaa atc tgc cca gtg ccc     1127
   tgc agt gag gag cac ggc aca tgt gta gat ggc ttg tgt gtg tgc cac     1175
   gat ggc ttt gca ggc gat gac tgc aac aag cct ctg tgt ctc aac aat     1223
   tgc tac aac cgt gga cga tgc gtg gag aat gag tgc gtg tgt gat gag     1271
   ggt ttc acg ggc gaa gac tgc agt gag ctc atc tgc ccc aat gac tgc     1319
   ttc gac cgg ggc cgc tgc atc aat ggc acc tgc tac tgc gaa gaa ggc     1367
   ttc aca ggt gaa gac tgc ggg aaa ccc acc tgc cca cat gcc tgc cac     1415
   acc cag ggc cgg tgt gag gag ggg cag tgt gta tgt gat gag ggc ttt     1463
   gcc ggt gtg gac tgc agc gag aag agg tgt cct gct gac tgt cac aat     1511
   cgt ggc cgc tgt gta gac ggg cgg tgt gag tgt gat gat ggt ttc act     1559
   gga gct gac tgt ggg gag ctc aag tgt ccc aat ggc tgc agt ggc cat     1607
   ggc cgc tgt gtc aat ggg cag tgt gtg tgt gat gag ggc tat act ggg     1655
   gag gac tgc agc cag cta cgg tgc ccc aat gac tgt cac agt cgg ggc     1703
   cgc tgt gtc gag ggc aaa tgt gta tgt gag caa ggc ttc aag ggc tat      1751
   gac tgc agt gac atg agc tgc cct aat gac tgt cac cag cac ggc cgc      1799
   tgt gtg aat ggc atg tgt gtt tgt gat gac ggc tac aca ggg gaa gac      1847
   tgc cgg gat cgc caa tgc ccc agg gac tgc agc aac agg ggc ctc tgt      1895
   gtg gac gga cag tgc gtc tgt gag gac ggc ttc acc ggc cct gac tgt      1943
   gca gaa ctc tcc tgt cca aat gac tgc cat ggc cag ggt cgc tgt gtg      1991
   aat ggg cag tgc gtg tgc cat gaa gga ttt atg ggc aaa gac tgc aag      2039
   gag caa aga tgt ccc agt gac tgt cat ggc cag ggc cgc tgc gtg gac      2087
   ggc cag tgc atc tgc cac gag ggc ttc aca ggc ctg gac tgt ggc cag      2135
   cac tcc tgc ccc agt gac tgc aac aac tta gga caa tgc gtc tcg ggc      2183
   cgc tgc atc tgc aac gag ggc tac agc gga gaa gac tgc tca gag gtg      2231
   tct cct ccc aaa gac ctc gtt gtg aca gaa gtg acg gaa gag acg gtc      2279
   aac ctg gcc tgg gac aat gag atg cgg gtc aca gag tac ctt gtc gtg      2327
   tac acg ccc acc cac gag ggt ggt ctg gaa atg cag ttc cgt gtg cct      2375
   ggg gac cag acg tcc acc atc atc cag gag ctg gag cct ggt gtg gag      2423
   tac ttt atc cgt gta ttt gcc atc ctg gag aac aag aag agc att cct      2471
   gtc agc gcc agg gtg gcc acg tac tta cct gca cct gaa ggc ctg aaa      2519
   ttc aag tcc atc aag gag aca tct gtg gaa gtg gag tgg gat cct cta      2567
   gac att gct ttt gaa acc tgg gag atc atc ttc cgg aat atg aat aaa      2615
   gaa gat gag gga gag atc acc aaa agc ctg agg agg cca gag acc tct      2663
   tac cgg caa act ggt cta gct cct ggg caa gag tat gag ata tct ctg      2711
   cac ata gtg aaa aac aat acc cgg ggc cct ggc ctg aag agg gtg acc      2759
   acc aca cgc ttg gat gcc ccc agc cag atc gag gtg aaa gat gtc aca      2807
   gac acc act gcc ttg atc acc tgg ttc aag ccc ctg gct gag atc gat      2855
   ggc att gag ctg acc tac ggc atc aaa gac gtg cca gga gac cgt acc      2903
   acc atc gat ctc aca gag gac gag aac cag tac tcc atc ggg aac ctg      2951
   aag cct gac act gag tac gag gtg tcc ctc atc tcc cgc aga ggt gac      2999
   atg tca agc aac cca gcc aaa gag acc ttc aca aca ggc ctc gat gct      3047
   ccc agg aat ctt cga cgt gtt tcc cag aca gat aac agc atc acc ctg      3095
   gaa tgg agg aat ggc aag gca gct att gac agt tac aga att aag tat      3143
   gcc ccc atc tct gga ggg gac cac gct gag gtt gat gtt cca aag agc      3191
   caa caa gcc aca acc aaa acc aca ctc aca ggt ctg agg ccg gga act      3239
   gaa tat ggg att gga gtt tct gct gtg aag gaa gac aag gag agc aat      3287
   cca gcg acc atc aac gca gcc aca gag ttg gac acg ccc aag gac ctt      3335
   cag gtt tct gaa act gca gag acc agc  ctg acc ctg ctc tgg  aag aca    3383
   ccg ttg gcc  aaa ttt gac cgc tac  cgc ctc aat tac agt  ctc ccc       3428
   aca ggc cag  tgg gtg gga gtg cag  ctt cca aga aac acc  act tcc       3473
   tat gtc ctg  aga ggc ctg gaa cca  gga cag gag tac aat  gtc ctc       3518
   ctg aca gcc  gag aaa ggc aga cac  aag agc aag ccc gca  cgt gtg       3563
   aag gca tcc  act gaa caa gcc cct  gag ctg gaa aac ctc  acc gtg       3608
   act gag gtt  ggc tgg gat ggc ctc  aga ctc aac tgg acc  gca gct       3653
   gac cag gcc  tat gag cac ttt atc  att cag gtg cag gag  gcc aac       3698
   aag gtg gag  gca gct cgg aac ctc  acc gtg cct ggc agc  ctt cgg       3743
   gct gtg gac  ata ccg ggc ctc aag  gct gct acg cct tat  aca gtc       3788
   tcc atc tat  ggg gtg atc cag ggc  tat aga aca cca gtg  ctc tct       3833
   gct gag gcc  tcc aca ggg gaa act  ccc aat ttg gga gag  gtc gtg       3878
   gtg gcc gag  gtg ggc tgg gat gcc  ctc aaa ctc aac tgg  act gct       3923
   cca gaa ggg  gcc tat gag tac ttt  ttc att cag gtg cag  gag gct      3968
   gac aca gta  gag gca gcc cag aac  ctc acc gtc cca gga  gga ctg      4013
   agg tcc aca  gac ctg cct ggg ctc  aaa gca gcc act cat  tat acc      4058
   atc acc atc  cgc ggg gtc act cag  gac ttc agc aca acc  cct ctc      4103
   tct gtt gaa  gtc ttg aca gag gag  gtt cca gat atg gga  aac ctc      4148
   aca gtg acc  gag gtt agc tgg gat  gct ctc aga ctg aac  tgg acc      4193
   acg cca gat  gga acc tat gac cag  ttt act att cag gtc  cag gag      4238
   gct gac cag  gtg gaa gag gct cac  aat ctc acg gtt cct  ggc agc      4283
   ctg cgt tcc  atg gaa atc cca ggc  ctc agg gct ggc act  cct tac      4328
   aca gtc acc  ctg cac ggc gag gtc  agg ggc cac agc act  cga ccc      4373
   ctt gct gta  gag gtc gtc aca gag  gat ctc cca cag ctg  gga gat      4418
   tta gcc gtg  tct gag gtt ggc tgg  gat ggc ctc aga ctc  aac tgg      4463
   acc gca gct  gac aat gcc tat gag  cac ttt gtc att cag  gtg cag      4508
   gag gtc aac  aaa gtg gag gca gcc  cag aac ctc acg ttg  cct ggc      4553
   agc ctc agg  gct gtg gac atc ccg  ggc ctc gag gct gcc  acg cct      4598
   tat aga gtc  tcc atc tat ggg gtg  atc cgg ggc tat aga  aca cca      4643
   gta ctc tct  gct gag gcc tcc aca  gcc aaa gaa cct gaa  att gga       4688
   aac tta aat  gtt tct gac ata act  ccc gag agc ttc aat  ctc tcc       4733
   tgg atg gct  acc gat ggg atc ttc  gag acc ttt acc att  gaa att       4778
   att gat tcc  aat agg ttg ctg gag  act gtg gaa tat aat  atc tct       4823
   ggt gct gaa  cga act gcc cat atc  tca ggg cta ccc cct  agt act       4868
   gat ttt att  gtc tac ctc tct gga  ctt gct ccc agc atc  cgg acc       4913
   aaa acc atc  agt gcc aca gcc acg  aca gag gcc ctg ccc  ctt ctg       4958
   gaa aac cta  acc att tcc gac att  aat ccc tac ggg ttc  aca gtt       5003
   tcc tgg atg  gca tcg gag aat gcc  ttt gac agc ttt cta  gta acg       5048
   gtg gtg gat  tct ggg aag ctg ctg  gac ccc cag gaa ttc  aca ctt       5093
   tca gga acc  cag agg aag ctg gag  ctt aga ggc ctc ata  act ggc       5138
   att ggc tat  gag gtt atg gtc tct  ggc ttc acc caa ggg  cat caa       5183
   acc aag ccc  ttg agg gct gag att  gtt aca gaa gcc gaa  ccg gaa       5228
   gtt gac aac  ctt ctg gtt tca gat  gcc acc cca gac ggt  ttc cgt       5273
   ctg tcc tgg  aca gct gat gaa ggg  gtc ttc gac aat ttt  gtt ctc       5318
   aaa atc aga  gat acc aaa aag cag  tct gag cca ctg gaa  ata acc      5363
   cta ctt gcc  ccc gaa cgt acc agg  gac ata aca ggt ctc  aga gag      5408
   gct act gaa  tac gaa att gaa ctc  tat gga ata agc aaa  gga agg      5453
   cga tcc cag  aca gtc agt gct ata  gca aca aca gcc atg  ggc tcc      5498
   cca aag gaa  gtc att ttc tca gac  atc act gaa aat tcg  gct act      5543
   gtc agc tgg  agg gca ccc aca gcc  caa gtg gag agc ttc  cgg att      5588
   acc tat gtg  ccc att aca gga ggt  aca ccc tcc atg gta  act gtg      5633
   gac gga acc  aag act cag acc agg  ctg gtg aaa ctc ata  cct ggc      5678
   gtg gag tac  ctt gtc agc atc atc  gcc atg aag ggc ttt  gag gaa      5723
   agt gaa cct  gtc tca ggg tca ttc  acc aca gct ctg gat  ggc cca      5768
   tct ggc ctg  gtg aca gcc aac atc  act gac tca gaa gcc  ttg gcc      5813
   agg tgg cag  cca gcc att gcc act  gtg gac agt tat gtc  atc tcc      5858
   tac aca ggc  gag aaa gtg cca gaa  att aca cgc acg gtg  tcc ggg      5903
   aac aca gtg  gag tat gct ctg acc  gac ctc gag cct gcc  acg gaa      5948
   tac aca ctg  aga atc ttt gca gag  aaa ggg ccc cag aag  agc tca      5993
   acc atc act  gcc aag ttc aca aca  gac ctc gat tct cca  aga gac      6038
   ttg act gct  act gag gtt cag tcg  gaa act gcc ctc ctt  acc tgg      6083
   cga ccc ccc  cgg gca tca gtc acc  ggt tac ctg ctg gtc  tat gaa      6128
   tca gtg gat  ggc aca gtc aag gaa  gtc att gtg ggt cca  gat acc      6173
   acc tcc tac  agc ctg gca gac ctg  agc cca tcc acc cac  tac aca      6218
   gcc aag atc  cag gca ctc aat ggg  ccc ctg agg agc aat  atg atc      6263
   cag acc atc  ttc acc aca att gga  ctc ctg tac ccc ttc  ccc aag      6308
   gac tgc tcc  caa gca atg ctg aat  gga gac acg acc tct  ggc ctc      6353
   tac acc att  tat ctg aat ggt gat  aag gct gag gcg ctg  gaa gtc      6398
   ttc tgt gac  atg acc tct gat ggg  ggt gga tgg att gtg  ttc ctg      6443
   aga cgc aaa  aac gga cgc gag aac  ttc tac caa aac tgg  aag gca      6488
   tat gct gct  gga ttt ggg gac cgc  aga gaa gaa ttc tgg  ctt ggg      6533
   ctg gac aac  ctg aac aaa atc aca  gcc cag ggg cag tac  gag ctc      6578
   cgg gtg gac  ctg cgg gac cat ggg  gag aca gcc ttt gct  gtc tat      6623
   gac aag ttc  agc gtg gga gat gcc  aag act cgc tac aag  ctg aag      6668
   gtg gag ggg  tac agt ggg aca gca  ggt gac tcc atg gcc  tac cac      6713
   aat ggc aga  tcc ttc tcc acc ttt  gac aag gac aca gat  tca gcc      6758
   atc acc aac  tgt gct ctg tcc tac  aaa ggg gct ttc tgg  tac agg      6803
   aac tgt cac  cgt gtc aac ctg atg  ggg aga tat ggg gac  aat aac      6848
   cac agt cag  ggc gtt aac tgg ttc  cac tgg aag ggc cac  gaa cac      6893
   tca atc cag  ttt gct gag atg aag  ctg aga cca agc aac  ttc aga      6938
   aat ctt gaa  ggc agg cgc aaa cgg  gca taa attccaggga ccactgggtg     6988
   agagaggaat aaggcccaga gcgaggaaag gattttacca aagcatcaat acaaccagcc   7048
   caaccatcgg tccacacctg ggcatttggt gagagtcaaa gctgaccatg gatccctggg   7108
   gccaacggca acagcatggg cctcacctcc tctgtgattt ctttctttgc accaaagaca   7168
   tcagtctcca acatgtttct gttttgttgt ttgattcagc aaaaatctcc cagtgacaac   7228
   atcgcaatag ttttttactt ctcttaggtg gctctgggaa tgggagaggg gtaggatgta  7288
   caggggtagt ttgttttaga accagccgta ttttacatga agctgtataa ttaattgtca   7348
   ttatttttgt tagcaaagat taaatgtgtc attggaagcc atcccttttt ttacatttca   7408
   tacaacagaa accagaaaag caatactgtt tccattttaa ggatatgatt aatattatta   7468
   atataataat gatgatgatg atgatgaaaa ctaaggattt ttcaagagat ctttctttcc   7528
   aaaacatttc tggacagtac ctgattgtat ttttttttta aataaaagca caagtacttt   7588
   tgagtttgtt aaaaaaaaaa aaaaaaaa                                      7616
<210> 6
   <211> 2201
   <212> PRT
   <213> Homo sapiens
<400> 6
<210> 7
   <211> 6314
   <212> DNA
   <213> Homo sapiens
<220>
   <221> CDS
   <222> (151)..(2193)
   <223>
<400> 7
gtcggcgagg agggtccggc cggagttgaa ggattgaact ttccggctca gtcgcggcgt      60
   ctgcctggtc ctcagcagtg cagccccggc gcggagcagg gagcctcggc ccgcgcccgg     120
   cgccctcgcc ctcgccctcg acccgcagcc atg gtg ccc ggg gtg ccc ggc gcg      174
   gtc ctg acc ctc tgc ctc tgg ctg gcg gcc tcc agc ggc tgc ctg gcg       222
   gcc ggc ccc ggc gcg gct gct gcg cgg cgg ctg gac gag tcg ctg tct       270
   gcc ggg agc gtc cag cgc gct cgc tgc gcc tcc agg tgc ctg agc ctg       318
   cag atc act cgc atc tcc gcc ttc ttc cag cac ttc cag aac aat ggt       366
   tcc ctg gtt tgg tgc cag aat cac aag caa tgt tct aag tgc ctg gag       414
   ccc tgc aag gaa tca ggg gac ctg agg aaa cac cag tgc caa agc ttt       462
   tgt gag cct ctc ttc ccc aag aag agc tac gaa tgc ttg acc agc tgt       510
   gag ttc ctc aaa tac atc ctg ttg gtg aag cag ggg gac tgt ccg gct       558
   cct gag aaa gcc agt gga ttt gcg gcc gcc tgt gtt gaa agc tgc gaa       606
   gtt gac aat gag tgc tct ggg gtg aag aaa tgt tgt tcg aat ggg tgt       654
   gga cac acc tgt caa gta ccc aag act ctg tac aaa ggt gtc ccc ctg       702
   aag ccc aga aaa gag tta cga ttt aca gaa ctg cag tct gga cag ctg       750
   gag gtt aag tgg tcc tcg aaa ttc aat att tct att gag cct gtg atc       798
   tat gtg gta caa aga aga tgg aat tat gga atc cat cct agc gaa gat      846
   gac gcc act cac tgg cag aca gtg gcc cag acc aca gac gag cga gtt      894
   caa ctg act gac ata aga ccc agc cga tgg tac cag ttt cga gtg gct      942
   gct gtg aat gtg cat gga act cga ggc ttc act gcc ccc agc aaa cac      990
   ttc cgt tct tcc aaa gat cca tct gcc cca cca gca ccg gct aac ctc     1038
   cgg ctg gcc aac tcc acc gtc aac agt gat ggg agt gtg acc gtc act     1086
   ata gtt tgg gat ctc ccc gag gag ccg gac atc cct gtg cat cat tac     1134
   aag gtc ttt tgg agc tgg atg gtc agc agt aag tct ctt gtc cca aca     1182
   aag aag aag cgg aga aag act acg gat ggg ttt caa aat tct gtg atc     1230
   ctg gag aaa ctc cag cca gac tgt gac tat gtt gtg gaa ttg caa gcc     1278
   ata acg tac tgg gga cag aca cgg ctg aag agt gca aag gtg tcc ctt     1326
   cac ttc aca tcg aca cat gca acc aac aac aaa gaa cag ctt gtg aaa     1374
   act aga aaa ggt gga att caa aca caa ctc cct ttt caa aga cga cga     1422
   ccc act cgc ccg ctg gaa gtc gga gct ccc ttc tat cag gat ggc caa     1470
   ctg caa gtt aaa gtc tac tgg aag aag aca gaa gat ccc act gtc aac     1518
   cga tat cat gtg cgg tgg ttt cct gaa gcg tgt gcc cac aac aga aca     1566
   acc gga tca gag gca tca tct ggc atg acc cac gaa aat tac ata att     1614
   ctt caa gat ctg tca ttt tcc tgc aag tat aag gtg act gtc caa cca     1662
   ata cgg cca aaa agt cac tcc aag gca gaa gct gtt ttc ttc act act     1710
   cca cca tgc tct gct ctt aag ggg aag agc cac aag cct gtt ggc tgc     1758
   ctg ggc gaa gca ggt cat gtt ctt tct aag gtg cta gct aag cct gag     1806
   aac ctt tct gct tca ttc atc gtc cag gat gtg aac atc acc ggt cac     1854
   ttt tct tgg aag atg gcc aag gcc aat ctc tat cag ccc atg act ggg     1902
   ttt caa gtg act tgg gct gag gtc act acg gaa agc aga cag aac agc     1950
   cta ccc aac agc att att tca cag tcc cag atc ctg cct tcc gat cat     1998
   tat gtc cta aca gtg ccc aat ctg aga cca tct act ctt tac cga ctg     2046
   gaa gtg caa gtg ctg acc cca gga ggg gag ggg ccg gcc acc atc aag     2094
   acg ttc cgg acg ccg gag ctc cca ccc tct tca gca cac aga tct cat     2142
   ctt aag cat cgt cat cca cat cat tac aag cct tct cca gaa aga tac     2190
   taa actgttcaaa aagattttgt gaaattgcac agatgtgtaa gcttgttgaa          2243
   cttcggccac gagacatgca cacttccaga ggcagtggga actgctcaga ggcccggact   2303
   ctcctatgtg actttagtgc aggaagaact tctgtcaatc atggacgcat ctggagacaa   2363
   gtgagaaaca gtagattggt gaagacagac accagttccc tacaagcatg gagaaaatga   2423
   agaataggcc tgtttaatgc taaattttgt tttcatgtat ggtgtcgctc atttctattg 2483
   aattacaaca gaactcagtt ttccctgaat ttggagcacc aaactccgcc ccaaaaagga 2543
   gagtaacaaa tacacaattc acacataaca ctaagcgtaa atctaatcaa taaaatatat 2603
   ttttgactaa attattgatt cgatatgaaa aatcaactaa gattacacag ctttgttttt 2663
   ttgaatcttt cctaagatca tttttatcct aggtgatttt taaatgaaaa tgtgtaatct 2723
   aaaatatacc agcgaattta aatctaaaaa tgctcctact ttaagtacct tgtgctgctc 2783
   tttatgcaaa ggtaaatcaa agttccctct ataaattatg atttacaaaa gacacccaag 2843
   ccagaggaac tcaatgaaat aagctgctaa tcagatttta ccttggagaa atgaaaatta 2903
   tttcttgggg atgcctttta atatttgatc ctattatgtg agagattttc ctgatatgtt 2963
   atcttattta tattttccct tattttcctc aatgcagata atagcttttg gtgcactttt 3023
   gtttcaccat ctgaaaattc acaaaacttc ttgcttcaaa tgaaaaaatc ccaactattg 3083
   agcatgttta aatctttgca gagatttgcc ttttcttaat caaagaaagg tctttgtgtg 3143
   ctagaatatt attggtaatg ttttaaaaat tcctttgatt gatagagaag gacagttatt 3203
   tgcatttaat tcacccatat gctttcaaat ctagtatatc ttactttttg gaaatgtttt 3263
   atgctacaaa ttagtgcctt gtagcatgaa cttaagtcaa aacgtgttat caatatagag 3323
   tgttgcagtg tatattgtaa caacctaaaa cgcagagaag tttaatttaa tactgttttt 3383
   tttcttgaag gaatactcac atacatggtt tgaaatgtgc atagatatgc atgtctatat 3443
   aattataaat gcatgtgtat atatatgcaa atatatgtac atatacatgt atatacacac 3503
   agacacatgc atatacatga atataccttg agcatgaatc cctggagaaa tcgttttcgt 3563
   agctcaccaa tggtgagtaa agatacagct cttttaaagg tcataaggat aatatatttt 3623
   ccccatcaat gctgattctg agaaaagagc aatttatcaa aattaaacac tgtaaaagaa 3683
   aggtgtccat atgtctttac ctacctaagt aaaacaggaa gaaaatcagt aacattatcc 3743
   ttaggttttg acaatggtac ttgcttcttg ttgttttatt gtttcctgaa ttcatgcaga 3803
   tgcctggcca ttcctgggaa gagtggataa ctcagaagtc actgtactcc acagagcctc 3863
   actgcagtgt ctaaaggtag atgcaaatta aaatgcaggg aaaataactt ttctgatgtt 3923
   gatgcatgtc tttgggaaac acatttataa acatggatac ctgataatag atattgaaac 3983
   ccatttcctg tgtgttaaaa tatttaaaaa gtggatattc caggaatgtt ttgcagcttt 4043
   gtacaagtaa cataaattgg acacctcaga atgaaagttc atgttggttc tgaatggttc 4103
   actgcagctc ctgtcacaag ctgggatgga tttatcacat tgagttatga aattacctgg 4163
   ttctaagaat ttttgagtgg caaaaataga aaacaatctt catttgaaaa catccctaag 4223
   cttgaataaa tggataccat agatagcttc tcttttttat tctggtgtca ttaccagcat 4283
   ctgaatttca agttcttaaa atttcaaaaa ttaaaatttt tcattattag ctatccattt 4343
   atcttttaca tgaacttgtc atgaacaaat tcaaatgttt atgccagcaa atttttgtac 4403
   tgttgcatag ttaaaaatgc tgggagtctc tgcatagata caaaatatta ttaaattatt 4463
   acataaattt aattttataa aatttaatca tgcttctttt gtctgggtaa tagacattgg 4523
   acagatattt ttagttcaga tggtgattct gaagcttaca tctcccttaa aaaaatctaa 4583
   agcagctctt atgggcttct aattttaata taaataaata atttaaattt tattggtgtt 4643
   attggaagaa aaatgctatt aatgggctaa taaaaaacat gtgtttctct tatggatttt 4703
   aataagctcc agtattattc aaatgatcaa aaatatagtt ataatttttt gaattttaaa 4763
   aatgtgattg ctctaataaa gaataaaatc tatgcttttt aacaaacata gttttggtgc 4823
   ctaattctgt aatatgtttt attgaaatta gattcatttc tctaatgtga gaaaaatata 4883
   tccagtaata gtattgactg tttaaaaaat tgagctcatc aaaaatattg tcatcaaata 4943
   caggtggtta atctgacata cattgcagtt acatgcatta tttttattta caacatttgc 5003
   tccttaatga tgaatttatc tgtgttaccc tgtttttcta cctggaactc catagaatga 5063
   tgtttgcaaa ccaacatgtg ctcttttcag tcattcactg ttttaatatg acatggtaga 5123
   gaagataagg tttatggcag gtaatttttt gtaatgtgta ttaaacgaag ttcaaagatt 5183
   agaaatacat ctgtgtcctg aaaaccttag atacatagcc gactgtatac agaggttcat 5243
   ctcaacctca acactattga cttttggggc tggatagttc tctgttgtgg gggtttgtct 5303
   tgtgcactgt aggtttttag tagcatccac actttctcct caccagatgc cagttgcacc 5363
   ctcccccaag ttgagacaac caaaaatgtc tccagatatt gccagctacc ccttgaggga 5423
   tggtacctct ggttgagaac cattgctaga gaatgatctt tactgaattt gccctttata 5483
   agaaacccag tgaatttcta gagcaagtcc caaaaactaa gggacagcta agaagttatt 5543
   atggttgact tcaaaggcct aaactgtgtt ttttatgtcc actaaacaac ttgattaaaa 5603
   gacggaattt tgactcgtgt ctgtatcata caagtacaaa tactaatttt gccctatgta 5663
   tccgtaaatg tcatttgtga ttttgactta tttatttaat gccctttctt atgccgtggg 5723
   ttttcaagtt tactcatttc tatggttgca aataactcta aaacttatta tataaacttt 5783
   catattatag gcagaacaca atggctaaat atctgttgca tgtactttaa agtttattat 5843
   aaaatataaa cagatatata aagatgttga ctcttacctg tgattttgca tggtcagact 5903
   cggtgtcagg tacggagagg attctcatga ctgtcttacc tctactgaat attctagtga 5963
   gttatatgat ttacggagtg attaacagag gtctatataa agttactttt cccctttact 6023
   taattatatt gtagtgtgca gataacaaaa ctgctacctt ctcatccaag tggtctgtag 6083
   aattcatgtc ccttacagtg gtcatttaaa gtcaatattt atttatgtat gtaataaaaa 6143
   aagttggatt tttgtgtatg tctgtcacat tatttagaga gaagtaatct tgtaaaaatg 6203
   ttttgtaaaa aacaaaaaag tattgtaaat agtcttgata ttctgtgact cattattttc   6263
   atgttagagt ttgtacatac tggttcaata ataaagtatc cttaaaccag a            6314
<210> 8
   <211> 680
   <212> PRT
   <213> Homo sapiens
<400> 8
<210> 9
   <211> 1497
   <212> DNA
   <213> Homo sapiens
<220>
   <221> CDS
   <222> (196)..(1152)
   <223>
<400> 9
   aaagtgctgg gattacaggc atgagccgcc gcgccccgcc ccacgctcag tcttgaaatt     60
   gtctggaacg ggaaacggca aacagcgaga tatccgagcg agagtcccgc cctgcatcag    120
   tttgcggaac cgccttggta gaaggagaga aggggagtgg aggaagcacg ggactggagg    180
   gaccaaagtt ccccg atg gcg gcc cag ggg tgc gcg gca tcg cgg ctg ctc     231
                    Met Ala Ala Gln Gly Cys Ala Ala Ser Arg Leu Leu
                    1               5                   10
   cag ctg ctg ctg cag ctt ctg ctt cta ctg ttg ctg ctg gcg gcg ggc      279
   Gln Leu Leu Leu Gln Leu Leu Leu Leu Leu Leu Leu Leu Ala Ala Gly
           15                  20                  25
   ggg gcg cgc gcg cgg tgg cgc ggg gag ggc acc agc gca cac ttg cgg      327
   Gly Ala Arg Ala Arg Trp Arg Gly Glu Gly Thr Ser Ala His Leu Arg
       30                  35                  40
   gac atc ttc ctg ggc cgc tgc gcc gag tac cgc gca ctg ctg agt ccc      375
   Asp Ile Phe Leu Gly Arg Cys Ala Glu Tyr Arg Ala Leu Leu Ser Pro
   45                  50                  55                  60
   gag cag cgg aac aag aac tgc aca gcc atc tgg gaa gcc ttt aaa gtg      423
   Glu Gln Arg Asn Lys Asn Cys Thr Ala Ile Trp Glu Ala Phe Lys Val
                   65                  70                  75
   gcg ctg gac aag gat ccc tgc tcc gtg ctg ccc tca gac tat gac ctt      471
   Ala Leu Asp Lys Asp Pro Cys Ser Val Leu Pro Ser Asp Tyr Asp Leu
               80                  85                  90
   ttt att aac ttg tcc agg cac tct att ccc aga gat aag tcc ctg ttc      519
   Phe Ile Asn Leu Ser Arg His Ser Ile Pro Arg Asp Lys Ser Leu Phe
           95                  100                 105
   tgg gaa aat agc cac ctc ctt gtt aac agc ttt gca gac aac acc cgt      567
   Trp Glu Asn Ser His Leu Leu Val Asn Ser Phe Ala Asp Asn Thr Arg
       110                 115                 120
   cgt ttt atg ccc ctg agc gat gtt ctg tat ggc agg gtt gca gat ttc      615
   Arg Phe Met Pro Leu Ser Asp Val Leu Tyr Gly Arg Val Ala Asp Phe
   125                 130                 135                 140
   ttg agc tgg tgt cga cag aaa aat gac tct gga ctc gat tac caa tcc      663
   Leu Ser Trp Cys Arg Gln Lys Asn Asp Ser Gly Leu Asp Tyr Gln Ser
                   145                 150                 155
   tgc cct aca tca gaa gac tgt gaa aat aat cct gtg gat tcc ttt tgg      711
   Cys Pro Thr Ser Glu Asp Cys Glu Asn Asn Pro Val Asp Ser Phe Trp
               160                 165                 170
   aaa agg gca tcc atc cag tat tcc aag gat agt tct ggg gtg atc cac      759
   Lys Arg Ala Ser Ile Gln Tyr Ser Lys Asp Ser Ser Gly Val Ile His
           175                 180                 185
   gtc atg ctg aat ggt tca gag cca aca gga gcc tat ccc atc aaa ggt      807
   Val Met Leu Asn Gly Ser Glu Pro Thr Gly Ala Tyr Pro Ile Lys Gly
       190                 195                 200
   ttt ttt gca gat tat gaa att cca aac ctc cag aag gaa aaa att aca      855
   Phe Phe Ala Asp Tyr Glu Ile Pro Asn Leu Gln Lys Glu Lys Ile Thr
   205                 210                 215                 220
   cga atc gag atc tgg gtt atg cat gaa att ggg gga ccc aat gtg gaa      903
   Arg Ile Glu Ile Trp Val Met His Glu Ile Gly Gly Pro Asn Val Glu
                   225                 230                 235
   tcc tgc ggg gaa ggc agc atg aaa gtc ctg gaa aag agg ctg aag gac      951
   Ser Cys Gly Glu Gly Ser Met Lys Val Leu Glu Lys Arg Leu Lys Asp
               240                 245                 250
   atg ggg ttc cag tac agc tgt att aat gat tac cga cca gtg aag ctc      999
   Met Gly Phe Gln Tyr Ser Cys Ile Asn Asp Tyr Arg Pro Val Lys Leu
           255                 260                 265
   tta cag tgc gtg gac cac agc acc cat cct gac tgt gcc tta aag tcg     1047
   Leu Gln Cys Val Asp His Ser Thr His Pro Asp Cys Ala Leu Lys Ser
       270                 275                 280
   gca gca gcc gct act caa aga aaa gcc cca agt ctt tat aca gaa caa     1095
   Ala Ala Ala Ala Thr Gln Arg Lys Ala Pro Ser Leu Tyr Thr Glu Gln
   285                 290                 295                 300
   agg gcg ggt ctt atc att ccc ctc ttt ctg gtg ctg gct tcc agg act     1143
   Arg Ala Gly Leu Ile Ile Pro Leu Phe Leu Val Leu Ala Ser Arg Thr
                   305                 310                 315
   caa ctg taa ctggaaactg tgttgctcta accctcctcc agccctgcag             1192
   Gln Leu
   cctccccttg cagtcatcat tcgtgttctg tgtataccaa atgattctgt tatctaaaga   1252
   agctttttgc tgggaaaacg atgtcctgaa aatggtattt caatgaggca tatgttcagg   1312
   atttcagaaa caagaagtta gttctattta gcaggttaaa aaatgctgca ttagaattaa   1372
   agcaagttat tttcttattt gtataatgac acaaagcatt gggagtcaga ctgcttgtat   1432
   attatcaaac attttaagag aattctaata aagctgtatt ttacatcaaa aaaaaaaaaa   1492
   aaaaa                                                               1497
<210> 10
   <211> 318
   <212> PRT
   <213> Homo sapiens
<400> 10
   Met Ala Ala Gln Gly Cys Ala Ala Ser Arg Leu Leu Gln Leu Leu Leu
   1               5                   10                  15
   Gln Leu Leu Leu Leu Leu Leu Leu Leu Ala Ala Gly Gly Ala Arg Ala
               20                  25                  30
   Arg Trp Arg Gly Glu Gly Thr Ser Ala His Leu Arg Asp Ile Phe Leu
           35                  40                  45
   Gly Arg Cys Ala Glu Tyr Arg Ala Leu Leu Ser Pro Glu Gln Arg Asn
       50                  55                  60
   Lys Asn Cys Thr Ala Ile Trp Glu Ala Phe Lys Val Ala Leu Asp Lys
   65                  70                  75                  80
   Asp Pro Cys Ser Val Leu Pro Ser Asp Tyr Asp Leu Phe Ile Asn Leu
                   85                  90                  95
   Ser Arg His Ser Ile Pro Arg Asp Lys Ser Leu Phe Trp Glu Asn Ser
               100                 105                 110
   His Leu Leu Val Asn Ser Phe Ala Asp Asn Thr Arg Arg Phe Met Pro
           115                 120                 125
   Leu Ser Asp Val Leu Tyr Gly Arg Val Ala Asp Phe Leu Ser Trp Cys
       130                 135                 140
   Arg Gln Lys Asn Asp Ser Gly Leu Asp Tyr Gln Ser Cys Pro Thr Ser
   145                 150                 155                 160
   Glu Asp Cys Glu Asn Asn Pro Val Asp Ser Phe Trp Lys Arg Ala Ser
                   165                 170                 175
   Ile Gln Tyr Ser Lys Asp Ser Ser Gly Val Ile His Val Met Leu Asn
               180                 185                 190
   Gly Ser Glu Pro Thr Gly Ala Tyr Pro Ile Lys Gly Phe Phe Ala Asp
           195                 200                 205
   Tyr Glu Ile Pro Asn Leu Gln Lys Glu Lys Ile Thr Arg Ile Glu Ile
       210                 215                 220
   Trp Val Met His Glu Ile Gly Gly Pro Asn Val Glu Ser Cys Gly Glu
   225                 230                 235                 240
   Gly Ser Met Lys Val Leu Glu Lys Arg Leu Lys Asp Met Gly Phe Gln
                   245                 250                 255
   Tyr Ser Cys Ile Asn Asp Tyr Arg Pro Val Lys Leu Leu Gln Cys Val
               260                 265                 270
   Asp His Ser Thr His Pro Asp Cys Ala Leu Lys Ser Ala Ala Ala Ala
           275                 280                 285
   Thr Gln Arg Lys Ala Pro Ser Leu Tyr Thr Glu Gln Arg Ala Gly Leu
   290 295 300
   Ile Ile Pro Leu Phe Leu Val Leu Ala Ser Arg Thr Gln Leu
   305 310 315
<210> 11
   <211> 3020
   <212> DNA
   <213> Homo sapiens
<220>
   <221> CDS
   <222> (212)..(1045)
   <223>
<400> 11
   cacgcccctg ggcggtgcgc gcgggcggtg ggtaggccgg gcaggctcac gtgatgcggg      60
   ccccgcggcc cgccatataa acccgcgcgc ccgccaggcg ctgcggccgt cccgggccgt     120
   gactcctcct ttcccccgcc ccgcctccgt tcggagagcc ggcgggcggg cgcctctcgg     180
   ccaggaagcg cctcttggac gcgtgtgacc g atg ccc aga ttg cac gac cac        232
                                      Met Pro Arg Leu His Asp His
                                      1               5
   ttc tgg agc tgc tcc tgt gcg cac agc gcg agg cgc cga ggc ccc ccg       280
   Phe Trp Ser Cys Ser Cys Ala His Ser Ala Arg Arg Arg Gly Pro Pro
           10                  15                  20
   cga gcc agc gcc gcg ggg ctg gcg gcc aag gtt ggg gag atg atc aac       328
   Arg Ala Ser Ala Ala Gly Leu Ala Ala Lys Val Gly Glu Met Ile Asn
       25                  30                  35
   gtt tcc gtg tcc ggg ccc tcc ctg ctg gcg gcc cac ggt gcc ccg gac       376
   Val Ser Val Ser Gly Pro Ser Leu Leu Ala Ala His Gly Ala Pro Asp
   40                  45                  50                  55
   gct gac ccc gcg ccc agg ggc cgc agt gct gcg atg agc ggc ccc gag       424
   Ala Asp Pro Ala Pro Arg Gly Arg Ser Ala Ala Met Ser Gly Pro Glu
                   60                  65                  70
   ccc ggc agc ccc tac ccc aac acc tgg cat cat cgc ctg ttg cag agg       472
   Pro Gly Ser Pro Tyr Pro Asn Thr Trp His His Arg Leu Leu Gln Arg
               75                  80                  85
   agc ctc gtg ctc ttc tcg gtt ggg gtg gtc cta gcc ctg gtg ctc aac       520
   Ser Leu Val Leu Phe Ser Val Gly Val Val Leu Ala Leu Val Leu Asn
           90                  95                  100
   ctg ctg cag atc cag agg aat gtc act ctc ttc ccc gag gag gtg atc       568
   Leu Leu Gln Ile Gln Arg Asn Val Thr Leu Phe Pro Glu Glu Val Ile
       105                 110                 115
   gcc acc atc ttt tcc tcc gcc tgg tgg gtc cct ccc tgc tgc ggg aca       616
   Ala Thr Ile Phe Ser Ser Ala Trp Trp Val Pro Pro Cys Cys Gly Thr
   120                 125                 130                 135
   gca gct gct gtt gtt ggc cta ctg tac ccc tgt atc gac agt cac ctc       664
   Ala Ala Ala Val Val Gly Leu Leu Tyr Pro Cys Ile Asp Ser His Leu
                   140                 145                 150
   gga gaa ccc cac aaa ttt aag aga gaa tgg gcc agt gtc atg cgc tgc      712
   Gly Glu Pro His Lys Phe Lys Arg Glu Trp Ala Ser Val Met Arg Cys
               155                 160                 165
   ata gca gtt ttt gtt ggc att aac cac gcc agt gct aaa ttg gat ttt      760
   Ile Ala Val Phe Val Gly Ile Asn His Ala Ser Ala Lys Leu Asp Phe
           170                 175                 180
   gcc aat aat gtc cag ctg tcc ttg act tta gca gcc cta tct ttg ggc      808
   Ala Asn Asn Val Gln Leu Ser Leu Thr Leu Ala Ala Leu Ser Leu Gly
       185                 190                 195
   ctt tgg tgg aca ttt gat cgt tcc aga agt ggc ctt ggg ctg ggg atc      856
   Leu Trp Trp Thr Phe Asp Arg Ser Arg Ser Gly Leu Gly Leu Gly Ile
   200                 205                 210                 215
   acc ata gct ttt cta gct acg ctg atc acg cag ttt ctc gtg tat aat      904
   Thr Ile Ala Phe Leu Ala Thr Leu Ile Thr Gln Phe Leu Val Tyr Asn
                   220                 225                 230
   ggt gtc tat cag tat aca tcc cca gat ttc ctc tat att cgt tct tgg      952
   Gly Val Tyr Gln Tyr Thr Ser Pro Asp Phe Leu Tyr Ile Arg Ser Trp
               235                 240                 245
   ctc cct tgt ata ttt ttc tca gga ggc gtc acg gtg ggg aac ata gga     1000
   Leu Pro Cys Ile Phe Phe Ser Gly Gly Val Thr Val Gly Asn Ile Gly
           250                 255                 260
   cga cag tta gct atg ggt gtt cct gaa aag ccc cat agt gat tga         1045
   Arg Gln Leu Ala Met Gly Val Pro Glu Lys Pro His Ser Asp
       265                 270                 275
   gtcttcaaaa ccaccgattc tgagagcaag gaagattttg gaagaaaatc tgactgtgga   1105
   ttatgacaaa gattatcttt tttcttaagt aatctattta gatcgggctg actgtacaaa   1165
   tgactcctgg aaaaaactct tcacctagtc tagaataggg aggtggagaa tgatgactta   1225
   ccctgaagtc ttcccttgac tgcccgcact ggcgcctgtc tgtgccctgg agcattctgc   1285
   ccaggctacg tgggttcagg caggtggcag cttcccaagt attcgatttc attcatgtga   1345
   ttaaaacaag ttgccatatt tcaaagcctt gaactaagac tcaattacca acccgcagtt   1405
   ttgtgtcagt gcccaaagga ggtaggttga tggtgcttaa caaacatgaa gtatggtgta   1465
   ataggaataa tatttatcca aaagattttt aaaaataggg ctgtgtttaa aaaaaaaaac   1525
   aaaacaagaa aagcagcagt gattatagag aggtcacact ctaagtgggg tcgcggcgtg   1585
   gccacgcttc acggtcacgc tcgtccgtcc tgcagtggcg tgtttacatg gtcacacgtg   1645
   tgtgtatcac cagtgggtca actgcttgtc attcctcccg tggcagtttg tgtagacaat   1705
   cttactgagc aaaaggcaat gaaaagtctt ggttcccaca ctgcgatata ttggaatttt   1765
   cacctcagtt tatgaagttt atttcgaaat ccatagtcat ctaagaatga atacctgtct   1825
   gccatgtatt tcaatcttag tgagccaaaa ttgtttgttt gttactacag aatagagatg   1885
   actgtttttt gccacagccc tatggaattt gcaatctgtg attgccttgt aaaaaggaga   1945
   gtgcatatgg cactgcatta aacgtgtggt gtttctagtc aatgatattg gtgagcacaa   2005
   tgtattcatt taatggcata gaccatacca gacctaattt gcaagtattg ggtcttaaac   2065
   ttcaagtgca atgtatatga aaaccaatct gagccttgta tctcttaaat atttattttt   2125
   tttaacgtgt gagatgttcg agagaaggtt ctccattcat ttcagtgctg cctggaggaa   2185
   actcggcaat gatttctttc agttgtgaag ttcctttcgt gttacaccct ccactgaacc   2245
   ctcaaccttc gaaatactcc agttttgtgg gtttggtcat ttttacttat aaatttacct   2305
   ttttgtattt tgcaatttac atgtgtttgg tttgttttaa attctgtgaa agtggcttga   2365
   ttaaaagact ccttttaaat ggaagccacc agtcagcaga atggaagctt agaggaactt   2425
   gcctgtgagc gctggtcttt gtgtttggtt ttgtgatgta acgatctttg ctggggtttt   2485
   ttgctttgtt ttgagggaaa tgtcttggag taaattttaa gttcctggag ttaatttgtt   2545
   ttacaggaat tttgtttttt aaaaaaatag gatcattctg aactttggaa tgaccccctt   2605
   atatattttc tgaaaatgaa aacagttaca tgaaaaaaat ttccaatgaa gatgtcagca   2665
   ttttatgaaa aaccagaagt tattagatga aagcagcgag tgaatcttta aaacagactt   2725
   gatcacgcac acacaataag tctttctctc cgaaaccgga agtaaatcta tatctgttag   2785
   aaataatgta gccaaaagaa tgtaaatttg aggatttttt tgccaatagt ttatagaaaa   2845
   tatatgaacc aaagtgattt gagtttgtaa aaatgtaaaa tagtatgaac aaaatttgca   2905
   ctctaccaga tttgaacatc tagtgaggtt cacattcata ctaagttttc aacattgtgt   2965
   tctttttgca ttcatttttt acttttatta aaggttcaaa accaaaaaaa aaaaa        3020
<210> 12
   <211> 277
   <212> PRT
   <213> Homo sapiens
<400> 12
   Met Pro Arg Leu His Asp His Phe Trp Ser Cys Ser Cys Ala His Ser
   1               5                   10                  15
   Ala Arg Arg Arg Gly Pro Pro Arg Ala Ser Ala Ala Gly Leu Ala Ala
               20                  25                  30
   Lys Val Gly Glu Met Ile Asn Val Ser Val Ser Gly Pro Ser Leu Leu
           35                  40                  45
   Ala Ala His Gly Ala Pro Asp Ala Asp Pro Ala Pro Arg Gly Arg Ser
       50                  55                  60
   Ala Ala Met Ser Gly Pro Glu Pro Gly Ser Pro Tyr Pro Asn Thr Trp
   65                  70                  75                  80
   His His Arg Leu Leu Gln Arg Ser Leu Val Leu Phe Ser Val Gly Val
                   85                  90                  95
   Val Leu Ala Leu Val Leu Asn Leu Leu Gln Ile Gln Arg Asn Val Thr
               100                 105                 110
   Leu Phe Pro Glu Glu Val Ile Ala Thr Ile Phe Ser Ser Ala Trp Trp
           115                 120                 125
   Val Pro Pro Cys Cys Gly Thr Ala Ala Ala Val Val Gly Leu Leu Tyr
       130                 135                 140
   Pro Cys Ile Asp Ser His Leu Gly Glu Pro His Lys Phe Lys Arg Glu
   145                 150                 155                 160
   Trp Ala Ser Val Met Arg Cys Ile Ala Val Phe Val Gly Ile Asn His
                   165                 170                 175
   Ala Ser Ala Lys Leu Asp Phe Ala Asn Asn Val Gln Leu Ser Leu Thr
               180                 185                 190
   Leu Ala Ala Leu Ser Leu Gly Leu Trp Trp Thr Phe Asp Arg Ser Arg
           195                 200                 205
   Ser Gly Leu Gly Leu Gly Ile Thr Ile Ala Phe Leu Ala Thr Leu Ile
       210                 215                 220
   Thr Gln Phe Leu Val Tyr Asn Gly Val Tyr Gln Tyr Thr Ser Pro Asp
   225                 230                 235                 240
   Phe Leu Tyr Ile Arg Ser Trp Leu Pro Cys Ile Phe Phe Ser Gly Gly
                   245                 250                 255
   Val Thr Val Gly Asn Ile Gly Arg Gln Leu Ala Met Gly Val Pro Glu
               260                 265                 270
   Lys Pro His Ser Asp
           275
<210> 13
   <211> 5473
   <212> DNA
   <213> Homo sapiens
<220>
   <221> CDS
   <222> (491)..(1570)
   <223>
<400> 13
   ggcaggacga ggtggcacca aattcccttc ggccaatgac gagccggagt ttacagaagc      60
   ctcattagca tttccccaga ggcaggggca ggggcagagg ccgggtggtg tggtgtcggt     120
   gtcggcagca tccccggcgc cctgctgcgg tcgccgcgag cctcggcctc tgtctcctcc     180
   ccctcccgcc cttacctcca cgcgggaccg cccgcgccag tcaactcctc gcactttgcc     240
   cctgcttggc agcggataaa agggggctga ggaaataccg gacacggtca cccgttgcca      300
   gctctagcct ttaaattccc ggctcgggga cctccacgca ccgcggctag cgccgacaac     360
   cagctagcgt gcaaggcgcc gcggctcagc gcgtaccggc gggcttcgaa accgcagtcc     420
   tccggcgacc ccgaactccg ctccggagcc tcagccccct ggaaagtgat cccggcatcc     480
   gagagccaag atg ccg gcc cac ttg ctg cag gac gat atc tct agc tcc        529
              Met Pro Ala His Leu Leu Gln Asp Asp Ile Ser Ser Ser
              1               5                   10
   tat acc acc acc acc acc att aca gcg cct ccc tcc agg gtc ctg cag       577
   Tyr Thr Thr Thr Thr Thr Ile Thr Ala Pro Pro Ser Arg Val Leu Gln
       15                  20                  25
   aat gga gga gat aag ttg gag acg atg ccc ctc tac ttg gaa gac gac       625
   Asn Gly Gly Asp Lys Leu Glu Thr Met Pro Leu Tyr Leu Glu Asp Asp
   30                  35                  40                  45
   att cgc cct gat ata aaa gat gat ata tat gac ccc acc tac aag gat       673
   Ile Arg Pro Asp Ile Lys Asp Asp Ile Tyr Asp Pro Thr Tyr Lys Asp
                   50                  55                  60
   aag gaa ggc cca agc ccc aag gtt gaa tat gtc tgg aga aac atc atc       721
   Lys Glu Gly Pro Ser Pro Lys Val Glu Tyr Val Trp Arg Asn Ile Ile
               65                  70                  75
   ctt atg tct ctg cta cac ttg gga gcc ctg tat ggg atc act ttg att       769
   Leu Met Ser Leu Leu His Leu Gly Ala Leu Tyr Gly Ile Thr Leu Ile
           80                  85                  90
   cct acc tgc aag ttc tac acc tgg ctt tgg ggg gta ttc tac tat ttt       817
   Pro Thr Cys Lys Phe Tyr Thr Trp Leu Trp Gly Val Phe Tyr Tyr Phe
       95                  100                 105
   gtc agt gcc ctg ggc ata aca gca gga gct cat cgt ctg tgg agc cac       865
   Val Ser Ala Leu Gly Ile Thr Ala Gly Ala His Arg Leu Trp Ser His
   110                 115                 120                 125
   cgc tct tac aaa gct cgg ctg ccc cta cgg ctc ttt ctg atc att gcc       913
   Arg Ser Tyr Lys Ala Arg Leu Pro Leu Arg Leu Phe Leu Ile Ile Ala
                   130                 135                 140
   aac aca atg gca ttc cag aat gat gtc tat gaa tgg gct cgt gac cac       961
   Asn Thr Met Ala Phe Gln Asn Asp Val Tyr Glu Trp Ala Arg Asp His
               145                 150                 155
   cgt gcc cac cac aag ttt tca gaa aca cat gct gat cct cat aat tcc      1009
   Arg Ala His His Lys Phe Ser Glu Thr His Ala Asp Pro His Asn Ser
           160                 165                 170
   cga cgt ggc ttt ttc ttc tct cac gtg ggt tgg ctg ctt gtg cgc aaa      1057
   Arg Arg Gly Phe Phe Phe Ser His Val Gly Trp Leu Leu Val Arg Lys
       175                 180                 185
   cac cca gct gtc aaa gag aag ggg agt acg cta gac ttg tct gac cta      1105
   His Pro Ala Val Lys Glu Lys Gly Ser Thr Leu Asp Leu Ser Asp Leu
   190                 195                 200                 205
   gaa gct gag aaa ctg gtg atg ttc cag agg agg tac tac aaa cct ggc      1153
   Glu Ala Glu Lys Leu Val Met Phe Gln Arg Arg Tyr Tyr Lys Pro Gly
                   210                 215                 220
   ttg ctg atg atg tgc ttc atc ctg ccc acg ctt gtg ccc tgg tat ttc      1201
   Leu Leu Met Met Cys Phe Ile Leu Pro Thr Leu Val Pro Trp Tyr Phe
               225                 230                 235
   tgg ggt gaa act ttt caa aac agt gtg ttc gtt gcc act ttc ttg cga      1249
   Trp Gly Glu Thr Phe Gln Asn Ser Val Phe Val Ala Thr Phe Leu Arg
           240                 245                 250
   tat gct gtg gtg ctt aat gcc acc tgg ctg gtg aac agt gct gcc cac      1297
   Tyr Ala Val Val Leu Asn Ala Thr Trp Leu Val Asn Ser Ala Ala His
       255                 260                 265
   ctc ttc gga tat cgt cct tat gac aag aac att agc ccc cgg gag aat      1345
   Leu Phe Gly Tyr Arg Pro Tyr Asp Lys Asn Ile Ser Pro Arg Glu Asn
   270                 275                 280                 285
   atc ctg gtt tca ctt gga gct gtg ggt gag ggc ttc cac aac tac cac      1393
   Ile Leu Val Ser Leu Gly Ala Val Gly Glu Gly Phe His Asn Tyr His
                   290                 295                 300
   cac tcc ttt ccc tat gac tac tct gcc agt gag tac cgc tgg cac atc      1441
   His Ser Phe Pro Tyr Asp Tyr Ser Ala Ser Glu Tyr Arg Trp His Ile
               305                 310                 315
   aac ttc acc aca ttc ttc att gat tgc atg gcc gcc ctc ggt ctg gcc      1489
   Asn Phe Thr Thr Phe Phe Ile Asp Cys Met Ala Ala Leu Gly Leu Ala
           320                 325                 330
   tat gac cgg aag aaa gtc tcc aag gcc gcc atc ttg gcc agg att aaa      1537
   Tyr Asp Arg Lys Lys Val Ser Lys Ala Ala Ile Leu Ala Arg Ile Lys
       335                 340                 345
   aga acc gga gat gga aac tac aag agt ggc tga gtttggggtc cctcaggttc   1590
   Arg Thr Gly Asp Gly Asn Tyr Lys Ser Gly
   350                 355
   ctttttcaaa aaccagccag gcagaggttt taatgtctgt ttattaacta ctgaataatg   1650
   ctaccaggat gctaaagatg atgatgttaa cccattccag tacagtattc ttttaaaatt   1710
   caaaagtatt gaaagccaac aactctgcct ttatgatgct aagctgatat tatttcttct   1770
   cttatcctct ctctcttcta ggcccattgt cctccttttc actttattgc tatcgccctc   1830
   ctttccctta ttgcctccca ggcaagcagc tggtcagtct ttgctcagtg tccagcttcc   1890
   aaagcctaga caacctttct gtagcctaaa acgaatggtc tttgctccag ataactctct   1950
   ttccttgagc tgttgtgagc tttgaagtag gtggcttgag ctagagataa aacagaatct   2010
   tctgggtagt cccctgttga ttatcttcag cccaggcttt tgctagatgg aatggaaaag   2070
   caacttcatt tgacacaaag cttctaaagc aggtaaattg tcgggggaga gagttagcat   2130
   gtatgaatgt aaggatgagg gaagcgaagc aagaggaacc tctcgccatg atcagacata 2190
   cagctgccta cctaatgagg acttcaagcc ccaccacata gcatgcttcc tttctctcct 2250
   ggctcggggt aaaaagtggc tgcggtgttt ggcaatgcta attcaatgcc gcaacatata 2310
   gttgaggccg aggataaaga aaagacattt taagtttgta gtaaaagtgg tctctgctgg 2370
   ggaagggttt tcttttcttt ttttctttaa taacaaggag atttcttagt tcatatatca 2430
   agaagtcttg aagttgggtg tttccagaat tggtaaaaac agcagctcat agaattttga 2490
   gtattccatg agctgctcat tacagttctt tcctctttct gctctgccat cttcaggata 2550
   ttggttcttc ccctcatagt aataagatgg ctgtggcatt tccaaacatc caaaaaaagg 2610
   gaaggattta aggaggtgaa gtcgggtcaa aaataaaata tatatacata tatacattgc 2670
   ttagaacgtt aaactattag agtatttccc ttccaaagag ggatgtttgg aaaaaactct 2730
   gaaggagagg aggaattagt tgggatgcca atttcctctc cactgctgga catgagatgg 2790
   agaggctgag ggacaggatc tataggcagc ttctaagagc gaacttcaca taggaaggga 2850
   tctgagaaca cgttgccagg ggcttgagaa ggttactgag tgagttattg ggagtcttaa 2910
   taaaataaac tagatattag gtccattcat taattagttc cagtttctcc ttgaaatgag 2970
   taaaaactag aaggcttctc tccacagtgt tgtgcccctt cactcatttt tttttgagga 3030
   gaagggggtc tctgttaaca tctagcctaa agtatacaac tgcctggggg gcagggttag 3090
   gaatctcttc actaccctga ttcttgattc ctggctctac cctgtctgtc ccttttcttt 3150
   gaccagatct ttctcttccc tgaacgtttt cttctttccc tggacaggca gcctcctttg 3210
   tgtgtattca gaggcagtga tgacttgctg tccaggcagc tccctcctgc acacagaatg 3270
   ctcagggtca ctgaaccact gcttctcttt tgaaagtaga gctagctgcc actttcacgt 3330
   ggcctccgca gtgtctccac ctacacccct gtgctcccct gccacactga tggctcaaga 3390
   caaggctggc aaaccctccc agaaacatct ctggcccaga aagcctctct ctccctccct 3450
   ctctcatgag gcacagccaa gccaagcgct catgttgagc cagtgggcca gccacagagc 3510
   aaaagagggt ttattttcag tcccctctct ctgggtcaga accagagggc atgctgaatg 3570
   ccccctgctt acttggtgag ggtgccccgc ctgagtcagt gctctcagct ggcagtgcaa 3630
   tgcttgtaga agtaggagga aacagttctc actgggaaga agcaagggca agaacccaag 3690
   tgcctcacct cgaaaggagg ccctgttccc tggagtcagg gtgaactgca aagctttggc 3750
   tgagacctgg gatttgagat accacaaacc ctgctgaaca cagtgtctgt tcagcaaact 3810
   aaccagcatt ccctacagcc tagggcagac aatagtatag aagtctggaa aaaaacaaaa 3870
   acagaatttg agaaccttgg accactcctg tccctgtagc tcagtcatca aagcagaagt 3930
   ctggctttgc tctattaaga ttggaaatgt acactaccaa acactcagtc cactgttgag 3990
   ccccagtgct ggaagggagg aaggcctttc ttctgtgtta attgcgtaga ggctacaggg 4050
   gttagcctgg actaaaggca tccttgtctt ttgagctatt cacctcagta gaaaaggatc 4110
   taagggaaga tcactgtagt ttagttctgt tgacctgtgc acctacccct tggaaatgtc 4170
   tgctggtatt tctaattcca caggtcatca gatgcctgct tgataatata taaacaataa 4230
   aaacaacttt cacttcttcc tattgtaatc gtgtgccatg gatctgatct gtaccatgac 4290
   cctacataag gctggatggc acctcaggct gagggcccca atgtatgtgt ggctgtgggt 4350
   gtgggtggga gtgtgtctgc tgagtaagga acacgatttt caagattcta aagctcaatt 4410
   caagtgacac attaatgata aactcagatc tgatcaagag tccggatttc taacagtcct 4470
   tgctttgggg ggtgtgctga caacttagct caggtgcctt acatcttttc taatcacagt 4530
   gttgcatatg agcctgccct cactccctct gcagaatccc tttgcacctg agaccctact 4590
   gaagtggctg gtagaaaaag gggcctgagt ggaggattat cagtatcacg atttgcagga 4650
   ttcccttctg ggcttcattc tggaaacttt tgttagggct gcttttctta agtgcccaca 4710
   tttgatggag ggtggaaata atttgaatgt atttgattta taagtttttt tttttttttt 4770
   gggttaaaag atggttgtag catttaaaat ggaaaatttt ctccttggtt tgctagtatc 4830
   ttgggtgtat tctctgtaag tgtagctcaa ataggtcatc atgaaaggtt aaaaaagcga 4890
   ggtggccatg ttatgctggt ggttaaggcc agggcctctc caaccactgt gccactgact 4950
   tgctgtgtga ccctgggcaa gtcacttaac tataaggtgc ctcagttttc cttctgttaa 5010
   aatggggata ataatactga cctacctcaa agggcagttt tgaggcatga ctaatgcttt 5070
   ttagaaagca ttttgggatc cttcagcaca ggaattctca agacctgagt attttttata 5130
   ataggaatgt ccaccatgaa cttgatacgt ccgtgtgtcc cagatgctgt cattagtcta 5190
   tatggttctc caagaaactg aatgaatcca ttggagaagc ggtggataac tagccagaca 5250
   aaatttgaga atacataaac aacgcattgc cacggaaaca tacagaggat gccttttctg 5310
   tgattgggtg ggattttttc cctttttatg tgggatatag tagttacttg tgacaagaat 5370
   aattttggaa taatttctat taatatcaac tctgaagcta attgtactaa tctgagattg 5430
   tgtttgttca taataaaagt gaagtgaatc tgattgcaaa aaa 5473
<210> 14
   <211> 359
   <212> PRT
   <213> Homo sapiens
<400> 14
   Met Pro Ala His Leu Leu Gln Asp Asp Ile Ser Ser Ser Tyr Thr Thr
   1               5                   10                  15
   Thr Thr Thr Ile Thr Ala Pro Pro Ser Arg Val Leu Gln Asn Gly Gly
               20                  25                  30
   Asp Lys Leu Glu Thr Met Pro Leu Tyr Leu Glu Asp Asp Ile Arg Pro
           35                  40                  45
   Asp Ile Lys Asp Asp Ile Tyr Asp Pro Thr Tyr Lys Asp Lys Glu Gly
       50                  55                  60
   Pro Ser Pro Lys Val Glu Tyr Val Trp Arg Asn Ile Ile Leu Met Ser
   65                  70                  75                  80
   Leu Leu His Leu Gly Ala Leu Tyr Gly Ile Thr Leu Ile Pro Thr Cys
                   85                  90                  95
   Lys Phe Tyr Thr Trp Leu Trp Gly Val Phe Tyr Tyr Phe Val Ser Ala
               100                 105                 110
   Leu Gly Ile Thr Ala Gly Ala His Arg Leu Trp Ser His Arg Ser Tyr
           115                 120                 125
   Lys Ala Arg Leu Pro Leu Arg Leu Phe Leu Ile Ile Ala Asn Thr Met
       130                 135                 140
   Ala Phe Gln Asn Asp Val Tyr Glu Trp Ala Arg Asp His Arg Ala His
   145                 150                 155                 160
   His Lys Phe Ser Glu Thr His Ala Asp Pro His Asn Ser Arg Arg Gly
                   165                 170                 175
   Phe Phe Phe Ser His Val Gly Trp Leu Leu Val Arg Lys His Pro Ala
               180                 185                 190
   Val Lys Glu Lys Gly Ser Thr Leu Asp Leu Ser Asp Leu Glu Ala Glu
           195                 200                 205
   Lys Leu Val Met Phe Gln Arg Arg Tyr Tyr Lys Pro Gly Leu Leu Met
       210                 215                 220
   Met Cys Phe Ile Leu Pro Thr Leu Val Pro Trp Tyr Phe Trp Gly Glu
   225                 230                 235                 240
   Thr Phe Gln Asn Ser Val Phe Val Ala Thr Phe Leu Arg Tyr Ala Val
                   245                 250                 255
   Val Leu Asn Ala Thr Trp Leu Val Asn Ser Ala Ala His Leu Phe Gly
               260                 265                 270
   Tyr Arg Pro Tyr Asp Lys Asn Ile Ser Pro Arg Glu Asn Ile Leu Val
           275                 280                 285
   Ser Leu Gly Ala Val Gly Glu Gly Phe His Asn Tyr His His Ser Phe
       290                 295                 300
   Pro Tyr Asp Tyr Ser Ala Ser Glu Tyr Arg Trp His Ile Asn Phe Thr
   305                 310                 315                 320
   Thr Phe Phe Ile Asp Cys Met Ala Ala Leu Gly Leu Ala Tyr Asp Arg
                   325                 330                 335
   Lys Lys Val Ser Lys Ala Ala Ile Leu Ala Arg Ile Lys Arg Thr Gly
               340                 345                 350
   Asp Gly Asn Tyr Lys Ser Gly
           355
<210> 15
   <211> 1689
   <212> DNA
   <213> Homo sapiens
<220>
   <221> CDS
   <222> (146)..(1267)
   <223>
<400> 15
   aggaaatgag gtgagaagta cgtccactct tctgtccagc ttttaacaat ctaactaatg     60
   ccctctccag ggtcacccta gaatcagatc tgctccccag catcttctgt ttcctggtga    120
   gtgattcctg ctactttgga tggcc atg acg ggc tgg agc tgc ctt gtg aca      172
                               Met Thr Gly Trp Ser Cys Leu Val Thr
                               1               5
   gga gca gga ggg ttt ctg gga cag agg atc atc cgc ctc ttg gtg aag      220
   Gly Ala Gly Gly Phe Leu Gly Gln Arg Ile Ile Arg Leu Leu Val Lys
   10                  15                  20                  25
   gag aag gag ctg aag gag atc agg gtc ttg gac aag gcc ttc gga cca      268
   Glu Lys Glu Leu Lys Glu Ile Arg Val Leu Asp Lys Ala Phe Gly Pro
                   30                  35                  40
   gaa ttg aga gag gaa ttt tct aaa ctc cag aac aag acc aag ctg aca      316
   Glu Leu Arg Glu Glu Phe Ser Lys Leu Gln Asn Lys Thr Lys Leu Thr
               45                  50                  55
   gtg ctg gaa gga gac att ctg gat gag cca ttc ctg aag aga gcc tgc      364
   Val Leu Glu Gly Asp Ile Leu Asp Glu Pro Phe Leu Lys Arg Ala Cys
           60                  65                  70
   cag gac gtc tcg gtc atc atc cac acc gcc tgt atc att gat gtc ttc      412
   Gln Asp Val Ser Val Ile Ile His Thr Ala Cys Ile Ile Asp Val Phe
       75                  80                  85
   ggt gtc act cac aga gag tct atc atg aat gtc aat gtg aaa ggt acc      460
   Gly Val Thr His Arg Glu Ser Ile Met Asn Val Asn Val Lys Gly Thr
   90                  95                  100                 105
   cag ctc ctg tta gag gcc tgt gtc caa gct agt gtg cca gtc ttc atc      508
   Gln Leu Leu Leu Glu Ala Cys Val Gln Ala Ser Val Pro Val Phe Ile
                   110                 115                 120
   tac acc agt agc ata gag gta gcc ggg ccc aac tcc tac aag gaa atc      556
   Tyr Thr Ser Ser Ile Glu Val Ala Gly Pro Asn Ser Tyr Lys Glu Ile
               125                 130                 135
   atc cag aat ggc cat gaa gaa gag cct ctg gaa aac aca tgg ccc gct      604
   Ile Gln Asn Gly His Glu Glu Glu Pro Leu Glu Asn Thr Trp Pro Ala
           140                 145                 150
   cca tac cca cac agc aaa aag ctt gct gag aag gct gta ctg gcg gct      652
   Pro Tyr Pro His Ser Lys Lys Leu Ala Glu Lys Ala Val Leu Ala Ala
       155                 160                 165
   aac ggg tgg aat ctg aaa aac ggc ggc acc ctg tac act tgt gcc tta      700
   Asn Gly Trp Asn Leu Lys Asn Gly Gly Thr Leu Tyr Thr Cys Ala Leu
   170                 175                 180                 185
   cga ccc atg tat atc tat ggg gaa gga agc cga ttc ctt tct gct agt      748
   Arg Pro Met Tyr Ile Tyr Gly Glu Gly Ser Arg Phe Leu Ser Ala Ser
                   190                 195                 200
   ata aac gag gcc ctg aac aac aat ggg atc ctg tca agt gtt gga aag      796
   Ile Asn Glu Ala Leu Asn Asn Asn Gly Ile Leu Ser Ser Val Gly Lys
               205                 210                 215
   ttc tcc act gtt aac cca gtc tat gtt ggc aat gtg gcc tgg gcc cac      844
   Phe Ser Thr Val Asn Pro Val Tyr Val Gly Asn Val Ala Trp Ala His
           220                 225                 230
   att ctg gcc ttg agg gcc ctg cag gac ccc aag aag gcc cca agc atc      892
   Ile Leu Ala Leu Arg Ala Leu Gln Asp Pro Lys Lys Ala Pro Ser Ile
       235                 240                 245
   cga gga cag ttc tac tat atc tca gat gac acg cct cac caa agc tat      940
   Arg Gly Gln Phe Tyr Tyr Ile Ser Asp Asp Thr Pro His Gln Ser Tyr
   250                 255                 260                 265
   gat aac ctt aat tac acc ctg agc aaa gag ttc ggc ctc cgc ctt gat      988
   Asp Asn Leu Asn Tyr Thr Leu Ser Lys Glu Phe Gly Leu Arg Leu Asp
                   270                 275                 280
   tcc aga tgg agc ttt cct tta tcc ctg atg tat tgg att ggc ttc ctg     1036
   Ser Arg Trp Ser Phe Pro Leu Ser Leu Met Tyr Trp Ile Gly Phe Leu
               285                 290                 295
   ctg gaa ata gtg agc ttc cta ctc agg cca att tac acc tat cga ccg     1084
   Leu Glu Ile Val Ser Phe Leu Leu Arg Pro Ile Tyr Thr Tyr Arg Pro
           300                 305                 310
   ccc ttc aac cgc cac ata gtc aca ttg tca aat agc gta ttc acc ttc     1132
   Pro Phe Asn Arg His Ile Val Thr Leu Ser Asn Ser Val Phe Thr Phe
       315                 320                 325
   tct tat aag aag gct cag cga gat ctg gcg tat aag cca ctc tac agc     1180
   Ser Tyr Lys Lys Ala Gln Arg Asp Leu Ala Tyr Lys Pro Leu Tyr Ser
   330                 335                 340                 345
   tgg gag gaa gcc aag cag aaa acg gtg gag tgg gtt ggt tcc ctt gtg     1228
   Trp Glu Glu Ala Lys Gln Lys Thr Val Glu Trp Val Gly Ser Leu Val
                   350                 355                 360
   gac cgg cac aag gag acc ctg aag tcc aag act cag tga tttaaggatg      1277
   Asp Arg His Lys Glu Thr Leu Lys Ser Lys Thr Gln
               365                 370
   acagagatgt gcatgtgggt attgttagga gatgtcatca agctccaccc tcctggcctc   1337
   atacagaaag tgacaagggc acaagctcag gtcctgctgc ctccctttca tacaatggcc   1397
   aacttattgt attcctcatg tcatcaaaac ctgcgcagtc attggcccaa caagaaggtt   1457
   tctgtcctaa tcatatacca gaggaaagac catgtggttt gctgttacca aatctcagta   1517
   gctgattctg aacaatttag ggactctttt aacttgaggg tcgttttgac tactagagct   1577
   ccatttctac tcttaaatga gaaaggattt cctttctttt taatcttcca ttccttcaca   1637
   tagtttgata aaaagatcaa taaatgtttg aatgtttaat gtgaaaaaaa aa           1689
<210> 16
   <211> 373
   <212> PRT
   <213> Homo sapiens
<400> 16
   Met Thr Gly Trp Ser Cys Leu Val Thr Gly Ala Gly Gly Phe Leu Gly
   1               5                   10                  15
   Gln Arg Ile Ile Arg Leu Leu Val Lys Glu Lys Glu Leu Lys Glu Ile
               20                  25                  30
   Arg Val Leu Asp Lys Ala Phe Gly Pro Glu Leu Arg Glu Glu Phe Ser
           35                  40                  45
   Lys Leu Gln Asn Lys Thr Lys Leu Thr Val Leu Glu Gly Asp Ile Leu
       50                  55                  60
   Asp Glu Pro Phe Leu Lys Arg Ala Cys Gln Asp Val Ser Val Ile Ile
   65                  70                  75                  80
   His Thr Ala Cys Ile Ile Asp Val Phe Gly Val Thr His Arg Glu Ser
                   85                  90                  95
   Ile Met Asn Val Asn Val Lys Gly Thr Gln Leu Leu Leu Glu Ala Cys
               100                 105                 110
   Val Gln Ala Ser Val Pro Val Phe Ile Tyr Thr Ser Ser Ile Glu Val
           115                 120                 125
   Ala Gly Pro Asn Ser Tyr Lys Glu Ile Ile Gln Asn Gly His Glu Glu
       130                 135                 140
   Glu Pro Leu Glu Asn Thr Trp Pro Ala Pro Tyr Pro His Ser Lys Lys
   145                 150                 155                 160
   Leu Ala Glu Lys Ala Val Leu Ala Ala Asn Gly Trp Asn Leu Lys Asn
                   165                 170                 175
   Gly Gly Thr Leu Tyr Thr Cys Ala Leu Arg Pro Met Tyr Ile Tyr Gly
               180                 185                 190
   Glu Gly Ser Arg Phe Leu Ser Ala Ser Ile Asn Glu Ala Leu Asn Asn
           195                 200                 205
   Asn Gly Ile Leu Ser Ser Val Gly Lys Phe Ser Thr Val Asn Pro Val
       210                 215                 220
   Tyr Val Gly Asn Val Ala Trp Ala His Ile Leu Ala Leu Arg Ala Leu
   225                 230                 235                 240
   Gln Asp Pro Lys Lys Ala Pro Ser Ile Arg Gly Gln Phe Tyr Tyr Ile
                   245                 250                 255
   Ser Asp Asp Thr Pro His Gln Ser Tyr Asp Asn Leu Asn Tyr Thr Leu
               260                 265                 270
   Ser Lys Glu Phe Gly Leu Arg Leu Asp Ser Arg Trp Ser Phe Pro Leu
           275                 280                 285
   Ser Leu Met Tyr Trp Ile Gly Phe Leu Leu Glu Ile Val Ser Phe Leu
       290                 295                 300
   Leu Arg Pro Ile Tyr Thr Tyr Arg Pro Pro Phe Asn Arg His Ile Val
   305                 310                 315                 320
   Thr Leu Ser Asn Ser Val Phe Thr Phe Ser Tyr Lys Lys Ala Gln Arg
                   325                 330                 335
   Asp Leu Ala Tyr Lys Pro Leu Tyr Ser Trp Glu Glu Ala Lys Gln Lys
               340                 345                 350
   Thr Val Glu Trp Val Gly Ser Leu Val Asp Arg His Lys Glu Thr Leu
           355                 360                 365
   Lys Ser Lys Thr Gln
       370
<210> 17
   <211> 1753
   <212> DNA
   <213> Homo sapiens
<220>
   <221> CDS
   <222> (128)..(1537)
   <223>
<400> 17
   cagccccgcc cctacctgtg gaagcccagc cgcccgctcc cgcggataaa aggcgcggag      60
   tgtccccgag gtcagcgagt gcgcgctcct cctcgcccgc cgctaggtcc atcccggccc     120
   agccacc atg tcc atc cac ttc agc tcc ccg gta ttc acc tcg cgc tca       169
           Met Ser Ile His Phe Ser Ser Pro Val Phe Thr Ser Arg Ser
           1               5                   10
   gcc gcc ttc tcg ggc cgc ggc gcc cag gtg cgc ctg agc tcc gct cgc       217
   Ala Ala Phe Ser Gly Arg Gly Ala Gln Val Arg Leu Ser Ser Ala Arg
   15                  20                  25                  30
   ccc ggc ggc ctt ggc agc agc agc ctc tac ggc ctc ggc gcc tca cgg       265
   Pro Gly Gly Leu Gly Ser Ser Ser Leu Tyr Gly Leu Gly Ala Ser Arg
                   35                  40                  45
   ccg cgc gtg gcc gtg cgc tct gcc tat ggg ggc ccg gtg ggc gcc ggc       313
   Pro Arg Val Ala Val Arg Ser Ala Tyr Gly Gly Pro Val Gly Ala Gly
               50                  55                  60
   atc cgc gag gtc acc att aac cag agc ctg ctg gcc ccg ctg cgg ctg       361
   Ile Arg Glu Val Thr Ile Asn Gln Ser Leu Leu Ala Pro Leu Arg Leu
           65                  70                  75
   gac gcc gac ccc tcc ctc cag cgg gtg cgc cag gag gag agc gag cag       409
   Asp Ala Asp Pro Ser Leu Gln Arg Val Arg Gln Glu Glu Ser Glu Gln
       80                  85                  90
   atc aag acc ctc aac aac aag ttt gcc tcc ttc atc gac aag gtg cgg       457
   Ile Lys Thr Leu Asn Asn Lys Phe Ala Ser Phe Ile Asp Lys Val Arg
   95                  100                 105                 110
   ttt ctg gag cag cag aac aag ctg ctg gag acc aag tgg acg ctg ctg       505
   Phe Leu Glu Gln Gln Asn Lys Leu Leu Glu Thr Lys Trp Thr Leu Leu
                   115                 120                 125
   cag gag cag aag tcg gcc aag agc agc cgc ctc cca gac atc ttt gag       553
   Gln Glu Gln Lys Ser Ala Lys Ser Ser Arg Leu Pro Asp Ile Phe Glu
               130                 135                 140
   gcc cag att gct ggc ctt cgg ggt cag ctt gag gca ctg cag gtg gat       601
   Ala Gln Ile Ala Gly Leu Arg Gly Gln Leu Glu Ala Leu Gln Val Asp
           145                 150                 155
   ggg ggc cgc ctg gag gcg gag ctg cgg agc atg cag gat gtg gtg gag       649
   Gly Gly Arg Leu Glu Ala Glu Leu Arg Ser Met Gln Asp Val Val Glu
       160                 165                 170
   gac ttc aag aat aag tac gaa gat gaa att aac cac cgc aca gct gct       697
   Asp Phe Lys Asn Lys Tyr Glu Asp Glu Ile Asn His Arg Thr Ala Ala
   175                 180                 185                 190
   gag aat gag ttt gtg gtg ctg aag aag gat gtg gat gct gcc tac atg       745
   Glu Asn Glu Phe Val Val Leu Lys Lys Asp Val Asp Ala Ala Tyr Met
                   195                 200                 205
   agc aag gtg gag ctg gag gcc aag gtg gat gcc ctg aat gat gag atc       793
   Ser Lys Val Glu Leu Glu Ala Lys Val Asp Ala Leu Asn Asp Glu Ile
               210                 215                 220
   aac ttc ctc agg acc ctc aat gag acg gag ttg aca gag ctg cag tcc       841
   Asn Phe Leu Arg Thr Leu Asn Glu Thr Glu Leu Thr Glu Leu Gln Ser
           225                 230                 235
   cag atc tcc gac aca tct gtg gtg ctg tcc atg gac aac agt cgc tcc       889
   Gln Ile Ser Asp Thr Ser Val Val Leu Ser Met Asp Asn Ser Arg Ser
       240                 245                 250
   ctg gac ctg gac ggc atc atc gct gag gtc aag gcg cag tat gag gag       937
   Leu Asp Leu Asp Gly Ile Ile Ala Glu Val Lys Ala Gln Tyr Glu Glu
   255                 260                 265                 270
   atg gcc aaa tgc agc cgg gct gag gct gaa gcc tgg tac cag acc aag       985
   Met Ala Lys Cys Ser Arg Ala Glu Ala Glu Ala Trp Tyr Gln Thr Lys
                   275                 280                 285
   ttt gag acc ctc cag gcc cag gct ggg aag cat ggg gac gac ctc cgg      1033
   Phe Glu Thr Leu Gln Ala Gln Ala Gly Lys His Gly Asp Asp Leu Arg
               290                 295                 300
   aat acc cgg aat gag att tca gag atg aac cgg gcc atc cag agg ctg      1081
   Asn Thr Arg Asn Glu Ile Ser Glu Met Asn Arg Ala Ile Gln Arg Leu
           305                 310                 315
   cag gct gag atc gac aac atc aag aac cag cgt gcc aag ttg gag gcc      1129
   Gln Ala Glu Ile Asp Asn Ile Lys Asn Gln Arg Ala Lys Leu Glu Ala
       320                 325                 330
   gcc att gcc gag gct gag gag cgt ggg gag ctg gcg ctc aag gat gct      1177
   Ala Ile Ala Glu Ala Glu Glu Arg Gly Glu Leu Ala Leu Lys Asp Ala
   335                 340                 345                 350
   cgt gcc aag cag gag gag ctg gaa gcc gcc ctg cag cgg ggc aag cag      1225
   Arg Ala Lys Gln Glu Glu Leu Glu Ala Ala Leu Gln Arg Gly Lys Gln
                   355                 360                 365
   gat atg gca cgg cag ctg cgt gag tac cag gaa ctc atg agc gtg aag      1273
   Asp Met Ala Arg Gln Leu Arg Glu Tyr Gln Glu Leu Met Ser Val Lys
               370                 375                 380
   ctg gcc ctg gac atc gag atc gcc acc tac cgc aag ctg ctg gag ggc      1321
   Leu Ala Leu Asp Ile Glu Ile Ala Thr Tyr Arg Lys Leu Leu Glu Gly
           385                 390                 395
   gag gag agc cgg ttg gct gga gat gga gtg gga gcc gtg aat atc tct      1369
   Glu Glu Ser Arg Leu Ala Gly Asp Gly Val Gly Ala Val Asn Ile Ser
       400                 405                 410
   gtg atg aat tcc act ggt ggc agt agc agt ggc ggt ggc att ggg ctg      1417
   Val Met Asn Ser Thr Gly Gly Ser Ser Ser Gly Gly Gly Ile Gly Leu
   415                 420                 425                 430
   acc ctc ggg gga acc atg ggc agc aat gcc ctg agc ttc tcc agc agt      1465
   Thr Leu Gly Gly Thr Met Gly Ser Asn Ala Leu Ser Phe Ser Ser Ser
                   435                 440                 445
   gcg ggt cct ggg ctc ctg aag gct tat tcc atc cgg acc gca tcc gcc      1513
   Ala Gly Pro Gly Leu Leu Lys Ala Tyr Ser Ile Arg Thr Ala Ser Ala
               450                 455                 460
   agt cgc agg agt gcc cgc gac tga gccgcctccc accactccac tcctccagcc     1567
   Ser Arg Arg Ser Ala Arg Asp
           465
   accacccaca atcacaagaa gattcccacc cctgcctccc atgcctggtc ccaagacagt   1627
   gagacagtct ggaaagtgat gtcagaatag cttccaataa agcagcctca ttctgaggcc   1687
   tgagtgatcc acgtgaaaaa aaaaaaaaaa aaaaaaaaaa aaaaaaaaaa aaaaaaaaaa   1747
   aaaaaa                                                               1753
<210> 18
   <211> 469
   <212> PRT
   <213> Homo sapiens
<400> 18
   Met Ser Ile His Phe Ser Ser Pro Val Phe Thr Ser Arg Ser Ala Ala
   1               5                   10                  15
   Phe Ser Gly Arg Gly Ala Gln Val Arg Leu Ser Ser Ala Arg Pro Gly
               20                  25                  30
   Gly Leu Gly Ser Ser Ser Leu Tyr Gly Leu Gly Ala Ser Arg Pro Arg
           35                  40                  45
   Val Ala Val Arg Ser Ala Tyr Gly Gly Pro Val Gly Ala Gly Ile Arg
       50                  55                  60
   Glu Val Thr Ile Asn Gln Ser Leu Leu Ala Pro Leu Arg Leu Asp Ala
   65                  70                  75                  80
   Asp Pro Ser Leu Gln Arg Val Arg Gln Glu Glu Ser Glu Gln Ile Lys
                   85                  90                  95
   Thr Leu Asn Asn Lys Phe Ala Ser Phe Ile Asp Lys Val Arg Phe Leu
               100                 105                 110
   Glu Gln Gln Asn Lys Leu Leu Glu Thr Lys Trp Thr Leu Leu Gln Glu
           115                 120                 125
   Gln Lys Ser Ala Lys Ser Ser Arg Leu Pro Asp Ile Phe Glu Ala Gln
       130                 135                 140
   Ile Ala Gly Leu Arg Gly Gln Leu Glu Ala Leu Gln Val Asp Gly Gly
   145                 150                 155                 160
   Arg Leu Glu Ala Glu Leu Arg Ser Met Gln Asp Val Val Glu Asp Phe
                   165                 170                 175
   Lys Asn Lys Tyr Glu Asp Glu Ile Asn His Arg Thr Ala Ala Glu Asn
               180                 185                 190
   Glu Phe Val Val Leu Lys Lys Asp Val Asp Ala Ala Tyr Met Ser Lys
           195                 200                 205
   Val Glu Leu Glu Ala Lys Val Asp Ala Leu Asn Asp Glu Ile Asn Phe
       210                 215                 220
   Leu Arg Thr Leu Asn Glu Thr Glu Leu Thr Glu Leu Gln Ser Gln Ile
   225                 230                 235                 240
   Ser Asp Thr Ser Val Val Leu Ser Met Asp Asn Ser Arg Ser Leu Asp
                   245                 250                 255
   Leu Asp Gly Ile Ile Ala Glu Val Lys Ala Gln Tyr Glu Glu Met Ala
               260                 265                 270
   Lys Cys Ser Arg Ala Glu Ala Glu Ala Trp Tyr Gln Thr Lys Phe Glu
           275                 280                 285
   Thr Leu Gln Ala Gln Ala Gly Lys His Gly Asp Asp Leu Arg Asn Thr
       290                 295                 300
   Arg Asn Glu Ile Ser Glu Met Asn Arg Ala Ile Gln Arg Leu Gln Ala
   305                 310                 315                 320
   Glu Ile Asp Asn Ile Lys Asn Gln Arg Ala Lys Leu Glu Ala Ala Ile
                   325                 330                 335
   Ala Glu Ala Glu Glu Arg Gly Glu Leu Ala Leu Lys Asp Ala Arg Ala
               340                 345                 350
   Lys Gln Glu Glu Leu Glu Ala Ala Leu Gln Arg Gly Lys Gln Asp Met
           355                 360                 365
   Ala Arg Gln Leu Arg Glu Tyr Gln Glu Leu Met Ser Val Lys Leu Ala
       370                 375                 380
   Leu Asp Ile Glu Ile Ala Thr Tyr Arg Lys Leu Leu Glu Gly Glu Glu
   385                 390                 395                 400
   Ser Arg Leu Ala Gly Asp Gly Val Gly Ala Val Asn Ile Ser Val Met
                   405                 410                 415
   Asn Ser Thr Gly Gly Ser Ser Ser Gly Gly Gly Ile Gly Leu Thr Leu
               420                 425                 430
   Gly Gly Thr Met Gly Ser Asn Ala Leu Ser Phe Ser Ser Ser Ala Gly
           435                 440                 445
   Pro Gly Leu Leu Lys Ala Tyr Ser Ile Arg Thr Ala Ser Ala Ser Arg
       450                 455                 460
   Arg Ser Ala Arg Asp
   465
<210> 19
   <211> 5623
   <212> DNA
   <213> Homo sapiens
<220>
   <221> CDS
   <222> (1)..(3744)
   <223>
<400> 19
   atg tca ctt ttt tta agg gta gta ttt agt ttc aca atg ttt ggg gac      48
   Met Ser Leu Phe Leu Arg Val Val Phe Ser Phe Thr Met Phe Gly Asp
   1               5                   10                  15
   ctg ttt gaa gag gag tat tcc act gtg tct aat aat cag tat gga aaa      96
   Leu Phe Glu Glu Glu Tyr Ser Thr Val Ser Asn Asn Gln Tyr Gly Lys
               20                  25                  30
   ggg aag aaa tta aag act aaa gct ttg gag cca cct gct cct aga gaa     144
   Gly Lys Lys Leu Lys Thr Lys Ala Leu Glu Pro Pro Ala Pro Arg Glu
           35                  40                  45
   ttc acc aat tta agc gga atc aga aat cag ggt gga acc tgt tac ctc     192
   Phe Thr Asn Leu Ser Gly Ile Arg Asn Gln Gly Gly Thr Cys Tyr Leu
       50                  55                  60
   aat tcc ctt ctt cag act ctt cat ttc aca cct gaa ttc aga gaa gct     240
   Asn Ser Leu Leu Gln Thr Leu His Phe Thr Pro Glu Phe Arg Glu Ala
   65                  70                  75                  80
   cta ttt tct ctt ggc cca gaa gag ctt ggt ttg ttt gaa gat aag gat     288
   Leu Phe Ser Leu Gly Pro Glu Glu Leu Gly Leu Phe Glu Asp Lys Asp
                   85                  90                  95
   aaa ccc gat gca aag gtt cga atc atc cct tta cag tta cag cgc ttg     336
   Lys Pro Asp Ala Lys Val Arg Ile Ile Pro Leu Gln Leu Gln Arg Leu
               100                 105                 110
   ttt gct cag ctt ctg ctc tta gac cag gaa gct gca tcc aca gca gac     384
   Phe Ala Gln Leu Leu Leu Leu Asp Gln Glu Ala Ala Ser Thr Ala Asp
           115                 120                 125
   ctc act gac agc ttt ggg tgg acc agt aat gag gaa atg agg caa cat      432
   Leu Thr Asp Ser Phe Gly Trp Thr Ser Asn Glu Glu Met Arg Gln His
       130                 135                 140
   gat gtg cag gaa ctg aat cga atc ctc ttc agc gct ttg gaa act tct      480
   Asp Val Gln Glu Leu Asn Arg Ile Leu Phe Ser Ala Leu Glu Thr Ser
   145                 150                 155                 160
   tta gtt ggg acc tcc ggt cat gac ctc atc tat cgt ctg tac cat gga      528
   Leu Val Gly Thr Ser Gly His Asp Leu Ile Tyr Arg Leu Tyr His Gly
                   165                 170                 175
   acc att gtt aac cag att gtt tgt aaa gaa tgt aag aac gtt agc gag      576
   Thr Ile Val Asn Gln Ile Val Cys Lys Glu Cys Lys Asn Val Ser Glu
               180                 185                 190
   agg cag gaa gac ttc tta gat cta aca gta gca gtc aaa aat gta tcc      624
   Arg Gln Glu Asp Phe Leu Asp Leu Thr Val Ala Val Lys Asn Val Ser
           195                 200                 205
   ggt ttg gaa gat gct ctc tgg aac atg tat gta gaa gag gaa gtt ttt      672
   Gly Leu Glu Asp Ala Leu Trp Asn Met Tyr Val Glu Glu Glu Val Phe
       210                 215                 220
   gat tgt gac aac ttg tac cac tgt gga act tgt gac agg ctg gtt aaa      720
   Asp Cys Asp Asn Leu Tyr His Cys Gly Thr Cys Asp Arg Leu Val Lys
   225                 230                 235                 240
   gca gca aag tcg gcc aaa tta cgt aag ctg cct cct ttt ctt act gtt      768
   Ala Ala Lys Ser Ala Lys Leu Arg Lys Leu Pro Pro Phe Leu Thr Val
                   245                 250                 255
   tca tta cta aga ttt aat ttt gat ttt gtg aaa tgc gaa cgc tac aag      816
   Ser Leu Leu Arg Phe Asn Phe Asp Phe Val Lys Cys Glu Arg Tyr Lys
               260                 265                 270
   gaa act agc tgt tat aca ttc cct ctc cgg att aat ctc aag ccc ttt      864
   Glu Thr Ser Cys Tyr Thr Phe Pro Leu Arg Ile Asn Leu Lys Pro Phe
           275                 280                 285
   tgt gaa cag agt gaa ttg gat gac tta gaa tat ata tat gac ctc ttc      912
   Cys Glu Gln Ser Glu Leu Asp Asp Leu Glu Tyr Ile Tyr Asp Leu Phe
       290                 295                 300
   tca gtt att ata cac aaa ggt ggc tgc tac gga ggc cat tac cat gta      960
   Ser Val Ile Ile His Lys Gly Gly Cys Tyr Gly Gly His Tyr His Val
   305                 310                 315                 320
   tat att aaa gat gtt gat cat ttg gga aac tgg cag ttt caa gag gaa     1008
   Tyr Ile Lys Asp Val Asp His Leu Gly Asn Trp Gln Phe Gln Glu Glu
                   325                 330                 335
   aaa agt aaa cca gat gtg aat ctg aaa gat ctc cag agt gaa gaa gag     1056
   Lys Ser Lys Pro Asp Val Asn Leu Lys Asp Leu Gln Ser Glu Glu Glu
               340                 345                 350
   att gat cat cca ctg atg att cta aaa gca atc tta tta gag gag aat     1104
   Ile Asp His Pro Leu Met Ile Leu Lys Ala Ile Leu Leu Glu Glu Asn
           355                 360                 365
   aat cta att cct gtt gat cag ctg ggc cag aaa ctt ttg aaa aag ata     1152
   Asn Leu Ile Pro Val Asp Gln Leu Gly Gln Lys Leu Leu Lys Lys Ile
       370                 375                 380
   gga ata tct tgg aac aag aag tac aga aaa cag cat gga cca ctg cgg     1200
   Gly Ile Ser Trp Asn Lys Lys Tyr Arg Lys Gln His Gly Pro Leu Arg
   385                 390                 395                 400
   aag ttc tta cag ctc cat tct cag ata ttt cta ctc agt tca gat gaa     1248
   Lys Phe Leu Gln Leu His Ser Gln Ile Phe Leu Leu Ser Ser Asp Glu
                   405                 410                 415
   agt aca gtt cgt ctc ttg aag aat agt tct ctc cag gct gag tct gat     1296
   Ser Thr Val Arg Leu Leu Lys Asn Ser Ser Leu Gln Ala Glu Ser Asp
               420                 425                 430
   ttc caa agg aat gac cag caa att ttc aag atg ctt cct cca gaa tcc     1344
   Phe Gln Arg Asn Asp Gln Gln Ile Phe Lys Met Leu Pro Pro Glu Ser
           435                 440                 445
   cca ggt tta aac aat agc atc tcc tgt ccc cac tgg ttt gat ata aat     1392
   Pro Gly Leu Asn Asn Ser Ile Ser Cys Pro His Trp Phe Asp Ile Asn
       450                 455                 460
   gat tct aaa gtc cag cca atc agg gaa aag gat att gaa cag caa ttt     1440
   Asp Ser Lys Val Gln Pro Ile Arg Glu Lys Asp Ile Glu Gln Gln Phe
   465                 470                 475                 480
   cag ggt aaa gaa agt gcc tac atg ttg ttt tat cgg aaa tcc cag ttg     1488
   Gln Gly Lys Glu Ser Ala Tyr Met Leu Phe Tyr Arg Lys Ser Gln Leu
                   485                 490                 495
   cag aga ccc cct gaa gct cga gct aat cca aga tat ggg gtt cca tgt     1536
   Gln Arg Pro Pro Glu Ala Arg Ala Asn Pro Arg Tyr Gly Val Pro Cys
               500                 505                 510
   cat tta ctg aat gaa atg gat gca gct aac att gaa ctg caa acc aaa     1584
   His Leu Leu Asn Glu Met Asp Ala Ala Asn Ile Glu Leu Gln Thr Lys
           515                 520                 525
   agg gca gaa tgt gat tct gca aac aat act ttt gaa ttg cat ctt cac     1632
   Arg Ala Glu Cys Asp Ser Ala Asn Asn Thr Phe Glu Leu His Leu His
       530                 535                 540
   ctg ggc cct cag tat cat ttc ttc aat ggg gct ctg cac cca gta gtc     1680
   Leu Gly Pro Gln Tyr His Phe Phe Asn Gly Ala Leu His Pro Val Val
   545                 550                 555                 560
   tct caa aca gaa agc gtg tgg gat ttg acc ttt gat aaa aga aaa act     1728
   Ser Gln Thr Glu Ser Val Trp Asp Leu Thr Phe Asp Lys Arg Lys Thr
                   565                 570                 575
   tta gga gat ctc cgg cag tca ata ttt cag ctg tta gaa ttt tgg gaa     1776
   Leu Gly Asp Leu Arg Gln Ser Ile Phe Gln Leu Leu Glu Phe Trp Glu
               580                 585                 590
   gga gac atg gtt ctt agt gtt gca aag ctt gta cca gca gga ctt cac     1824
   Gly Asp Met Val Leu Ser Val Ala Lys Leu Val Pro Ala Gly Leu His
           595                 600                 605
   att tac cag tca ctt ggc ggg gat gaa ctg aca ctg tgt gaa act gaa     1872
   Ile Tyr Gln Ser Leu Gly Gly Asp Glu Leu Thr Leu Cys Glu Thr Glu
       610                 615                 620
   att gct gat ggg gaa gac atc ttt gtg tgg aat ggg gtg gag gtt ggt     1920
   Ile Ala Asp Gly Glu Asp Ile Phe Val Trp Asn Gly Val Glu Val Gly
   625 630 635 640
   gga gtc cac att caa act ggt att gac tgc gaa cct cta ctt tta aat 1968
   Gly Val His Ile Gln Thr Gly Ile Asp Cys Glu Pro Leu Leu Leu Asn
   645 650 655
   gtt ctt cat cta gac aca agc agt gat gga gaa aag tgt tgt cag gtg 2016
   Val Leu His Leu Asp Thr Ser Ser Asp Gly Glu Lys Cys Cys Gln Val
   660 665 670
   ata gaa tct cca cat gtc ttt cca gct aat gca gaa gtg ggc act gtc 2064
   Ile Glu Ser Pro His Val Phe Pro Ala Asn Ala Glu Val Gly Thr Val
   675 680 685
   ctc aca gcc tta gca atc cca gca ggt gtc atc ttc atc aac agt gct 2112
   Leu Thr Ala Leu Ala Ile Pro Ala Gly Val Ile Phe Ile Asn Ser Ala
   690 695 700
   gga tgt cca ggt ggg gag ggt tgg acg gcc atc ccc aag gaa gac atg 2160
   Gly Cys Pro Gly Gly Glu Gly Trp Thr Ala Ile Pro Lys Glu Asp Met
   705 710 715 720
   agg aag acg ttc agg gag caa ggg ctc aga aat gga agc tca att tta 2208
   Arg Lys Thr Phe Arg Glu Gln Gly Leu Arg Asn Gly Ser Ser Ile Leu
   725 730 735
   att cag gat tct cat gat gat aac agc ttg ttg acc aag gaa gag aaa 2256
   Ile Gln Asp Ser His Asp Asp Asn Ser Leu Leu Thr Lys Glu Glu Lys
   740 745 750
   tgg gtc act agt atg aat gag att gac tgg ctc cac gtt aaa aat tta 2304
   Trp Val Thr Ser Met Asn Glu Ile Asp Trp Leu His Val Lys Asn Leu
   755 760 765
   tgc cag tta gaa tct gaa gag aag caa gtt aaa ata tca gca act gtt 2352
   Cys Gln Leu Glu Ser Glu Glu Lys Gln Val Lys Ile Ser Ala Thr Val
   770 775 780
   aac aca atg gtg ttt gat att cga att aaa gcc ata aag gaa tta aaa 2400
   Asn Thr Met Val Phe Asp Ile Arg Ile Lys Ala Ile Lys Glu Leu Lys
   785 790 795 800
   tta atg aag gaa cta gct gac aac agc tgt ttg aga cct att gat aga 2448
   Leu Met Lys Glu Leu Ala Asp Asn Ser Cys Leu Arg Pro Ile Asp Arg
   805 810 815
   aat ggg aag ctt ctt tgt cca gtg ccg gac agc tat act ttg aag gaa 2496
   Asn Gly Lys Leu Leu Cys Pro Val Pro Asp Ser Tyr Thr Leu Lys Glu
   820 825 830
   gca gaa ttg aag atg gga agt tca ttg gga ctg tgt ctt gga aaa gca 2544
   Ala Glu Leu Lys Met Gly Ser Ser Leu Gly Leu Cys Leu Gly Lys Ala
   835 840 845
   cca agt tcg tct cag ttg ttc ctg ttt ttt gca atg ggg agt gac gtt 2592
   Pro Ser Ser Ser Gln Leu Phe Leu Phe Phe Ala Met Gly Ser Asp Val
   850 855 860
   caa cct ggg aca gaa atg gaa atc gta gta gaa gaa aca ata tct gtg 2640
   Gln Pro Gly Thr Glu Met Glu Ile Val Val Glu Glu Thr Ile Ser Val
   865 870 875 880
   aga gat tgt tta aag tta atg ctg aag aaa tct ggc cta caa gga gat 2688
   Arg Asp Cys Leu Lys Leu Met Leu Lys Lys Ser Gly Leu Gln Gly Asp
                   885                 890                 895
   gcc tgg cat tta cga aaa atg gat tgg tgc tat gaa gct gga gag cct      2736
   Ala Trp His Leu Arg Lys Met Asp Trp Cys Tyr Glu Ala Gly Glu Pro
               900                 905                 910
   tta tgt gaa gaa gat gca aca ctg aaa gaa ctt ctg ata tgt tct gga      2784
   Leu Cys Glu Glu Asp Ala Thr Leu Lys Glu Leu Leu Ile Cys Ser Gly
           915                 920                 925
   gat act ttg ctt tta att gaa gga caa ctt cct cct ctg ggt ttc ctg      2832
   Asp Thr Leu Leu Leu Ile Glu Gly Gln Leu Pro Pro Leu Gly Phe Leu
       930                 935                 940
   aag gtg ccc atc tgg tgg tac cag ctt cag ggt ccc tca gga cac tgg      2880
   Lys Val Pro Ile Trp Trp Tyr Gln Leu Gln Gly Pro Ser Gly His Trp
   945                 950                 955                 960
   gag agt cat cag gac cag acc aac tgt act tcg tct tgg ggc aga gtt      2928
   Glu Ser His Gln Asp Gln Thr Asn Cys Thr Ser Ser Trp Gly Arg Val
                   965                 970                 975
   tgg aga gcc act tcc agc caa ggt gct tct ggg aac gag cct gcg caa      2976
   Trp Arg Ala Thr Ser Ser Gln Gly Ala Ser Gly Asn Glu Pro Ala Gln
               980                 985                 990
   gtt tct ctc ctc tac ttg gga gac  ata gag atc tca gaa  gat gcc acg    3024
   Val Ser Leu Leu Tyr Leu Gly Asp  Ile Glu Ile Ser Glu  Asp Ala Thr
           995                 1000                 1005
   ctg gcg  gag ctg aag tct cag  gcc atg acc ttg cct  cct ttc ctg       3069
   Leu Ala  Glu Leu Lys Ser Gln  Ala Met Thr Leu Pro  Pro Phe Leu
       1010                 1015                 1020
   gag ttc  ggt gtc ccg tcc cca  gcc cac ctc aga gcc  tgg acg gtg       3114
   Glu Phe  Gly Val Pro Ser Pro  Ala His Leu Arg Ala  Trp Thr Val
       1025                 1030                 1035
   gag agg  aag cgc cca ggc agg  ctt tta cga act gac  cgg cag cca       3159
   Glu Arg  Lys Arg Pro Gly Arg  Leu Leu Arg Thr Asp  Arg Gln Pro
       1040                 1045                 1050
   ctc agg  gaa tat aaa cta gga  cgg aga att gag atc  tgc tta gag       3204
   Leu Arg  Glu Tyr Lys Leu Gly  Arg Arg Ile Glu Ile  Cys Leu Glu
       1055                 1060                 1065
   ccc ctt  cag aaa ggc gaa aac  ttg ggc ccc cag gac  gtg ctg ctg       3249
   Pro Leu  Gln Lys Gly Glu Asn  Leu Gly Pro Gln Asp  Val Leu Leu
       1070                 1075                 1080
   agg aca  cag gtg cgc atc cct  ggt gag agg acc tat  gcc cct gcc       3294
   Arg Thr  Gln Val Arg Ile Pro  Gly Glu Arg Thr Tyr  Ala Pro Ala
       1085                 1090                 1095
   ctg gac  ctg gtg tgg aac gcg  gcc cag ggt ggg act  gcc ggc tcc       3339
   Leu Asp  Leu Val Trp Asn Ala  Ala Gln Gly Gly Thr  Ala Gly Ser
       1100                 1105                 1110
   ctg agg  cag aga gtt gcc gat  ttc tat cgt ctt ccc  gtg gag aag       3384
   Leu Arg  Gln Arg Val Ala Asp  Phe Tyr Arg Leu Pro  Val Glu Lys
       1115                 1120                 1125
   att gaa att gcc aaa tac ttt ccc gaa aag ttc gag tgg ctt ccg      3429
   Ile Glu Ile Ala Lys Tyr Phe Pro Glu Lys Phe Glu Trp Leu Pro
       1130                1135                1140
   ata tct agc tgg aac caa caa ata acc aag agg aaa aag aaa aaa      3474
   Ile Ser Ser Trp Asn Gln Gln Ile Thr Lys Arg Lys Lys Lys Lys
       1145                1150                1155
   aaa caa gat tat ttg caa ggg gca ccg tat tac ttg aaa gac gga      3519
   Lys Gln Asp Tyr Leu Gln Gly Ala Pro Tyr Tyr Leu Lys Asp Gly
       1160                1165                1170
   gat act att ggt gtt aag aat ctc ctg att gac gac gat gat gat      3564
   Asp Thr Ile Gly Val Lys Asn Leu Leu Ile Asp Asp Asp Asp Asp
       1175                1180                1185
   ttc agt aca atc aga gat gac act gga aaa gaa aag cag aaa caa      3609
   Phe Ser Thr Ile Arg Asp Asp Thr Gly Lys Glu Lys Gln Lys Gln
       1190                1195                1200
   cgg gcc ctg ggg aga agg aaa agc caa gaa gcc ctc cat gag cag      3654
   Arg Ala Leu Gly Arg Arg Lys Ser Gln Glu Ala Leu His Glu Gln
       1205                1210                1215
   agc agc tac atc ctc tcc agt gca gag acg cct gcc cgg ccc cga      3699
   Ser Ser Tyr Ile Leu Ser Ser Ala Glu Thr Pro Ala Arg Pro Arg
       1220                1225                1230
   gcc ccg gaa act tct ctc tcc atc cac gtg ggg agc ttc aga taa      3744
   Ala Pro Glu Thr Ser Leu Ser Ile His Val Gly Ser Phe Arg
       1235                1240                1245
   ccgcgccgct gcacggctct actcccgatg aactctccgg ctgatgccac aaacgtgggt   3804
   ttcctgggca tggggactgg ctgcctggcg cctccaatcc caaatcctct gcttcctttg   3864
   agcacaggga cggctcctct gaggcctggc cagtgcatgt agtcacttag ctctgcaaca   3924
   cgtggcagcc acgggggctg gtgcagctct ggatgtcgcc cacccagctg ccagtaggtg   3984
   ctgggctctc tcacacagca cccggcccca gctgcctttt tttttctttt aaccagaaaa   4044
   tgcacaacgt gtgcgtgaac cgcaggtatg gaggcagcgg catgccgttg ctccgctgtg   4104
   ggaggtgtgt ggggtcaggc cagccacttt cctccgtgtt cagatgactc tcgttcgccc   4164
   tgaccggctt ctcacagtgt ctcaggccac tgcgccaccg cgctggtgct gagcagaagc   4224
   gggcagaagt ggggtctgct ttcaggactt catttccccc actcgttccg gccccgcatg   4284
   ctccacgtct gccctttggt ctgagttaaa actgcgatgc tgaaaagtgc gagctctttc   4344
   cacgaggagg agccacacag ggtggcctcc gagggtgagt cgctctgcta agcaagggca   4404
   gccgctgcac gtcagcccgc aggccaaggg tccagcttat cctgggtgct ctgtgatcag   4464
   aaggtccttg gatcccgagg actgcagctg ctgccgtcca cagcgcctca gcctctcttc   4524
   ctgtgtggaa gcggggcagg cagggctgtg gcagacaggc ctggcatagc ccaggttcca   4584
   ggtgtcctgg gcagcctgac cggtttcctg agtagcctca atgaaaacac ctgagaaaaa   4644
   gaaaatgtaa actaaggaag ggttttccca cttaatccaa cccagatttc tattcaactt   4704
   aaaggattta attggcattt gtgagaaata atagtacctg tgtcactgag ttgattgatt   4764
   tgggacatag acggttgact ttcccagaca gcagcttagc ggaaagcagc atcccgaaga   4824
   cggtgtcgca tgtcctgagc ctgcttgtca cctgggcctc acggtttctc gtcagccccg   4884
   tcactgatgg gaagcagcac tgaagtgtga gggcagaaag gacaccgctg gagtcaggcg   4944
   ctcccctgcc tgtgaccctt ttccaacaag ctgatgctga gccagcggcc tgctttgtcc   5004
   ctcatactcc tcccaacagt ccaaacccct accagacaga agccacgtcg cttctgcaca   5064
   cgtgtctgag agcgtctgcc atagacgtct gtgcgtttcc gtgaagcact gcgccctaag   5124
   gacccaactc tgactagcag ctgcctccca ctcccaaaca ctaaccccgc tcaggagact   5184
   cgcactgggt gtagactgcg tctttgcaga gggagatgat agaggcttct gatgacgcca   5244
   gagctgtaag tgtcgtgacg agctgggcgc ccagcccttc ttaagctgtt ctgtttctgt   5304
   ggctttaact gacatatttc tgtagcatct gccttcatct catctcagcg taatgaaata   5364
   ttaatgaaat cgctgaaaag ctttgccttc gagaggccag aagcctcgcg gaatgtctgc   5424
   aagtccaaag acgcgtgtgg gttgtgccct gaagtgccgt ccagcaggcg cgtgcggccg   5484
   ggccggcctg tgcgtgtggc ctttgccttc ttccctttct tcctgttttc tgttttttta   5544
   atttggggat tgagaaagct gtacgatttt gttaaagaaa aaaataaacc attttttaaa   5604
   gttgtagccc ttaaaaaaa                                                 5623
<210> 20
   <211> 1247
   <212> PRT
   <213> Homo sapiens
<400> 20
   Met Ser Leu Phe Leu Arg Val Val Phe Ser Phe Thr Met Phe Gly Asp
   1               5                   10                  15
   Leu Phe Glu Glu Glu Tyr Ser Thr Val Ser Asn Asn Gln Tyr Gly Lys
               20                  25                  30
   Gly Lys Lys Leu Lys Thr Lys Ala Leu Glu Pro Pro Ala Pro Arg Glu
           35                  40                  45
   Phe Thr Asn Leu Ser Gly Ile Arg Asn Gln Gly Gly Thr Cys Tyr Leu
       50                  55                  60
   Asn Ser Leu Leu Gln Thr Leu His Phe Thr Pro Glu Phe Arg Glu Ala
   65                  70                  75                  80
   Leu Phe Ser Leu Gly Pro Glu Glu Leu Gly Leu Phe Glu Asp Lys Asp
                   85                  90                  95
   Lys Pro Asp Ala Lys Val Arg Ile Ile Pro Leu Gln Leu Gln Arg Leu
   100 105 110
   Phe Ala Gln Leu Leu Leu Leu Asp Gln Glu Ala Ala Ser Thr Ala Asp
   115 120 125
   Leu Thr Asp Ser Phe Gly Trp Thr Ser Asn Glu Glu Met Arg Gln His
   130 135 140
   Asp Val Gln Glu Leu Asn Arg Ile Leu Phe Ser Ala Leu Glu Thr Ser
   145 150 155 160
   Leu Val Gly Thr Ser Gly His Asp Leu Ile Tyr Arg Leu Tyr His Gly
   165 170 175
   Thr Ile Val Asn Gln Ile Val Cys Lys Glu Cys Lys Asn Val Ser Glu
   180 185 190
   Arg Gln Glu Asp Phe Leu Asp Leu Thr Val Ala Val Lys Asn Val Ser
   195 200 205
   Gly Leu Glu Asp Ala Leu Trp Asn Met Tyr Val Glu Glu Glu Val Phe
   210 215 220
   Asp Cys Asp Asn Leu Tyr His Cys Gly Thr Cys Asp Arg Leu Val Lys
   225 230 235 240
   Ala Ala Lys Ser Ala Lys Leu Arg Lys Leu Pro Pro Phe Leu Thr Val
   245 250 255
   Ser Leu Leu Arg Phe Asn Phe Asp Phe Val Lys Cys Glu Arg Tyr Lys
   260 265 270
   Glu Thr Ser Cys Tyr Thr Phe Pro Leu Arg Ile Asn Leu Lys Pro Phe
   275 280 285
   Cys Glu Gln Ser Glu Leu Asp Asp Leu Glu Tyr Ile Tyr Asp Leu Phe
   290 295 300
   Ser Val Ile Ile His Lys Gly Gly Cys Tyr Gly Gly His Tyr His Val
   305 310 315 320
   Tyr Ile Lys Asp Val Asp His Leu Gly Asn Trp Gln Phe Gln Glu Glu
   325 330 335
   Lys Ser Lys Pro Asp Val Asn Leu Lys Asp Leu Gln Ser Glu Glu Glu
               340                 345                 350
   Ile Asp His Pro Leu Met Ile Leu Lys Ala Ile Leu Leu Glu Glu Asn
           355                 360                 365
   Asn Leu Ile Pro Val Asp Gln Leu Gly Gln Lys Leu Leu Lys Lys Ile
       370                 375                 380
   Gly Ile Ser Trp Asn Lys Lys Tyr Arg Lys Gln His Gly Pro Leu Arg
   385                 390                 395                 400
   Lys Phe Leu Gln Leu His Ser Gln Ile Phe Leu Leu Ser Ser Asp Glu
                   405                 410                 415
   Ser Thr Val Arg Leu Leu Lys Asn Ser Ser Leu Gln Ala Glu Ser Asp
               420                 425                 430
   Phe Gln Arg Asn Asp Gln Gln Ile Phe Lys Met Leu Pro Pro Glu Ser
           435                 440                 445
   Pro Gly Leu Asn Asn Ser Ile Ser Cys Pro His Trp Phe Asp Ile Asn
       450                 455                 460
   Asp Ser Lys Val Gln Pro Ile Arg Glu Lys Asp Ile Glu Gln Gln Phe
   465                 470                 475                 480
   Gln Gly Lys Glu Ser Ala Tyr Met Leu Phe Tyr Arg Lys Ser Gln Leu
                   485                 490                 495
   Gln Arg Pro Pro Glu Ala Arg Ala Asn Pro Arg Tyr Gly Val Pro Cys
               500                 505                 510
   His Leu Leu Asn Glu Met Asp Ala Ala Asn Ile Glu Leu Gln Thr Lys
           515                 520                 525
   Arg Ala Glu Cys Asp Ser Ala Asn Asn Thr Phe Glu Leu His Leu His
       530                 535                 540
   Leu Gly Pro Gln Tyr His Phe Phe Asn Gly Ala Leu His Pro Val Val
   545                 550                 555                 560
   Ser Gln Thr Glu Ser Val Trp Asp Leu Thr Phe Asp Lys Arg Lys Thr
                   565                 570                 575
   Leu Gly Asp Leu Arg Gln Ser Ile Phe Gln Leu Leu Glu Phe Trp Glu
               580                 585                 590
   Gly Asp Met Val Leu Ser Val Ala Lys Leu Val Pro Ala Gly Leu His
   595 600 605
   Ile Tyr Gln Ser Leu Gly Gly Asp Glu Leu Thr Leu Cys Glu Thr Glu
   610 615 620
   Ile Ala Asp Gly Glu Asp Ile Phe Val Trp Asn Gly Val Glu Val Gly
   625 630 635 640
   Gly Val His Ile Gln Thr Gly Ile Asp Cys Glu Pro Leu Leu Leu Asn
   645 650 655
   Val Leu His Leu Asp Thr Ser Ser Asp Gly Glu Lys Cys Cys Gln Val
   660 665 670
   Ile Glu Ser Pro His Val Phe Pro Ala Asn Ala Glu Val Gly Thr Val
   675 680 685
   Leu Thr Ala Leu Ala Ile Pro Ala Gly Val Ile Phe Ile Asn Ser Ala
   690 695 700
   Gly Cys Pro Gly Gly Glu Gly Trp Thr Ala Ile Pro Lys Glu Asp Met
   705 710 715 720
   Arg Lys Thr Phe Arg Glu Gln Gly Leu Arg Asn Gly Ser Ser Ile Leu
   725 730 735
   Ile Gln Asp Ser His Asp Asp Asn Ser Leu Leu Thr Lys Glu Glu Lys
   740 745 750
   Trp Val Thr Ser Met Asn Glu Ile Asp Trp Leu His Val Lys Asn Leu
   755 760 765
   Cys Gln Leu Glu Ser Glu Glu Lys Gln Val Lys Ile Ser Ala Thr Val
   770 775 780
   Asn Thr Met Val Phe Asp Ile Arg Ile Lys Ala Ile Lys Glu Leu Lys
   785 790 795 800
   Leu Met Lys Glu Leu Ala Asp Asn Ser Cys Leu Arg Pro Ile Asp Arg
   805 810 815
   Asn Gly Lys Leu Leu Cys Pro Val Pro Asp Ser Tyr Thr Leu Lys Glu
   820 825 830
   Ala Glu Leu Lys Met Gly Ser Ser Leu Gly Leu Cys Leu Gly Lys Ala
   835 840 845
   Pro Ser Ser Ser Gln Leu Phe Leu Phe Phe Ala Met Gly Ser Asp Val
       850                 855                 860
   Gln Pro Gly Thr Glu Met Glu Ile Val Val Glu Glu Thr Ile Ser Val
   865                 870                 875                 880
   Arg Asp Cys Leu Lys Leu Met Leu Lys Lys Ser Gly Leu Gln Gly Asp
                   885                 890                 895
   Ala Trp His Leu Arg Lys Met Asp Trp Cys Tyr Glu Ala Gly Glu Pro
               900                 905                 910
   Leu Cys Glu Glu Asp Ala Thr Leu Lys Glu Leu Leu Ile Cys Ser Gly
           915                 920                 925
   Asp Thr Leu Leu Leu Ile Glu Gly Gln Leu Pro Pro Leu Gly Phe Leu
       930                 935                 940
   Lys Val Pro Ile Trp Trp Tyr Gln Leu Gln Gly Pro Ser Gly His Trp
   945                 950                 955                 960
   Glu Ser His Gln Asp Gln Thr Asn Cys Thr Ser Ser Trp Gly Arg Val
                   965                 970                 975
   Trp Arg Ala Thr Ser Ser Gln Gly Ala Ser Gly Asn Glu Pro Ala Gln
               980                 985                 990
   Val Ser Leu Leu Tyr Leu Gly Asp  Ile Glu Ile Ser Glu  Asp Ala Thr
           995                 1000                 1005
   Leu Ala  Glu Leu Lys Ser Gln  Ala Met Thr Leu Pro  Pro Phe Leu
       1010                 1015                 1020
   Glu Phe  Gly Val Pro Ser Pro  Ala His Leu Arg Ala  Trp Thr Val
       1025                 1030                 1035
   Glu Arg  Lys Arg Pro Gly Arg  Leu Leu Arg Thr Asp  Arg Gln Pro
       1040                 1045                 1050
   Leu Arg  Glu Tyr Lys Leu Gly  Arg Arg Ile Glu Ile  Cys Leu Glu
       1055                 1060                 1065
   Pro Leu  Gln Lys Gly Glu Asn  Leu Gly Pro Gln Asp  Val Leu Leu
       1070                 1075                 1080
   Arg Thr  Gln Val Arg Ile Pro  Gly Glu Arg Thr Tyr  Ala Pro Ala
       1085                 1090                 1095
   Leu Asp Leu Val Trp Asn Ala Ala Gln Gly Gly Thr Ala Gly Ser
       1100                1105                1110
   Leu Arg Gln Arg Val Ala Asp Phe Tyr Arg Leu Pro Val Glu Lys
       1115                1120                1125
   Ile Glu Ile Ala Lys Tyr Phe Pro Glu Lys Phe Glu Trp Leu Pro
       1130                1135                1140
   Ile Ser Ser Trp Asn Gln Gln Ile Thr Lys Arg Lys Lys Lys Lys
       1145                1150                1155
   Lys Gln Asp Tyr Leu Gln Gly Ala Pro Tyr Tyr Leu Lys Asp Gly
       1160                1165                1170
   Asp Thr Ile Gly Val Lys Asn Leu Leu Ile Asp Asp Asp Asp Asp
       1175                1180                1185
   Phe Ser Thr Ile Arg Asp Asp Thr Gly Lys Glu Lys Gln Lys Gln
       1190                1195                1200
   Arg Ala Leu Gly Arg Arg Lys Ser Gln Glu Ala Leu His Glu Gln
       1205                1210                1215
   Ser Ser Tyr Ile Leu Ser Ser Ala Glu Thr Pro Ala Arg Pro Arg
       1220                1225                1230
   Ala Pro Glu Thr Ser Leu Ser Ile His Val Gly Ser Phe Arg
       1235                1240                1245
<210> 21
   <211> 1805
   <212> DNA
   <213> Homo sapiens
<220>
   <221> CDS
   <222> (114)..(998)
   <223>
<400> 21
   caaatgcaga agtggttctc atcttttttt gcagcttaag atctgccttg gtatttgaag      60
   agatataaac tagatcaatt tctttcacag gatcaactaa acagtgtacc aca atg        116
                                                              Met
                                                              1
   aat tct gaa ctt gac tat tat gaa aag ttt gaa gaa gtc cat ggg att       164
   Asn Ser Glu Leu Asp Tyr Tyr Glu Lys Phe Glu Glu Val His Gly Ile
               5                   10                  15
   cta atg tat aaa gat ttt gtc aaa tat tgg gat aat gtg gaa gcg ttc      212
   Leu Met Tyr Lys Asp Phe Val Lys Tyr Trp Asp Asn Val Glu Ala Phe
           20                  25                  30
   cag gca aga cca gat gat ctt gtc att gcc acc tac cct aaa tct ggt      260
   Gln Ala Arg Pro Asp Asp Leu Val Ile Ala Thr Tyr Pro Lys Ser Gly
       35                  40                  45
   aca acc tgg gtt agt gaa att gtg tat atg atc tat aaa gag ggt gat      308
   Thr Thr Trp Val Ser Glu Ile Val Tyr Met Ile Tyr Lys Glu Gly Asp
   50                  55                  60                  65
   gtg gaa aag tgc aaa gaa gat gta att ttt aat cga ata cct ttc ctg      356
   Val Glu Lys Cys Lys Glu Asp Val Ile Phe Asn Arg Ile Pro Phe Leu
                   70                  75                  80
   gaa tgc aga aaa gaa aac ctc atg aat gga gta aaa caa tta gat gag      404
   Glu Cys Arg Lys Glu Asn Leu Met Asn Gly Val Lys Gln Leu Asp Glu
               85                  90                  95
   atg aat tct cct aga att gtg aag act cat ttg cca cct gaa ctt ctt      452
   Met Asn Ser Pro Arg Ile Val Lys Thr His Leu Pro Pro Glu Leu Leu
           100                 105                 110
   cct gcc tca ttt tgg gaa aag gat tgt aag ata atc tat ctt tgc cgg      500
   Pro Ala Ser Phe Trp Glu Lys Asp Cys Lys Ile Ile Tyr Leu Cys Arg
       115                 120                 125
   aat gca aag gat gtg gct gtt tcc ttt tat tat ttc ttt cta atg gtg      548
   Asn Ala Lys Asp Val Ala Val Ser Phe Tyr Tyr Phe Phe Leu Met Val
   130                 135                 140                 145
   gct ggt cat cca aat cct gga tcc ttt cca gag ttt gtg gag aaa ttc      596
   Ala Gly His Pro Asn Pro Gly Ser Phe Pro Glu Phe Val Glu Lys Phe
                   150                 155                 160
   atg caa gga cag gtt cct tat ggt tcc tgg tat aaa cat gta aaa tct      644
   Met Gln Gly Gln Val Pro Tyr Gly Ser Trp Tyr Lys His Val Lys Ser
               165                 170                 175
   tgg tgg gaa aag gga aag agt cca cgt gta cta ttt ctt ttc tac gaa      692
   Trp Trp Glu Lys Gly Lys Ser Pro Arg Val Leu Phe Leu Phe Tyr Glu
           180                 185                 190
   gac ctg aaa gag gat atc aga aaa gag gtg ata aaa ttg ata cat ttc      740
   Asp Leu Lys Glu Asp Ile Arg Lys Glu Val Ile Lys Leu Ile His Phe
       195                 200                 205
   ctg gaa agg aag cca tca gag gag ctt gtg gac agg att ata cat cat      788
   Leu Glu Arg Lys Pro Ser Glu Glu Leu Val Asp Arg Ile Ile His His
   210                 215                 220                 225
   act tcg ttc caa gag atg aag aac aat cca tcc aca aat tac aca aca      836
   Thr Ser Phe Gln Glu Met Lys Asn Asn Pro Ser Thr Asn Tyr Thr Thr
                   230                 235                 240
   ctg cca gac gaa att atg aac cag aaa ttg tcg ccc ttc atg aga aag      884
   Leu Pro Asp Glu Ile Met Asn Gln Lys Leu Ser Pro Phe Met Arg Lys
               245                 250                 255
   gga att aca gga gac tgg aaa aat cac ttt aca gta gcc ctg aat gaa      932
   Gly Ile Thr Gly Asp Trp Lys Asn His Phe Thr Val Ala Leu Asn Glu
           260                 265                 270
   aaa ttt gat aaa cat tat gag cag caa atg aag gaa tct aca ctg aag      980
   Lys Phe Asp Lys His Tyr Glu Gln Gln Met Lys Glu Ser Thr Leu Lys
       275                 280                 285
   ttt cga act gag atc taa gaaggtcttt ctttacttaa catatctgat            1028
   Phe Arg Thr Glu Ile
   290
   attaaagatt tcttttcatt attctccact ttttcttatt ttagattgct agaaaagaca   1088
   taatcatgga ttatgttgac attttctttt taaatttttg tttaactttt tttttttttt   1148
   tttgagacag agtctcactc tgttgcctag gctggaggac agtggcacaa tcatggctga   1208
   ttgcagcctt gacctccttg actcaattga tcctcccatc tcagcctccc aagtagctag   1268
   gactacagac atgtgcaacc atgtttggct aattttttta atgttttttt gtagagatga   1328
   ggtcttatta tattgtccag gctggtcttg aattcctggg ctcaagcttc ccaagtagct   1388
   gcaacaacag gcacacacca ccatgctcaa ctaattttat ttctattttt tgtatagaca   1448
   ggggcttgct atagtgtcca ggctggtctg aaacccttga gctcaagtga tcttcccaca   1508
   ccagcctccc aaaatactgg gattacaggc ttgagcctcc atgcctggcc caggtaacat   1568
   gtttattgag ctgtacatgc atatgagaaa taagaaactt ttttttccta ctatcatctc   1628
   ttaaattttg ttttcttttt cttttgcttc ctcttcttct tttctatttt ttataaatat   1688
   catgcacaac tataacctat gggaatgatg tagtaacaca gattattcat cttgttagag   1748
   ttgtattaaa aataaacaag catttcaaat taaaaaaaaa aaaaaaaaaa aaaaaaa      1805
<210> 22
   <211> 294
   <212> PRT
   <213> Homo sapiens
<400> 22
   Met Asn Ser Glu Leu Asp Tyr Tyr Glu Lys Phe Glu Glu Val His Gly
   1               5                   10                  15
   Ile Leu Met Tyr Lys Asp Phe Val Lys Tyr Trp Asp Asn Val Glu Ala
               20                  25                  30
   Phe Gln Ala Arg Pro Asp Asp Leu Val Ile Ala Thr Tyr Pro Lys Ser
           35                  40                  45
   Gly Thr Thr Trp Val Ser Glu Ile Val Tyr Met Ile Tyr Lys Glu Gly
       50                  55                  60
   Asp Val Glu Lys Cys Lys Glu Asp Val Ile Phe Asn Arg Ile Pro Phe
   65                  70                  75                  80
   Leu Glu Cys Arg Lys Glu Asn Leu Met Asn Gly Val Lys Gln Leu Asp
                   85                  90                  95
   Glu Met Asn Ser Pro Arg Ile Val Lys Thr His Leu Pro Pro Glu Leu
               100                 105                 110
   Leu Pro Ala Ser Phe Trp Glu Lys Asp Cys Lys Ile Ile Tyr Leu Cys
           115                 120                 125
   Arg Asn Ala Lys Asp Val Ala Val Ser Phe Tyr Tyr Phe Phe Leu Met
       130                 135                 140
   Val Ala Gly His Pro Asn Pro Gly Ser Phe Pro Glu Phe Val Glu Lys
   145                 150                 155                 160
   Phe Met Gln Gly Gln Val Pro Tyr Gly Ser Trp Tyr Lys His Val Lys
                   165                 170                 175
   Ser Trp Trp Glu Lys Gly Lys Ser Pro Arg Val Leu Phe Leu Phe Tyr
               180                 185                 190
   Glu Asp Leu Lys Glu Asp Ile Arg Lys Glu Val Ile Lys Leu Ile His
           195                 200                 205
   Phe Leu Glu Arg Lys Pro Ser Glu Glu Leu Val Asp Arg Ile Ile His
       210                 215                 220
   His Thr Ser Phe Gln Glu Met Lys Asn Asn Pro Ser Thr Asn Tyr Thr
   225                 230                 235                 240
   Thr Leu Pro Asp Glu Ile Met Asn Gln Lys Leu Ser Pro Phe Met Arg
                   245                 250                 255
   Lys Gly Ile Thr Gly Asp Trp Lys Asn His Phe Thr Val Ala Leu Asn
               260                 265                 270
   Glu Lys Phe Asp Lys His Tyr Glu Gln Gln Met Lys Glu Ser Thr Leu
           275                 280                 285
   Lys Phe Arg Thr Glu Ile
       290
<210> 23
   <211> 20
   <212> DNA
   <213> Artificial
<220>
   <223> PCR primer
<400> 23
   cccctgagct gatgtttgat 20
<210> 24
   <211> 20
   <212> DNA
   <213> Artificial
<220>
   <223> PCR primer
<400> 24
   gacgcccaga ttcaaaggta 20
<210> 25
   <211> 20
   <212> DNA
   <213> Artificial
<220>
   <223> PCR primer
<400> 25
   gcagacacac ctgttggaaa 20
<210> 26
   <211> 20
   <212> DNA
   <213> Artificial
<220>
   <223> PCR primer
<400> 26
   gtcacgggga tttctttgaa 20
<210> 27
   <211> 20
   <212> DNA
   <213> Artificial
<220>
   <223> PCR primer
<400> 27
   gtcaccgtgt caacctgatg 20
<210> 28
   <211> 20
   <212> DNA
   <213> Artificial
<220>
   <223> PCR primer
<400> 28
   gcctgccttc aagatttctg 20
<210> 29
   <211> 20
   <212> DNA
   <213> Artificial
<220>
   <223> PCR primer
<400> 29
   ggctgaggtc actacggaaa 20
<210> 30
   <211> 20
   <212> DNA
   <213> Artificial
<220>
   <223> PCR primer
<400> 30
   ggtctcagat tgggcactgt 20
<210> 31
   <211> 22
   <212> DNA
   <213> Artificial
<220>
   <223> PCR primer
<400> 31
   gtgctgccct cagactatga cc 22
<210> 32
   <211> 23
   <212> DNA
   <213> Artificial
<220>
   <223> PCR primer
<400> 32
   ctgccataca gaacatcgct cag 23
<210> 33
   <211> 20
   <212> DNA
   <213> Artificial
<220>
   <223> PCR primer
<400> 33
   ggtggacatt tgatcgttcc 20
<210> 34
   <211> 20
   <212> DNA
   <213> Artificial
<220>
   <223> PCR primer
<400> 34
   tgacgcctcc tgagaaaaat 20
<210> 35
   <211> 20
   <212> DNA
   <213> Artificial
<220>
   <223> PCR primer
<400> 35
   tggtttcact tggagctgtg 20
<210> 36
   <211> 20
   <212> DNA
   <213> Artificial
<220>
   <223> PCR primer
<400> 36
   gccatgcaat caatgaagaa 20
<210> 37
   <211> 20
   <212> DNA
   <213> Artificial
<220>
   <223> PCR primer
<400> 37
   aggacgtctc ggtcatcatc 20
<210> 38
   <211> 20
   <212> DNA
   <213> Artificial
<220>
   <223> PCR primer
<400> 38
   ctggcacact agcttggaca 20
<210> 39
   <211> 20
   <212> DNA
   <213> Artificial
<220>
   <223> PCR primer
<400> 39
   caggaactca tgagcgtgaa 20
<210> 40
   <211> 20
   <212> DNA
   <213> Artificial
<220>
   <223> PCR primer
<400> 40
   gatattcacg gctcccactc 20
<210> 41
   <211> 20
   <212> DNA
   <213> Artificial
<220>
   <223> PCR primer
<400> 41
   ggggcaccgt attacttgaa 20
<210> 42
   <211> 20
   <212> DNA
   <213> Artificial
<220>
   <223> PCR primer
<400> 42
   ggcttcttgg cttttccttc 20
<210> 43
   <211> 20
   <212> DNA
   <213> Artificial
<220>
   <223> PCR primer
<400> 43
   cagaaattgt cgcccttcat 20
<210> 44
   <211> 21
   <212> DNA
   <213> Artificial
<220>
   <223> PCR primer
<400> 44
   gattccttca tttgctgctc a 21
<210> 45
   <211> 1567
   <212> DNA
   <213> Homo sapiens
<220>
   <221> CDS
   <222> (132)..(1325)
   <223>
<400> 45
   ggtgcgcggg gaggtggagg gcgaggggcg gggctacctc aggtcccgcc cgcggcaggc     60
   ctgtgggctg cgaggaggag ctttgcctag cttgcaggca gcgcagggca gacggcggca    120
   ggagaagcaa g atg aat gca ggc tca gat cct gtg gtc atc gtc tcg gcg     170
                Met Asn Ala Gly Ser Asp Pro Val Val Ile Val Ser Ala
                1               5                   10
   gcg cgg acc atc ata ggt tcc ttc aat ggt gcc tta gct gct gtt cct      218
   Ala Arg Thr Ile Ile Gly Ser Phe Asn Gly Ala Leu Ala Ala Val Pro
       15                  20                  25
   gtc cag gac ctg ggc tcc act gtc atc aaa gaa gtc ttg aag agg gcc      266
   Val Gln Asp Leu Gly Ser Thr Val Ile Lys Glu Val Leu Lys Arg Ala
   30                  35                  40                  45
   act gtg gct ccg gaa gat gtg tct gag gtc atc ttt gga cat gtc ttg      314
   Thr Val Ala Pro Glu Asp Val Ser Glu Val Ile Phe Gly His Val Leu
                   50                  55                  60
   gca gca ggc tgt ggg cag aat cct gtt aga caa gcc agt gtg ggt gca      362
   Ala Ala Gly Cys Gly Gln Asn Pro Val Arg Gln Ala Ser Val Gly Ala
               65                  70                  75
   gga att ccc tac tct gtt cca gca tgg agc tgc cag atg atc tgt ggg      410
   Gly Ile Pro Tyr Ser Val Pro Ala Trp Ser Cys Gln Met Ile Cys Gly
           80                  85                  90
   tca ggc cta aaa gct gtg tgc ctt gca gtc cag tca ata ggg ata gga      458
   Ser Gly Leu Lys Ala Val Cys Leu Ala Val Gln Ser Ile Gly Ile Gly
       95                  100                 105
   gac tcc agc att gtg gtt gca gga ggc atg gaa aat atg agc aag gct      506
   Asp Ser Ser Ile Val Val Ala Gly Gly Met Glu Asn Met Ser Lys Ala
   110                 115                 120                 125
   cct cac ttg gct tac ttg aga aca gga gta aag ata ggt gag atg cca      554
   Pro His Leu Ala Tyr Leu Arg Thr Gly Val Lys Ile Gly Glu Met Pro
                   130                 135                 140
   ctg act gac agt ata ctc tgt gat ggt ctt aca gat gca ttt cac aac      602
   Leu Thr Asp Ser Ile Leu Cys Asp Gly Leu Thr Asp Ala Phe His Asn
               145                 150                 155
   tgt cat atg ggt att aca gct gaa aat gta gcc aaa aaa tgg caa gtg      650
   Cys His Met Gly Ile Thr Ala Glu Asn Val Ala Lys Lys Trp Gln Val
           160                 165                 170
   agt aga gaa gat cag gac aag gtt gca gtt ctg tcc cag aac agg aca      698
   Ser Arg Glu Asp Gln Asp Lys Val Ala Val Leu Ser Gln Asn Arg Thr
       175                 180                 185
   gag aat gca cag aaa gct ggc cat ttt gac aaa gag att gta cca gtt      746
   Glu Asn Ala Gln Lys Ala Gly His Phe Asp Lys Glu Ile Val Pro Val
   190                 195                 200                 205
   ttg gtg tca act aga aaa ggt ctt att gaa gtt aaa aca gat gag ttt      794
   Leu Val Ser Thr Arg Lys Gly Leu Ile Glu Val Lys Thr Asp Glu Phe
                   210                 215                 220
   cct cgc cat ggg agc aac ata gaa gcc atg tcc aag cta aag cct tac      842
   Pro Arg His Gly Ser Asn Ile Glu Ala Met Ser Lys Leu Lys Pro Tyr
               225                 230                 235
   ttt ctt act gat gga acg gga aca gtc acc cca gcc aat gct tca gga      890
   Phe Leu Thr Asp Gly Thr Gly Thr Val Thr Pro Ala Asn Ala Ser Gly
           240                 245                 250
   ata aat gat ggt gct gca gct gtc gtt ctt atg aag aag tca gaa gct      938
   Ile Asn Asp Gly Ala Ala Ala Val Val Leu Met Lys Lys Ser Glu Ala
       255                 260                 265
   gat aaa cgt ggg ctt aca cct tta gca cgg ata gtt tcc tgg tcc caa      986
   Asp Lys Arg Gly Leu Thr Pro Leu Ala Arg Ile Val Ser Trp Ser Gln
   270                 275                 280                 285
   gtg ggt gtg gag cct tcc att atg gga ata gga cca att cca gcc ata     1034
   Val Gly Val Glu Pro Ser Ile Met Gly Ile Gly Pro Ile Pro Ala Ile
                   290                 295                 300
   aag caa gct gtt aca aaa gca ggt tgg tca ctg gaa gat gtt gac ata     1082
   Lys Gln Ala Val Thr Lys Ala Gly Trp Ser Leu Glu Asp Val Asp Ile
               305                 310                 315
   ttt gaa atc aat gaa gcc ttt gca gct gtc tct gct gca ata gtt aaa     1130
   Phe Glu Ile Asn Glu Ala Phe Ala Ala Val Ser Ala Ala Ile Val Lys
           320                 325                 330
   gaa ctt gga tta aac cca gag aag gtc aat att gaa gga ggg gct ata     1178
   Glu Leu Gly Leu Asn Pro Glu Lys Val Asn Ile Glu Gly Gly Ala Ile
       335                 340                 345
   gcc ttg ggc cac cct ctt gga gca tct ggc tgt cga att ctt gtg acc     1226
   Ala Leu Gly His Pro Leu Gly Ala Ser Gly Cys Arg Ile Leu Val Thr
   350                 355                 360                 365
   ctg tta cac aca ctg gag aga atg ggc aga agt cgt ggt gtt gca gcc     1274
   Leu Leu His Thr Leu Glu Arg Met Gly Arg Ser Arg Gly Val Ala Ala
                   370                 375                 380
   ctg tgc att ggg ggt ggg atg gga ata gca atg tgt gtt cag aga gaa     1322
   Leu Cys Ile Gly Gly Gly Met Gly Ile Ala Met Cys Val Gln Arg Glu
               385                 390                 395
   tga attgcttaaa ctttgaacaa cctcaatttc tttttaaact aataaagtac          1375
   taggttgcaa tatgtgaaat cagaggacca aagtacagat ggaaaccatt tcctacatca   1435
   caaaaaccca agtttacagc ttgtacttta ctttaatgtg taatactcaa ctcaaggtac   1495
   aagacaattg catttaacat tgttataaat aaaaggaaca tcagatcaat cattaaaaaa 1555
   aaaaaaaaaa aa 1567
<210> 46
   <211> 397
   <212> PRT
   <213> Homo sapiens
<400> 46
   Met Asn Ala Gly Ser Asp Pro Val Val Ile Val Ser Ala Ala Arg Thr
   1               5                   10                  15
   Ile Ile Gly Ser Phe Asn Gly Ala Leu Ala Ala Val Pro Val Gln Asp
               20                  25                  30
   Leu Gly Ser Thr Val Ile Lys Glu Val Leu Lys Arg Ala Thr Val Ala
           35                  40                  45
   Pro Glu Asp Val Ser Glu Val Ile Phe Gly His Val Leu Ala Ala Gly
       50                  55                  60
   Cys Gly Gln Asn Pro Val Arg Gln Ala Ser Val Gly Ala Gly Ile Pro
   65                  70                  75                  80
   Tyr Ser Val Pro Ala Trp Ser Cys Gln Met Ile Cys Gly Ser Gly Leu
                   85                  90                  95
   Lys Ala Val Cys Leu Ala Val Gln Ser Ile Gly Ile Gly Asp Ser Ser
               100                 105                 110
   Ile Val Val Ala Gly Gly Met Glu Asn Met Ser Lys Ala Pro His Leu
           115                 120                 125
   Ala Tyr Leu Arg Thr Gly Val Lys Ile Gly Glu Met Pro Leu Thr Asp
       130                 135                 140
   Ser Ile Leu Cys Asp Gly Leu Thr Asp Ala Phe His Asn Cys His Met
   145                 150                 155                 160
   Gly Ile Thr Ala Glu Asn Val Ala Lys Lys Trp Gln Val Ser Arg Glu
                   165                 170                 175
   Asp Gln Asp Lys Val Ala Val Leu Ser Gln Asn Arg Thr Glu Asn Ala
               180                 185                 190
   Gln Lys Ala Gly His Phe Asp Lys Glu Ile Val Pro Val Leu Val Ser
           195                 200                 205
   Thr Arg Lys Gly Leu Ile Glu Val Lys Thr Asp Glu Phe Pro Arg His
       210                 215                 220
   Gly Ser Asn Ile Glu Ala Met Ser Lys Leu Lys Pro Tyr Phe Leu Thr
   225                 230                 235                 240
   Asp Gly Thr Gly Thr Val Thr Pro Ala Asn Ala Ser Gly Ile Asn Asp
                   245                 250                 255
   Gly Ala Ala Ala Val Val Leu Met Lys Lys Ser Glu Ala Asp Lys Arg
               260                 265                 270
   Gly Leu Thr Pro Leu Ala Arg Ile Val Ser Trp Ser Gln Val Gly Val
           275                 280                 285
   Glu Pro Ser Ile Met Gly Ile Gly Pro Ile Pro Ala Ile Lys Gln Ala
       290                 295                 300
   Val Thr Lys Ala Gly Trp Ser Leu Glu Asp Val Asp Ile Phe Glu Ile
   305                 310                 315                 320
   Asn Glu Ala Phe Ala Ala Val Ser Ala Ala Ile Val Lys Glu Leu Gly
                   325                 330                 335
   Leu Asn Pro Glu Lys Val Asn Ile Glu Gly Gly Ala Ile Ala Leu Gly
               340                 345                 350
   His Pro Leu Gly Ala Ser Gly Cys Arg Ile Leu Val Thr Leu Leu His
           355                 360                 365
   Thr Leu Glu Arg Met Gly Arg Ser Arg Gly Val Ala Ala Leu Cys Ile
       370                 375                 380
   Gly Gly Gly Met Gly Ile Ala Met Cys Val Gln Arg Glu
   385                 390                 395
<210> 47
   <211> 3105
   <212> DNA
   <213> Homo sapiens
<220>
   <221> CDS
   <222> (179)..(1720)
   <223>
<400> 47
caactggggg cgccccggac gaccatgaga gataaggact gagggccagg aaggggaagc      60
   gagcccgccg agaggtggcg gggactgctc acgccaaggg ccacagcggc cgcgctccgg     120
   cctcgctccg ccgctccacg cctcgcggga tccgcggggg cagcccggcc gggcgggg      178
   atg ccg ggg ctg ggg cgg agg gcg cag tgg ctg tgc tgg tgg tgg ggg      226
   ctg ctg tgc agc tgc tgc ggg ccc ccg ccg ctg cgg ccg ccc ttg ccc      274
   gct gcc gcg gcc gcc gcc gcc ggg ggg cag ctg ctg ggg gac ggc ggg      322
   agc ccc ggc cgc acg gag cag ccg ccg ccg tcg ccg cag tcc tcc tcg      370
   ggc ttc ctg tac cgg cgg ctc aag acg cag gag aag cgg gag atg cag      418
   aag gag atc ttg tcg gtg ctg ggg ctc ccg cac cgg ccc cgg ccc ctg      466
   cac ggc ctc caa cag ccg cag ccc ccg gcg ctc cgg cag cag gag gag      514
   cag cag cag cag cag cag ctg cct cgc gga gag ccc cct ccc ggg cga      562
   ctg aag tcc gcg ccc ctc ttc atg ctg gat ctg tac aac gcc ctg tcc      610
   gcc gac aac gac gag gac ggg gcg tcg gag ggg gag agg cag cag tcc      658
   tgg ccc cac gaa gca gcc agc tcg tcc cag cgt cgg cag ccg ccc ccg      706
   ggc gcc gcg cac ccg ctc aac cgc aag agc ctt ctg gcc ccc gga tct      754
   ggc agc ggc ggc gcg tcc cca ctg acc agc gcg cag gac agc gcc ttc      802
   ctc aac gac gcg gac atg gtc atg agc ttt gtg aac ctg gtg gag tac      850
   gac aag gag ttc tcc cct cgt cag cga cac cac aaa gag ttc aag ttc      898
   aac tta tcc cag att cct gag ggt gag gtg gtg acg gct gca gaa ttc      946
   cgc atc tac aag gac tgt gtt atg ggg agt ttt aaa aac caa act ttt      994
   ctt atc agc att tat caa gtc tta cag gag cat cag cac aga gac tct     1042
   gac ctg ttt ttg ttg gac acc cgt gta gta tgg gcc tca gaa gaa ggc     1090
   tgg ctg gaa ttt gac atc acg gcc act agc aat ctg tgg gtt gtg act     1138
   cca cag cat aac atg ggg ctt cag ctg agc gtg gtg aca agg gat gga     1186
   gtc cac gtc cac ccc cga gcc gca ggc ctg gtg ggc aga gac ggc cct     1234
   tac gac aag cag ccc ttc atg gtg gct ttc ttc aaa gtg agt gag gtg     1282
   cac gtg cgc acc acc agg tca gcc tcc agc cgg cgc cga caa cag agt     1330
   cgt aat cgc tct acc cag tcc cag gac gtg gcg cgg gtc tcc agt gct     1378
   tca gat tac aac agc agt gaa ttg aaa aca gcc tgc agg aag cat gag     1426
   ctg tat gtg agt ttc caa gac ctg gga tgg cag gac tgg atc att gca     1474
   ccc aag ggc tat gct gcc aat tac tgt gat gga gaa tgc tcc ttc cca     1522
   ctc aac gca cac atg aat gca acc aac cac gcg att gtg cag acc ttg     1570
   gtt cac ctt atg aac ccc gag tat gtc ccc aaa ccg tgc tgt gcg cca     1618
   act aag cta aat gcc atc tcg gtt ctt tac ttt gat gac aac tcc aat     1666
   gtc att ctg aaa aaa tac agg aat atg gtt gta aga gct tgt gga tgc      1714
   cac taa ctcgaaacca gatgctgggg acacacattc tgccttggat tcctagatta       1770
   catctgcctt aaaaaaacac ggaagcacag ttggaggtgg gacgatgaga ctttgaaact   1830
   atctcatgcc agtgccttat tacccaggaa gattttaaag gacctcatta ataatttgct   1890
   cacttggtaa atgacgtgag tagttgttgg tctgtagcaa gctgagtttg gatgtctgta   1950
   gcataaggtc tggtaactgc agaaacataa ccgtgaagct cttcctaccc tcctccccca   2010
   aaaacccacc aaaattagtt ttagctgtag atcaagctat ttggggtgtt tgttagtaaa   2070
   tagggaaaat aatctcaaag gagttaaatg tattcttggc taaaggatca gctggttcag   2130
   tactgtctat caaaggtaga ttttacagag aacagaaatc ggggaagtgg ggggaacgcc   2190
   tctgttcagt tcattcccag aagtccacag gacgcacagc ccaggccaca gccagggctc   2250
   cacggggcgc ccttgtctca gtcattgctg ttgtatgttc gtgctggagt tttgttggtg   2310
   tgaaaataca cttatttcag ccaaaacata ccatttctac acctcaatcc tccatttgct   2370
   gtactctttg ctagtaccaa aagtagactg attacactga ggtgaggcta caaggggtgt   2430
   gtaaccgtgt aacacgtgaa ggcaatgctc acctcttctt taccagaacg gttctttgac   2490
   cagcacatta acttctggac tgccggctct agtacctttt cagtaaagtg gttctctgcc   2550
   tttttactat acagcatacc acgccacagg gttagaacca acgaagaaaa taaaatgagg   2610
   gtgcccagct tataagaatg gtgttagggg gatgagcatg ctgtttatga acggaaatca   2670
   tgatttccct tgtagaaagt gaggctcaga ttaaatttta gaatattttc taaatgtctt   2730
   tttcacaatc atgtactggg aaggcaattt catactaaac tgattaaata atacatttat   2790
   aatctacaac tgtttgcact tacagctttt tttgtaaata taaactataa tttattgtct   2850
   attttatatc tgttttgctg taacattgaa ggaaagacca gacttttaaa aaaaaagagt   2910
   ttatttagaa agtatcatag tgtaaacaaa caaattgtac cactttgatt ttcttggaat   2970
   acaagactcg tgatgcaaag ctgaagttgt gtgtacaaga ctcttgacag ttgtgcttct   3030
   ctaggaggtt gggttttttt aaaaaaagaa ttatctgtga accatacgtg attaataaag   3090
   atttccttta aggca                                                     3105
<210> 48
   <211> 513
   <212> PRT
   <213> Homo sapiens
<400> 48
<210> 49
   <211> 3691
   <212> DNA
   <213> Homo sapiens
<220>
   <221> CDS
   <222> (109)..(981)
   <223>
<400> 49
   ggcgcaacgc tgagcagctg gcgcgtcccg cgcggcccca gttctgcgca gcttcccgag      60
   gctccgcacc agccgcgctt ctgtccgcct gcagggcatt ccagaaag atg agg ata     117
                                                        Met Arg Ile
                                                        1
   ttt gct gtc ttt ata ttc atg acc tac tgg cat ttg ctg aac gca ttt      165
   Phe Ala Val Phe Ile Phe Met Thr Tyr Trp His Leu Leu Asn Ala Phe
       5                   10                  15
   act gtc acg gtt ccc aag gac cta tat gtg gta gag tat ggt agc aat      213
   Thr Val Thr Val Pro Lys Asp Leu Tyr Val Val Glu Tyr Gly Ser Asn
   20                  25                  30                  35
   atg aca att gaa tgc aaa ttc cca gta gaa aaa caa tta gac ctg gct      261
   Met Thr Ile Glu Cys Lys Phe Pro Val Glu Lys Gln Leu Asp Leu Ala
                   40                  45                  50
   gca cta att gtc tat tgg gaa atg gag gat aag aac att att caa ttt      309
   Ala Leu Ile Val Tyr Trp Glu Met Glu Asp Lys Asn Ile Ile Gln Phe
               55                  60                  65
   gtg cat gga gag gaa gac ctg aag gtt cag cat agt agc tac aga cag      357
   Val His Gly Glu Glu Asp Leu Lys Val Gln His Ser Ser Tyr Arg Gln
           70                  75                  80
   agg gcc cgg ctg ttg aag gac cag ctc tcc ctg gga aat gct gca ctt      405
   Arg Ala Arg Leu Leu Lys Asp Gln Leu Ser Leu Gly Asn Ala Ala Leu
       85                  90                  95
   cag atc aca gat gtg aaa ttg cag gat gca ggg gtg tac cgc tgc atg      453
   Gln Ile Thr Asp Val Lys Leu Gln Asp Ala Gly Val Tyr Arg Cys Met
   100                 105                 110                 115
   atc agc tat ggt ggt gcc gac tac aag cga att act gtg aaa gtc aat      501
   Ile Ser Tyr Gly Gly Ala Asp Tyr Lys Arg Ile Thr Val Lys Val Asn
                   120                 125                 130
   gcc cca tac aac aaa atc aac caa aga att ttg gtt gtg gat cca gtc      549
   Ala Pro Tyr Asn Lys Ile Asn Gln Arg Ile Leu Val Val Asp Pro Val
               135                 140                 145
   acc tct gaa cat gaa ctg aca tgt cag gct gag ggc tac ccc aag gcc      597
   Thr Ser Glu His Glu Leu Thr Cys Gln Ala Glu Gly Tyr Pro Lys Ala
           150                 155                 160
   gaa gtc atc tgg aca agc agt gac cat caa gtc ctg agt ggt aag acc      645
   Glu Val Ile Trp Thr Ser Ser Asp His Gln Val Leu Ser Gly Lys Thr
       165                 170                 175
   acc acc acc aat tcc aag aga gag gag aag ctt ttc aat gtg acc agc      693
   Thr Thr Thr Asn Ser Lys Arg Glu Glu Lys Leu Phe Asn Val Thr Ser
   180                 185                 190                 195
   aca ctg aga atc aac aca aca act aat gag att ttc tac tgc act ttt      741
   Thr Leu Arg Ile Asn Thr Thr Thr Asn Glu Ile Phe Tyr Cys Thr Phe
                   200                 205                 210
   agg aga tta gat cct gag gaa aac cat aca gct gaa ttg gtc atc cca      789
   Arg Arg Leu Asp Pro Glu Glu Asn His Thr Ala Glu Leu Val Ile Pro
               215                 220                 225
   gaa cta cct ctg gca cat cct cca aat gaa agg act cac ttg gta att      837
   Glu Leu Pro Leu Ala His Pro Pro Asn Glu Arg Thr His Leu Val Ile
           230                 235                 240
   ctg gga gcc atc tta tta tgc ctt ggt gta gca ctg aca ttc atc ttc      885
   Leu Gly Ala Ile Leu Leu Cys Leu Gly Val Ala Leu Thr Phe Ile Phe
       245                 250                 255
   cgt tta aga aaa ggg aga atg atg gat gtg aaa aaa tgt ggc atc caa      933
   Arg Leu Arg Lys Gly Arg Met Met Asp Val Lys Lys Cys Gly Ile Gln
   260                 265                 270                 275
   gat aca aac tca aag aag caa agt gat aca cat ttg gag gag acg taa      981
   Asp Thr Asn Ser Lys Lys Gln Ser Asp Thr His Leu Glu Glu Thr
                   280                 285                 290
   tccagcattg gaacttctga tcttcaagca gggattctca acctgtggtt taggggttca   1041
   tcggggctga gcgtgacaag aggaaggaat gggcccgtgg gatgcaggca atgtgggact   1101
   taaaaggccc aagcactgaa aatggaacct ggcgaaagca gaggaggaga atgaagaaag   1161
   atggagtcaa acagggagcc tggagggaga ccttgatact ttcaaatgcc tgaggggctc   1221
   atcgacgcct gtgacaggga gaaaggatac ttctgaacaa ggagcctcca agcaaatcat   1281
   ccattgctca tcctaggaag acgggttgag aatccctaat ttgagggtca gttcctgcag   1341
   aagtgccctt tgcctccact caatgcctca atttgttttc tgcatgactg agagtctcag   1401
   tgttggaacg ggacagtatt tatgtatgag tttttcctat ttattttgag tctgtgaggt   1461
   cttcttgtca tgtgagtgtg gttgtgaatg atttcttttg aagatatatt gtagtagatg   1521
   ttacaatttt gtcgccaaac taaacttgct gcttaatgat ttgctcacat ctagtaaaac   1581
   atggagtatt tgtaaggtgc ttggtctcct ctataactac aagtatacat tggaagcata   1641
   aagatcaaac cgttggttgc ataggatgtc acctttattt aacccattaa tactctggtt   1701
   gacctaatct tattctcaga cctcaagtgt ctgtgcagta tctgttccat ttaaatatca   1761
   gctttacaat tatgtggtag cctacacaca taatctcatt tcatcgctgt aaccaccctg   1821
   ttgtgataac cactattatt ttacccatcg tacagctgag gaagcaaaca gattaagtaa   1881
   cttgcccaaa ccagtaaata gcagacctca gactgccacc cactgtcctt ttataataca   1941
   atttacagct atattttact ttaagcaatt cttttattca aaaaccattt attaagtgcc 2001
   cttgcaatat caatcgctgt gccaggcatt gaatctacag atgtgagcaa gacaaagtac 2061
   ctgtcctcaa ggagctcata gtataatgag gagattaaca agaaaatgta ttattacaat 2121
   ttagtccagt gtcatagcat aaggatgatg cgaggggaaa acccgagcag tgttgccaag 2181
   aggaggaaat aggccaatgt ggtctgggac ggttggatat acttaaacat cttaataatc 2241
   agagtaattt tcatttacaa agagaggtcg gtacttaaaa taaccctgaa aaataacact 2301
   ggaattcctt ttctagcatt atatttattc ctgatttgcc tttgccatat aatctaatgc 2361
   ttgtttatat agtgtctggt attgtttaac agttctgtct tttctattta aatgccacta   2421
   aattttaaat tcataccttt ccatgattca aaattcaaaa gatcccatgg gagatggttg   2481
   gaaaatctcc acttcatcct ccaagccatt caagtttcct ttccagaagc aactgctact 2541
   gcctttcatt catatgttct tctaaagata gtctacattt ggaaatgtat gttaaaagca 2601
   cgtattttta aaattttttt cctaaatagt aacacattgt atgtctgctg tgtactttgc 2661
   tatttttatt tattttagtg tttcttatat agcagatgga atgaatttga agttcccagg 2721
   gctgaggatc catgccttct ttgtttctaa gttatctttc ccatagcttt tcattatctt 2781
   tcatatgatc cagtatatgt taaatatgtc ctacatatac atttagacaa ccaccatttg 2841
   ttaagtattt gctctaggac agagtttgga tttgtttatg tttgctcaaa aggagaccca 2901
   tgggctctcc agggtgcact gagtcaatct agtcctaaaa agcaatctta ttattaactc 2961
   tgtatgacag aatcatgtct ggaacttttg ttttctgctt tctgtcaagt ataaacttca 3021
   ctttgatgct gtacttgcaa aatcacattt tctttctgga aattccggca gtgtaccttg 3081
   actgctagct accctgtgcc agaaaagcct cattcgttgt gcttgaaccc ttgaatgcca 3141
   ccagctgtca tcactacaca gccctcctaa gaggcttcct ggaggtttcg agattcagat 3201
   gccctgggag atcccagagt ttcctttccc tcttggccat attctggtgt caatgacaag 3261
   gagtaccttg gctttgccac atgtcaaggc tgaagaaaca gtgtctccaa cagagctcct 3321
   tgtgttatct gtttgtacat gtgcatttgt acagtaattg gtgtgacagt gttctttgtg 3381
   tgaattacag gcaagaattg tggctgagca aggcacatag tctactcagt ctattcctaa 3441
   gtcctaactc ctccttgtgg tgttggattt gtaaggcact ttatcccttt tgtctcatgt 3501
   ttcatcgtaa atggcatagg cagagatgat acctaattct gcatttgatt gtcacttttt 3561
   gtacctgcat taatttaata aaatattctt atttattttg ttacttggta caccagcatg 3621
   tccattttct tgtttatttt gtgtttaata aaatgttcag tttaacatcc cagtggagaa 3681
   agttaaaaaa 3691
<210> 50
   <211> 290
   <212> PRT
   <213> Homo sapiens
<400> 50
   Met Arg Ile Phe Ala Val Phe Ile Phe Met Thr Tyr Trp His Leu Leu
   1               5                   10                  15
   Asn Ala Phe Thr Val Thr Val Pro Lys Asp Leu Tyr Val Val Glu Tyr
               20                  25                  30
   Gly Ser Asn Met Thr Ile Glu Cys Lys Phe Pro Val Glu Lys Gln Leu
           35                  40                  45
   Asp Leu Ala Ala Leu Ile Val Tyr Trp Glu Met Glu Asp Lys Asn Ile
       50                  55                  60
   Ile Gln Phe Val His Gly Glu Glu Asp Leu Lys Val Gln His Ser Ser
   65                  70                  75                  80
   Tyr Arg Gln Arg Ala Arg Leu Leu Lys Asp Gln Leu Ser Leu Gly Asn
                   85                  90                  95
   Ala Ala Leu Gln Ile Thr Asp Val Lys Leu Gln Asp Ala Gly Val Tyr
               100                 105                 110
   Arg Cys Met Ile Ser Tyr Gly Gly Ala Asp Tyr Lys Arg Ile Thr Val
           115                 120                 125
   Lys Val Asn Ala Pro Tyr Asn Lys Ile Asn Gln Arg Ile Leu Val Val
       130                 135                 140
   Asp Pro Val Thr Ser Glu His Glu Leu Thr Cys Gln Ala Glu Gly Tyr
   145                 150                 155                 160
   Pro Lys Ala Glu Val Ile Trp Thr Ser Ser Asp His Gln Val Leu Ser
                   165                 170                 175
   Gly Lys Thr Thr Thr Thr Asn Ser Lys Arg Glu Glu Lys Leu Phe Asn
               180                 185                 190
   Val Thr Ser Thr Leu Arg Ile Asn Thr Thr Thr Asn Glu Ile Phe Tyr
           195                 200                 205
   Cys Thr Phe Arg Arg Leu Asp Pro Glu Glu Asn His Thr Ala Glu Leu
       210                 215                 220
   Val Ile Pro Glu Leu Pro Leu Ala His Pro Pro Asn Glu Arg Thr His
   225                 230                 235                 240
   Leu Val Ile Leu Gly Ala Ile Leu Leu Cys Leu Gly Val Ala Leu Thr
                   245                 250                 255
   Phe Ile Phe Arg Leu Arg Lys Gly Arg Met Met Asp Val Lys Lys Cys
               260                 265                 270
   Gly Ile Gln Asp Thr Asn Ser Lys Lys Gln Ser Asp Thr His Leu Glu
           275                 280                 285
   Glu Thr
       290
<210> 51
   <211> 2358
   <212> DNA
   <213> Homo sapiens
<220>
   <221> CDS
   <222> (207)..(1256)
   <223>
<400> 51
   aaactcacac aacaactctt ccccgctgag aggagacagc cagtgcgact ccaccctcca      60
   gctcgacggc agccgccccg gccgacagcc ccgagacgac agcccggcgc gtcccggtcc     120
   ccacctccga ccaccgccag cgctccaggc cccgccgctc cccgctcgcc gccaccgcgc     180
   cctccgctcc gcccgcagtg ccaacc atg acc gcc gcc agt atg ggc ccc gtc      233
                                Met Thr Ala Ala Ser Met Gly Pro Val
                                1               5
   cgc gtc gcc ttc gtg gtc ctc ctc gcc ctc tgc agc cgg ccg gcc gtc       281
   Arg Val Ala Phe Val Val Leu Leu Ala Leu Cys Ser Arg Pro Ala Val
   10                  15                  20                  25
   ggc cag aac tgc agc ggg ccg tgc cgg tgc ccg gac gag ccg gcg ccg       329
   Gly Gln Asn Cys Ser Gly Pro Cys Arg Cys Pro Asp Glu Pro Ala Pro
                   30                  35                  40
   cgc tgc ccg gcg ggc gtg agc ctc gtg ctg gac ggc tgc ggc tgc tgc       377
   Arg Cys Pro Ala Gly Val Ser Leu Val Leu Asp Gly Cys Gly Cys Cys
               45                  50                  55
   cgc gtc tgc gcc aag cag ctg ggc gag ctg tgc acc gag cgc gac ccc       425
   Arg Val Cys Ala Lys Gln Leu Gly Glu Leu Cys Thr Glu Arg Asp Pro
           60                  65                  70
   tgc gac ccg cac aag ggc ctc ttc tgt gac ttc ggc tcc ccg gcc aac       473
   Cys Asp Pro His Lys Gly Leu Phe Cys Asp Phe Gly Ser Pro Ala Asn
       75                  80                  85
   cgc aag atc ggc gtg tgc acc gcc aaa gat ggt gct ccc tgc atc ttc       521
   Arg Lys Ile Gly Val Cys Thr Ala Lys Asp Gly Ala Pro Cys Ile Phe
   90                  95                  100                 105
   ggt ggt acg gtg tac cgc agc gga gag tcc ttc cag agc agc tgc aag      569
   Gly Gly Thr Val Tyr Arg Ser Gly Glu Ser Phe Gln Ser Ser Cys Lys
                   110                 115                 120
   tac cag tgc acg tgc ctg gac ggg gcg gtg ggc tgc atg ccc ctg tgc      617
   Tyr Gln Cys Thr Cys Leu Asp Gly Ala Val Gly Cys Met Pro Leu Cys
               125                 130                 135
   agc atg gac gtt cgt ctg ccc agc cct gac tgc ccc ttc ccg agg agg      665
   Ser Met Asp Val Arg Leu Pro Ser Pro Asp Cys Pro Phe Pro Arg Arg
           140                 145                 150
   gtc aag ctg ccc ggg aaa tgc tgc gag gag tgg gtg tgt gac gag ccc      713
   Val Lys Leu Pro Gly Lys Cys Cys Glu Glu Trp Val Cys Asp Glu Pro
       155                 160                 165
   aag gac caa acc gtg gtt ggg cct gcc ctc gcg gct tac cga ctg gaa      761
   Lys Asp Gln Thr Val Val Gly Pro Ala Leu Ala Ala Tyr Arg Leu Glu
   170                 175                 180                 185
   gac acg ttt ggc cca gac cca act atg att aga gcc aac tgc ctg gtc      809
   Asp Thr Phe Gly Pro Asp Pro Thr Met Ile Arg Ala Asn Cys Leu Val
                   190                 195                 200
   cag acc aca gag tgg agc gcc tgt tcc aag acc tgt ggg atg ggc atc      857
   Gln Thr Thr Glu Trp Ser Ala Cys Ser Lys Thr Cys Gly Met Gly Ile
               205                 210                 215
   tcc acc cgg gtt acc aat gac aac gcc tcc tgc agg cta gag aag cag      905
   Ser Thr Arg Val Thr Asn Asp Asn Ala Ser Cys Arg Leu Glu Lys Gln
           220                 225                 230
   agc cgc ctg tgc atg gtc agg cct tgc gaa gct gac ctg gaa gag aac      953
   Ser Arg Leu Cys Met Val Arg Pro Cys Glu Ala Asp Leu Glu Glu Asn
       235                 240                 245
   att aag aag ggc aaa aag tgc atc cgt act ccc aaa atc tcc aag cct     1001
   Ile Lys Lys Gly Lys Lys Cys Ile Arg Thr Pro Lys Ile Ser Lys Pro
   250                 255                 260                 265
   atc aag ttt gag ctt tct ggc tgc acc agc atg aag aca tac cga gct     1049
   Ile Lys Phe Glu Leu Ser Gly Cys Thr Ser Met Lys Thr Tyr Arg Ala
                   270                 275                 280
   aaa ttc tgt gga gta tgt acc gac ggc cga tgc tgc acc ccc cac aga     1097
   Lys Phe Cys Gly Val Cys Thr Asp Gly Arg Cys Cys Thr Pro His Arg
               285                 290                 295
   acc acc acc ctg ccg gtg gag ttc aag tgc cct gac ggc gag gtc atg     1145
   Thr Thr Thr Leu Pro Val Glu Phe Lys Cys Pro Asp Gly Glu Val Met
           300                 305                 310
   aag aag aac atg atg ttc atc aag acc tgt gcc tgc cat tac aac tgt     1193
   Lys Lys Asn Met Met Phe Ile Lys Thr Cys Ala Cys His Tyr Asn Cys
       315                 320                 325
   ccc gga gac aat gac atc ttt gaa tcg ctg tac tac agg aag atg tac     1241
   Pro Gly Asp Asn Asp Ile Phe Glu Ser Leu Tyr Tyr Arg Lys Met Tyr
   330                 335                 340                 345
   gga gac atg gca tga agccagagag tgagagacat taactcatta gactggaact     1296
   Gly Asp Met Ala
   tgaactgatt cacatctcat ttttccgtaa aaatgatttc agtagcacaa gttatttaaa   1356
   tctgtttttc taactggggg aaaagattcc cacccaattc aaaacattgt gccatgtcaa   1416
   acaaatagtc tatcaacccc agacactggt ttgaagaatg ttaagacttg acagtggaac   1476
   tacattagta cacagcacca gaatgtatat taaggtgtgg ctttaggagc agtgggaggg   1536
   taccagcaga aaggttagta tcatcagata gcatcttata cgagtaatat gcctgctatt   1596
   tgaagtgtaa ttgagaagga aaattttagc gtgctcactg acctgcctgt agccccagtg   1656
   acagctagga tgtgcattct ccagccatca agagactgag tcaagttgtt ccttaagtca   1716
   gaacagcaga ctcagctctg acattctgat tcgaatgaca ctgttcagga atcggaatcc   1776
   tgtcgattag actggacagc ttgtggcaag tgaatttgcc tgtaacaagc cagatttttt   1836
   aaaatttata ttgtaaatat tgtgtgtgtg tgtgtgtgtg tatatatata tatatgtaca   1896
   gttatctaag ttaatttaaa gttgtttgtg cctttttatt tttgttttta atgctttgat   1956
   atttcaatgt tagcctcaat ttctgaacac cataggtaga atgtaaagct tgtctgatcg   2016
   ttcaaagcat gaaatggata cttatatgga aattctgctc agatagaatg acagtccgtc   2076
   aaaacagatt gtttgcaaag gggaggcatc agtgtccttg gcaggctgat ttctaggtag   2136
   gaaatgtggt agcctcactt ttaatgaaca aatggccttt attaaaaact gagtgactct   2196
   atatagctga tcagtttttt cacctggaag catttgtttc tactttgata tgactgtttt   2256
   tcggacagtt tatttgttga gagtgtgacc aaaagttaca tgtttgcacc tttctagttg   2316
   aaaataaagt gtatattttt tctataaaaa aaaaaaaaaa aa                      2358
<210> 52
   <211> 349
   <212> PRT
   <213> Homo sapiens
<400> 52
   Met Thr Ala Ala Ser Met Gly Pro Val Arg Val Ala Phe Val Val Leu
   1               5                   10                  15
   Leu Ala Leu Cys Ser Arg Pro Ala Val Gly Gln Asn Cys Ser Gly Pro
               20                  25                  30
   Cys Arg Cys Pro Asp Glu Pro Ala Pro Arg Cys Pro Ala Gly Val Ser
           35                  40                  45
   Leu Val Leu Asp Gly Cys Gly Cys Cys Arg Val Cys Ala Lys Gln Leu
       50                  55                  60
   Gly Glu Leu Cys Thr Glu Arg Asp Pro Cys Asp Pro His Lys Gly Leu
   65                  70                  75                  80
   Phe Cys Asp Phe Gly Ser Pro Ala Asn Arg Lys Ile Gly Val Cys Thr
                   85                  90                  95
   Ala Lys Asp Gly Ala Pro Cys Ile Phe Gly Gly Thr Val Tyr Arg Ser
               100                 105                 110
   Gly Glu Ser Phe Gln Ser Ser Cys Lys Tyr Gln Cys Thr Cys Leu Asp
           115                 120                 125
   Gly Ala Val Gly Cys Met Pro Leu Cys Ser Met Asp Val Arg Leu Pro
       130                 135                 140
   Ser Pro Asp Cys Pro Phe Pro Arg Arg Val Lys Leu Pro Gly Lys Cys
   145                 150                 155                 160
   Cys Glu Glu Trp Val Cys Asp Glu Pro Lys Asp Gln Thr Val Val Gly
                   165                 170                 175
   Pro Ala Leu Ala Ala Tyr Arg Leu Glu Asp Thr Phe Gly Pro Asp Pro
               180                 185                 190
   Thr Met Ile Arg Ala Asn Cys Leu Val Gln Thr Thr Glu Trp Ser Ala
           195                 200                 205
   Cys Ser Lys Thr Cys Gly Met Gly Ile Ser Thr Arg Val Thr Asn Asp
       210                 215                 220
   Asn Ala Ser Cys Arg Leu Glu Lys Gln Ser Arg Leu Cys Met Val Arg
   225                 230                 235                 240
   Pro Cys Glu Ala Asp Leu Glu Glu Asn Ile Lys Lys Gly Lys Lys Cys
                   245                 250                 255
   Ile Arg Thr Pro Lys Ile Ser Lys Pro Ile Lys Phe Glu Leu Ser Gly
               260                 265                 270
   Cys Thr Ser Met Lys Thr Tyr Arg Ala Lys Phe Cys Gly Val Cys Thr
           275                 280                 285
   Asp Gly Arg Cys Cys Thr Pro His Arg Thr Thr Thr Leu Pro Val Glu
       290                 295                 300
   Phe Lys Cys Pro Asp Gly Glu Val Met Lys Lys Asn Met Met Phe Ile
   305                 310                 315                 320
   Lys Thr Cys Ala Cys His Tyr Asn Cys Pro Gly Asp Asn Asp Ile Phe
   325 330 335
   Glu Ser Leu Tyr Tyr Arg Lys Met Tyr Gly Asp Met Ala
   340 345
<210> 53
   <211> 5742
   <212> DNA
   <213> Homo sapiens
<220>
   <221> CDS
   <222> (237)..(2972)
   <223>
<400> 53
   gttttagcgg ggactacgat ccaggctgga gttgcgctcg gccggtctga gcgctggcgc     60
   tgcccggacg ccgcggggtc cccgccagcc cagggcactc ggcgcgggga tctgcgcgcc    120
   tcgctctccc ttcccgatgc cgccgcccgg ctgctgatcg ccgcaccacc ttccctcatc    180
   ggcttgggtc cgtggaggtc cctgcagagg caggaagcct ccttaggaaa gcaggg atg    239
                                                                 Met
                                                                 1
   gag gta aat tgt tta aca cta aaa gac ctg atc agc ccc agg cag ccc      287
   Glu Val Asn Cys Leu Thr Leu Lys Asp Leu Ile Ser Pro Arg Gln Pro
           5                   10                  15
   aga cta gat ttt gca gtt gaa gat ggg gaa aat gca caa aag gaa aat      335
   Arg Leu Asp Phe Ala Val Glu Asp Gly Glu Asn Ala Gln Lys Glu Asn
       20                  25                  30
   ata ttt gtt gat cga tca agg atg gcc ccg aag act cca ata aaa aat      383
   Ile Phe Val Asp Arg Ser Arg Met Ala Pro Lys Thr Pro Ile Lys Asn
       35                  40                  45
   gaa cca att gat tta tcg aag caa aaa aaa ttt act cca gaa aga aat      431
   Glu Pro Ile Asp Leu Ser Lys Gln Lys Lys Phe Thr Pro Glu Arg Asn
   50                  55                  60                  65
   ccc att act cca gtt aag ttt gtt gac aga cag caa gcg gaa cca tgg      479
   Pro Ile Thr Pro Val Lys Phe Val Asp Arg Gln Gln Ala Glu Pro Trp
                   70                  75                  80
   aca ccc aca gct aac ctg aag atg ctc att agt gct gcc agc cca gat      527
   Thr Pro Thr Ala Asn Leu Lys Met Leu Ile Ser Ala Ala Ser Pro Asp
               85                  90                  95
   ata agg gac cgg gag aag aaa aag gga cta ttc cga ccc att gaa aac      575
   Ile Arg Asp Arg Glu Lys Lys Lys Gly Leu Phe Arg Pro Ile Glu Asn
           100                 105                 110
   aag gac gat gca ttt aca gat tct cta cag ctt gat gtt gtt ggg gac      623
   Lys Asp Asp Ala Phe Thr Asp Ser Leu Gln Leu Asp Val Val Gly Asp
       115                 120                 125
   agt gct gtg gac gaa ttt gaa aag caa agg cca agc aga aaa cag aaa      671
   Ser Ala Val Asp Glu Phe Glu Lys Gln Arg Pro Ser Arg Lys Gln Lys
   130 135 140 145
   agt tta gga ctc ctg tgc cag aag ttt cta gct cgc tat cca agt tat 719
   Ser Leu Gly Leu Leu Cys Gln Lys Phe Leu Ala Arg Tyr Pro Ser Tyr
   150 155 160
   ccc ttg tca act gag aaa act acc atc tcc cta gat gaa gtt gct gtc 767
   Pro Leu Ser Thr Glu Lys Thr Thr Ile Ser Leu Asp Glu Val Ala Val
   165 170 175
   agt ctt ggt gtg gaa agg aga cgc atc tat gac att gta aat gtg ctg 815
   Ser Leu Gly Val Glu Arg Arg Arg Ile Tyr Asp Ile Val Asn Val Leu
   180 185 190
   gag tcg ctg cat ctg gtc agc cgg gtg gct aag aat cag tat ggc tgg 863
   Glu Ser Leu His Leu Val Ser Arg Val Ala Lys Asn Gln Tyr Gly Trp
   195 200 205
   cat gga cgg cac agc ctg cca aaa acc ctg agg aac ctc cag aga cta 911
   His Gly Arg His Ser Leu Pro Lys Thr Leu Arg Asn Leu Gln Arg Leu
   210 215 220 225
   gga gag gag cag aaa tat gaa gag caa atg gcc tac ctc caa cag aaa 959
   Gly Glu Glu Gln Lys Tyr Glu Glu Gln Met Ala Tyr Leu Gln Gln Lys
   230 235 240
   gag ctg gac ctg ata gat tat aaa ttt gga gaa cgt aaa aaa gat ggt 1007
   Glu Leu Asp Leu Ile Asp Tyr Lys Phe Gly Glu Arg Lys Lys Asp Gly
   245 250 255
   gat cca gat tcc cag gaa caa cag tta ctg gat ttc tct gaa ccc gac 1055
   Asp Pro Asp Ser Gln Glu Gln Gln Leu Leu Asp Phe Ser Glu Pro Asp
   260 265 270
   tgt ccc tct tca tct gca aac agt aga aaa gac aag tct ctg aga att 1103
   Cys Pro Ser Ser Ser Ala Asn Ser Arg Lys Asp Lys Ser Leu Arg Ile
   275 280 285
   atg agc cag aag ttt gtc atg ctg ttc ctc gtc tcc aaa acc aag att 1151
   Met Ser Gln Lys Phe Val Met Leu Phe Leu Val Ser Lys Thr Lys Ile
   290 295 300 305
   gtc act ctg gat gtg gct gcc aaa ata ctg ata gaa gaa agc caa gat 1199
   Val Thr Leu Asp Val Ala Ala Lys Ile Leu Ile Glu Glu Ser Gln Asp
   310 315 320
   gcc cca gac cat agt aaa ttt aaa aca aag gta cga cgc ctc tat gac 1247
   Ala Pro Asp His Ser Lys Phe Lys Thr Lys Val Arg Arg Leu Tyr Asp
   325 330 335
   ata gcc aat gtt ctg acc agc ttg gct ctg ata aag aaa gtg cat gta 1295
   Ile Ala Asn Val Leu Thr Ser Leu Ala Leu Ile Lys Lys Val His Val
   340 345 350
   aca gaa gag cga ggt cgt aaa cca gcc ttc aag tgg atc ggg cct gtg 1343
   Thr Glu Glu Arg Gly Arg Lys Pro Ala Phe Lys Trp Ile Gly Pro Val
   355 360 365
   gac ttc agc tca agt gat gaa gaa ctg gtg gat gtt tct gca tct gtc 1391
   Asp Phe Ser Ser Ser Asp Glu Glu Leu Val Asp Val Ser Ala Ser Val
   370 375 380 385
   tta cca gaa ttg aaa aga gaa aca tat ggc cag att caa gtc tgt gca 1439
   Leu Pro Glu Leu Lys Arg Glu Thr Tyr Gly Gln Ile Gln Val Cys Ala
                       390                 395                 400
   aaa cag aag ctg gct cgc cat ggt tct ttt aac aca gtt cag gct tct      1487
   Lys Gln Lys Leu Ala Arg His Gly Ser Phe Asn Thr Val Gln Ala Ser
               405                 410                 415
   gag agg atc cag agg aaa gtg aac tca gaa ccg agc agc ccg tac aga      1535
   Glu Arg Ile Gln Arg Lys Val Asn Ser Glu Pro Ser Ser Pro Tyr Arg
           420                 425                 430
   gaa gaa caa gga tca ggt ggc tac tct tta gaa att gga agc ctg gca      1583
   Glu Glu Gln Gly Ser Gly Gly Tyr Ser Leu Glu Ile Gly Ser Leu Ala
       435                 440                 445
   gct gtc tat aga cag aaa ata gaa gac aat tca cag gga aaa gcc ttt      1631
   Ala Val Tyr Arg Gln Lys Ile Glu Asp Asn Ser Gln Gly Lys Ala Phe
   450                 455                 460                 465
   gcc agt aag aga gtg gtg cct cca tca agc agc ttg gac cct gtt gct      1679
   Ala Ser Lys Arg Val Val Pro Pro Ser Ser Ser Leu Asp Pro Val Ala
                   470                 475                 480
   cct ttc cct gtc ctc tct gtt gac cca gaa tat tgt gtt aat cct tta      1727
   Pro Phe Pro Val Leu Ser Val Asp Pro Glu Tyr Cys Val Asn Pro Leu
               485                 490                 495
   gcc cac cca gta ttt tct gtt gct cag acg gac ctg cag gca ttc tcc      1775
   Ala His Pro Val Phe Ser Val Ala Gln Thr Asp Leu Gln Ala Phe Ser
           500                 505                 510
   atg cag aac ggt ctg aat gga caa gtg gat gtc tca ctt gct tct gca      1823
   Met Gln Asn Gly Leu Asn Gly Gln Val Asp Val Ser Leu Ala Ser Ala
       515                 520                 525
   gcc tct gct gtg gag agc ctg aag cca gca ctc ctt gct ggc cag cct      1871
   Ala Ser Ala Val Glu Ser Leu Lys Pro Ala Leu Leu Ala Gly Gln Pro
   530                 535                 540                 545
   cta gtg tat gtg ccc tct gcc tca ctg ttc atg ctg tat gga agt ctg      1919
   Leu Val Tyr Val Pro Ser Ala Ser Leu Phe Met Leu Tyr Gly Ser Leu
                   550                 555                 560
   cag gag gga cca gcg tca ggg tca ggg tca gag agg gat gac aga agc      1967
   Gln Glu Gly Pro Ala Ser Gly Ser Gly Ser Glu Arg Asp Asp Arg Ser
               565                 570                 575
   tca gaa gcc cca gcc aca gta gag ctg tca tct gca ccc tca gct cag      2015
   Ser Glu Ala Pro Ala Thr Val Glu Leu Ser Ser Ala Pro Ser Ala Gln
           580                 585                 590
   aag cgc ctc tgt gag gag agg aaa cct cag gag gag gat gag cca gcc      2063
   Lys Arg Leu Cys Glu Glu Arg Lys Pro Gln Glu Glu Asp Glu Pro Ala
       595                 600                 605
   act aaa agg caa agt agg gaa tat gaa gac ggc ccg ctg tcg ctt gtc      2111
   Thr Lys Arg Gln Ser Arg Glu Tyr Glu Asp Gly Pro Leu Ser Leu Val
   610                 615                 620                 625
   atg ccc aag aaa ccc tca gat tcc aca gac ctt gcc tct ccc aag act      2159
   Met Pro Lys Lys Pro Ser Asp Ser Thr Asp Leu Ala Ser Pro Lys Thr
                   630                 635                 640
   atg ggt aac agg gca tct ata ccc ctc aaa gac att cat gtg aat ggc      2207
   Met Gly Asn Arg Ala Ser Ile Pro Leu Lys Asp Ile His Val Asn Gly
                   645                 650                 655
   caa ctc cct gct gca gaa gag att tca gga aag gca aca gca aac tct      2255
   Gln Leu Pro Ala Ala Glu Glu Ile Ser Gly Lys Ala Thr Ala Asn Ser
           660                 665                 670
   ctt gtt tct tct gag tgg gga aat cct tca aga aat aca gat gtt gaa      2303
   Leu Val Ser Ser Glu Trp Gly Asn Pro Ser Arg Asn Thr Asp Val Glu
       675                 680                 685
   aag cct tca aaa gaa aat gaa agc acc aaa gag cct tct ttg cta caa      2351
   Lys Pro Ser Lys Glu Asn Glu Ser Thr Lys Glu Pro Ser Leu Leu Gln
   690                 695                 700                 705
   tat ctt tgt gtg cag tct cct gca gga tta aat ggt ttc aat gta ctt      2399
   Tyr Leu Cys Val Gln Ser Pro Ala Gly Leu Asn Gly Phe Asn Val Leu
                   710                 715                 720
   tta tct ggc agt caa acc ccc cct act gtg ggc ccg tcc tca ggt cag      2447
   Leu Ser Gly Ser Gln Thr Pro Pro Thr Val Gly Pro Ser Ser Gly Gln
                   725                 730                 735
   ctg ccg tct ttc agt gtc cct tgc atg gtc tta cca tct cca cct ctg      2495
   Leu Pro Ser Phe Ser Val Pro Cys Met Val Leu Pro Ser Pro Pro Leu
           740                 745                 750
   ggc cct ttt cct gtt ctc tat tct cct gca atg ccg ggc ccg gtt tct      2543
   Gly Pro Phe Pro Val Leu Tyr Ser Pro Ala Met Pro Gly Pro Val Ser
       755                 760                 765
   tct act ctt ggt gct ctc cca aac aca gga cct gtg aat ttc agc ttg      2591
   Ser Thr Leu Gly Ala Leu Pro Asn Thr Gly Pro Val Asn Phe Ser Leu
   770                 775                 780                 785
   cct ggc ctt gga tca ata gcc cag ctt ctc gtc ggc ccc aca gct gtg      2639
   Pro Gly Leu Gly Ser Ile Ala Gln Leu Leu Val Gly Pro Thr Ala Val
                   790                 795                 800
   gtt aat cca aag tcg tcc aca ctc cct tct gca gac cct cag ctt cag      2687
   Val Asn Pro Lys Ser Ser Thr Leu Pro Ser Ala Asp Pro Gln Leu Gln
                   805                 810                 815
   agt cag ccc tca cta aac cta agt cca gtg atg tca agg tca cac agt      2735
   Ser Gln Pro Ser Leu Asn Leu Ser Pro Val Met Ser Arg Ser His Ser
           820                 825                 830
   gtc gtc caa caa cct gag tcc ccc gtt tac gtg gga cat cca gtc tca      2783
   Val Val Gln Gln Pro Glu Ser Pro Val Tyr Val Gly His Pro Val Ser
       835                 840                 845
   gta gta aaa tta cat cag tca cca gtt cca gtg acc ccc aag agc atc      2831
   Val Val Lys Leu His Gln Ser Pro Val Pro Val Thr Pro Lys Ser Ile
   850                 855                 860                 865
   caa cgc aca cat cgt gag acg ttt ttc aag aca ccc ggc agc ctt gga      2879
   Gln Arg Thr His Arg Glu Thr Phe Phe Lys Thr Pro Gly Ser Leu Gly
                   870                 875                 880
   gac cct gtc ctg aag aga aga gaa agg aac cag tca cga aac acc agc      2927
   Asp Pro Val Leu Lys Arg Arg Glu Arg Asn Gln Ser Arg Asn Thr Ser
                   885                 890                 895
   tcg gcc cag agg aga cta gaa atc ccc agc ggc ggc gct gac taa        2972
   Ser Ala Gln Arg Arg Leu Glu Ile Pro Ser Gly Gly Ala Asp
           900                 905                 910
   cctgccgctt tgccaggtgg gggtgggatc aaacgccctg agagtcccgg atgtccgagg   3032
   cgggatgcaa accatcccgt cctgagcacg ggtccttcct ctctctttca tccacacttc   3092
   tgttaacttc ccaccaccat caatcatctg atttcctgaa agtaattaat tgtgcattta   3152
   ataccagtta gagttccgac tctgcatggt gtcacagtga aagcgccgac tgacttatgg   3212
   ttttgattca agaatcgtct tattctggaa gtagatctga ataggctacc ggagccttgt   3272
   ttttctaaag gggggcgctg tctagcactt aactagggta agcattctta acatgtattt   3332
   ccacttgccc tgagtaaatc tgtggtgaga gaagcttcct ttctgcagtt taaaaaagct   3392
   actgcttcct taggcttcat caggaagcca ccttcagttg tgaatcctat ggtgttattt   3452
   attttgttcc tgaaatggga tttagtgcaa aaagtttaca actacagtct ttaacacatt   3512
   tttttcaggg tatgacgact tgaatgttta tacttttatt ctataatttg ccctgcactt   3572
   attttacaac ctagtaataa tgtggataaa tgtatctaca tgacacatgt caagaccaaa   3632
   ataactgtga atgacacacc ttgctgtaaa tgaactgtgc taaccctgac tgtgggcttg   3692
   agaacaaaga tgaactctag aactctagca gcctaactgc tgcttctcaa ataactgtgt   3752
   gaacagtgag atattactgt ttgtttctaa aaatcctact gtgcccagtt tccttcacta   3812
   catgccctgc attttttatt taaatattta gctgtagcgc catcagatat ggatgccttc   3872
   taacaattgc tgtttgtaaa ataaatcagg atggtagaaa gtgattatat ggaaaattgg   3932
   aacctggatg agaccttttc gttgaattct gaagagtaat gatgtgaaaa ttgatacagg   3992
   gcaagagatg attcttttgt ttttcttcta cttcatgtcc agaagagtaa gagggaaaat   4052
   ggacatatgt ttcatatcca agggtattca aactgtagtt agttggtacc tctgaaaaat   4112
   gagaatggtg agcgcacggg ttggttgttc tagcatgaat acaattctgg aaactgttat   4172
   gcaatttccc ttttttaacc cacattactt taggggtgca ttaagtcgcc aaactatact   4232
   agttctttgt attcctagac ttgctgatat ttacctctct cttgtctctt cagagtaaat   4292
   ggttcccttc tttccttcct actttccttc attctctctt ccttccctcc ttcctacttc   4352
   ttttcttcct tcctcttcct ctcttaaaac tatcttagat gtagaatcct ggtgtagggt   4412
   tttattttat ttttattttt tgacccaata aaatgttata tgaaagaatg aaaatattaa   4472
   tttaagagac tctgggagtc tgaataaagt agctttatat taactacagg ataatattag   4532
   ccttattacc cccacaagat tttttaaaac ttgaggtagg tagctacatt aaataaattt   4592
   gctacttata taaaaatttt tatcaacact aaacttttaa agtttacaag tttttttttt    4652
   cttttttaca gtcttctata gagttaggtt aaaaatgtgg ttctaaccat caacaattgc   4712
   atggttaaat gaccctgaac taaaactgat gggttcccta tcaaaacaaa taaaaatata   4772
   cctttttcag gtttcaatct gtgcagggta tatgcatgtt aattctacca tgcttaagaa   4832
   cttccacaaa atatttcatg gagaggtctg catttagacg gaaacagaaa ttgcttttcc   4892
   cctcactgtt cctgaatgct ctatacttgt tttaacattt ttgctatctt tttttattat   4952
   tctgatcatg atatgaccat ttaacctcag aattcataat tcctgagggg tgttaagaag   5012
   cagtcccatt ggtgaggata ttatgacttg gtgaccattc ttaggagtag aaaaccaagg   5072
   acaattgctt ctgtattcag tatccacttc ttaatgtggc tttatatgta aaaataataa   5132
   tgcagtggtt gtttctgtca ggaaaataaa tcttacagaa caactggtgg aattgaagct   5192
   gctgcgctag acttggatat tttgggtagt gaagaagcaa tggcaatctt gagtctatta   5252
   ttgtataatt tagtaaaaga aaaaaataat cgttggtggt cctactaaga gaatgcagct   5312
   tttttgagtt gtcacagagg ctgtgtgtgc cctacactga ccagggtttg taaaaccctt   5372
   tcattctggt acaagagtcg ggggtataac ttttatactt gaatctacct accaagttta   5432
   catttctcaa ttcctttttg taaggtgcta tttctgtatt taaataactt tcttttaacg   5492
   taaagctgct ttctgcttat cttattgcac tgctagttgt atgtaggtat taattttatt   5552
   gctgcttact gcttttgttt tcttattatt tagctctgct ctttttccta atggctatat   5612
   tatctatagc tatttacttg taactgtact acatgtaaac tgattttttg ttctgatttt   5672
   ttttctaata tttttaggaa aatattaagc tttataaaat agcaataaaa aataattcat   5732
   ttaagaagaa                                                           5742
<210> 54
   <211> 911
   <212> PRT
   <213> Homo sapiens
<400> 54
   Met Glu Val Asn Cys Leu Thr Leu Lys Asp Leu Ile Ser Pro Arg Gln
   1               5                   10                  15
   Pro Arg Leu Asp Phe Ala Val Glu Asp Gly Glu Asn Ala Gln Lys Glu
               20                  25                  30
   Asn Ile Phe Val Asp Arg Ser Arg Met Ala Pro Lys Thr Pro Ile Lys
           35                  40                  45
   Asn Glu Pro Ile Asp Leu Ser Lys Gln Lys Lys Phe Thr Pro Glu Arg
       50                  55                  60
   Asn Pro Ile Thr Pro Val Lys Phe Val Asp Arg Gln Gln Ala Glu Pro
   65                  70                  75                  80
   Trp Thr Pro Thr Ala Asn Leu Lys Met Leu Ile Ser Ala Ala Ser Pro
                   85                  90                  95
   Asp Ile Arg Asp Arg Glu Lys Lys Lys Gly Leu Phe Arg Pro Ile Glu
               100                 105                 110
   Asn Lys Asp Asp Ala Phe Thr Asp Ser Leu Gln Leu Asp Val Val Gly
           115                 120                 125
   Asp Ser Ala Val Asp Glu Phe Glu Lys Gln Arg Pro Ser Arg Lys Gln
       130                 135                 140
   Lys Ser Leu Gly Leu Leu Cys Gln Lys Phe Leu Ala Arg Tyr Pro Ser
   145                 150                 155                 160
   Tyr Pro Leu Ser Thr Glu Lys Thr Thr Ile Ser Leu Asp Glu Val Ala
                   165                 170                 175
   Val Ser Leu Gly Val Glu Arg Arg Arg Ile Tyr Asp Ile Val Asn Val
               180                 185                 190
   Leu Glu Ser Leu His Leu Val Ser Arg Val Ala Lys Asn Gln Tyr Gly
           195                 200                 205
   Trp His Gly Arg His Ser Leu Pro Lys Thr Leu Arg Asn Leu Gln Arg
       210                 215                 220
   Leu Gly Glu Glu Gln Lys Tyr Glu Glu Gln Met Ala Tyr Leu Gln Gln
   225                 230                 235                 240
   Lys Glu Leu Asp Leu Ile Asp Tyr Lys Phe Gly Glu Arg Lys Lys Asp
                   245                 250                 255
   Gly Asp Pro Asp Ser Gln Glu Gln Gln Leu Leu Asp Phe Ser Glu Pro
               260                 265                 270
   Asp Cys Pro Ser Ser Ser Ala Asn Ser Arg Lys Asp Lys Ser Leu Arg
           275                 280                 285
   Ile Met Ser Gln Lys Phe Val Met Leu Phe Leu Val Ser Lys Thr Lys
       290                 295                 300
   Ile Val Thr Leu Asp Val Ala Ala Lys Ile Leu Ile Glu Glu Ser Gln
   305                 310                 315                 320
   Asp Ala Pro Asp His Ser Lys Phe Lys Thr Lys Val Arg Arg Leu Tyr
                   325                 330                 335
   Asp Ile Ala Asn Val Leu Thr Ser Leu Ala Leu Ile Lys Lys Val His
   340 345 350
   Val Thr Glu Glu Arg Gly Arg Lys Pro Ala Phe Lys Trp Ile Gly Pro
   355 360 365
   Val Asp Phe Ser Ser Ser Asp Glu Glu Leu Val Asp Val Ser Ala Ser
   370 375 380
   Val Leu Pro Glu Leu Lys Arg Glu Thr Tyr Gly Gln Ile Gln Val Cys
   385 390 395 400
   Ala Lys Gln Lys Leu Ala Arg His Gly Ser Phe Asn Thr Val Gln Ala
   405 410 415
   Ser Glu Arg Ile Gln Arg Lys Val Asn Ser Glu Pro Ser Ser Pro Tyr
   420 425 430
   Arg Glu Glu Gln Gly Ser Gly Gly Tyr Ser Leu Glu Ile Gly Ser Leu
   435 440 445
   Ala Ala Val Tyr Arg Gln Lys Ile Glu Asp Asn Ser Gln Gly Lys Ala
   450 455 460
   Phe Ala Ser Lys Arg Val Val Pro Pro Ser Ser Ser Leu Asp Pro Val
   465 470 475 480
   Ala Pro Phe Pro Val Leu Ser Val Asp Pro Glu Tyr Cys Val Asn Pro
   485 490 495
   Leu Ala His Pro Val Phe Ser Val Ala Gln Thr Asp Leu Gln Ala Phe
   500 505 510
   Ser Met Gln Asn Gly Leu Asn Gly Gln Val Asp Val Ser Leu Ala Ser
   515 520 525
   Ala Ala Ser Ala Val Glu Ser Leu Lys Pro Ala Leu Leu Ala Gly Gln
   530 535 540
   Pro Leu Val Tyr Val Pro Ser Ala Ser Leu Phe Met Leu Tyr Gly Ser
   545 550 555 560
   Leu Gln Glu Gly Pro Ala Ser Gly Ser Gly Ser Glu Arg Asp Asp Arg
   565 570 575
   Ser Ser Glu Ala Pro Ala Thr Val Glu Leu Ser Ser Ala Pro Ser Ala
   580 585 590
   Gln Lys Arg Leu Cys Glu Glu Arg Lys Pro Gln Glu Glu Asp Glu Pro
   595 600 605
   Ala Thr Lys Arg Gln Ser Arg Glu Tyr Glu Asp Gly Pro Leu Ser Leu
   610 615 620
   Val Met Pro Lys Lys Pro Ser Asp Ser Thr Asp Leu Ala Ser Pro Lys
   625 630 635 640
   Thr Met Gly Asn Arg Ala Ser Ile Pro Leu Lys Asp Ile His Val Asn
   645 650 655
   Gly Gln Leu Pro Ala Ala Glu Glu Ile Ser Gly Lys Ala Thr Ala Asn
   660 665 670
   Ser Leu Val Ser Ser Glu Trp Gly Asn Pro Ser Arg Asn Thr Asp Val
   675 680 685
   Glu Lys Pro Ser Lys Glu Asn Glu Ser Thr Lys Glu Pro Ser Leu Leu
   690 695 700
   Gln Tyr Leu Cys Val Gln Ser Pro Ala Gly Leu Asn Gly Phe Asn Val
   705 710 715 720
   Leu Leu Ser Gly Ser Gln Thr Pro Pro Thr Val Gly Pro Ser Ser Gly
   725 730 735
   Gln Leu Pro Ser Phe Ser Val Pro Cys Met Val Leu Pro Ser Pro Pro
   740 745 750
   Leu Gly Pro Phe Pro Val Leu Tyr Ser Pro Ala Met Pro Gly Pro Val
   755 760 765
   Ser Ser Thr Leu Gly Ala Leu Pro Asn Thr Gly Pro Val Asn Phe Ser
   770 775 780
   Leu Pro Gly Leu Gly Ser Ile Ala Gln Leu Leu Val Gly Pro Thr Ala
   785 790 795 800
   Val Val Asn Pro Lys Ser Ser Thr Leu Pro Ser Ala Asp Pro Gln Leu
   805 810 815
   Gln Ser Gln Pro Ser Leu Asn Leu Ser Pro Val Met Ser Arg Ser His
   820 825 830
   Ser Val Val Gln Gln Pro Glu Ser Pro Val Tyr Val Gly His Pro Val
   835 840 845
   Ser Val Val Lys Leu His Gln Ser Pro Val Pro Val Thr Pro Lys Ser
   850 855 860
   Ile Gln Arg Thr His Arg Glu Thr Phe Phe Lys Thr Pro Gly Ser Leu
   865 870 875 880
   Gly Asp Pro Val Leu Lys Arg Arg Glu Arg Asn Gln Ser Arg Asn Thr
   885 890 895
   Ser Ser Ala Gln Arg Arg Leu Glu Ile Pro Ser Gly Gly Ala Asp
   900 905 910
<210> 55
   <211> 2112
   <212> DNA
   <213> Homo sapiens
<220>
   <221> CDS
   <222> (335)..(973)
   <223>
<400> 55
   ggagctgttt acccccactc taataggggt tcaatataaa aagccggcag agagctgtcc     60
   aagtcagacg cgcctctgca tctgcgccag gcgaacgggt cctgcgcctc ctgcagtccc    120
   agctctccac cgccgcgtgc gcctgcagac gctccgctcg ctgccttctc tcctggcagg    180
   cgctgccttt tctccccgtt aaaagggcac ttgggctgaa ggatcgcttt gagatctgag    240
   gaacccgcag cgctttgagg gacctgaagc tgtttttctt cgttttcctt tgggttcagt    300
   ttgaacggga ggtttttgat cccttttttt caga atg gat tat ttg ctc atg att    355
                                         Met Asp Tyr Leu Leu Met Ile
                                         1               5
   ttc tct ctg ctg ttt gtg gct tgc caa gga gct cca gaa aca gca gtc      403
   Phe Ser Leu Leu Phe Val Ala Cys Gln Gly Ala Pro Glu Thr Ala Val
           10                  15                  20
   tta ggc gct gag ctc agc gcg gtg ggt gag aac ggc ggg gag aaa ccc      451
   Leu Gly Ala Glu Leu Ser Ala Val Gly Glu Asn Gly Gly Glu Lys Pro
       25                  30                  35
   act ccc agt cca ccc tgg cgg ctc cgc cgg tcc aag cgc tgc tcc tgc      499
   Thr Pro Ser Pro Pro Trp Arg Leu Arg Arg Ser Lys Arg Cys Ser Cys
   40                  45                  50                  55
   tcg tcc ctg atg gat aaa gag tgt gtc tac ttc tgc cac ctg gac atc      547
   Ser Ser Leu Met Asp Lys Glu Cys Val Tyr Phe Cys His Leu Asp Ile
                   60                  65                  70
   att tgg gtc aac act ccc gag cac gtt gtt ccg tat gga ctt gga agc      595
   Ile Trp Val Asn Thr Pro Glu His Val Val Pro Tyr Gly Leu Gly Ser
   75 80 85
   cct agg tcc aag aga gcc ttg gag aat tta ctt ccc aca aag gca aca 643
   Pro Arg Ser Lys Arg Ala Leu Glu Asn Leu Leu Pro Thr Lys Ala Thr
   90 95 100
   gac cgt gaa aat aga tgc caa tgt gct agc caa aaa gac aag aag tgc 691
   Asp Arg Glu Asn Arg Cys Gln Cys Ala Ser Gln Lys Asp Lys Lys Cys
   105 110 115
   tgg aat ttt tgc caa gca gga aaa gaa ctc agg gct gaa gac att atg 739
   Trp Asn Phe Cys Gln Ala Gly Lys Glu Leu Arg Ala Glu Asp Ile Met
   120 125 130 135
   gag aaa gac tgg aat aat cat aag aaa gga aaa gac tgt tcc aag ctt 787
   Glu Lys Asp Trp Asn Asn His Lys Lys Gly Lys Asp Cys Ser Lys Leu
   140 145 150
   ggg aaa aag tgt att tat cag cag tta gtg aga gga aga aaa atc aga 835
   Gly Lys Lys Cys Ile Tyr Gln Gln Leu Val Arg Gly Arg Lys Ile Arg
   155 160 165
   aga agt tca gag gaa cac cta aga caa acc agg tcg gag acc atg aga 883
   Arg Ser Ser Glu Glu His Leu Arg Gln Thr Arg Ser Glu Thr Met Arg
   170 175 180
   aac agc gtc aaa tca tct ttt cat gat ccc aag ctg aaa ggc aag ccc 931
   Asn Ser Val Lys Ser Ser Phe His Asp Pro Lys Leu Lys Gly Lys Pro
   185 190 195
   tcc aga gag cgt tat gtg acc cac aac cga gca cat tgg tga 973
   Ser Arg Glu Arg Tyr Val Thr His Asn Arg Ala His Trp
   200 205 210
   cagaccttcg gggcctgtct gaagccatag cctccacgga gagccctgtg gccgactctg 1033
   cactctccac cctggctggg atcagagcag gagcatcctc tgctggttcc tgactggcaa 1093
   aggaccagcg tcctcgttca aaacattcca agaaaggtta aggagttccc ccaaccatct 1153
   tcactggctt ccatcagtgg taactgcttt ggtctcttct ttcatctggg gatgacaatg 1213
   gacctctcag cagaaacaca cagtcacatt cgaattcggg tggcatcctc cggagagaga 1273
   gagaggaagg agattccaca caggggtgga gtttctgacg aaggtcctaa gggagtgttt 1333
   gtgtctgact caggcgcctg gcacatttca gggagaaact ccaaagtcca cacaaagatt 1393
   ttctaaggaa tgcacaaatt gaaaacacac tcaaaagaca aacatgcaag taaagaaaaa 1453
   aaaaagaaag acttttgttt aaatttgtaa aatgcaaaac tgaatgaaac tgttactacc 1513
   ataaatcagg atatgtttca tgaatatgag tctacctcac ctatattgca ctctggcaga 1573
   agtatttccc acatttaatt attgcctccc caaactcttc ccacccctgc tgccccttcc 1633
   tccatccccc atactaaatc ctagcctcgt agaagtctgg tctaatgtgt cagcagtaga 1693
   tataatattt tcatggtaat ctactagctc tgatccataa gaaaaaaaag atcattaaat 1753
   caggagattc cctgtccttg atttttggag acacaatggt atagggttgt ttatgaaata 1813
   tattgaaaag taagtgtttg ttacgcttta aagcagtaaa attattttcc tttatataac 1873
   cggctaatga aagaggttgg attgaatttt gatgtactta tttttttata gatatttata 1933
   ttcaaacaat ttattcctta tatttaccat gttaaatatc tgtttgggca ggccatattg 1993
   gtctatgtat ttttaaaata tgtatttcta aatgaaattg agaacatgct ttgttttgcc 2053
   tgtcaaggta atgactttag aaaataaata tttttttcct tactgtaaaa aaaaaaaaa 2112
<210> 56
   <211> 212
   <212> PRT
   <213> Homo sapiens
<400> 56
   Met Asp Tyr Leu Leu Met Ile Phe Ser Leu Leu Phe Val Ala Cys Gln
   1               5                   10                  15
   Gly Ala Pro Glu Thr Ala Val Leu Gly Ala Glu Leu Ser Ala Val Gly
               20                  25                  30
   Glu Asn Gly Gly Glu Lys Pro Thr Pro Ser Pro Pro Trp Arg Leu Arg
           35                  40                  45
   Arg Ser Lys Arg Cys Ser Cys Ser Ser Leu Met Asp Lys Glu Cys Val
       50                  55                  60
   Tyr Phe Cys His Leu Asp Ile Ile Trp Val Asn Thr Pro Glu His Val
   65                  70                  75                  80
   Val Pro Tyr Gly Leu Gly Ser Pro Arg Ser Lys Arg Ala Leu Glu Asn
                   85                  90                  95
   Leu Leu Pro Thr Lys Ala Thr Asp Arg Glu Asn Arg Cys Gln Cys Ala
               100                 105                 110
   Ser Gln Lys Asp Lys Lys Cys Trp Asn Phe Cys Gln Ala Gly Lys Glu
           115                 120                 125
   Leu Arg Ala Glu Asp Ile Met Glu Lys Asp Trp Asn Asn His Lys Lys
       130                 135                 140
   Gly Lys Asp Cys Ser Lys Leu Gly Lys Lys Cys Ile Tyr Gln Gln Leu
   145                 150                 155                 160
   Val Arg Gly Arg Lys Ile Arg Arg Ser Ser Glu Glu His Leu Arg Gln
                   165                 170                 175
   Thr Arg Ser Glu Thr Met Arg Asn Ser Val Lys Ser Ser Phe His Asp
               180                 185                 190
   Pro Lys Leu Lys Gly Lys Pro Ser Arg Glu Arg Tyr Val Thr His Asn
           195                 200                 205
   Arg Ala His Trp
       210
<210> 57
   <211> 3182
   <212> DNA
   <213> Homo sapiens
<220>
   <221> CDS
   <222> (935)..(2206)
   <223>
<400> 57
   ggcaggcgag ccggacgccg ctcgcagcac cggagagggc gcactgcaaa ggcgggcagc      60
   agaccgtgga gagcccggga gcggagctgg acaccgcctc ggagggaaga aatgaggtag     120
   cggcggttcc cggacccggc catgcccgtc ccctgttctc ggagcccagc gccgtctcgg     180
   ccaggccagc ccggacactg agcgggccga gcgcgagtcc ccggcgtccg gcggagcgaa     240
   gatgcagtga gtccccgcgg gactgctgcg cggggcccgc cgcggccagc cggacccagc     300
   atccgaccgc actttgggcg agctgctgac ttgagaccag cccaaacggg gggctttcca     360
   tctccagcac ccctcggagg tggggagcac cggcccctag gcacactcgc tgtagagttt     420
   ccgcgggttt tggccccagt cctgagggtt gtgtgtgtta ggggacccac ctcacgttcg     480
   ccgaggagtt cctgcatatt cttgcaccat cccgggtgct gttgctgggg ctctctttat     540
   ttgcacgcgc gcttctagct tttttcctga cattttccac tctacgctcg ttctttctcc     600
   cttgcatttt gttgcttgcc tgagactctt tgctctcgcc cttgcccagg ctggggtaat     660
   tctgtgtgcg cgcgtgtctc ccccgcacca accttttttg cacactcgaa gctgaatatt     720
   ttctttttta gagaatttac ccgcacctcc tgcggggttc ctaagcactc tctctgctcc     780
   cctcccccca actccctacc acagggccgc tcccagtagt tttattcttc gatcttgccc     840
   gggccgagcc tggcaggggc cggtggctca gcgggcctcg cacccggcgc tccgccgccg     900
   ccgccgccca gctgcgccgg ggcgccctcc ggag atg ctg ccg tgg aag aag cac     955
                                        Met Leu Pro Trp Lys Lys His
                                        1               5
   aag ttc gag ctg ctg gcc gag gcg ccg ccg cgg cag gcg tcc aag ccc     1003
   Lys Phe Glu Leu Leu Ala Glu Ala Pro Pro Arg Gln Ala Ser Lys Pro
       10                  15                  20
   aag ggc tac gct gtg agc ctg cac tac tcg gcg ctc agc tcg ctg gcg     1051
   Lys Gly Tyr Ala Val Ser Leu His Tyr Ser Ala Leu Ser Ser Leu Ala
       25                  30                  35
   cgg gcg tgc ccc gaa ggc gcg ctt agc cgg gtg ggc agc atg ttc cgc      1099
   Arg Ala Cys Pro Glu Gly Ala Leu Ser Arg Val Gly Ser Met Phe Arg
   40                  45                  50                  55
   tcc aag cgc aag aag ctg cac atc act agc gag gac cca act tac acc      1147
   Ser Lys Arg Lys Lys Leu His Ile Thr Ser Glu Asp Pro Thr Tyr Thr
                   60                  65                  70
   gtg ctc tac ctg ggc aat gcc acc acc atc cag gcg cgc ggc gac ggc      1195
   Val Leu Tyr Leu Gly Asn Ala Thr Thr Ile Gln Ala Arg Gly Asp Gly
               75                  80                  85
   tgc acc gac ctt gct gtg ggc aag atc tgg agc aag agc gag gcg ggc      1243
   Cys Thr Asp Leu Ala Val Gly Lys Ile Trp Ser Lys Ser Glu Ala Gly
           90                  95                  100
   cgt cag ggc acc aag atg aag ctg acg gtg agt gcg cag ggt atc cgc      1291
   Arg Gln Gly Thr Lys Met Lys Leu Thr Val Ser Ala Gln Gly Ile Arg
       105                 110                 115
   atg gtg cac gcc gag gag cgc gcg ctg cgc cgc ccg ggc cac ctc tac      1339
   Met Val His Ala Glu Glu Arg Ala Leu Arg Arg Pro Gly His Leu Tyr
   120                 125                 130                 135
   ctg ctg cac cgc gtc acc tac tgc gtg gcc gac gcg cgg ctg ccc aag      1387
   Leu Leu His Arg Val Thr Tyr Cys Val Ala Asp Ala Arg Leu Pro Lys
                   140                 145                 150
   gtc ttc gcc tgg gtg tac cgg cac gag ctg aag cac aag gcc gtg atg      1435
   Val Phe Ala Trp Val Tyr Arg His Glu Leu Lys His Lys Ala Val Met
               155                 160                 165
   ctg cgc tgc cac gcc gtg ctg gtg tcc aag ccc gaa aag gcg cag gcc      1483
   Leu Arg Cys His Ala Val Leu Val Ser Lys Pro Glu Lys Ala Gln Ala
           170                 175                 180
   atg gcc ctg ctg ctc tac cag acg tcg gcc aac gcg ctg gcg gaa ttt      1531
   Met Ala Leu Leu Leu Tyr Gln Thr Ser Ala Asn Ala Leu Ala Glu Phe
       185                 190                 195
   aaa cgc ctc aag cgg cgg gac gac gcg cgt cac cag cag cag gag ctg      1579
   Lys Arg Leu Lys Arg Arg Asp Asp Ala Arg His Gln Gln Gln Glu Leu
   200                 205                 210                 215
   gtg ggc gca cac acc atc ccg cta gtg ccg ctg cgc aag ctg ctc cta      1627
   Val Gly Ala His Thr Ile Pro Leu Val Pro Leu Arg Lys Leu Leu Leu
                   220                 225                 230
   cac gga ccc tgc tgc tat aaa ccg ccg gtg gag cgc agc cgc agc gcg      1675
   His Gly Pro Cys Cys Tyr Lys Pro Pro Val Glu Arg Ser Arg Ser Ala
               235                 240                 245
   ccc aag ctt ggc tcc atc acc gag gac ctg ctc ggc gaa cag ctg gag      1723
   Pro Lys Leu Gly Ser Ile Thr Glu Asp Leu Leu Gly Glu Gln Leu Glu
           250                 255                 260
   cag gag ctg cag gag gaa gag gaa gag gag caa ccc gag ggc tgc ccg      1771
   Gln Glu Leu Gln Glu Glu Glu Glu Glu Glu Gln Pro Glu Gly Cys Pro
       265                 270                 275
   gag gag gag gag aac cgt gcg gca gag gga gat cca gca gag gag gag      1819
   Glu Glu Glu Glu Asn Arg Ala Ala Glu Gly Asp Pro Ala Glu Glu Glu
   280                 285                 290                 295
   gcc gag gcg cag cgt gcg cta gtg gtc gcc atg cac ttt gag tgc ggg     1867
   Ala Glu Ala Gln Arg Ala Leu Val Val Ala Met His Phe Glu Cys Gly
                   300                 305                 310
   gac ttg ttg gat act ctg gag aat ggc cgt ggg gag gcg cta gga ggc     1915
   Asp Leu Leu Asp Thr Leu Glu Asn Gly Arg Gly Glu Ala Leu Gly Gly
               315                 320                 325
   ggc ggg ggc tcc ctg ggc ccg ggg gcc ggg ccg ccg cct ctg ctg ctg     1963
   Gly Gly Gly Ser Leu Gly Pro Gly Ala Gly Pro Pro Pro Leu Leu Leu
           330                 335                 340
   ggc agc gcc tcc gac atg aag gct gag ctg tcg caa ctt att agc gac     2011
   Gly Ser Ala Ser Asp Met Lys Ala Glu Leu Ser Gln Leu Ile Ser Asp
       345                 350                 355
   ctg ggc gag ctc agc ttc ggc aac gac gtg cgc acc ctg cag gcc gac     2059
   Leu Gly Glu Leu Ser Phe Gly Asn Asp Val Arg Thr Leu Gln Ala Asp
   360                 365                 370                 375
   ttg cgg gtg acg cgc ctg ctg tca ggc gac agc acg ggc agc gag agc     2107
   Leu Arg Val Thr Arg Leu Leu Ser Gly Asp Ser Thr Gly Ser Glu Ser
                   380                 385                 390
   tcc atc gag ggc ggg ggc cct gac gcc acc tcc gcc acc gcc ggg gac     2155
   Ser Ile Glu Gly Gly Gly Pro Asp Ala Thr Ser Ala Thr Ala Gly Asp
               395                 400                 405
   tcg tcc cgc cag gcc gac ggc gcc agt gca gac gag ccc cac tcg ggc     2203
   Ser Ser Arg Gln Ala Asp Gly Ala Ser Ala Asp Glu Pro His Ser Gly
           410                 415                 420
   tga gctcctccgc gcgtcgccgg cgctccaccg tggctaccca tccgtggtcc          2256
   cgacaacctc cctgtccctt gcccgccccc aggaaggggg aaatggggca tttggggccc   2316
   agacctacac ttggagccca ggtccagcgt tcccccgacc gctttcccct acctcccggc   2376
   ccccgctccc gccccagcac ttttggctct gttgcgcgtg gggatgcggg gagatttgag   2436
   aggggaaaac cccgccagga gggagagaga ggcacccctc tgggatgcgg gtgagggaag    2496
   gttggctgaa gttcctagtc tcaggccgta ggtgcctggc cagtttcctg tttgtggggc   2556
   agctggggcc tgaggaggag gggttcactt cctcctccca ccccctggga gcggccctgc   2616
   gctgtcactg acatctcatt aaaaaaaaaa aaaaatttgc tctcaaggtg tttgaggctt   2676
   taatgcaacc ctttagccct tggttctttt tggtgcaaga attctggctg tttacctcag   2736
   actcagaccc ctgaaatgtt gccaaattct tcaaataact gtttgggggg tggggggaga  2796
   tgaaagagag tcgcgttttg tttacagtta aagacatcca atatcttaaa aaggagtttt   2856
   cctttagaaa cacacacacc cttcctcttg ctcaaaagat ctcactccat gatactgtgt   2916
   aaaatatttt tgcactgttg tgaagtattt ttgacttttt tctgtacata actgtgttct   2976
   cagagctgaa tgtttatatc ttttgctgtg caaaagaaac atgtaaaatg ttgttcagtt   3036
   gtatatacag aaatgtgtat aaaacatttt gttatttttt aaaagtagca ctgttctggt   3096
   tctgtttgca cgccagtggg gagagaataa agaggaaaat ttaacagaaa aaaaaaaaaa 3156
   aaaaaaaaaa aaaaaaaaaa aaaaaa 3182
<210> 58
   <211> 423
   <212> PRT
   <213> Homo sapiens
<400> 58
   Met Leu Pro Trp Lys Lys His Lys Phe Glu Leu Leu Ala Glu Ala Pro
   1               5                   10                  15
   Pro Arg Gln Ala Ser Lys Pro Lys Gly Tyr Ala Val Ser Leu His Tyr
               20                  25                  30
   Ser Ala Leu Ser Ser Leu Ala Arg Ala Cys Pro Glu Gly Ala Leu Ser
           35                  40                  45
   Arg Val Gly Ser Met Phe Arg Ser Lys Arg Lys Lys Leu His Ile Thr
       50                  55                  60
   Ser Glu Asp Pro Thr Tyr Thr Val Leu Tyr Leu Gly Asn Ala Thr Thr
   65                  70                  75                  80
   Ile Gln Ala Arg Gly Asp Gly Cys Thr Asp Leu Ala Val Gly Lys Ile
                   85                  90                  95
   Trp Ser Lys Ser Glu Ala Gly Arg Gln Gly Thr Lys Met Lys Leu Thr
               100                 105                 110
   Val Ser Ala Gln Gly Ile Arg Met Val His Ala Glu Glu Arg Ala Leu
           115                 120                 125
   Arg Arg Pro Gly His Leu Tyr Leu Leu His Arg Val Thr Tyr Cys Val
       130                 135                 140
   Ala Asp Ala Arg Leu Pro Lys Val Phe Ala Trp Val Tyr Arg His Glu
   145                 150                 155                 160
   Leu Lys His Lys Ala Val Met Leu Arg Cys His Ala Val Leu Val Ser
                   165                 170                 175
   Lys Pro Glu Lys Ala Gln Ala Met Ala Leu Leu Leu Tyr Gln Thr Ser
               180                 185                 190
   Ala Asn Ala Leu Ala Glu Phe Lys Arg Leu Lys Arg Arg Asp Asp Ala
           195                 200                 205
   Arg His Gln Gln Gln Glu Leu Val Gly Ala His Thr Ile Pro Leu Val
       210                 215                 220
   Pro Leu Arg Lys Leu Leu Leu His Gly Pro Cys Cys Tyr Lys Pro Pro
   225                 230                 235                 240
   Val Glu Arg Ser Arg Ser Ala Pro Lys Leu Gly Ser Ile Thr Glu Asp
                   245                 250                 255
   Leu Leu Gly Glu Gln Leu Glu Gln Glu Leu Gln Glu Glu Glu Glu Glu
               260                 265                 270
   Glu Gln Pro Glu Gly Cys Pro Glu Glu Glu Glu Asn Arg Ala Ala Glu
           275                 280                 285
   Gly Asp Pro Ala Glu Glu Glu Ala Glu Ala Gln Arg Ala Leu Val Val
       290                 295                 300
   Ala Met His Phe Glu Cys Gly Asp Leu Leu Asp Thr Leu Glu Asn Gly
   305                 310                 315                 320
   Arg Gly Glu Ala Leu Gly Gly Gly Gly Gly Ser Leu Gly Pro Gly Ala
                   325                 330                 335
   Gly Pro Pro Pro Leu Leu Leu Gly Ser Ala Ser Asp Met Lys Ala Glu
               340                 345                 350
   Leu Ser Gln Leu Ile Ser Asp Leu Gly Glu Leu Ser Phe Gly Asn Asp
           355                 360                 365
   Val Arg Thr Leu Gln Ala Asp Leu Arg Val Thr Arg Leu Leu Ser Gly
       370                 375                 380
   Asp Ser Thr Gly Ser Glu Ser Ser Ile Glu Gly Gly Gly Pro Asp Ala
   385                 390                 395                 400
   Thr Ser Ala Thr Ala Gly Asp Ser Ser Arg Gln Ala Asp Gly Ala Ser
                   405                 410                 415
   Ala Asp Glu Pro His Ser Gly
               420
<210> 59
   <211> 2529
   <212> DNA
   <213> Homo sapiens
<220>
   <221> CDS
   <222> (203)..(1996)
   <223>
<400> 59
   ggaggcgagc gaacagaggg agggacccgc ccgccgcgcc ccggccgctg ggcatgtgtg     60
   tccgcaggcg cccgacgctg ccgatgtccc ggggctgagc cgcgcccagg tgtcccggac    120
   agtgcgtgcg agcgtgtgtg tccgcgcagg cgagcaccgc gccggccctg agcctcccgc    180
   tcgctcccca cggccgcggt gc atg ttc gcc tcc tgc cac tgt gtg ccg aga     232
                            Met Phe Ala Ser Cys His Cys Val Pro Arg
                            1               5                   10
   ggc agg agg acc atg aaa atg atc cac ttt cgg agc tcc agc gtc aaa      280
   Gly Arg Arg Thr Met Lys Met Ile His Phe Arg Ser Ser Ser Val Lys
                   15                  20                  25
   tcg ctc agc cag gag atg aga tgc acc atc cgg ctg ctg gac gac tcg      328
   Ser Leu Ser Gln Glu Met Arg Cys Thr Ile Arg Leu Leu Asp Asp Ser
               30                  35                  40
   gag atc tcc tgc cac atc cag agg gaa acc aaa ggg cag ttt ctc att      376
   Glu Ile Ser Cys His Ile Gln Arg Glu Thr Lys Gly Gln Phe Leu Ile
           45                  50                  55
   gac cac atc tgc aac tac tac agc ctg ctg gag aag gac tac ttt ggc      424
   Asp His Ile Cys Asn Tyr Tyr Ser Leu Leu Glu Lys Asp Tyr Phe Gly
       60                  65                  70
   att cgc tat gtg gac cca gag aag caa agg cac tgg ctt gaa cct aac      472
   Ile Arg Tyr Val Asp Pro Glu Lys Gln Arg His Trp Leu Glu Pro Asn
   75                  80                  85                  90
   aag tcc atc ttc aag caa atg aaa act cat cca cca tac acc atg tgc      520
   Lys Ser Ile Phe Lys Gln Met Lys Thr His Pro Pro Tyr Thr Met Cys
                   95                  100                 105
   ttt aga gtg aaa ttc tac cca cat gaa ccc ttg aag att aaa gaa gag      568
   Phe Arg Val Lys Phe Tyr Pro His Glu Pro Leu Lys Ile Lys Glu Glu
               110                 115                 120
   ctc aca aga tac ctt tta tac ctt cag att aaa agg gac att ttt cat      616
   Leu Thr Arg Tyr Leu Leu Tyr Leu Gln Ile Lys Arg Asp Ile Phe His
           125                 130                 135
   ggc cgc ctg ctg tgc tcc ttt tct gat gct gcc tac ctg ggt gcc tgt      664
   Gly Arg Leu Leu Cys Ser Phe Ser Asp Ala Ala Tyr Leu Gly Ala Cys
       140                 145                 150
   att gtt caa gct gag ctt ggt gat tac gat cct gat gag cat cct gag      712
   Ile Val Gln Ala Glu Leu Gly Asp Tyr Asp Pro Asp Glu His Pro Glu
   155                 160                 165                 170
   aat tac atc agt gag ttt gag att ttc ccc aag cag tca cag aag ctg      760
   Asn Tyr Ile Ser Glu Phe Glu Ile Phe Pro Lys Gln Ser Gln Lys Leu
                   175                 180                 185
   gaa aga aaa ata gtg gaa att cat aaa aat gaa ctc agg ggg cag agc      808
   Glu Arg Lys Ile Val Glu Ile His Lys Asn Glu Leu Arg Gly Gln Ser
               190                 195                 200
   cca cca gtt gct gaa ttt aac ttg ctc ctg aaa gct cac act ttg gaa      856
   Pro Pro Val Ala Glu Phe Asn Leu Leu Leu Lys Ala His Thr Leu Glu
           205                 210                 215
   acc tac ggg gtg gat cct cac cca tgc aag gat tca aca ggc aca aca      904
   Thr Tyr Gly Val Asp Pro His Pro Cys Lys Asp Ser Thr Gly Thr Thr
       220                 225                 230
   aca ttt tta gga ttc aca gct gca ggc ttt gtg gtc ttt cag gga aat      952
   Thr Phe Leu Gly Phe Thr Ala Ala Gly Phe Val Val Phe Gln Gly Asn
   235                 240                 245                 250
   aag aga atc cat ttg ata aaa tgg cca gat gtc tgc aaa ttg aag ttt     1000
   Lys Arg Ile His Leu Ile Lys Trp Pro Asp Val Cys Lys Leu Lys Phe
                   255                 260                 265
   gaa ggg aag aca ttt tat gtg att ggc acc cag aag gag aaa aaa gcc     1048
   Glu Gly Lys Thr Phe Tyr Val Ile Gly Thr Gln Lys Glu Lys Lys Ala
               270                 275                 280
   atg ttg gca ttc cat act tca aca cca gct gcc tgc aaa cat ctt tgg     1096
   Met Leu Ala Phe His Thr Ser Thr Pro Ala Ala Cys Lys His Leu Trp
           285                 290                 295
   aag tgt gga gtg gaa aac cag gcc ttt tat aag tat gca aaa tcc agt     1144
   Lys Cys Gly Val Glu Asn Gln Ala Phe Tyr Lys Tyr Ala Lys Ser Ser
       300                 305                 310
   cag atc aag act gta tca agc agc aag ata ttt ttt aaa gga agt aga     1192
   Gln Ile Lys Thr Val Ser Ser Ser Lys Ile Phe Phe Lys Gly Ser Arg
   315                 320                 325                 330
   ttt cga tat agt ggg aaa gtt gcc aaa gag gtg gtg gag gcc agt tcc     1240
   Phe Arg Tyr Ser Gly Lys Val Ala Lys Glu Val Val Glu Ala Ser Ser
                   335                 340                 345
   aag atc cag agg gag cct cct gag gtg cac aga gcc aac att act cag     1288
   Lys Ile Gln Arg Glu Pro Pro Glu Val His Arg Ala Asn Ile Thr Gln
               350                 355                 360
   agc cgc agt tcc cac tcc ttg aac aaa cag ctc atc att aac atg gaa     1336
   Ser Arg Ser Ser His Ser Leu Asn Lys Gln Leu Ile Ile Asn Met Glu
           365                 370                 375
   ccc ctg cag ccc ctg ctt cct tcc ccc agc gag caa gaa gaa gaa ctt     1384
   Pro Leu Gln Pro Leu Leu Pro Ser Pro Ser Glu Gln Glu Glu Glu Leu
       380                 385                 390
   cct ctg ggt gag ggt gtt cca ttg cct aaa gag gag aac att tct gct     1432
   Pro Leu Gly Glu Gly Val Pro Leu Pro Lys Glu Glu Asn Ile Ser Ala
   395                 400                 405                 410
   ccc ttg atc tcc agc tcc cca gtg aag gca gcc cgg gag tat gaa gat     1480
   Pro Leu Ile Ser Ser Ser Pro Val Lys Ala Ala Arg Glu Tyr Glu Asp
                   415                 420                 425
   ccc cct agt gaa gag gaa gat aaa ata aaa gaa gaa cct tta acc atc     1528
   Pro Pro Ser Glu Glu Glu Asp Lys Ile Lys Glu Glu Pro Leu Thr Ile
               430                 435                 440
   tct gaa cta gtg tac aac cca agt gcc agc ctg ctc ccc acc cct gtg     1576
   Ser Glu Leu Val Tyr Asn Pro Ser Ala Ser Leu Leu Pro Thr Pro Val
           445                 450                 455
   gat gac gat gag att gac atg ctc ttt gac tgt cct tct agg ctt gag     1624
   Asp Asp Asp Glu Ile Asp Met Leu Phe Asp Cys Pro Ser Arg Leu Glu
       460                 465                 470
   ttg gaa aga gaa gac aca gat tca ttt gag gat ctg gaa gca gat gaa     1672
   Leu Glu Arg Glu Asp Thr Asp Ser Phe Glu Asp Leu Glu Ala Asp Glu
   475                 480                 485                 490
   aac gcc ttt ttg att gct gaa gaa gag gag ctg aag gag gct cgc cgt     1720
   Asn Ala Phe Leu Ile Ala Glu Glu Glu Glu Leu Lys Glu Ala Arg Arg
                   495                 500                 505
   gct ttg tcg tgg agc tat gac att ctg act ggc cat att cgg gtg aac     1768
   Ala Leu Ser Trp Ser Tyr Asp Ile Leu Thr Gly His Ile Arg Val Asn
               510                 515                 520
   cca ctg gtc aag agt ttt tcc agg ctc ctt gtg gtg ggc ctg gga ctg     1816
   Pro Leu Val Lys Ser Phe Ser Arg Leu Leu Val Val Gly Leu Gly Leu
           525                 530                 535
   ctg ctc ttt gta ttt ccc ctg ctc ctc ctc ctt ttg gag tca ggt att     1864
   Leu Leu Phe Val Phe Pro Leu Leu Leu Leu Leu Leu Glu Ser Gly Ile
       540                 545                 550
   gat ctc tcc ttc tta tgc gaa atc cgc cag aca cca gag ttt gag cag     1912
   Asp Leu Ser Phe Leu Cys Glu Ile Arg Gln Thr Pro Glu Phe Glu Gln
   555                 560                 565                 570
   ttt cac tat gaa tac tac tgt ccc ctc aag gag tgg gtg gct ggg aaa     1960
   Phe His Tyr Glu Tyr Tyr Cys Pro Leu Lys Glu Trp Val Ala Gly Lys
                   575                 580                 585
   gtc cac ctc atc ctc tac atg ctg ggt tgc tca tga agttaatctc          2006
   Val His Leu Ile Leu Tyr Met Leu Gly Cys Ser
               590                 595
   tcacgtgact aagggctata ttcaatgcta gtgatttctt tttttcagca aatgcctggt   2066
   tctgaagggt cacggggctg tcaacaggtg ttccttactc ataattgatt attcaaacct   2126
   ttaagttagc tttccataat tcactgcact taaataagtt taaatcaaat acagttattt   2186
   tagttacagg ttaggaagat ggtctttaaa taaccaaaaa tatgtttatt ttttattata   2246
   gtgtagacat acccttcatc tattatatca taatacatgt tacattggac tgaattagat   2306
   tttcccattt ctaatagttg gcaccattat aagctataag gttcagaatc agaattttag   2366
   taacaactca agagaaagtt gttgaatata atccttagtg aaaacagtgt cctctaacca   2426
   atgcctatac aactaaattt atgctgggtt tttggttctg tttttttaaa aatattttta   2486
   tgtgttcaaa ctattttggt aaatttttag caaaaaaaaa aaa                     2529
<210> 60
   <211> 597
   <212> PRT
   <213> Homo sapiens
<400> 60
   Met Phe Ala Ser Cys His Cys Val Pro Arg Gly Arg Arg Thr Met Lys
   1 5 10 15
   Met Ile His Phe Arg Ser Ser Ser Val Lys Ser Leu Ser Gln Glu Met
   20 25 30
   Arg Cys Thr Ile Arg Leu Leu Asp Asp Ser Glu Ile Ser Cys His Ile
   35 40 45
   Gln Arg Glu Thr Lys Gly Gln Phe Leu Ile Asp His Ile Cys Asn Tyr
   50 55 60
   Tyr Ser Leu Leu Glu Lys Asp Tyr Phe Gly Ile Arg Tyr Val Asp Pro
   65 70 75 80
   Glu Lys Gln Arg His Trp Leu Glu Pro Asn Lys Ser Ile Phe Lys Gln
   85 90 95
   Met Lys Thr His Pro Pro Tyr Thr Met Cys Phe Arg Val Lys Phe Tyr
   100 105 110
   Pro His Glu Pro Leu Lys Ile Lys Glu Glu Leu Thr Arg Tyr Leu Leu
   115 120 125
   Tyr Leu Gln Ile Lys Arg Asp Ile Phe His Gly Arg Leu Leu Cys Ser
   130 135 140
   Phe Ser Asp Ala Ala Tyr Leu Gly Ala Cys Ile Val Gln Ala Glu Leu
   145 150 155 160
   Gly Asp Tyr Asp Pro Asp Glu His Pro Glu Asn Tyr Ile Ser Glu Phe
   165 170 175
   Glu Ile Phe Pro Lys Gln Ser Gln Lys Leu Glu Arg Lys Ile Val Glu
   180 185 190
   Ile His Lys Asn Glu Leu Arg Gly Gln Ser Pro Pro Val Ala Glu Phe
   195 200 205
   Asn Leu Leu Leu Lys Ala His Thr Leu Glu Thr Tyr Gly Val Asp Pro
   210 215 220
   His Pro Cys Lys Asp Ser Thr Gly Thr Thr Thr Phe Leu Gly Phe Thr
   225 230 235 240
   Ala Ala Gly Phe Val Val Phe Gln Gly Asn Lys Arg Ile His Leu Ile
   245 250 255
   Lys Trp Pro Asp Val Cys Lys Leu Lys Phe Glu Gly Lys Thr Phe Tyr
               260                 265                 270
   Val Ile Gly Thr Gln Lys Glu Lys Lys Ala Met Leu Ala Phe His Thr
           275                 280                 285
   Ser Thr Pro Ala Ala Cys Lys His Leu Trp Lys Cys Gly Val Glu Asn
       290                 295                 300
   Gln Ala Phe Tyr Lys Tyr Ala Lys Ser Ser Gln Ile Lys Thr Val Ser
   305                 310                 315                 320
   Ser Ser Lys Ile Phe Phe Lys Gly Ser Arg Phe Arg Tyr Ser Gly Lys
                   325                 330                 335
   Val Ala Lys Glu Val Val Glu Ala Ser Ser Lys Ile Gln Arg Glu Pro
               340                 345                 350
   Pro Glu Val His Arg Ala Asn Ile Thr Gln Ser Arg Ser Ser His Ser
           355                 360                 365
   Leu Asn Lys Gln Leu Ile Ile Asn Met Glu Pro Leu Gln Pro Leu Leu
       370                 375                 380
   Pro Ser Pro Ser Glu Gln Glu Glu Glu Leu Pro Leu Gly Glu Gly Val
   385                 390                 395                 400
   Pro Leu Pro Lys Glu Glu Asn Ile Ser Ala Pro Leu Ile Ser Ser Ser
                   405                 410                 415
   Pro Val Lys Ala Ala Arg Glu Tyr Glu Asp Pro Pro Ser Glu Glu Glu
               420                 425                 430
   Asp Lys Ile Lys Glu Glu Pro Leu Thr Ile Ser Glu Leu Val Tyr Asn
           435                 440                 445
   Pro Ser Ala Ser Leu Leu Pro Thr Pro Val Asp Asp Asp Glu Ile Asp
       450                 455                 460
   Met Leu Phe Asp Cys Pro Ser Arg Leu Glu Leu Glu Arg Glu Asp Thr
   465                 470                 475                 480
   Asp Ser Phe Glu Asp Leu Glu Ala Asp Glu Asn Ala Phe Leu Ile Ala
                   485                 490                 495
   Glu Glu Glu Glu Leu Lys Glu Ala Arg Arg Ala Leu Ser Trp Ser Tyr
               500                 505                 510
   Asp Ile Leu Thr Gly His Ile Arg Val Asn Pro Leu Val Lys Ser Phe
           515                 520                 525
   Ser Arg Leu Leu Val Val Gly Leu Gly Leu Leu Leu Phe Val Phe Pro
       530                 535                 540
   Leu Leu Leu Leu Leu Leu Glu Ser Gly Ile Asp Leu Ser Phe Leu Cys
   545                 550                 555                 560
   Glu Ile Arg Gln Thr Pro Glu Phe Glu Gln Phe His Tyr Glu Tyr Tyr
                   565                 570                 575
   Cys Pro Leu Lys Glu Trp Val Ala Gly Lys Val His Leu Ile Leu Tyr
               580                 585                 590
   Met Leu Gly Cys Ser
           595
<210> 61
   <211> 1777
   <212> DNA
   <213> Homo sapiens
<220>
   <221> CDS
   <222> (38)..(1468)
   <223>
<400> 61
   agaagcccag tagacaaaga aggtaagggc agtgaga atg atg cat ctt gca ttc      55
                                            Met Met His Leu Ala Phe
                                            1               5
   ctt gtg ctg ttg tgt ctg cca gtc tgc tct gcc tat cct ctg agt ggg      103
   Leu Val Leu Leu Cys Leu Pro Val Cys Ser Ala Tyr Pro Leu Ser Gly
               10                  15                  20
   gca gca aaa gag gag gac tcc aac aag gat ctt gcc cag caa tac cta      151
   Ala Ala Lys Glu Glu Asp Ser Asn Lys Asp Leu Ala Gln Gln Tyr Leu
           25                  30                  35
   gaa aag tac tac aac ctc gaa aag gat gtg aaa cag ttt aga aga aag      199
   Glu Lys Tyr Tyr Asn Leu Glu Lys Asp Val Lys Gln Phe Arg Arg Lys
       40                  45                  50
   gac agt aat ctc att gtt aaa aaa atc caa gga atg cag aag ttc ctt      247
   Asp Ser Asn Leu Ile Val Lys Lys Ile Gln Gly Met Gln Lys Phe Leu
   55                  60                  65                  70
   ggg ttg gag gtg aca ggg aag cta gac act gac act ctg gag gtg atg      295
   Gly Leu Glu Val Thr Gly Lys Leu Asp Thr Asp Thr Leu Glu Val Met
                   75                  80                  85
   cgc aag ccc agg tgt gga gtt cct gac gtt ggt cac ttc agc tcc ttt      343
   Arg Lys Pro Arg Cys Gly Val Pro Asp Val Gly His Phe Ser Ser Phe
               90                  95                  100
   cct ggc atg ccg aag tgg agg aaa acc cac ctt aca tac agg att gtg      391
   Pro Gly Met Pro Lys Trp Arg Lys Thr His Leu Thr Tyr Arg Ile Val
           105                 110                 115
   aat tat aca cca gat ttg cca aga gat gct gtt gat tct gcc att gag      439
   Asn Tyr Thr Pro Asp Leu Pro Arg Asp Ala Val Asp Ser Ala Ile Glu
       120                 125                 130
   aaa gct ctg aaa gtc tgg gaa gag gtg act cca ctc aca ttc tcc agg      487
   Lys Ala Leu Lys Val Trp Glu Glu Val Thr Pro Leu Thr Phe Ser Arg
   135                 140                 145                 150
   ctg tat gaa gga gag gct gat ata atg atc tct ttt gca gtt aaa gaa      535
   Leu Tyr Glu Gly Glu Ala Asp Ile Met Ile Ser Phe Ala Val Lys Glu
                   155                 160                 165
   cat gga gac ttt tac tct ttt gat ggc cca gga cac agt ttg gct cat      583
   His Gly Asp Phe Tyr Ser Phe Asp Gly Pro Gly His Ser Leu Ala His
               170                 175                 180
   gcc tac cca cct gga cct ggg ctt tat gga gat att cac ttt gat gat      631
   Ala Tyr Pro Pro Gly Pro Gly Leu Tyr Gly Asp Ile His Phe Asp Asp
           185                 190                 195
   gat gaa aaa tgg aca gaa gat gca tca ggc acc aat tta ttc ctc gtt      679
   Asp Glu Lys Trp Thr Glu Asp Ala Ser Gly Thr Asn Leu Phe Leu Val
       200                 205                 210
   gct gct cat gaa ctt ggc cac tcc ctg ggg ctc ttt cac tca gcc aac      727
   Ala Ala His Glu Leu Gly His Ser Leu Gly Leu Phe His Ser Ala Asn
   215                 220                 225                 230
   act gaa gct ttg atg tac cca ctc tac aac tca ttc aca gag ctc gcc      775
   Thr Glu Ala Leu Met Tyr Pro Leu Tyr Asn Ser Phe Thr Glu Leu Ala
                   235                 240                 245
   cag ttc cgc ctt tcg caa gat gat gtg aat ggc att cag tct ctc tac      823
   Gln Phe Arg Leu Ser Gln Asp Asp Val Asn Gly Ile Gln Ser Leu Tyr
               250                 255                 260
   gga cct ccc cct gcc tct act gag gaa ccc ctg gtg ccc aca aaa tct      871
   Gly Pro Pro Pro Ala Ser Thr Glu Glu Pro Leu Val Pro Thr Lys Ser
           265                 270                 275
   gtt cct tcg gga tct gag atg cca gcc aag tgt gat cct gct ttg tcc      919
   Val Pro Ser Gly Ser Glu Met Pro Ala Lys Cys Asp Pro Ala Leu Ser
       280                 285                 290
   ttc gat gcc atc agc act ctg agg gga gaa tat ctg ttc ttt aaa gac      967
   Phe Asp Ala Ile Ser Thr Leu Arg Gly Glu Tyr Leu Phe Phe Lys Asp
   295                 300                 305                 310
   aga tat ttt tgg cga aga tcc cac tgg aac cct gaa cct gaa ttt cat     1015
   Arg Tyr Phe Trp Arg Arg Ser His Trp Asn Pro Glu Pro Glu Phe His
                   315                 320                 325
   ttg att tct gca ttt tgg ccc tct ctt cca tca tat ttg gat gct gca     1063
   Leu Ile Ser Ala Phe Trp Pro Ser Leu Pro Ser Tyr Leu Asp Ala Ala
               330                 335                 340
   tat gaa gtt aac agc agg gac acc gtt ttt att ttt aaa gga aat gag     1111
   Tyr Glu Val Asn Ser Arg Asp Thr Val Phe Ile Phe Lys Gly Asn Glu
           345                 350                 355
   ttc tgg gcc atc aga gga aat gag gta caa gca ggt tat cca aga ggc      1159
   Phe Trp Ala Ile Arg Gly Asn Glu Val Gln Ala Gly Tyr Pro Arg Gly
       360                 365                 370
   atc cat acc ctg ggt ttt cct cca acc ata agg aaa att gat gca gct      1207
   Ile His Thr Leu Gly Phe Pro Pro Thr Ile Arg Lys Ile Asp Ala Ala
   375                 380                 385                 390
   gtt tct gac aag gaa aag aag aaa aca tac ttc ttt gca gcg gac aaa      1255
   Val Ser Asp Lys Glu Lys Lys Lys Thr Tyr Phe Phe Ala Ala Asp Lys
                   395                 400                 405
   tac tgg aga ttt gat gaa aat agc cag tcc atg gag caa ggc ttc cct      1303
   Tyr Trp Arg Phe Asp Glu Asn Ser Gln Ser Met Glu Gln Gly Phe Pro
               410                 415                 420
   aga cta ata gct gat gac ttt cca gga gtt gag cct aag gtt gat gct      1351
   Arg Leu Ile Ala Asp Asp Phe Pro Gly Val Glu Pro Lys Val Asp Ala
           425                 430                 435
   gta tta cag gca ttt gga ttt ttc tac ttc ttc agt gga tca tca cag      1399
   Val Leu Gln Ala Phe Gly Phe Phe Tyr Phe Phe Ser Gly Ser Ser Gln
       440                 445                 450
   ttt gag ttt gac ccc aat gcc agg atg gtg aca cac ata tta aag agt      1447
   Phe Glu Phe Asp Pro Asn Ala Arg Met Val Thr His Ile Leu Lys Ser
   455                 460                 465                 470
   aac agc tgg tta cat tgc tag gcgagatagg gggaagacag atatgggtgt       1498
   Asn Ser Trp Leu His Cys
                   475
   ttttaataaa tctaataatt attcatctaa tgtattatga gccaaaatgg ttaatttttc   1558
   ctgcatgttc tgtgactgaa gaagatgagc cttgcagata tctgcatgtg tcatgaagaa   1618
   tgtttctgga attcttcact tgcttttgaa ttgcactgaa cagaattaag aaatactcat   1678
   gtgcaatagg tgagagaatg tattttcata gatgtgttat tacttcctca ataaaaagtt   1738
   ttattttggg cctgttcctt aaaaaaaaaa aaaaaaaaa                          1777
<210> 62
   <211> 476
   <212> PRT
   <213> Homo sapiens
<400> 62
   Met Met His Leu Ala Phe Leu Val Leu Leu Cys Leu Pro Val Cys Ser
   1               5                   10                  15
   Ala Tyr Pro Leu Ser Gly Ala Ala Lys Glu Glu Asp Ser Asn Lys Asp
               20                  25                  30
   Leu Ala Gln Gln Tyr Leu Glu Lys Tyr Tyr Asn Leu Glu Lys Asp Val
           35                  40                  45
   Lys Gln Phe Arg Arg Lys Asp Ser Asn Leu Ile Val Lys Lys Ile Gln
       50                  55                  60
   Gly Met Gln Lys Phe Leu Gly Leu Glu Val Thr Gly Lys Leu Asp Thr
   65                  70                  75                  80
   Asp Thr Leu Glu Val Met Arg Lys Pro Arg Cys Gly Val Pro Asp Val
                   85                  90                  95
   Gly His Phe Ser Ser Phe Pro Gly Met Pro Lys Trp Arg Lys Thr His
               100                 105                 110
   Leu Thr Tyr Arg Ile Val Asn Tyr Thr Pro Asp Leu Pro Arg Asp Ala
           115                 120                 125
   Val Asp Ser Ala Ile Glu Lys Ala Leu Lys Val Trp Glu Glu Val Thr
       130                 135                 140
   Pro Leu Thr Phe Ser Arg Leu Tyr Glu Gly Glu Ala Asp Ile Met Ile
   145                 150                 155                 160
   Ser Phe Ala Val Lys Glu His Gly Asp Phe Tyr Ser Phe Asp Gly Pro
                   165                 170                 175
   Gly His Ser Leu Ala His Ala Tyr Pro Pro Gly Pro Gly Leu Tyr Gly
               180                 185                 190
   Asp Ile His Phe Asp Asp Asp Glu Lys Trp Thr Glu Asp Ala Ser Gly
           195                 200                 205
   Thr Asn Leu Phe Leu Val Ala Ala His Glu Leu Gly His Ser Leu Gly
       210                 215                 220
   Leu Phe His Ser Ala Asn Thr Glu Ala Leu Met Tyr Pro Leu Tyr Asn
   225                 230                 235                 240
   Ser Phe Thr Glu Leu Ala Gln Phe Arg Leu Ser Gln Asp Asp Val Asn
                   245                 250                 255
   Gly Ile Gln Ser Leu Tyr Gly Pro Pro Pro Ala Ser Thr Glu Glu Pro
               260                 265                 270
   Leu Val Pro Thr Lys Ser Val Pro Ser Gly Ser Glu Met Pro Ala Lys
           275                 280                 285
   Cys Asp Pro Ala Leu Ser Phe Asp Ala Ile Ser Thr Leu Arg Gly Glu
   290 295 300
   Tyr Leu Phe Phe Lys Asp Arg Tyr Phe Trp Arg Arg Ser His Trp Asn
   305 310 315 320
   Pro Glu Pro Glu Phe His Leu Ile Ser Ala Phe Trp Pro Ser Leu Pro
   325 330 335
   Ser Tyr Leu Asp Ala Ala Tyr Glu Val Asn Ser Arg Asp Thr Val Phe
   340 345 350
   Ile Phe Lys Gly Asn Glu Phe Trp Ala Ile Arg Gly Asn Glu Val Gln
   355 360 365
   Ala Gly Tyr Pro Arg Gly Ile His Thr Leu Gly Phe Pro Pro Thr Ile
   370 375 380
   Arg Lys Ile Asp Ala Ala Val Ser Asp Lys Glu Lys Lys Lys Thr Tyr
   385 390 395 400
   Phe Phe Ala Ala Asp Lys Tyr Trp Arg Phe Asp Glu Asn Ser Gln Ser
   405 410 415
   Met Glu Gln Gly Phe Pro Arg Leu Ile Ala Asp Asp Phe Pro Gly Val
   420 425 430
   Glu Pro Lys Val Asp Ala Val Leu Gln Ala Phe Gly Phe Phe Tyr Phe
   435 440 445
   Phe Ser Gly Ser Ser Gln Phe Glu Phe Asp Pro Asn Ala Arg Met Val
   450 455 460
   Thr His Ile Leu Lys Ser Asn Ser Trp Leu His Cys
   465 470 475
<210> 63
   <211> 2305
   <212> DNA
   <213> Homo sapiens
<220>
   <221> CDS
   <222> (451)..(1392)
   <223>
<400> 63
   gttactgtcc cgcgaaccca catccctaca aagcaggaaa gtatgcttgg gagaggccaa     60
   gtgagtgggg aatcagccca aagccaggcg tccagggtct ccctcacctg aagctgactt    120
   tttccccacc ttggacagag ggcgggagat gccatcccca ctgaacccag tgctttcacc    180
   agccatatta gctcccactc accccccgtc gtggaagcct cggccgtcac acctgcaggg    240
   ccggggcgtg catggcctca gggatggcct gttcagctgc tgggtgactc gggtccaggt    300
   gcctcaccac ctgctgagct ctgtgtgatt tctggacact tctgctcgtt gcctttgggc    360
   tcagtgaaga gtctggagtt tatctggagt gaggtggccg gttcttggtg ggatctgagc    420
   aggacagcgt ctggctcctt ccctcggctc atg gcc ctc aga atc tgc gtc aca    474
                                    Met Ala Leu Arg Ile Cys Val Thr
                                    1               5
   tac acc cca gct ctc ccg ata ggt ctc tgc act cgc tgt tgc ctc tgc      522
   Tyr Thr Pro Ala Leu Pro Ile Gly Leu Cys Thr Arg Cys Cys Leu Cys
       10                  15                  20
   ctg gaa cag tct ccc tcc tgg tgt cat tgt ctc cgt ggt gtg tcc ttc      570
   Leu Glu Gln Ser Pro Ser Trp Cys His Cys Leu Arg Gly Val Ser Phe
   25                  30                  35                  40
   ctg acc ttc cac ctc cac cag tct gtc ccc ctt ggg gac agg gac tcg      618
   Leu Thr Phe His Leu His Gln Ser Val Pro Leu Gly Asp Arg Asp Ser
                   45                  50                  55
   ttg ctc atg ttc acc cgg cag gct gga cac ttc gtg gag ggc tcc aaa      666
   Leu Leu Met Phe Thr Arg Gln Ala Gly His Phe Val Glu Gly Ser Lys
               60                  65                  70
   gcc ggc aga tcc cgg ggc cgc ctc tgt ctc tcc cag gcc ctg cgt gtt      714
   Ala Gly Arg Ser Arg Gly Arg Leu Cys Leu Ser Gln Ala Leu Arg Val
           75                  80                  85
   gcg gtg aga gga gca ttt gtg tct ctg tgg ttt gct gct gga gct ggt      762
   Ala Val Arg Gly Ala Phe Val Ser Leu Trp Phe Ala Ala Gly Ala Gly
       90                  95                  100
   gac cgg gag aga aac aag gga gac aag ggt gcc cag aca ggt gcg ggg      810
   Asp Arg Glu Arg Asn Lys Gly Asp Lys Gly Ala Gln Thr Gly Ala Gly
   105                 110                 115                 120
   ctc agc cag gag gca gaa gac gtg gac gtg tcc cgg gcc agg agg gtc      858
   Leu Ser Gln Glu Ala Glu Asp Val Asp Val Ser Arg Ala Arg Arg Val
                   125                 130                 135
   aca gat gca cca caa ggc act ctg tgt ggc act ggg aac agg aat tct      906
   Thr Asp Ala Pro Gln Gly Thr Leu Cys Gly Thr Gly Asn Arg Asn Ser
               140                 145                 150
   ggg agt cag tct gca agg gtg gtg ggc gtt gct cac ctg gga gaa gcc      954
   Gly Ser Gln Ser Ala Arg Val Val Gly Val Ala His Leu Gly Glu Ala
           155                 160                 165
   ttt aga gtg ggc gtt gag cag gcc att agc tcg tgc cct gag gag gtg     1002
   Phe Arg Val Gly Val Glu Gln Ala Ile Ser Ser Cys Pro Glu Glu Val
       170                 175                 180
   cat ggg cgg cat ggg ctc tcc atg gaa att atg tgg gcg cga atg gat     1050
   His Gly Arg His Gly Leu Ser Met Glu Ile Met Trp Ala Arg Met Asp
   185                 190                 195                 200
   gtg gct ctg cgc tca cct ggg cga gga ctt ctg gcc ggt gcc ggg gca     1098
   Val Ala Leu Arg Ser Pro Gly Arg Gly Leu Leu Ala Gly Ala Gly Ala
                   205                 210                 215
   ctc tgc atg acc ctg gca gaa tcg agc tgc cct gac tat gaa agg gga      1146
   Leu Cys Met Thr Leu Ala Glu Ser Ser Cys Pro Asp Tyr Glu Arg Gly
               220                 225                 230
   aga aga gca tgc ctg acc ctc cac cgg cac ccc acc cct cac tgc tcc      1194
   Arg Arg Ala Cys Leu Thr Leu His Arg His Pro Thr Pro His Cys Ser
           235                 240                 245
   acc tgg ggc ctg cct ctg cgg gtg gct ggg tcc tgg ctg act gtt gtg      1242
   Thr Trp Gly Leu Pro Leu Arg Val Ala Gly Ser Trp Leu Thr Val Val
       250                 255                 260
   act gtt gag gcc ctg ggg ggg tgg cgc atg gga gtt agg agg act ggc      1290
   Thr Val Glu Ala Leu Gly Gly Trp Arg Met Gly Val Arg Arg Thr Gly
   265                 270                 275                 280
   cag gtg ggg ccc act atg cac cca ccc cca gtg tca ggt gct tct cct      1338
   Gln Val Gly Pro Thr Met His Pro Pro Pro Val Ser Gly Ala Ser Pro
                   285                 290                 295
   ctc ctc ctc cac cac ctc ctc ctc ctc ctc ctc atc atc atc ctc act      1386
   Leu Leu Leu His His Leu Leu Leu Leu Leu Leu Ile Ile Ile Leu Thr
               300                 305                 310
   tgt tga ggacgtcctg tgtgccaagt ggtttatatg cccagcctca tttaatcctc      1442
   Cys
   agaatgactc catgaggtag ctactaaaac cccccactta acagatgagg aaactgaggc   1502
   ctagagaagc tcaacaagtt gcctaagttc caagtttcct ctcccaactc tcctaccctc   1562
   tcctcttcct tctcctttct cccattcttc cctgcctctt ccctaactag acaatttttt   1622
   attgagtgtc tcccaggtgc aggcatgagc caggtgctgg gaaaatcatg ataacccagc   1682
   tccttctggt cattttctca gctggttaga ggctgggagg acacgcaagt tcagctccag   1742
   ccgactgggg cattggtggt agcccctgga gacattgtgc aatggggcta cgaggctgca   1802
   tctggctcca gggaagcgtg ttgcaatcca tgagtgatgt ctgccatgcg tacaggcatg   1862
   gagagtgagg cgcctgtact gtctttctgt agaccctaga ctggtggggc ctctgaaatg   1922
   catccagaca ctgtgctggg tgtgttgcat ggccctccca accaattcag tatttttctc   1982
   cccattttcc agggagaaat ctaaggcgtc agaatgtaag gttcttattg gaacccaggc   2042
   tccagggtcc ctggttttct gtgacatcat gctgcaggac cctgtttctc tctttgcttt   2102
   ggctgctggg gagctcagaa gggagcttta ggcttgctct cagtcaccac attagctcag   2162
   gggtttgggc atttttgtcg ccgtctcctt tgggctctgt ctcctccctg ctgtgcttcc   2222
   tgaggagcag gccggatgta agttatcaac tataaataaa accaagactt ccgttctggc   2282
   tctcaaaaaa aaaaaaaaaa aaa                                           2305
<210> 64
   <211> 313
   <212> PRT
   <213> Homo sapiens
<400> 64
   Met Ala Leu Arg Ile Cys Val Thr Tyr Thr Pro Ala Leu Pro Ile Gly
   1               5                   10                  15
   Leu Cys Thr Arg Cys Cys Leu Cys Leu Glu Gln Ser Pro Ser Trp Cys
               20                  25                  30
   His Cys Leu Arg Gly Val Ser Phe Leu Thr Phe His Leu His Gln Ser
           35                  40                  45
   Val Pro Leu Gly Asp Arg Asp Ser Leu Leu Met Phe Thr Arg Gln Ala
       50                  55                  60
   Gly His Phe Val Glu Gly Ser Lys Ala Gly Arg Ser Arg Gly Arg Leu
   65                  70                  75                  80
   Cys Leu Ser Gln Ala Leu Arg Val Ala Val Arg Gly Ala Phe Val Ser
                   85                  90                  95
   Leu Trp Phe Ala Ala Gly Ala Gly Asp Arg Glu Arg Asn Lys Gly Asp
               100                 105                 110
   Lys Gly Ala Gln Thr Gly Ala Gly Leu Ser Gln Glu Ala Glu Asp Val
           115                 120                 125
   Asp Val Ser Arg Ala Arg Arg Val Thr Asp Ala Pro Gln Gly Thr Leu
       130                 135                 140
   Cys Gly Thr Gly Asn Arg Asn Ser Gly Ser Gln Ser Ala Arg Val Val
   145                 150                 155                 160
   Gly Val Ala His Leu Gly Glu Ala Phe Arg Val Gly Val Glu Gln Ala
                   165                 170                 175
   Ile Ser Ser Cys Pro Glu Glu Val His Gly Arg His Gly Leu Ser Met
               180                 185                 190
   Glu Ile Met Trp Ala Arg Met Asp Val Ala Leu Arg Ser Pro Gly Arg
           195                 200                 205
   Gly Leu Leu Ala Gly Ala Gly Ala Leu Cys Met Thr Leu Ala Glu Ser
       210                 215                 220
   Ser Cys Pro Asp Tyr Glu Arg Gly Arg Arg Ala Cys Leu Thr Leu His
   225                 230                 235                 240
   Arg His Pro Thr Pro His Cys Ser Thr Trp Gly Leu Pro Leu Arg Val
   245 250 255
   Ala Gly Ser Trp Leu Thr Val Val Thr Val Glu Ala Leu Gly Gly Trp
   260 265 270
   Arg Met Gly Val Arg Arg Thr Gly Gln Val Gly Pro Thr Met His Pro
   275 280 285
   Pro Pro Val Ser Gly Ala Ser Pro Leu Leu Leu His His Leu Leu Leu
   290 295 300
   Leu Leu Leu Ile Ile Ile Leu Thr Cys
   305 310
<210> 65
   <211> 3210
   <212> DNA
   <213> Homo sapiens
<220>
   <221> CDS
   <222> (1688)..(2959)
   <223>
<400> 65
cctctttctc ccccctctct ccctcctctc ctctctctct cctctcttct ctgaacctct 60
   cttctctttc tcttttctta catattctac tagttgtttt ccccttcttc ttctcctttc 120
   tctccttttt ctccctcctc cttgtctctt ttactccatt cctgcagaag agagagggct 180
   aaacttaaaa aaaaaaaaaa ggaggaggag gaggaggagg cacccccttc gtattcttct 240
   tatcgttatt ttatacatat atgatttttt ttggagggag ggtgttggtt gccggctgaa 300
   gagcacttat ttaaaatact aaaaaaagaa catttttggg cgatctccag ggttttttta 360
   actagctctg tgtgttatag cagaagaagc agaagaagga gcaagaaaga ggaaaagaag 420
   aggattattt attcgaccta ctttggatgt ctctctcgct tttccttttt cctttttttg 480
   gcaattattt tcttctgatt tttatttttt ctatttcgct gtgatttcgt cgccggcgtg 540
   aattatcccg tatttttctc ccccttccgt cacctcccga aagaagaagg cagcgagagc 600
   ccggcgccac cggcacaaca aaaagagcaa agtgtgtgat cttcctcgcc ggctgcctcc 660
   cgctctccag cgctgccttc ctgaatggct ggctgcgtcc ggccctggac ctggcccccc 720
   gacacccgcg cgccctgatc gccggcggca gcctcgccca gcgccctgct cggctcaccg 780
   cgctccccgc actcccgagc ccggcgaggg ctcccgccgg gacagcggcg gcgccgcggg 840
   cggccccggc ctccgctcgc gctccggctg cggccccgac tcctgctcgg actccggccc 900
   gggtcccggc tcctccagcg gcgctcgccg cagcagctcc ggcggcagct ccagcggcgc     960
   ctgcagccgc gacctcctcc tcctccgccg ccgccgccgc ctccgccctc gccggcttcc    1020
   tctatgtcgg ctcagcccgc gcgctgcgcg tagcccgagc ggccggcggg cgggcgcccg    1080
   gcgcgggtga gcgactgtgt gtgcgagtgt gtgtgtgcgc gggggtgcgg gcgaggcgga    1140
   gggcgagtgt gtgcgcgcgc cgtggcccat gcccgccgcc cccggcgctg cgccccgcgc    1200
   cgctcccggc tgccgcctgt gccatttctg attttgcaac ttggggaaga agaaaaaagc    1260
   gagagaaggg agcttgctcg ccggggggtg gggagggggg aaggagagcg cggccccccc     1320
   aggaacggag cgcgggggga gcgggcgagg ggagcagggg tgttgggggg ggagcctgag     1380
   agcctggggg ggctgcaaaa agagagaaag aaaacagcag gaaccacaac aaaacgccag    1440
   cagggcgggc gggcgcgcag cagcagcggg gcggccgagg cagtagcggc ggcagcggcg    1500
   gcggcggcgg aggcagcggc cggtgtccgg ctcgggctcg gctcctgcga ccccggggcg    1560
   cccggcgggc cccccgcccc ctccccctcc ccccttcccc ttccccttcc cctcccagcg     1620
   cgcccgcgcg ccccgcggcc ctcggcgagc agctcggctc cccccagcgc tccccgggcc    1680
   caaagat atg gca atg gta gtt agc agc tgg cga gat ccg cag gac gac      1729
   gtg gcc ggg ggc aac ccc ggc ggc ccc aac ccc gca gcg cag gcg gcc      1777
   cgc ggc ggc ggc ggc ggc gcc ggc gag cag cag cag cag gcg ggc tcg      1825
   ggc gcg ccg cac acg ccg cag acc ccg ggc cag ccc gga gcg ccc gcc      1873
   acc ccc ggc acg gcg ggg gac aag ggc cag ggc ccg ccc ggt tcg ggc      1921
   cag agc cag cag cac atc gag tgc gtg gtg tgc ggg gac aag tcg agc      1969
   ggc aag cac tac ggc caa ttc acc tgc gag ggc tgc aaa agt ttc ttc      2017
   aag agg agc gtc cgc agg aac tta act tac aca tgc cgt gcc aac agg      2065
   aac tgt ccc atc gac cag cac cac cgc aac cag tgc caa tac tgc cgc      2113
   ctc aag aag tgc ctc aaa gtg ggc atg agg cgg gaa gcg gtt cag cga      2161
   gga aga atg cct cca acc cag ccc aat cca ggc cag tac gca ctc acc      2209
   aac ggg gac ccc ctc aac ggc cac tgc tac ctg tcc ggc tac atc tcg      2257
   ctg ctg ctg cgc gcc gag ccc tac ccc acg tcg cgc tac ggc agc cag      2305
   tgc atg cag ccc aac aac att atg ggc atc gag aac atc tgc gag ctg      2353
   gcc gcg cgc ctg ctc ttc agc gcc gtc gag tgg gcc cgc aac atc ccc      2401
   ttc ttc ccg gat ctg cag atc acc gac cag gtg tcc ctg cta cgc ctc      2449
   acc tgg agc gag ctg ttc gtg ctc aac gcg gcc cag tgc tct atg ccg      2497
   ctg cac gtg gcg ccg ttg ctg gcc gcc gcc ggc ctg cat gcc tcg ccc      2545
   atg tct gcc gac cgc gtc gtg gcc ttc atg gac cac atc cgc atc ttc      2593
   cag gag cag gtg gag aag ctc aag gcg cta cac gtc gac tca gcc gag      2641
   tac agc tgc ctc aaa gcc atc gtg ctg ttc acg tca gac gcc tgt ggc      2689
   ctg tcg gat gcg gcc cac atc gag agc ctg cag gag aag tcg cag tgc      2737
   gca ctg gag gag tac gtg agg agc cag tac ccc aac cag ccc agc cgt      2785
   ttt ggc aaa ctg ctg ctg cga ctg ccc tcg ctg cgc acc gtg tcc tcc      2833
   tcc gtc atc gag cag ctc ttc ttc gtc cgt ttg gta ggt aaa acc ccc      2881
   atc gaa act ctc atc cgc gat atg tta ctg tct ggg agc agc ttc aac      2929
   tgg cct tac atg tcc atc cag tgc tcc tag accttgggcg cttcccacct        2979
   gccccgtccc cctagagact cagaggaccc acctgggcca aggactccaa agccgcgggg   3039
   acaccgggaa gtgcagcggg ccaggcaggc tgggtgggag ggaggagggc cgagacagga   3099
   gcagcccacc cagcagaaat acaatccgag ctacaaagca tgggaaaaag agactctttt   3159
   aggatcagat ctgtgagcac gttggcgagg aaaaacaaaa aaaaaaaaaa a             3210
<210> 66
   <211> 423
   <212> PRT
   <213> Homo sapiens
<400> 66
<210> 67
   <211> 6305
   <212> DNA
   <213> Homo sapiens
<220>
   <221> CDS
   <222> (107)..(4021)
   <223>
<400> 67
   tttcccgcat gaaaattact taaacgttgc acacaacgtt tcacaaaatc ttttgtgaaa      60
   gaagaaaagg aaattcagtg tgtgagtctc agcaggagtt aagcta atg cag ctt        115
                                                      Met Gln Leu
                                                      1
   aaa ata atg ccg aaa aag aag cgc tta tct gcg ggc aga gtg ccc ctg       163
   Lys Ile Met Pro Lys Lys Lys Arg Leu Ser Ala Gly Arg Val Pro Leu
       5                   10                  15
   att ctc ttc ctg tgc cag atg att agt gca ctg gaa gta cct ctt gat       211
   Ile Leu Phe Leu Cys Gln Met Ile Ser Ala Leu Glu Val Pro Leu Asp
   20                  25                  30                  35
   cca aaa ctt ctt gaa gac ttg gta cag cct cca acc atc acc caa cag       259
   Pro Lys Leu Leu Glu Asp Leu Val Gln Pro Pro Thr Ile Thr Gln Gln
                   40                  45                  50
   tct cca aaa gat tac att att gac cct cgg gag aat att gta atc cag       307
   Ser Pro Lys Asp Tyr Ile Ile Asp Pro Arg Glu Asn Ile Val Ile Gln
               55                  60                  65
   tgt gaa gcc aaa ggg aaa ccg ccc cca agc ttt tcc tgg acc cgt aat       355
   Cys Glu Ala Lys Gly Lys Pro Pro Pro Ser Phe Ser Trp Thr Arg Asn
           70                  75                  80
   ggg act cat ttt gac atc gat aaa gac cct ctg gtc acc atg aag cct       403
   Gly Thr His Phe Asp Ile Asp Lys Asp Pro Leu Val Thr Met Lys Pro
       85                  90                  95
   ggc aca gga acg ctc ata att aac atc atg agc gaa ggg aaa gct gag       451
   Gly Thr Gly Thr Leu Ile Ile Asn Ile Met Ser Glu Gly Lys Ala Glu
   100                 105                 110                 115
   acc tat gaa gga gtc tat cag tgt aca gca agg aac gaa cgc gga gct       499
   Thr Tyr Glu Gly Val Tyr Gln Cys Thr Ala Arg Asn Glu Arg Gly Ala
                   120                 125                 130
   gca gtt tct aat aac att gtt gtc cgc cca tcc aga tca cca ttg tgg       547
   Ala Val Ser Asn Asn Ile Val Val Arg Pro Ser Arg Ser Pro Leu Trp
               135                 140                 145
   acc aaa gaa aaa ctt gaa cca atc aca ctt caa agt ggt cag tct tta       595
   Thr Lys Glu Lys Leu Glu Pro Ile Thr Leu Gln Ser Gly Gln Ser Leu
           150                 155                 160
   gta ctt ccc tgc aga ccc cca att gga tta cca cca cct ata ata ttt      643
   Val Leu Pro Cys Arg Pro Pro Ile Gly Leu Pro Pro Pro Ile Ile Phe
       165                 170                 175
   tgg atg gat aat tcc ttt caa aga ctt cca caa agt gag aga gtt tct      691
   Trp Met Asp Asn Ser Phe Gln Arg Leu Pro Gln Ser Glu Arg Val Ser
   180                 185                 190                 195
   caa ggt ttg aat ggg gac ctt tat ttt tcc aat gtc ctc cca gag gac      739
   Gln Gly Leu Asn Gly Asp Leu Tyr Phe Ser Asn Val Leu Pro Glu Asp
                   200                 205                 210
   acc cgc gaa gac tat atc tgt tat gct aga ttt aat cat act caa acc      787
   Thr Arg Glu Asp Tyr Ile Cys Tyr Ala Arg Phe Asn His Thr Gln Thr
               215                 220                 225
   ata cag cag aag caa cct att tct gtg aag gtg att tca gtg gat gaa      835
   Ile Gln Gln Lys Gln Pro Ile Ser Val Lys Val Ile Ser Val Asp Glu
           230                 235                 240
   ttg aat gac act ata gct gct aat ttg agt gac act gag ttt tat ggt      883
   Leu Asn Asp Thr Ile Ala Ala Asn Leu Ser Asp Thr Glu Phe Tyr Gly
       245                 250                 255
   gct aaa tca agt aga gag agg cca cca aca ttt tta act cca gaa ggc      931
   Ala Lys Ser Ser Arg Glu Arg Pro Pro Thr Phe Leu Thr Pro Glu Gly
   260                 265                 270                 275
   aat gca agt aac aaa gag gaa tta aga gga aat gtg ctt tca ctg gag      979
   Asn Ala Ser Asn Lys Glu Glu Leu Arg Gly Asn Val Leu Ser Leu Glu
                   280                 285                 290
   tgc att gca gaa gga ctg cct acc cca att att tac tgg gca aag gaa     1027
   Cys Ile Ala Glu Gly Leu Pro Thr Pro Ile Ile Tyr Trp Ala Lys Glu
               295                 300                 305
   gat gga atg cta ccc aaa aac agg aca gtt tat aag aac ttt gag aaa     1075
   Asp Gly Met Leu Pro Lys Asn Arg Thr Val Tyr Lys Asn Phe Glu Lys
           310                 315                 320
   acc ttg cag atc att cat gtt tca gaa gca gac tct gga aat tac caa     1123
   Thr Leu Gln Ile Ile His Val Ser Glu Ala Asp Ser Gly Asn Tyr Gln
       325                 330                 335
   tgt ata gca aaa aac gca tta gga gcc atc cac cat acc att tct gtt     1171
   Cys Ile Ala Lys Asn Ala Leu Gly Ala Ile His His Thr Ile Ser Val
   340                 345                 350                 355
   aga gtt aaa gcg gct cca tac tgg atc aca gcc cct caa aat ctt gtg     1219
   Arg Val Lys Ala Ala Pro Tyr Trp Ile Thr Ala Pro Gln Asn Leu Val
                   360                 365                 370
   ctg tcc cca gga gag gat ggg acc ttg atc tgc aga gct aat ggc aac     1267
   Leu Ser Pro Gly Glu Asp Gly Thr Leu Ile Cys Arg Ala Asn Gly Asn
               375                 380                 385
   ccc aaa ccc aga att agc tgg tta aca aat gga gtc cca ata gaa att     1315
   Pro Lys Pro Arg Ile Ser Trp Leu Thr Asn Gly Val Pro Ile Glu Ile
           390                 395                 400
   gcc cct gat gac ccc agc aga aaa ata gat ggc gat acc att att ttt     1363
   Ala Pro Asp Asp Pro Ser Arg Lys Ile Asp Gly Asp Thr Ile Ile Phe
       405                 410                 415
   tca aat gtt caa gaa aga tca agt gca gtc tat cag tgc aat gcc tct      1411
   Ser Asn Val Gln Glu Arg Ser Ser Ala Val Tyr Gln Cys Asn Ala Ser
   420                 425                 430                 435
   aat gaa tat gga tat tta ctg gca aac gca ttt gta aat gtg ctg gct      1459
   Asn Glu Tyr Gly Tyr Leu Leu Ala Asn Ala Phe Val Asn Val Leu Ala
                   440                 445                 450
   gag cca cca cga atc ctc aca cct gca aac aca ctc tac cag gtc att      1507
   Glu Pro Pro Arg Ile Leu Thr Pro Ala Asn Thr Leu Tyr Gln Val Ile
               455                 460                 465
   gca aac agg cct gct tta cta gac tgt gcc ttc ttt ggg tct cct ctc      1555
   Ala Asn Arg Pro Ala Leu Leu Asp Cys Ala Phe Phe Gly Ser Pro Leu
           470                 475                 480
   cca acc atc gag tgg ttt aaa gga gct aaa gga agt gct ctt cat gaa      1603
   Pro Thr Ile Glu Trp Phe Lys Gly Ala Lys Gly Ser Ala Leu His Glu
       485                 490                 495
   gat att tat gtt tta cat gaa aat gga act ttg gaa att cct gtg gcc      1651
   Asp Ile Tyr Val Leu His Glu Asn Gly Thr Leu Glu Ile Pro Val Ala
   500                 505                 510                 515
   caa aag gac agt aca gga act tat acg tgt gtt gca agg aat aaa tta      1699
   Gln Lys Asp Ser Thr Gly Thr Tyr Thr Cys Val Ala Arg Asn Lys Leu
                   520                 525                 530
   ggg atg gcg aag aat gaa gtt cac tta gaa atc aaa gat cct aca tgg      1747
   Gly Met Ala Lys Asn Glu Val His Leu Glu Ile Lys Asp Pro Thr Trp
               535                 540                 545
   atc gtt aaa cag ccc gaa tat gca gtt gtg caa aga ggg agc atg gtg      1795
   Ile Val Lys Gln Pro Glu Tyr Ala Val Val Gln Arg Gly Ser Met Val
           550                 555                 560
   tcc ttt gaa tgc aaa gtg aaa cat gat cac acc tta tcc ctc act gtc      1843
   Ser Phe Glu Cys Lys Val Lys His Asp His Thr Leu Ser Leu Thr Val
       565                 570                 575
   ctg tgg ctg aag gac aac agg gaa ctg ccc agt gat gaa agg ttc act      1891
   Leu Trp Leu Lys Asp Asn Arg Glu Leu Pro Ser Asp Glu Arg Phe Thr
   580                 585                 590                 595
   gtt gac aag gat cat cta gtg gta gct gat gtc agt gac gat gac agc      1939
   Val Asp Lys Asp His Leu Val Val Ala Asp Val Ser Asp Asp Asp Ser
                   600                 605                 610
   ggg acc tac acg tgt gtg gcc aac acc act ctg gac agc gtc tcc gcc      1987
   Gly Thr Tyr Thr Cys Val Ala Asn Thr Thr Leu Asp Ser Val Ser Ala
               615                 620                 625
   agc gct gtg ctt agc gtt gtt gct cct act cca act cca gct ccc gtt      2035
   Ser Ala Val Leu Ser Val Val Ala Pro Thr Pro Thr Pro Ala Pro Val
           630                 635                 640
   tac gat gtc cca aat cct ccc ttt gac tta gaa ctg aca gat caa ctt      2083
   Tyr Asp Val Pro Asn Pro Pro Phe Asp Leu Glu Leu Thr Asp Gln Leu
       645                 650                 655
   gac aaa agt gtt cag ctg tca tgg acc cca ggc gat gac aac aat agc      2131
   Asp Lys Ser Val Gln Leu Ser Trp Thr Pro Gly Asp Asp Asn Asn Ser
   660                 665                 670                 675
   ccc att aca aaa ttc atc atc gaa tat gaa gat gca atg cac aag cca      2179
   Pro Ile Thr Lys Phe Ile Ile Glu Tyr Glu Asp Ala Met His Lys Pro
                   680                 685                 690
   ggg ctg tgg cac cac caa act gaa gtt tct gga aca cag acc aca gcc      2227
   Gly Leu Trp His His Gln Thr Glu Val Ser Gly Thr Gln Thr Thr Ala
               695                 700                 705
   cag ctg aag ctg tct cct tac gtg aac tac tcc ttc cgc gtg atg gca      2275
   Gln Leu Lys Leu Ser Pro Tyr Val Asn Tyr Ser Phe Arg Val Met Ala
           710                 715                 720
   gtg aac agc att ggg aag agc ttg ccc agc gag gcg tct gag cag tat      2323
   Val Asn Ser Ile Gly Lys Ser Leu Pro Ser Glu Ala Ser Glu Gln Tyr
       725                 730                 735
   ttg acg aaa gcc tca gaa cca gat aaa aac ccc aca gct gtg gaa gga      2371
   Leu Thr Lys Ala Ser Glu Pro Asp Lys Asn Pro Thr Ala Val Glu Gly
   740                 745                 750                 755
   ctg gga tca gag cct gat aat ttg gtg att acg tgg aag ccc ttg aat      2419
   Leu Gly Ser Glu Pro Asp Asn Leu Val Ile Thr Trp Lys Pro Leu Asn
                   760                 765                 770
   ggt ttc gaa tct aat ggg cca ggc ctt cag tac aaa gtt agc tgg cgc      2467
   Gly Phe Glu Ser Asn Gly Pro Gly Leu Gln Tyr Lys Val Ser Trp Arg
               775                 780                 785
   cag aaa gat ggt gat gat gaa tgg aca tct gtg gtt gtg gca aat gta      2515
   Gln Lys Asp Gly Asp Asp Glu Trp Thr Ser Val Val Val Ala Asn Val
           790                 795                 800
   tcc aaa tat att gtc tca ggc acg cca acc ttt gtt cca tac ctg atc      2563
   Ser Lys Tyr Ile Val Ser Gly Thr Pro Thr Phe Val Pro Tyr Leu Ile
       805                 810                 815
   aaa gtt cag gcc ctg aat gac atg ggg ttt gcc ccc gag cca gct gta      2611
   Lys Val Gln Ala Leu Asn Asp Met Gly Phe Ala Pro Glu Pro Ala Val
   820                 825                 830                 835
   gtc atg gga cat tct gga gaa gac ctc cca atg gtg gct cct ggg aac      2659
   Val Met Gly His Ser Gly Glu Asp Leu Pro Met Val Ala Pro Gly Asn
                   840                 845                 850
   gtg cgt gtg aat gtg gtg aac agt acc tta gcc gag gtg cac tgg gac      2707
   Val Arg Val Asn Val Val Asn Ser Thr Leu Ala Glu Val His Trp Asp
               855                 860                 865
   cca gta cct ctg aaa agc atc cga gga cac cta caa ggc tat cgg att      2755
   Pro Val Pro Leu Lys Ser Ile Arg Gly His Leu Gln Gly Tyr Arg Ile
           870                 875                 880
   tac tat tgg aag acc cag agt tca tct aaa aga aac aga cgt cac att      2803
   Tyr Tyr Trp Lys Thr Gln Ser Ser Ser Lys Arg Asn Arg Arg His Ile
       885                 890                 895
   gag aaa aag atc ctc acc ttc caa ggc agc aag act cat ggc atg ttg      2851
   Glu Lys Lys Ile Leu Thr Phe Gln Gly Ser Lys Thr His Gly Met Leu
   900                 905                 910                 915
   ccg ggg cta gag ccc ttt agc cac tac aca ctg aat gtc cga gtg gtc      2899
   Pro Gly Leu Glu Pro Phe Ser His Tyr Thr Leu Asn Val Arg Val Val
                   920                 925                 930
   aat ggg aaa ggg gag ggc cca gcc agc cct gac aga gtc ttt aat act      2947
   Asn Gly Lys Gly Glu Gly Pro Ala Ser Pro Asp Arg Val Phe Asn Thr
               935                 940                 945
   cca gaa gga gtc ccc agt gct ccc tcg tct ttg aag att gtg aat cca      2995
   Pro Glu Gly Val Pro Ser Ala Pro Ser Ser Leu Lys Ile Val Asn Pro
           950                 955                 960
   aca ctg gac tct ctc act ttg gaa tgg gat cca ccg agc cac ccg aat      3043
   Thr Leu Asp Ser Leu Thr Leu Glu Trp Asp Pro Pro Ser His Pro Asn
       965                 970                 975
   ggc att ttg aca gag tac acc tta aag tat cag cca att aac agc aca      3091
   Gly Ile Leu Thr Glu Tyr Thr Leu Lys Tyr Gln Pro Ile Asn Ser Thr
   980                 985                 990                 995
   cat gaa tta ggc cct  ctg gta gat ttg aaa  att cct gcc aac aag       3136
   His Glu Leu Gly Pro  Leu Val Asp Leu Lys  Ile Pro Ala Asn Lys
                   1000                 1005                 1010
   aca cgg tgg act tta  aaa aat tta aat ttc  agc act cga tat aag       3181
   Thr Arg Trp Thr Leu  Lys Asn Leu Asn Phe  Ser Thr Arg Tyr Lys
                   1015                 1020                 1025
   ttt tat ttc tat gca  caa aca tca gca gga  tca gga agt caa att       3226
   Phe Tyr Phe Tyr Ala  Gln Thr Ser Ala Gly  Ser Gly Ser Gln Ile
                   1030                 1035                 1040
   aca gag gaa gca gta  aca act gtg gat gaa  gct ggt att ctt cca       3271
   Thr Glu Glu Ala Val  Thr Thr Val Asp Glu  Ala Gly Ile Leu Pro
                   1045                 1050                 1055
   cct gat gta ggt gca  ggc aaa gtt caa gca  gta aat ccc agg atc       3316
   Pro Asp Val Gly Ala  Gly Lys Val Gln Ala  Val Asn Pro Arg Ile
                   1060                 1065                 1070
   agc aat ctt act gct  gca gct gct gag acc  tat gcc aat atc agt       3361
   Ser Asn Leu Thr Ala  Ala Ala Ala Glu Thr  Tyr Ala Asn Ile Ser
                   1075                 1080                 1085
   tgg gaa tat gag gga  cca gag cat gtg aac  ttt tat gtt gaa tat       3406
   Trp Glu Tyr Glu Gly  Pro Glu His Val Asn  Phe Tyr Val Glu Tyr
                   1090                 1095                 1100
   ggt gta gca ggc agc  aaa gaa gaa tgg aga  aaa gaa att gta aat       3451
   Gly Val Ala Gly Ser  Lys Glu Glu Trp Arg  Lys Glu Ile Val Asn
                   1105                 1110                 1115
   ggt tct cgg agc ttc  ttt ggg tta aag ggt  cta atg cca gga aca       3496
   Gly Ser Arg Ser Phe  Phe Gly Leu Lys Gly  Leu Met Pro Gly Thr
                   1120                 1125                 1130
   gca tac aaa gtt cga  gtt ggt gct gtg ggg  gac tct ggt ttt gtg       3541
   Ala Tyr Lys Val Arg  Val Gly Ala Val Gly  Asp Ser Gly Phe Val
                   1135                 1140                 1145
   agt tca gag gat gtg  ttt gag aca ggc cca  gcg atg gca agc cgg       3586
   Ser Ser Glu Asp Val  Phe Glu Thr Gly Pro  Ala Met Ala Ser Arg
                   1150                 1155                 1160
   cag gtg gat att gca  act cag ggc tgg ttc  att ggt ctg atg tgt       3631
   Gln Val Asp Ile Ala  Thr Gln Gly Trp Phe  Ile Gly Leu Met Cys
                   1165                 1170                 1175
   gct gtt gct ctc ctt  atc tta att ttg ctg  att gtt tgc ttc atc       3676
   Ala Val Ala Leu Leu  Ile Leu Ile Leu Leu  Ile Val Cys Phe Ile
                   1180                 1185                 1190
   aga aga aac aag ggt  ggt aaa tat cca gtt  aaa gaa aag gaa gat       3721
   Arg Arg Asn Lys Gly  Gly Lys Tyr Pro Val  Lys Glu Lys Glu Asp
                   1195                 1200                 1205
   gcc cat gct gac cct  gaa atc cag cct atg  aag gaa gat gat ggg       3766
   Ala His Ala Asp Pro  Glu Ile Gln Pro Met  Lys Glu Asp Asp Gly
                   1210                 1215                 1220
   aca ttt gga gaa tac  agt gat gca gaa gac  cac aag cct ttg aaa       3811
   Thr Phe Gly Glu Tyr  Ser Asp Ala Glu Asp  His Lys Pro Leu Lys
                   1225                 1230                 1235
   aaa gga agt cga act  cct tca gac agg act  gtg aaa aaa gaa gat       3856
   Lys Gly Ser Arg Thr  Pro Ser Asp Arg Thr  Val Lys Lys Glu Asp
                   1240                 1245                 1250
   agt gac gac agc cta  gtt gac tat gga gaa  ggg gtt aat ggc cag       3901
   Ser Asp Asp Ser Leu  Val Asp Tyr Gly Glu  Gly Val Asn Gly Gln
                   1255                 1260                 1265
   ttc aat gag gat ggc  tcc ttt att gga caa  tac agt ggt aag aaa       3946
   Phe Asn Glu Asp Gly  Ser Phe Ile Gly Gln  Tyr Ser Gly Lys Lys
                   1270                 1275                 1280
   gag aaa gag ccg gct  gaa gga aac gaa agc  tca gag gca cct tct       3991
   Glu Lys Glu Pro Ala  Glu Gly Asn Glu Ser  Ser Glu Ala Pro Ser
                   1285                 1290                 1295
   cct gtc aac gcc atg  aat tcc ttt gtt taa tttttaagct ctttgccaat      4041
   Pro Val Asn Ala Met  Asn Ser Phe Val
                   1300
   attccatttc tctagaatgt ttatcctaag cacttgtttg tcagccctct catactatga   4101
   acatatgggt agagagtata ttttctgctg tatgttagta ttatgagaat agttacagca   4161
   aaaacataac tcagtcaaat gatatgttaa tatgaactgg aatgcaaagt gcatactttt   4221
   tcattcaaaa tgggtattct tgatttcctc agaactgata aaaaataatg caacatcacc   4281
   aacagatcct gttatttcct ctgcaggata cagttcaata tgatgcatga aaaatgctcc   4341
   acatttaaag gacatacccg tgtatgttat gaaaacatgg tttgatactt tgtttatact   4401
   accctcagct gaacccctat atatgaattc cgttttcatt gtcaagaatg ttactgtagt   4461
   attctctaga acttcaatgt ctttgtggac attgttgtga aattggtgac tatgtatagc   4521
   tgtcgttagt ctttttggga gactgttagg aacagtttgt acagtatata cttgctaaat   4581
   gagttcatta tgacagtcac attgctgatg cttactgaga actattacct actcttggct   4641
   cctgttactc cgtaggcttc ttaatcttcc aggcattaca gcagcacagt gttctacttt   4701
   ttacatcatt tctatgttcg gttgttttta ggcataaaca atgtgtattg cagtgcattt   4761
   cggcatttgt gccatactga aagaatcaaa aacaaatcat ccaaattaaa tttcaaacat 4821
   tatttcagag aacacagggc aagacacata cagtgccttc agatattaag cattccacaa 4881
   catcgtgcat tctgtatcag ctggtccagt ccattctggg tcctagatta ctgtcattgt 4941
   ctaaaagtaa cttttaaaaa gcagagttca tgaaaactgc aatgctggga aaagaaggaa 5001
   acatgaaaat aaaaataaga cagtttatta gaaatagcat ttcctcataa gcataaaaag 5061
   aaaatctttg ttgccaactg aagcacatga tgattttgtg gtcctttatg gtttctatta 5121
   cattcagtaa gaaagatgtc aacatgctag aaaattaatt ttaaaactaa gttattccaa 5181
   cactaaaagc atacaacagc atgccaacag taatatatta ttctccaaga ctttacctat 5241
   gtaagtgttc aaaactctgc agcattaaac aacgtgtatg caaattgtta tggatacatt 5301
   tcagaatcta agaaatcagg caagtgctta aaaggccaac ggtccaaggg attacatctg 5361
   cagtttaaaa agtaaatata tattctatcg tattcataaa caatatctat caaatgggtt 5421
   acctccaaat atgaaaatct ataacaacct atggttgaag gaatgctcag tttcatttgc 5481
   caataaattg gtttctcata acttgcatca agtttaattt taagtaaagc tttttatatg 5541
   tagatatttt gttgaatttg taaatacact taaaatgtag atgctatatg cttataggtg 5601
   ttacatacaa ataaacatgc aatgtttatg ttgtactgta taagaggtaa gctaattaat 5661
   gcagtgaatg ggattggaaa gcatctactt aaatatctat tgggttcccc cctcccccca 5721
   ccttttttgc tgtgaaactg aaatagtgaa cttttctacg tattgacagc agatttttcg 5781
   atgaaatctt cagagctttg cctatggggc acagtaggcc tagtaacctg gcatgtttga 5841
   tatatgtagg taaagcataa tttaaagtaa tcccaggtaa agatggccct aaatactttc 5901
   atgtctctat attcattttt cacagatcca cctgtctctt gaaaatataa aaagacaaaa 5961
   caggtttgcc ttggcatcag agagcacaaa gattaaaagt tactttaaat ttgccaatat 6021
   tttgggagaa caataaaact acattttttc ctcttccata ctggtagatg cgaaatttat 6081
   ctgtgcatga aagggtcact tctgtaatag tgcaacagat ttggtattaa aaattaaatg 6141
   tggttttaaa agttcctctc tcttttgtaa tttatgttcc caattgagtg tgaatgtcca 6201
   agtaatggtg tatgtaatgg tacaggcaaa tgtgactgga tttccctcaa aaaagtaact 6261
   attaaacagt cttgatctct ttgtgacttt taactttttc aaac 6305
<210> 68
   <211> 1304
   <212> PRT
   <213> Homo sapiens
<400> 68
   Met Gln Leu Lys Ile Met Pro Lys Lys Lys Arg Leu Ser Ala Gly Arg
   1 5 10 15
   Val Pro Leu Ile Leu Phe Leu Cys Gln Met Ile Ser Ala Leu Glu Val
   20 25 30
   Pro Leu Asp Pro Lys Leu Leu Glu Asp Leu Val Gln Pro Pro Thr Ile
   35 40 45
   Thr Gln Gln Ser Pro Lys Asp Tyr Ile Ile Asp Pro Arg Glu Asn Ile
   50 55 60
   Val Ile Gln Cys Glu Ala Lys Gly Lys Pro Pro Pro Ser Phe Ser Trp
   65 70 75 80
   Thr Arg Asn Gly Thr His Phe Asp Ile Asp Lys Asp Pro Leu Val Thr
   85 90 95
   Met Lys Pro Gly Thr Gly Thr Leu Ile Ile Asn Ile Met Ser Glu Gly
   100 105 110
   Lys Ala Glu Thr Tyr Glu Gly Val Tyr Gln Cys Thr Ala Arg Asn Glu
   115 120 125
   Arg Gly Ala Ala Val Ser Asn Asn Ile Val Val Arg Pro Ser Arg Ser
   130 135 140
   Pro Leu Trp Thr Lys Glu Lys Leu Glu Pro Ile Thr Leu Gln Ser Gly
   145 150 155 160
   Gln Ser Leu Val Leu Pro Cys Arg Pro Pro Ile Gly Leu Pro Pro Pro
   165 170 175
   Ile Ile Phe Trp Met Asp Asn Ser Phe Gln Arg Leu Pro Gln Ser Glu
   180 185 190
   Arg Val Ser Gln Gly Leu Asn Gly Asp Leu Tyr Phe Ser Asn Val Leu
   195 200 205
   Pro Glu Asp Thr Arg Glu Asp Tyr Ile Cys Tyr Ala Arg Phe Asn His
   210 215 220
   Thr Gln Thr Ile Gln Gln Lys Gln Pro Ile Ser Val Lys Val Ile Ser
   225 230 235 240
   Val Asp Glu Leu Asn Asp Thr Ile Ala Ala Asn Leu Ser Asp Thr Glu
   245 250 255
   Phe Tyr Gly Ala Lys Ser Ser Arg Glu Arg Pro Pro Thr Phe Leu Thr
               260                 265                 270
   Pro Glu Gly Asn Ala Ser Asn Lys Glu Glu Leu Arg Gly Asn Val Leu
           275                 280                 285
   Ser Leu Glu Cys Ile Ala Glu Gly Leu Pro Thr Pro Ile Ile Tyr Trp
       290                 295                 300
   Ala Lys Glu Asp Gly Met Leu Pro Lys Asn Arg Thr Val Tyr Lys Asn
   305                 310                 315                 320
   Phe Glu Lys Thr Leu Gln Ile Ile His Val Ser Glu Ala Asp Ser Gly
                   325                 330                 335
   Asn Tyr Gln Cys Ile Ala Lys Asn Ala Leu Gly Ala Ile His His Thr
               340                 345                 350
   Ile Ser Val Arg Val Lys Ala Ala Pro Tyr Trp Ile Thr Ala Pro Gln
           355                 360                 365
   Asn Leu Val Leu Ser Pro Gly Glu Asp Gly Thr Leu Ile Cys Arg Ala
       370                 375                 380
   Asn Gly Asn Pro Lys Pro Arg Ile Ser Trp Leu Thr Asn Gly Val Pro
   385                 390                 395                 400
   Ile Glu Ile Ala Pro Asp Asp Pro Ser Arg Lys Ile Asp Gly Asp Thr
                   405                 410                 415
   Ile Ile Phe Ser Asn Val Gln Glu Arg Ser Ser Ala Val Tyr Gln Cys
               420                 425                 430
   Asn Ala Ser Asn Glu Tyr Gly Tyr Leu Leu Ala Asn Ala Phe Val Asn
           435                 440                 445
   Val Leu Ala Glu Pro Pro Arg Ile Leu Thr Pro Ala Asn Thr Leu Tyr
       450                 455                 460
   Gln Val Ile Ala Asn Arg Pro Ala Leu Leu Asp Cys Ala Phe Phe Gly
   465                 470                 475                 480
   Ser Pro Leu Pro Thr Ile Glu Trp Phe Lys Gly Ala Lys Gly Ser Ala
                   485                 490                 495
   Leu His Glu Asp Ile Tyr Val Leu His Glu Asn Gly Thr Leu Glu Ile
               500                 505                 510
   Pro Val Ala Gln Lys Asp Ser Thr Gly Thr Tyr Thr Cys Val Ala Arg
           515                 520                 525
   Asn Lys Leu Gly Met Ala Lys Asn Glu Val His Leu Glu Ile Lys Asp
       530                 535                 540
   Pro Thr Trp Ile Val Lys Gln Pro Glu Tyr Ala Val Val Gln Arg Gly
   545                 550                 555                 560
   Ser Met Val Ser Phe Glu Cys Lys Val Lys His Asp His Thr Leu Ser
                   565                 570                 575
   Leu Thr Val Leu Trp Leu Lys Asp Asn Arg Glu Leu Pro Ser Asp Glu
               580                 585                 590
   Arg Phe Thr Val Asp Lys Asp His Leu Val Val Ala Asp Val Ser Asp
           595                 600                 605
   Asp Asp Ser Gly Thr Tyr Thr Cys Val Ala Asn Thr Thr Leu Asp Ser
       610                 615                 620
   Val Ser Ala Ser Ala Val Leu Ser Val Val Ala Pro Thr Pro Thr Pro
   625                 630                 635                 640
   Ala Pro Val Tyr Asp Val Pro Asn Pro Pro Phe Asp Leu Glu Leu Thr
                   645                 650                 655
   Asp Gln Leu Asp Lys Ser Val Gln Leu Ser Trp Thr Pro Gly Asp Asp
               660                 665                 670
   Asn Asn Ser Pro Ile Thr Lys Phe Ile Ile Glu Tyr Glu Asp Ala Met
           675                 680                 685
   His Lys Pro Gly Leu Trp His His Gln Thr Glu Val Ser Gly Thr Gln
       690                 695                 700
   Thr Thr Ala Gln Leu Lys Leu Ser Pro Tyr Val Asn Tyr Ser Phe Arg
   705                 710                 715                 720
   Val Met Ala Val Asn Ser Ile Gly Lys Ser Leu Pro Ser Glu Ala Ser
                   725                 730                 735
   Glu Gln Tyr Leu Thr Lys Ala Ser Glu Pro Asp Lys Asn Pro Thr Ala
               740                 745                 750
   Val Glu Gly Leu Gly Ser Glu Pro Asp Asn Leu Val Ile Thr Trp Lys
           755                 760                 765
   Pro Leu Asn Gly Phe Glu Ser Asn Gly Pro Gly Leu Gln Tyr Lys Val
       770                 775                 780
   Ser Trp Arg Gln Lys Asp Gly Asp Asp Glu Trp Thr Ser Val Val Val
   785                 790                 795                 800
   Ala Asn Val Ser Lys Tyr Ile Val Ser Gly Thr Pro Thr Phe Val Pro
                   805                 810                 815
   Tyr Leu Ile Lys Val Gln Ala Leu Asn Asp Met Gly Phe Ala Pro Glu
               820                 825                 830
   Pro Ala Val Val Met Gly His Ser Gly Glu Asp Leu Pro Met Val Ala
           835                 840                 845
   Pro Gly Asn Val Arg Val Asn Val Val Asn Ser Thr Leu Ala Glu Val
       850                 855                 860
   His Trp Asp Pro Val Pro Leu Lys Ser Ile Arg Gly His Leu Gln Gly
   865                 870                 875                 880
   Tyr Arg Ile Tyr Tyr Trp Lys Thr Gln Ser Ser Ser Lys Arg Asn Arg
                   885                 890                 895
   Arg His Ile Glu Lys Lys Ile Leu Thr Phe Gln Gly Ser Lys Thr His
               900                 905                 910
   Gly Met Leu Pro Gly Leu Glu Pro Phe Ser His Tyr Thr Leu Asn Val
           915                 920                 925
   Arg Val Val Asn Gly Lys Gly Glu Gly Pro Ala Ser Pro Asp Arg Val
       930                 935                 940
   Phe Asn Thr Pro Glu Gly Val Pro Ser Ala Pro Ser Ser Leu Lys Ile
   945                 950                 955                 960
   Val Asn Pro Thr Leu Asp Ser Leu Thr Leu Glu Trp Asp Pro Pro Ser
                   965                 970                 975
   His Pro Asn Gly Ile Leu Thr Glu Tyr Thr Leu Lys Tyr Gln Pro Ile
               980                 985                 990
   Asn Ser Thr His Glu Leu Gly Pro  Leu Val Asp Leu Lys  Ile Pro Ala
           995                 1000                 1005
   Asn Lys  Thr Arg Trp Thr Leu  Lys Asn Leu Asn Phe  Ser Thr Arg
       1010                 1015                 1020
   Tyr Lys Phe Tyr Phe Tyr Ala Gln Thr Ser Ala Gly Ser Gly Ser
   1025 1030 1035
   Gln Ile Thr Glu Glu Ala Val Thr Thr Val Asp Glu Ala Gly Ile
   1040 1045 1050
   Leu Pro Pro Asp Val Gly Ala Gly Lys Val Gln Ala Val Asn Pro
   1055 1060 1065
   Arg Ile Ser Asn Leu Thr Ala Ala Ala Ala Glu Thr Tyr Ala Asn
   1070 1075 1080
   Ile Ser Trp Glu Tyr Glu Gly Pro Glu His Val Asn Phe Tyr Val
   1085 1090 1095
   Glu Tyr Gly Val Ala Gly Ser Lys Glu Glu Trp Arg Lys Glu Ile
   1100 1105 1110
   Val Asn Gly Ser Arg Ser Phe Phe Gly Leu Lys Gly Leu Met Pro
   1115 1120 1125
   Gly Thr Ala Tyr Lys Val Arg Val Gly Ala Val Gly Asp Ser Gly
   1130 1135 1140
   Phe Val Ser Ser Glu Asp Val Phe Glu Thr Gly Pro Ala Met Ala
   1145 1150 1155
   Ser Arg Gln Val Asp Ile Ala Thr Gln Gly Trp Phe Ile Gly Leu
   1160 1165 1170
   Met Cys Ala Val Ala Leu Leu Ile Leu Ile Leu Leu Ile Val Cys
   1175 1180 1185
   Phe Ile Arg Arg Asn Lys Gly Gly Lys Tyr Pro Val Lys Glu Lys
   1190 1195 1200
   Glu Asp Ala His Ala Asp Pro Glu Ile Gln Pro Met Lys Glu Asp
   1205 1210 1215
   Asp Gly Thr Phe Gly Glu Tyr Ser Asp Ala Glu Asp His Lys Pro
   1220 1225 1230
   Leu Lys Lys Gly Ser Arg Thr Pro Ser Asp Arg Thr Val Lys Lys
   1235 1240 1245
   Glu Asp Ser Asp Asp Ser Leu Val Asp Tyr Gly Glu Gly Val Asn
       1250                1255                1260
   Gly Gln  Phe Asn Glu Asp Gly  Ser Phe Ile Gly Gln  Tyr Ser Gly
       1265                1270                1275
   Lys Lys  Glu Lys Glu Pro Ala  Glu Gly Asn Glu Ser  Ser Glu Ala
       1280                1285                1290
   Pro Ser  Pro Val Asn Ala Met  Asn Ser Phe Val
       1295                1300
<210> 69
   <211> 5166
   <212> DNA
   <213> Homo sapiens
<220>
   <221> CDS
   <222> (240)..(2501)
   <223>
<400> 69
atttccattc tggctgggaa gggctggggc tccactcagc ctggagaccg aagcgcttca     60
   ctgagcgctc gccgccgccc agcctctcct ctcgcgcctc ctagctcttc gcagagcaac    120
   caggagccag gagtggtcta gagcccgagg gtgggaaggg ggagtctgtc tggcttttct    180
   cctatcttgc ttctttttcc tcttcccttc ccactcttgt tcaagcgagt gtgtgagct     239
   atg gag cga aga gcc tgg agt ctg cag tgc act gct ttc gtc ctc ttt      287
   tgc gct tgg tgt gca ctg aac agt gca aaa gcg aaa agg caa ttt gtc      335
   aat gaa tgg gca gcg gag atc ccc ggg ggc ccg gaa gca gcc tcg gcc      383
   atc gcc gag gag ctg ggc tat gac ctt ttg ggt cag att ggt tca ctt      431
   gaa aat cac tac tta ttc aaa cat aaa aac cac ccc aga agg tct cga      479
   agg agt gcc ttt cat atc act aag aga tta tct gat gat gat cgt gtg      527
   ata tgg gct gaa caa cag tat gaa aaa gaa aga agt aaa cgt tca gct      575
   cta agg gac tca gca cta aat ctc ttc aat gat ccc atg tgg aat cag      623
   caa tgg tac ttg caa gat acc agg atg acg gca gcc ctg ccc aag ctg       671
   gac ctt cat gtg ata cct gtt tgg caa aaa ggc att acg ggc aaa gga       719
   gtt gtt atc acc gta ctg gat gat ggt ttg gag tgg aat cac acg gac       767
   att tat gcc aac tat gat cca gag gct agc tat gat ttt aat gat aat       815
   gac cat gat cca ttt ccc cga tat gat ccc aca aac gag aac aaa cac       863
   ggg acc aga tgt gca gga gaa att gcc atg caa gca aat aat cac aaa       911
   tgc ggg gtt gga gtt gca tac aat tcc aaa gtt gga ggc ata aga atg       959
   ctg gat ggc att gtg acg gat gct att gag gcc agt tca att gga ttc      1007
   aat cct gga cac gtg gat att tac agt gca agc tgg ggc cct aat gat      1055
   gat ggg aaa act gtg gag ggg cct ggc cgg cta gcc cag aag gct ttt      1103
   gaa tat ggt gtc aaa cag ggg aga cag ggg aag ggg tcc atc ttc gtc      1151
   tgg gct tcg gga aac ggg ggg cgt cag gga gat aat tgt gac tgt gat      1199
   ggc tac aca gac agc atc tac acc atc tcc atc agc agt gcc tcc cag      1247
   caa ggc cta tcc ccc tgg tac gct gag aag tgc tcc tcc aca ctg gcc      1295
   acc tct tac agc agc gga gat tac acc gac cag aga atc acg agc gct      1343
   gac ctg cac aat gac tgc acg gag acg cac aca ggc acc tcg gcc tct      1391
   gca cct ctg gct gct ggc atc ttc gct ctg gcc ctg gaa gca aac cca      1439
   aat ctc acc tgg cga gat atg cag cac ctg gtt gtc tgg acc tct gag      1487
   tat gac ccg ctg gcc aat aac cct gga tgg aaa aag aat gga gca ggc      1535
   ttg atg gtg aat agt cga ttt gga ttt ggc ttg cta aat gcc aaa gct      1583
   ctg gtg gat tta gct gac ccc agg acc tgg agg agc gtg cct gag aag      1631
   aaa gag tgt gtt gta aag gac aat gac ttt gag ccc aga gcc ctg aaa      1679
   gct aat gga gaa gtt atc att gaa att cca aca aga gct tgt gaa gga      1727
   caa gaa aat gct atc aag tcc ctg gag cat gta caa ttt gaa gca aca      1775
   att gaa tat tcc cga aga gga gac ctt cat gtc aca ctt act tct gct      1823
   gct gga act agc act gtg ctc ttg gct gaa aga gaa cgg gat aca tct      1871
   cct aat ggc ttt aag aat tgg gac ttc atg tct gtt cac aca tgg gga      1919
   gag aac cct ata ggt act tgg act ttg aga att aca gac atg tct gga      1967
   aga att caa aat gaa gga aga att gtg aac tgg aag ctg att ttg cac      2015
   ggg acc tct tct cag cca gag cat atg aag cag cct cgt gtg tac acg      2063
   tcc tac aac act gtt cag aat gac aga aga ggg gtg gag aag atg gtg      2111
   gat cca ggg gag gag cag ccc aca caa gag aac cct aag gag aac acc     2159
   ctg gtg tcc aaa agc ccc agc agc agc agc gta ggg ggc cgg agg gat     2207
   gag ttg gag gag gga gcc cct tcc cag gcc atg ctg cga ctc ctg caa     2255
   agt gct ttc agt aaa aac tca ccg cca aag caa tca cca aag aag tcc     2303
   cca agt gca aag ctc aac atc cct tat gaa aac ttc tac gaa gcc ctg     2351
   gaa aag ctg aac aaa cct tcc cag ctt aaa gac tct gaa gac agt ctg     2399
   tat aat gac tat gtt gat gtt ttt tat aac act aaa cct tac aag cac     2447
   aga gac gac cgg ctg ctt caa gct ctg gtg gac att ctg aat gag gaa     2495
   aat taa aataagtgtg tggtcccaag ttggaaatat tcatgcttct tccttaccct      2551
   gcgattttgc ctgtgtctga agtggttgtt ttgtcatgaa ttcttatgct tataatatcc   2611
   tttgtggcac cttttctttt tctccctaaa ctgtacatgt gaaggggatg agctcaagca   2671
   ggaagttcaa cttccagaat tgatcatagg tatttcaaaa cacatctttc ctgtctgcac   2731
   aagtgaagtg ttttgttctt tctggagtca cagttgacaa aaagctctta cactacatta   2791
   gaacactgca ttagagccca tttcaattct caaaagaaaa ggcaaaacct gggatatcaa   2851
   ttaatttgaa aacataatct gcaaagaatg agaaggagtc agaaactgtt tctgtagctt   2911
   gttccctgtc ttgtccatgt ggttcttcaa attttgatgc caagaaagta tttggtaggc   2971
   ctaatgaagg agttcactgt aagactcatt ccctagatct ttctattcca aagtgccact   3031
   cattcctgta gtcaaaatct ggtcatgttg gtcaaaagct ggattattta gatctagaaa   3091
   cagatcttga aatctgaatg ctctggtttg agcaattttc gaacattctt tgcctggtgc   3151
   actgtgtctg tggtgccaga ggcgtccgtg gatccagagg tggttatgac tcgtgctgca   3211
   tgcctggtct ttcctctgtt tctccttctg aaagttttct atacctgtct cctttctcag   3271
   ccacaaaata aatgttggga gaaatgatat ataccacttt cccagaaaaa aaaaaactta   3331
   cacttgggac ttggcaaatt cctagtcaca atttttttca gcagtaacag gaaaccactt 3391
   atcacatgga gacctaatgt aataatagaa aaatactcat aatagggaga aaccaagaga 3451
   agttttgttt ttgttttttt ccaactgtgt tcattagaac agcgtgttct aagtatttga 3511
   aactgaatgt ttattccttg atactaaaag ttcttctcca atcctatcac tgatagtgtc 3571
   caaattctca ccaaattgct cctaagcttc aaatcagaag cagaaactgg caggccatgg 3631
   accttaattg tccctcaggt agattttgtt tggtatgcag aatgttttta aaatatgagt 3691
   ggttattgaa aatatgatgt ttcacataaa acctcattct cggacccatc tttgctcatg 3751
   gcaacagtta gctggagctg agtagcagct gcctgattag atgactctca gtccccatgg 3811
   caccctgctc catgttacct agagcaggca cttgattcct tgctgggcag tatccaatag 3871
   gcatttgatt ttgcccactc ctacactaag cgaatgtgta caaagtgtaa atgcattagg 3931
   aaaaacaaac tacccgcatc ttctgttagg caggatctgt acaataataa ttatgagttt 3991
   gcttatgtaa tctcacctca cctggatgat cactaatact aattcattta ttactaacct 4051
   tctggcttcc ttctctcaat atgcttacaa agtctccagt cacctacaat gctggctttc 4111
   tcccactgag tttgctgttt gcaatttttc catgaagttt gaacttcata aggtaattca 4171
   tggcattgaa ctggttcatg aaaagaacac tagagtctgt catttgcttt ggcttgaagt 4231
   atggttggta acacaaattt tcacctgctc ttctaccatt tgaatttgtg tagagggtgt 4291
   ttgcagagca atgcccgtaa tgcttagaga atgttctcct aaaagacttg cggaatcact 4351
   ctgtccttgg aagtttcata tattgtttga tatgaagtgt tagatagaat ttccaatatt 4411
   ggagcatatc aaaaagtatt aaaactaaaa aggaccagag aattcttaga ttggcccgga 4471
   aaggccaata aagagttaga atgaaaactc attacttttc cattcccaat ctagtgctag 4531
   atgtataaat ctttcttttg attcttccta acaaaatatt ttctgggtta aaaccccagc 4591
   caactcattg ggttgtagcc aaaggttcac tctcaagaag ctttaatatt taaataaaat 4651
   catattgaat gtttccaacc tggagtataa tattcagata taaaacagtt ttgtcagtct 4711
   ttcttagtgc ctgtgtggat ttttgtgaaa atgtcaaaga gaaaacttat atactatttc 4771
   ccttgaaatt ttaaactata ttttctttac aggtatttat aatataccaa tgcttttatc 4831
   aaacagaatt ttaaagagca taataaatta tattaaagaa ccaaaagttt tcctgagaat 4891
   aagaaagttt cacccaataa aatatttttg aaaggcatgt tcctctgtca atgaaaaaaa 4951
   gtacatgtat gtgttgtgat attaaaagtg acatttgtct aatagcctaa tacaacatgt 5011
   agctgagttt aacatgtgtg gtcttggtat tcttaaggga acttccacat tatacatttg 5071
   atgtattgac cagaatatgt aaaatatgct tataaatcag aaaaataaat tgtttctcac 5131
   taagtcaaaa gaaaaaaagc tcatgtttcc accac 5166
<210> 70
   <211> 753
   <212> PRT
   <213> Homo sapiens
<400> 70
<210> 71
   <211> 11457
   <212> DNA
   <213> Homo sapiens
<220>
   <221> CDS
   <222> (759)..(6443)
   <223>
<400> 71
cgccgccgcc gccgccaccg cgggagccag agctgccggg aggagcggca tccgcgccag 60
   actggagcgg gagggcggcg gagggcagtt gctgggaatt tttcagccga gagggcgagc 120
   gatccggaga gagaccccga gagcttggga gcggtagggc gtgcgagcgc cgcagccagc 180
   ggagcaaacc tcgaaataga tctggaaagc caggctcccg gaggaaatgg gactgtgaac 240
   gaaccggaga gcaagaaggg aaggaagcgc cgggattgct gatgtcagag gagcccggaa 300
   agtcgcgctg gaaaaatctg aagacagccg gggctctgct tcttcctcag gagagacacc 360
   gccggccgcc cccacacgcc ccctcggcgc ctccgggtgc cccctgagag ccggcgacag 420
   cgcccagccg ggctgctgcg gggcgacgga ggactgaggg gcgcgcggag cggagaccga 480
   ggagcgactt caggaataca gataagtgtc tggtggcttg acgtggattt taatgaattt 540
   ggactccatg tggatttggt cgtctccctg attccgagct gcgggcaggg agaggggcct 600
   cgcgccgccc tcagcagccg gcggcggccg aggtagaccg agcggggacg gaaggacaga 660
   ccgacgtcgc cgagctggaa tcatgtgagg gccaaccggg gaaggtggag cagatgagca 720
   cacacaggag ccgtctcctc accgccgccc ctctcagc atg gaa cag agg cgg ccc 776
   tgg ccc cgg gcc ctg gag gtg gac agc cgc tct gtg gtc ctg ctc tca 824
   gtg gtc tgg gtg ctg ctg gcc ccc cca gca gcc ggc atg cct cag ttc 872
   agc acc ttc cac tct gag aat cgt gac tgg acc ttc aac cac ttg acc 920
   gtc cac caa ggg acg ggg gcc gtc tat gtg ggg gcc atc aac cgg gtc 968
   tat aag ctg aca ggc aac ctg acc atc cag gtg gct cat aag aca ggg     1016
   cca gaa gag gac aac aag tct tgt tac ccg ccc ctc atc gtg cag ccc     1064
   tgc agc gaa gtg ctc acc ctc acc aac aat gtc aac aag ctg ctc atc     1112
   att gac tac tct gag aac cgc ctg ctg gcc tgt ggg agc ctc tac cag     1160
   ggg gtc tgc aag ctg ctg cgg ctg gat gac ctc ttc atc ctg gtg gag     1208
   cca tcc cac aag aag gag cac tac ctg tcc agt gtc aac aag acg ggc     1256
   acc atg tac ggg gtg att gtg cgc tct gag ggt gag gat ggc aag ctc     1304
   ttc atc ggc acg gct gtg gat ggg aag cag gat tac ttc ccg acc ctg     1352
   tcc agc cgg aag ctg ccc cga gac cct gag tcc tca gcc atg ctc gac     1400
   tat gag cta cac agc gat ttt gtc tcc tct ctc atc aag atc cct tca     1448
   gac acc ctg gcc ctg gtc tcc cac ttt gac atc ttc tac atc tac ggc     1496
   ttt gct agt ggg ggc ttt gtc tac ttt ctc act gtc cag ccc gag acc     1544
   cct gag ggt gtg gcc atc aac tcc gct gga gac ctc ttc tac acc tca     1592
   cgc atc gtg cgg ctc tgc aag gat gac ccc aag ttc cac tca tac gtg     1640
   tcc ctg ccc ttc ggc tgc acc cgg gcc ggg gtg gaa tac cgc ctc ctg     1688
   cag gct gct tac ctg gcc aag cct ggg gac tca ctg gcc cag gcc ttc     1736
   aat atc acc agc cag gac gat gta ctc ttt gcc atc ttc tcc aaa ggg      1784
   cag aag cag tat cac cac ccg ccc gat gac tct gcc ctg tgt gcc ttc      1832
   cct atc cgg gcc atc aac ttg cag atc aag gag cgc ctg cag tcc tgc      1880
   tac cag ggc gag ggc aac ctg gag ctc aac tgg ctg ctg ggg aag gac      1928
   gtc cag tgc acc aag gcg cct gtc ccc atc gat gat aac ttc tgt gga      1976
   ctg gac atc aac cag ccc ctg gga ggc tca act cca gtg gag ggc ctg      2024
   acc ctg tac acc acc agc agg gac cgc atg acc tct gtg gcc tcc tac      2072
   gtt tac aac ggc tac agc gtg gtt ttt gtg ggg act aag agt ggc aag      2120
   ctg aaa aag att cgg gcc gac ggt ccc ccc cat ggt ggg gtc cag tac      2168
   gag atg gtc tct gtg ctc aag gac gga agc ccc atc ctc cgg gac atg      2216
   gcc ttc tcc att gat cag cgc tac ctg tac gtc atg tct gag aga cag      2264
   gtc acc agg gtc ccc gtg gag tca tgt gag cag tat acg act tgt ggg      2312
   gag tgc ctg agc tct ggg gac cct cac tgt ggc tgg tgt gcc ctg cac      2360
   aac atg tgc tcc cgc agg gac aaa tgc caa cag gcc tgg gaa cct aat      2408
   cga ttt gct gcc agc atc agc cag tgt gtg agc ctt gca gtg cat ccc      2456
   agc agc atc tca gta tct gag cac agc cgg ttg ctt agc ctg gta gtg      2504
   agt gat gct cct gat cta tct gcg ggt atc gcc tgt gcc ttt ggg aac      2552
   ctg aca gag gtg gag ggg cag gtg tcc ggg agc cag gtc atc tgc atc      2600
   tca cct ggg ccc aag gat gtc cct gtc atc ccg ctg gat caa gac tgg      2648
   ttt ggg ctg gag cta cag ctg agg tcc aag gag aca ggg aag ata ttt      2696
   gtc agc acc gag ttc aag ttt tac aac tgc agt gcc cac caa ctg tgc      2744
   ctg tcc tgt gtc aac agc gcc ttc cgc tgc cat tgg tgc aag tac cgc      2792
   aac ctc tgc act cat gac ccc acc acc tgc tcc ttc cag gag ggc cgg      2840
   atc aat att tca gag gac tgt ccc cag ctg gtg ccc aca gag gag atc      2888
   ttg att cca gtc ggg gag gta aag cca atc acc ctt aag gcg cga aat      2936
   ctg ccc cag ccg cag tcc ggc cag cga ggc tat gag tgt gtc ctc aac      2984
   ata caa gga gcc atc cac cgg gtc ccc gct ctg cgc ttc aac agc tcc      3032
   agc gtt cag tgt cag aac agc tcg tac cag tat gat ggc atg gac atc      3080
   agc aat ctg gcc gtg gat ttc gct gtg gtg tgg aac ggc aat ttc atc      3128
   att gac aac cct cag gac ctg aaa gtc cat ctc tac aag tgt gca gcc      3176
   cag cgg gag agc tgc ggc ctc tgc ctc aag gcc gac cgg aag ttt gag      3224
   tgt ggc tgg tgc agc ggc gag cgc agg tgc acc ctc cac cag cac tgt      3272
   acc agc cct tcc agc ccc tgg ctc gac tgg tcc agc cac aat gtc aag      3320
   tgc tcc aac cct caa atc acc gag att ttg acg gtg tct gga ccg ccg      3368
   gaa gga ggg acg cga gtg acc atc cat ggc gtg aac ctg ggt ctg gac      3416
   ttc tcc gag atc gcc cac cat gtg cag gtg gct ggg gtg ccc tgc acg      3464
   ccc ctc cca ggg gaa tac atc atc gct gag cag att gtc tgt gag atg      3512
   ggc cat gcc ctc gtg gga acc acc tcc ggg cca gta cgc ctg tgt att      3560
   ggc gag tgt aag cca gag ttc atg acg aag tcc cat cag cag tac acc      3608
   ttc gtg aac cct tct gtg ctg tca ctc aac cca atc cga ggt ccc gag      3656
   tca gga ggc act atg gtg acc att acc ggc cat tac ctt ggg gct ggg      3704
   agc agc gtg gca gtc tac ctg ggc aac cag acc tgc gag ttc tac ggg      3752
   agg tca  atg agt gag atc gtg  tgt gtc tca ccc cca  tca tcc aat      3797
   ggc ctt  ggc ccg gtc cct gtt  tct gtg agt gtc gac  cga gcc cat      3842
   gtg gat  agc aac ctg cag ttt  gag tac ata gat gac  cct cgg gtc      3887
   cag cgc  atc gag cca gag tgg  agc att gcc agt ggc  cac aca ccc      3932
   ctg acc  atc aca ggc ttc aac  ctg gat gtc att cag  gag cca agg      3977
   atc cga  gtc aaa ttc aat ggc  aaa gaa tct gtc aat  gtg tgt aaa      4022
   gtt gtg  aac aca acc acc ctc  acc tgc ctg gca ccc  tct ctg acc      4067
   acg gac  tac cgc cct ggc ctg  gac act gtg gaa cgc  cca gat gag      4112
   ttt gga  ttt gtc ttt aac aat  gtc caa tcc ttg cta  att tac aac      4157
   gac acc  aag ttt atc tac tac  ccc aac ccg acc ttt  gaa ctg ctt      4202
   agc cct  act gga gtc ttg gat  caa aag cca gga tcg  ccc atc att      4247
   ctg aag  ggc aaa aac ctc tgc  cct cct gcc tct gga  ggg gcc aaa      4292
   ctc aac  tac act gtg ctc atc  gga gag acc cct tgt  gct gtc acc      4337
   gta tct  gag acc cag ctt ctc  tgc gag cct ccc aac  ctc acc ggg      4382
   cag cac  aag gtc atg gtt cac  gtg ggc ggg atg gtg  ttc tcg cct      4427
   ggc tcg  gtg agt gtc atc tca  gac agc ttg ctg acc  ctg cca gcc      4472
   atc gtc  agc atc gcg gcc ggc  ggc agc ctc ctc ctc  atc atc gtc      4517
   atc atc  gtc ctc att gcc tac  aag cgc aag tct cga  gaa aat gac      4562
   ctc act  ctc aag cgg ctg caa  atg cag atg gac aat  ctg gag tcc      4607
   cgt gtg  gcc ttg gag tgc aag  gaa gct ttt gct gag  ctc cag acg      4652
   gat atc  aat gag ttg acc agt  gac ctg gac cgc tca  gga atc cct      4697
   tac ctg  gac tat cgt acc tac  gct atg cga gtc ctg  ttc ccg ggc        4742
   atc gag  gac cac ccc gtc ctg  cgg gag ctg gag gta  caa gga aac        4787
   ggg cag  cag cac gtg gag aag  gcc ctg aag ctc ttt  gcc cag ctc        4832
   atc aac  aac aag gtg ttc ctg  ctg acc ttc atc cgc  acc ctg gag        4877
   ctg cag  cgc agt ttc tcc atg  cgc gac cgg ggc aac  gtg gct tcg        4922
   ctc atc  atg acc ggc ctg cag  ggc cgc ctg gaa tat  gcc act gat        4967
   gtc ctc  aag cag ctg ctc tct  gac ctc atc gat aag  aac ctg gag        5012
   aac aag  aac cac ccc aag ctg  cta ctc cgg agg aca  gag tct gtg        5057
   gct gaa  aag atg ctg acc aat  tgg ttc gcc ttc ctc  ctg cac aag        5102
   ttc cta  aag gag tgc gca ggg  gag cca ctc ttc atg  cta tac tgt        5147
   gcc atc  aag cag cag atg gag  aag ggc ccc att gat  gcc atc acg        5192
   ggc gag  gcc cgc tac tcc ctg  agc gag gac aag ctc  atc cgg cag        5237
   cag atc  gag tac aag acc ctg  atc ctg aac tgc gtc  aac cct gac        5282
   aac gag  aac agt cca gag atc  cca gtg aag gtg tta  aac tgt gac        5327
   acc atc  aca cag gtc aag gag  aag att ctt gat gcc  gtg tat aag        5372
   aat gtg ccc tat tcc cag cgg ccg agg gca gtg gac atg gac ttg      5417
   gag tgg cgc caa ggc cgg atc gcc cgg gtc gtg ctg caa gat gag      5462
   gac atc acc acc aag att gag ggt gac tgg aag cgg ctc aac aca      5507
   ctg atg cat tat cag gtg tca gac agg tcg gtg gtg gct ctg gtc      5552
   ccc aaa cag acc tcc tcc tac aac atc cct gcc tct gcc agc atc      5597
   tcc cgg acg tcc atc agc aga tac gac tcc tcc ttc agg tat acg      5642
   ggc agc ccc gac agc ctg cgg tcc cgg gcc ccg atg atc acc cca      5687
   gac ctg gaa agt ggg gtc aag gtg tgg cat ctg gtg aag aac cat      5732
   gac cac ggt gac cag aag gag ggt gac cgg ggc agc aag atg gtg      5777
   tcc gag atc tac ctg acc cgg cta ctg gcc acc aag ggc acc ctg      5822
   cag aag ttt gtg gac gac ttg ttt gag acc ttg ttc agc act gtg      5867
   cac cgg ggc agc gct ctc ccc ctg gcc atc aag tac atg ttt gat      5912
   ttc cta gat gag cag gca gac agg cac agc atc cat gac aca gat      5957
   gtg cgg cac acc tgg aaa agc aac tgc ctc cct ctg cgc ttc tgg      6002
   gtg aac gtg att aag aac ccc cag ttc gtg ttt gac atc cac aag      6047
   ggc agc atc acg gac gcc tgc ctc tct gtg gtg gcc cag acc ttc      6092
   atg gac  tct tgt tca acg tca  gag cac cgg ctg ggc  aag gac tcc      6137
   ccc tcc  aac aag ctg ctc tat  gcc aag gac atc ccc  agc tac aag      6182
   agc tgg  gtg gag aga tac tac  gca gac atc gcc aag  ctc cca gcc      6227
   atc agt  gac cag gac atg aat  gcc tac ctc gcc gag  cag tcc cgc      6272
   ctg cac  gcc gtg gag ttc aac  atg ctg agt gcc ctc  aat gag atc      6317
   tac tcc  tat gtc agc aag tat  agt gag gag ctc atc  ggg gcc cta      6362
   gag cag  gat gag cag gca cgg  cgg cag cgg ctg gct  tat aag gtg      6407
   gag cag  ctc att aat gcc atg  tcc att gag agc tga gaggaggagc        6453
   ctcgcattcc tgggaagagg gacctgtcca agctgtcaca ctgggagtct cagatggaag   6513
   gacaagtgat ggggatcagg ccccagagct tgctgtcccc tgagacccca tcctggggag   6573
   aggggaggac tcctctccct acgccagcca agtttcgtca tagccagttc cagctgggag   6633
   agacagtggg cgtcgtccat cctcagtgag aacaccagag aacccggggc cgggagaagg   6693
   tggttcttca agccgagagg cacgagctgg ggacagttct gcctctgtga ctgctgcttt   6753
   gcatgaaaac tcatttgatg tatattgggg aaataatgag aactttattt aattttttta   6813
   agaaaaaggg aaaaaaacag aaataaaaca aaaagccgcc ctgttaatcc cgtccaactt   6873
   ttgtttaatt ctgatttctg tctcccttcc atcttttctc ccattcctcc ttctttatat   6933
   aatgcctatt tccaaatgcc agagaaagca gagatgctga gagacattgg agagaaaatg   6993
   actgtctcct tttccttgaa attaaaaaaa aaaaaaaaaa gagaaagagg agaagaagaa   7053
   tgatgagcac aagtatgcac caaacacttc gcaaaaacag aggccagtaa aacctggaat   7113
   tatcccggca gccagaggag tatggaactt ccagaacttt gcacaaattg caaagccatc   7173
   aagagctcac cctggctgac tggaaactga gctttatcta ccacacacct gtatattctc   7233
   atcttttgag aggagatgtg tacctagata gtaccaatgc tttttgctac tgttttttgt    7293
   tttgttttat ttaatcctaa acctcaacaa atgaggagct ggtctttgat atgtttcctt   7353
   tcaatttccc taaagttact atgagaagtg gggtgaggtg ggcctctccc agaccagaca 7413
   cctggcagcc ctgcctcata tcaatccctg tcataaacca ggcaccctgg ggaaacggcc 7473
   tggaggtgtg tgggccaggc ctccacgagg ttccatttga aagttgattt ggagacatag 7533
   gtgtttgact ttggagttca ctccaatcat ccagtggtcc ctggcaatta aaaagaaaac 7593
   aaaaatcaaa cactgtttac agcaagcaat acttgaagag cataggttac agaagctgca 7653
   gtatttatta ttatgctttc tttctttcat tctctcgtgc ctggagaggg gagaccaccc 7713
   ttcgctcata tacggaagct cctgaccatc tgggcctaca gcacttcctc agtagaaatg 7773
   actgtggcat gcccacgtta ctaccttctg cctctctttc tgcctctcac ggacttgtga 7833
   gtgtgaagga caagtggatg acttctcact ggactttcct tctctgtctc ttctgatgat 7893
   gcctaaaact atggataacc aattctcctg agtgtagatt ccaaacaaag agaccaaagc 7953
   tccatgccca ggtccaaagg ccccatagaa gccagtggga gtcatcgaga ggaaaggcgc 8013
   tctgtgagtg tcaggacctt ggtggccagg atagccatca gagtgtccag gcctcccaca 8073
   ttaccttgga tccgagcagc cagctccaac tgttctgcaa gcagcactgg agtcaggggg 8133
   taggaggaca agtggaagca aaggtgtggg actggggaag acaaagagga acaagctgcc 8193
   cttcctcact tttcaaaggg tcaggaatcc taggctatga tgctgggaag ctagaccagc 8253
   tcctccaaga gagactagac cagggtcatt ttctctgtta ttaactctgg gctccagctt 8313
   ctccgtgccc tgctttacct ccaagtggtt ccaatttcca aaggccctgc tgaccacatg 8373
   tgattcccag gagagggctg ggggagggga gcgcagaggt ctggcttcca tccttggcgt 8433
   gtagctttgg atcgctgtct aaccacacag cagacgttgc ccggtctccc cagctctagt 8493
   ttcttgcctg aatgcggctg acaaatggga agagaaaagc attcagcaaa atactcagga 8553
   aacttgctgt tttcattata attcacaacc agccatgcca aggccacttt cttttgaaaa 8613
   tccacttctt taaagtttct caggccctat tagtagcctg aaggaaatac taatgactgg 8673
   ccttccgcac taagccaaag tgtttgctct tcatagcact caaagcttat cagcgcagag 8733
   cccataattt atggagataa aaggaaagga gatataggta agaagagtgt gaccaggaga 8793
   ccttatgcta cctgtaaaaa agttcagccc accccatcta acttctgagc tctgtttggg 8853
   tgaagatttt ctggccgcat ggctgctcag actggcatcc aggctttgct ccaccaagaa 8913
   gttaaaggca gtcggacatc tcagtagcat ctctaccagc ccttaactca atgcatctac 8973
   ctggcatctc ccagcagtta cttttggaga cgattcactg cccctggggc gtttccttga 9033
   aggtttgtgg agagcgtgga gaatgatggg gcaatggcca attggagggt ggagagtgaa 9093
   gagccggagc tggctgtgag tggtttggcc acatttctca ggattccatg agagacttgg 9153
   ggaacttggg ctgacaaagg aagtagcctg gggcatcctt aaggaaggaa ttaagaaaag 9213
   ggaaaaagct ggactcaagc cacgccatga gggtgaaagg ttataaggcc ctgccccctt 9273
   tccagctgcc cacctgttcc tcctccacac ctcttcgctt tgggccacca agaaccaatg 9333
   aagtccacac cctttggatg agaaaaagag ggagttggtt ggcctctctt ctccctgtta 9393
   tccaatttga ggatattttg accttgggta aggatgaagt gttaaagcca cagctcctct 9453
   ccacaagaag ccattcatct tgggggaggc agagagggaa gtctctctcc aaagtctatc 9513
   cagcttcgct tcgtttcatt gatctgcaca agagacaatg ctctggaaaa ggaagaggac 9573
   cccagaaggg tgcttggcaa gacagaggat gctaatgggc aatggagagc actccctcca 9633
   gctggcccct gctgctgcct cccgtcctct gcatggggtc aggtgcttct gtgcttgctg 9693
   tcctacctct ctccacagca gggctctcaa aaccattttg atcccccatt ggcagagggt 9753
   tcccctcttt acagagttca gtcattaaaa gcatggatca gctgttaatc tcattggagg 9813
   agggaactgt ttcctgcatt cattcatctg ggaaccttct tgagtagcca ctgtctgcca 9873
   gccactgctc tagagatggg aaaacagcac ggaacaaaac caaggtcttt cttccagcga 9933
   atttatatcc ttcaggaagc tggttcctgc caccaactta gcaggcaaca gttctcctcc 9993
   cctagtggca cagggtacca gttttgtagg aaaagtggtc cagcaaagga agaaagcaga 10053
   ccaacccagc tgccttacct tattctgggg ccattccccc agcgatgaga gctgctcttg 10113
   tttctactgc caccatctct tctggctgca cttcacctgc tgcttgagct tctgaccttc  10173
   cttcagttcc accaaatgag gacaggaaat agcagtcaag acccctggcc ctgctgagcg  10233
   tgaaacagaa gcaatggatg agtgctggac gaagaatggc ctgggcagaa caaataggga 10293
   gcatttgaaa gcttctggct gataaatctc caggtgcatc ccggttgcca cgcctgcccc 10353
   cattaacctg ctcctggtaa atactgatcc agcagctgct ccaggagagg ccgtcttttt 10413
   ttcccagcca cgctgtgtct tgcatgagac tcctggggcc tgggcacaga gagaaaagaa 10473
   ttgagactca ggaggctcag tgggtgagaa aatgcaaagt ggcttcacag acacagggct 10533
   gtgggagcag atcgacgggg aacttgggag atgaacttca gggccttccg acgccttgtc 10593
   tcaggaacat gctttgagaa aaatggtagc atcctttcca taactcagtc tctcttccct 10653
   agtttccctg aagtgtgacg ttttagtatc tggagctcag tgatccccat gaatgaggga 10713
   taaagtttca ctcttggtat tttctaacta gtgctaggga aagtcctgag acacgatcac 10773
   agccactgct tggcatacag ggcctccacc caataagcaa actggagatt cctcagcctc 10833
   tcgtggacac ccacatctca ttcttctcac agcagagaag ctctcccttc agcctgagct 10893
   gtcttctttc tgctgcagtg cagcctgctc cctcctaccc tggcctcaag gaaggtagga 10953
   aacatcttct gcatttcaaa gtcctcactt tgacttattt ggccttcatc ttggcatgga 11013
   aggtggcagg cagaatggaa atactccccc caaacagaac agatattctt gcgtgtgtaa 11073
   gggcagaagg gacaagctct ctatcccatg agactagggg ccggagccca cctgcctttc 11133
   cccacaactt ttcctgctca aacccactcc tcttgacaca ctggaatctg tattatatat  11193
   atttttaaga aaatacaatg atggttgtct ggttttgttg tttttacagg tgttgtggaa  11253
   taaaaactgt aagaaaatta agtatttaaa atgttccaat aaagtggggt tttttgttat  11313
   tctaatatat tattgtgtac ctattgtaaa tatgaaacac tcctattttg caagctgagg  11373
   acacaatttg tactgttgtt atatataaat aaagtttact gaattaaaaa aaccttaaat  11433
   ctttaaataa aaaaaaaaaa aaaa                                          11457
<210> 72
   <211> 1894
   <212> PRT
   <213> Homo sapiens
<400> 72
<210> 73
   <211> 2108
   <212> DNA
   <213> Homo sapiens
<220>
   <221> CDS
   <222> (187)..(1353)
   <223>
<400> 73
   ggctccggcg gcagcgggat ctgcagttcg gactccgcgc gccacagtcg ctgcagttcg     60
   ctccactctg gtggccccgc cgccctgcgg ggatcccggg ggtcggcggg ctgctcggac    120
   ttggcgcggg gccggcccgg cctctctctt cctcggtggg gcctagacgg tcggggcacc    180
   gggaac atg gag ccc tct cca gcc gct ggg ggc ttg gag acc act cgc       228
          Met Glu Pro Ser Pro Ala Ala Gly Gly Leu Glu Thr Thr Arg
          1               5                   10
   ctg gtg agc ccc cgg gac cgc ggt ggc gcc gga ggc agc ctg cgt ttg      276
   Leu Val Ser Pro Arg Asp Arg Gly Gly Ala Gly Gly Ser Leu Arg Leu
   15                  20                  25                  30
   aag agt ctc ttc aca gag ccc tca gag ccc ctc cct gag gag tcc aaa      324
   Lys Ser Leu Phe Thr Glu Pro Ser Glu Pro Leu Pro Glu Glu Ser Lys
                   35                  40                  45
   cct gtg gag atg ccc ttc cac cac tgc cac agg gac ccc ctt ccg ccg      372
   Pro Val Glu Met Pro Phe His His Cys His Arg Asp Pro Leu Pro Pro
               50                  55                  60
   ccg ggc ctt acc cct gag agg ctg cat gca cgg agg cag cta tat gct      420
   Pro Gly Leu Thr Pro Glu Arg Leu His Ala Arg Arg Gln Leu Tyr Ala
           65                  70                  75
   gcc tgt gcc gtt tgc ttt gtc ttc atg gct ggg gag gtg gtc ggc ggg      468
   Ala Cys Ala Val Cys Phe Val Phe Met Ala Gly Glu Val Val Gly Gly
       80                  85                  90
   tat ctg gca cac agc ctg gcc atc atg acc gat gca gcc cac ttg ctg      516
   Tyr Leu Ala His Ser Leu Ala Ile Met Thr Asp Ala Ala His Leu Leu
   95                  100                 105                 110
   gcg gat gtg ggc agc atg atg ggc agc ctc ttc tcc ctc tgg ctc tcc      564
   Ala Asp Val Gly Ser Met Met Gly Ser Leu Phe Ser Leu Trp Leu Ser
                   115                 120                 125
   acc cgt cca gcc acc cgc acc atg acc ttt ggc tgg cac cgt tca gag      612
   Thr Arg Pro Ala Thr Arg Thr Met Thr Phe Gly Trp His Arg Ser Glu
               130                 135                 140
   act ctg ggg gct ttg gcc tct gtg gtc tcc ctc tgg atg gtc act ggc      660
   Thr Leu Gly Ala Leu Ala Ser Val Val Ser Leu Trp Met Val Thr Gly
           145                 150                 155
   atc ctc ctg tac ctg gcc ttc gtc cgc ctg ctg cac agc gac tac cac      708
   Ile Leu Leu Tyr Leu Ala Phe Val Arg Leu Leu His Ser Asp Tyr His
       160                 165                 170
   atc gag ggg ggt gcc atg ctg ctg acc gcc agc atc gca gtc tgt gcc      756
   Ile Glu Gly Gly Ala Met Leu Leu Thr Ala Ser Ile Ala Val Cys Ala
   175                 180                 185                 190
   aac ctg tta atg gcc ttt gtg ctg cac cag gct ggg ccc ccc cac agc      804
   Asn Leu Leu Met Ala Phe Val Leu His Gln Ala Gly Pro Pro His Ser
                   195                 200                 205
   cac ggg tct agg gga gca gag tat gca ccg ctg gag gag ggg cct gaa      852
   His Gly Ser Arg Gly Ala Glu Tyr Ala Pro Leu Glu Glu Gly Pro Glu
               210                 215                 220
   gag ccc ctg ccc ctg ggg aac acc agc gtc cgg gcg gca ttt gtg cac      900
   Glu Pro Leu Pro Leu Gly Asn Thr Ser Val Arg Ala Ala Phe Val His
           225                 230                 235
   gtg ctg ggg gac ctc ctg cag agc ttt ggg gta ctg gct gcc tcc atc      948
   Val Leu Gly Asp Leu Leu Gln Ser Phe Gly Val Leu Ala Ala Ser Ile
       240                 245                 250
   ctc atc tac ttc aag cct caa tac aag gca gcc gac ccc atc agc acc      996
   Leu Ile Tyr Phe Lys Pro Gln Tyr Lys Ala Ala Asp Pro Ile Ser Thr
   255                 260                 265                 270
   ttc ctc ttc tcc atc tgt gcc ctt gga tcc acc gct ccc acc ctc cga     1044
   Phe Leu Phe Ser Ile Cys Ala Leu Gly Ser Thr Ala Pro Thr Leu Arg
                   275                 280                 285
   gac gtt ctt cga atc ctc atg gaa ggt acc ccc cgc aat gtg ggg ttc     1092
   Asp Val Leu Arg Ile Leu Met Glu Gly Thr Pro Arg Asn Val Gly Phe
               290                 295                 300
   gaa cct gtg cgg gat acg ctg ttg tcg gtg cca gga gtc cgg gca acc     1140
   Glu Pro Val Arg Asp Thr Leu Leu Ser Val Pro Gly Val Arg Ala Thr
           305                 310                 315
   cat gag ctg cac ctg tgg gcc ctt acg ctc act tac cat gtt gcc tct     1188
   His Glu Leu His Leu Trp Ala Leu Thr Leu Thr Tyr His Val Ala Ser
       320                 325                 330
   gca cac ctg gcc atc gac tcc acc gct gac cct gaa gcc gtc ctg gct     1236
   Ala His Leu Ala Ile Asp Ser Thr Ala Asp Pro Glu Ala Val Leu Ala
   335                 340                 345                 350
   gaa gcc tca tcc cgg ctc tac tcc cgg ttt gga ttc tcc agc tgc acc     1284
   Glu Ala Ser Ser Arg Leu Tyr Ser Arg Phe Gly Phe Ser Ser Cys Thr
                   355                 360                 365
   ctg cag gtc gag cag tat cag ccg gag atg gcc cag tgc ctg cgc tgc     1332
   Leu Gln Val Glu Gln Tyr Gln Pro Glu Met Ala Gln Cys Leu Arg Cys
               370                 375                 380
   cag gaa ccc ccc caa gcc tga gccatggccc tgccctcacc ccactgccag        1383
   Gln Glu Pro Pro Gln Ala
           385
   gccgaggctc agccccagac tctcagcatc tgctgccctg atcacagaga cgggaccgag   1443
   ccaggtccat accccttcct ctctccctcc taccacctgc cagtttcccc agcctcagcc 1503
   ccagccccag ccccagtggg caagaccaaa gtgtggcggg gagtggggtg ggagtcaggg 1563
   gaatagatgt gactagttca ggggcgggga ctcccaggcc tcagtgtggc agggtgtgtt 1623
   gaaggcctgt ggtgccatct ccccatggtt catgtggagc cacgaacatc ctttccctgc 1683
   agtccatttg tctgtgtggc aggctggctg gctgggggca tctgcctgtc tatgtgctgt 1743
   tggtgtgcct atgcctgggg gaggtcagta ggggccccct ccccacatgg ccctcgctct 1803
   gtctatgcag gggccccaaa gcccgcactt tgtccgtgtg tcttagccct gtggttttgt 1863
   ctgtgtgtgt gtgtgtgtgt gtgtgtgtgt gtgtgtgtgt gttcttggtg ctgtggcctg 1923
   tgtgtctctg tgcctatgtg gctgtgctat ggtttctatg agtctgctcc atccatgtgt 1983
   ctgtttgggg gtctatctct ccatccctct gttggtgctg tgcccttggc tatccctgaa 2043
   agagggagga ctccgctgca gctccaccaa taaacttgtg tctcactgca aaaaaaaaaa 2103
   aaaaa 2108
<210> 74
   <211> 388
   <212> PRT
   <213> Homo sapiens
<400> 74
   Met Glu Pro Ser Pro Ala Ala Gly Gly Leu Glu Thr Thr Arg Leu Val
   1               5                   10                  15
   Ser Pro Arg Asp Arg Gly Gly Ala Gly Gly Ser Leu Arg Leu Lys Ser
               20                  25                  30
   Leu Phe Thr Glu Pro Ser Glu Pro Leu Pro Glu Glu Ser Lys Pro Val
           35                  40                  45
   Glu Met Pro Phe His His Cys His Arg Asp Pro Leu Pro Pro Pro Gly
       50                  55                  60
   Leu Thr Pro Glu Arg Leu His Ala Arg Arg Gln Leu Tyr Ala Ala Cys
   65                  70                  75                  80
   Ala Val Cys Phe Val Phe Met Ala Gly Glu Val Val Gly Gly Tyr Leu
                   85                  90                  95
   Ala His Ser Leu Ala Ile Met Thr Asp Ala Ala His Leu Leu Ala Asp
               100                 105                 110
   Val Gly Ser Met Met Gly Ser Leu Phe Ser Leu Trp Leu Ser Thr Arg
           115                 120                 125
   Pro Ala Thr Arg Thr Met Thr Phe Gly Trp His Arg Ser Glu Thr Leu
       130                 135                 140
   Gly Ala Leu Ala Ser Val Val Ser Leu Trp Met Val Thr Gly Ile Leu
   145                 150                 155                 160
   Leu Tyr Leu Ala Phe Val Arg Leu Leu His Ser Asp Tyr His Ile Glu
                   165                 170                 175
   Gly Gly Ala Met Leu Leu Thr Ala Ser Ile Ala Val Cys Ala Asn Leu
               180                 185                 190
   Leu Met Ala Phe Val Leu His Gln Ala Gly Pro Pro His Ser His Gly
           195                 200                 205
   Ser Arg Gly Ala Glu Tyr Ala Pro Leu Glu Glu Gly Pro Glu Glu Pro
       210                 215                 220
   Leu Pro Leu Gly Asn Thr Ser Val Arg Ala Ala Phe Val His Val Leu
   225                 230                 235                 240
   Gly Asp Leu Leu Gln Ser Phe Gly Val Leu Ala Ala Ser Ile Leu Ile
                   245                 250                 255
   Tyr Phe Lys Pro Gln Tyr Lys Ala Ala Asp Pro Ile Ser Thr Phe Leu
               260                 265                 270
   Phe Ser Ile Cys Ala Leu Gly Ser Thr Ala Pro Thr Leu Arg Asp Val
           275                 280                 285
   Leu Arg Ile Leu Met Glu Gly Thr Pro Arg Asn Val Gly Phe Glu Pro
       290                 295                 300
   Val Arg Asp Thr Leu Leu Ser Val Pro Gly Val Arg Ala Thr His Glu
   305                 310                 315                 320
   Leu His Leu Trp Ala Leu Thr Leu Thr Tyr His Val Ala Ser Ala His
                   325                 330                 335
   Leu Ala Ile Asp Ser Thr Ala Asp Pro Glu Ala Val Leu Ala Glu Ala
               340                 345                 350
   Ser Ser Arg Leu Tyr Ser Arg Phe Gly Phe Ser Ser Cys Thr Leu Gln
           355                 360                 365
   Val Glu Gln Tyr Gln Pro Glu Met Ala Gln Cys Leu Arg Cys Gln Glu
   370 375 380
   Pro Pro Gln Ala
   385
<210> 75
   <211> 1722
   <212> DNA
   <213> Homo sapiens
<220>
   <221> CDS
   <222> (85)..(879)
   <223>
<400> 75
   attcattgcg ccgcggcacg gcctagcgag tggttcttct gcgctactgc tgcgcgaatc      60
   ggcgacccca gtgcctcgac cact atg ccg cgc tct ttc ctc gtc agg aag        111
                              Met Pro Arg Ser Phe Leu Val Arg Lys
                              1               5
   ccc tcc gac ccc aat cgg aag cct aac tac agc gag ctg cag gac tct      159
   Pro Ser Asp Pro Asn Arg Lys Pro Asn Tyr Ser Glu Leu Gln Asp Ser
   10                  15                  20                  25
   aat cca gag ttt acc ttc cag cag ccc tac gac cag gcc cac ctg ctg      207
   Asn Pro Glu Phe Thr Phe Gln Gln Pro Tyr Asp Gln Ala His Leu Leu
                   30                  35                  40
   gca gcc atc cca cct ccg gag atc ctc aac ccc acc gcc tcg ctg cca      255
   Ala Ala Ile Pro Pro Pro Glu Ile Leu Asn Pro Thr Ala Ser Leu Pro
               45                  50                  55
   atg ctc atc tgg gac tct gtc ctg gcg ccc caa gcc cag cca att gcc      303
   Met Leu Ile Trp Asp Ser Val Leu Ala Pro Gln Ala Gln Pro Ile Ala
           60                  65                  70
   tgg gcc tcc ctt cgg ctc cag gag agt ccc agg gtg gca gag ctg acc      351
   Trp Ala Ser Leu Arg Leu Gln Glu Ser Pro Arg Val Ala Glu Leu Thr
       75                  80                  85
   tcc ctg tca gat gag gac agt ggg aaa ggc tcc cag ccc ccc agc cca      399
   Ser Leu Ser Asp Glu Asp Ser Gly Lys Gly Ser Gln Pro Pro Ser Pro
   90                  95                  100                 105
   ccc tca ccg gct cct tcg tcc ttc tcc tct act tca gtc tct tcc ttg      447
   Pro Ser Pro Ala Pro Ser Ser Phe Ser Ser Thr Ser Val Ser Ser Leu
                   110                 115                 120
   gag gcc gag gcc tat gct gcc ttc cca ggc ttg ggc caa gtg ccc aag      495
   Glu Ala Glu Ala Tyr Ala Ala Phe Pro Gly Leu Gly Gln Val Pro Lys
               125                 130                 135
   cag ctg gcc cag ctc tct gag gcc aag gat ctc cag gct cga aag gcc      543
   Gln Leu Ala Gln Leu Ser Glu Ala Lys Asp Leu Gln Ala Arg Lys Ala
           140                 145                 150
   ttc aac tgc aaa tac tgc aac aag gaa tac ctc agc ctg ggt gcc ctc      591
   Phe Asn Cys Lys Tyr Cys Asn Lys Glu Tyr Leu Ser Leu Gly Ala Leu
       155                 160                 165
   aag atg cac atc cga agc cac acg ctg ccc tgc gtc tgc gga acc tgc      639
   Lys Met His Ile Arg Ser His Thr Leu Pro Cys Val Cys Gly Thr Cys
   170                 175                 180                 185
   ggg aag gcc ttc tct agg ccc tgg ctg cta caa ggc cat gtc cgg acc      687
   Gly Lys Ala Phe Ser Arg Pro Trp Leu Leu Gln Gly His Val Arg Thr
                   190                 195                 200
   cac act ggc gag aag ccc ttc tcc tgt ccc cac tgc agc cgt gcc ttc      735
   His Thr Gly Glu Lys Pro Phe Ser Cys Pro His Cys Ser Arg Ala Phe
               205                 210                 215
   gct gac cgc tcc aac ctg cgg gcc cac ctc cag acc cac tca gat gtc      783
   Ala Asp Arg Ser Asn Leu Arg Ala His Leu Gln Thr His Ser Asp Val
           220                 225                 230
   aag aag tac cag tgc cag gcg tgt gct cgg acc ttc tcc cga atg tcc      831
   Lys Lys Tyr Gln Cys Gln Ala Cys Ala Arg Thr Phe Ser Arg Met Ser
       235                 240                 245
   ctg ctc cac aag cac caa gag tcc ggc tgc tca gga tgt ccc cgc tga      879
   Leu Leu His Lys His Gln Glu Ser Gly Cys Ser Gly Cys Pro Arg
   250                 255                 260
   ccctcgaggc tccctcttcc tctccatacc tgcccctgcc tgacagcctt ccccagctcc    939
   agcaggaagg accccacatc cttctcactg ccatggaatt ccctcctgag tgccccactt    999
   ctggccacat cagccccaca ggactttgat gaagaccatt ttctggttct gtgtcctctg   1059
   cctgggctct ggaagaggcc ttcccatggc catttctgtg gagggagggc agctggcccc   1119
   cagccctggg ggattcctga gctggcctgt ctgcgtgggt ttttgtatcc agagctgttt   1179
   ggatacagct gctttgagct acaggacaaa ggctgacaga ctcactggga agctcccacc   1239
   ccactcaggg gaccccactc ccctcacaca caccccccca caaggaaccc tcaggccacc    1299
   ctccacgagg tgtgactaac tatgcaataa tccaccccca ggtgcagccc cagggcctgc    1359
   ggaggcggtg gcagactaga gtctgagatg ccccgagccc aggcagctat ttcagcctcc   1419
   tgtttggtgg ggtggcacct gtttcccggg caatttaaca atgtctgaaa agggactgtg   1479
   agtaatggct gtcacttgtc gggggcccaa gtggggtgct ctggtctgac cgatgtgtct   1539
   cccagaacta ttctgggggc ccgacaggtg ggcctgggag gaagatgttt acatttttaa   1599
   aggtacactg gtatttatat ttcaaacatt ttgtatcaag gaaacgtttt gtatagttat   1659
   atgtacagtt tattgatatt caataaagca gttaatttat atattaaaaa aaaaaaaaaa   1719
   aaa                                                                 1722
<210> 76
   <211> 264
   <212> PRT
   <213> Homo sapiens
<400> 76
   Met Pro Arg Ser Phe Leu Val Arg Lys Pro Ser Asp Pro Asn Arg Lys
   1 5 10 15
   Pro Asn Tyr Ser Glu Leu Gln Asp Ser Asn Pro Glu Phe Thr Phe Gln
   20 25 30
   Gln Pro Tyr Asp Gln Ala His Leu Leu Ala Ala Ile Pro Pro Pro Glu
   35 40 45
   Ile Leu Asn Pro Thr Ala Ser Leu Pro Met Leu Ile Trp Asp Ser Val
   50 55 60
   Leu Ala Pro Gln Ala Gln Pro Ile Ala Trp Ala Ser Leu Arg Leu Gln
   65 70 75 80
   Glu Ser Pro Arg Val Ala Glu Leu Thr Ser Leu Ser Asp Glu Asp Ser
   85 90 95
   Gly Lys Gly Ser Gln Pro Pro Ser Pro Pro Ser Pro Ala Pro Ser Ser
   100 105 110
   Phe Ser Ser Thr Ser Val Ser Ser Leu Glu Ala Glu Ala Tyr Ala Ala
   115 120 125
   Phe Pro Gly Leu Gly Gln Val Pro Lys Gln Leu Ala Gln Leu Ser Glu
   130 135 140
   Ala Lys Asp Leu Gln Ala Arg Lys Ala Phe Asn Cys Lys Tyr Cys Asn
   145 150 155 160
   Lys Glu Tyr Leu Ser Leu Gly Ala Leu Lys Met His Ile Arg Ser His
   165 170 175
   Thr Leu Pro Cys Val Cys Gly Thr Cys Gly Lys Ala Phe Ser Arg Pro
   180 185 190
   Trp Leu Leu Gln Gly His Val Arg Thr His Thr Gly Glu Lys Pro Phe
   195 200 205
   Ser Cys Pro His Cys Ser Arg Ala Phe Ala Asp Arg Ser Asn Leu Arg
   210 215 220
   Ala His Leu Gln Thr His Ser Asp Val Lys Lys Tyr Gln Cys Gln Ala
   225 230 235 240
   Cys Ala Arg Thr Phe Ser Arg Met Ser Leu Leu His Lys His Gln Glu
   245 250 255
   Ser Gly Cys Ser Gly Cys Pro Arg
   260
<210> 77
   <211> 3888
   <212> DNA
   <213> Homo sapiens
<220>
   <221> CDS
   <222> (59)..(3709)
   <223>
<400> 77
   aaaactcagg gaagcccagg gcccgtgttg tgcttttggc ccaggtaggt ggacagac        58
   atg tcc cag gcg ggg gac gta gaa ggc ccc agc aca gga gac cct gtg       106
   Met Ser Gln Ala Gly Asp Val Glu Gly Pro Ser Thr Gly Asp Pro Val
   1               5                   10                  15
   ctc agt ccc caa cac aac tgt gag ctt tta cag aac atg gaa gga gcc       154
   Leu Ser Pro Gln His Asn Cys Glu Leu Leu Gln Asn Met Glu Gly Ala
               20                  25                  30
   agc tcc atg cca ggc ctg tca cca gat ggg ccg gga gca agc tct ggg       202
   Ser Ser Met Pro Gly Leu Ser Pro Asp Gly Pro Gly Ala Ser Ser Gly
           35                  40                  45
   ccc gga gtc agg gct ggc agc aga agg aag atc ccc agg aag gag gcc       250
   Pro Gly Val Arg Ala Gly Ser Arg Arg Lys Ile Pro Arg Lys Glu Ala
       50                  55                  60
   ctt cga ggt ggc agc tcc cgg gct gca ggt gct gct gag gtc cgg cca       298
   Leu Arg Gly Gly Ser Ser Arg Ala Ala Gly Ala Ala Glu Val Arg Pro
   65                  70                  75                  80
   ggg gtc ttg gag ctg cta gct gtg gta cag agc cgg ggc tcg atg ctg       346
   Gly Val Leu Glu Leu Leu Ala Val Val Gln Ser Arg Gly Ser Met Leu
                   85                  90                  95
   gct cct ggg ctc cac atg cag ctg ccc tcg gtg cct act cag ggg aga       394
   Ala Pro Gly Leu His Met Gln Leu Pro Ser Val Pro Thr Gln Gly Arg
               100                 105                 110
   gct ctg acc tcc aag agg ctc cag gtt tct ctg tgt gac atc tta gat       442
   Ala Leu Thr Ser Lys Arg Leu Gln Val Ser Leu Cys Asp Ile Leu Asp
           115                 120                 125
   gac agt tgc ccc agg aaa ctt tgt agc agg tct gct ggc ctc cca gag       490
   Asp Ser Cys Pro Arg Lys Leu Cys Ser Arg Ser Ala Gly Leu Pro Glu
       130                 135                 140
   aga gct ctg gcc tgc agg gag agg ctt gca gga gtg gag gag gtg agc       538
   Arg Ala Leu Ala Cys Arg Glu Arg Leu Ala Gly Val Glu Glu Val Ser
   145                 150                 155                 160
   tgc ctc agg ccc agg gag gcc aga gac ggt gga atg agt tct cca ggg       586
   Cys Leu Arg Pro Arg Glu Ala Arg Asp Gly Gly Met Ser Ser Pro Gly
                   165                 170                 175
   tgt gac aga aga agc ccc aca ctc agc aaa gag gag ccc cct gga agg       634
   Cys Asp Arg Arg Ser Pro Thr Leu Ser Lys Glu Glu Pro Pro Gly Arg
               180                 185                 190
   ccc ctg aca tcc tca cca gac cca gtc cct gtg agg gta aga aag aaa       682
   Pro Leu Thr Ser Ser Pro Asp Pro Val Pro Val Arg Val Arg Lys Lys
           195                 200                 205
   tgg agg agg caa ggg gct cat tca gag tgt gag gaa ggg gct ggt gac       730
   Trp Arg Arg Gln Gly Ala His Ser Glu Cys Glu Glu Gly Ala Gly Asp
       210                 215                 220
   ttc ctg tgg ctt gat cag agc cct cgt ggg gac aac ctc ctg tct gtg       778
   Phe Leu Trp Leu Asp Gln Ser Pro Arg Gly Asp Asn Leu Leu Ser Val
   225                 230                 235                 240
   gga gac cct ccc caa gtt gct gac ctg gag tcc ttg gga ggc cct tgc       826
   Gly Asp Pro Pro Gln Val Ala Asp Leu Glu Ser Leu Gly Gly Pro Cys
                   245                 250                 255
   aga cct ccc tct cca aaa gac act ggg tct ggg cct gga gag cca ggt       874
   Arg Pro Pro Ser Pro Lys Asp Thr Gly Ser Gly Pro Gly Glu Pro Gly
               260                 265                 270
   gga agt ggg gca gga tgt gcc tca ggg act gag aaa ttt gga tat ttg       922
   Gly Ser Gly Ala Gly Cys Ala Ser Gly Thr Glu Lys Phe Gly Tyr Leu
           275                 280                 285
   ccc gct aca ggg gat ggg ccc cag cca ggc agc ccc tgt ggc cct gtc       970
   Pro Ala Thr Gly Asp Gly Pro Gln Pro Gly Ser Pro Cys Gly Pro Val
       290                 295                 300
   ggg ttc cca gtg ccc agt gga ggg gag tcc ctc agt tca gct gca cag      1018
   Gly Phe Pro Val Pro Ser Gly Gly Glu Ser Leu Ser Ser Ala Ala Gln
   305                 310                 315                 320
   gct cct cca cag agc gca gca ctg tgc ctg ggg gcg tca gca cag gcc      1066
   Ala Pro Pro Gln Ser Ala Ala Leu Cys Leu Gly Ala Ser Ala Gln Ala
                   325                 330                 335
   tct gca gag cag caa gaa gct gtg tgt gtc gtg cgg act ggc agc gat      1114
   Ser Ala Glu Gln Gln Glu Ala Val Cys Val Val Arg Thr Gly Ser Asp
               340                 345                 350
   gaa ggc cag gct cca gca cag gac cag gag gag ctg gag gcc aag gct      1162
   Glu Gly Gln Ala Pro Ala Gln Asp Gln Glu Glu Leu Glu Ala Lys Ala
           355                 360                 365
   cag cca gct tcc agg gga agg ctg gag caa gga ctc gct gcc ccc gct      1210
   Gln Pro Ala Ser Arg Gly Arg Leu Glu Gln Gly Leu Ala Ala Pro Ala
       370                 375                 380
   gac acc tgt gcc agc tcc cgg gag ccc ttg ggc ggc ctc agc tcc tcc      1258
   Asp Thr Cys Ala Ser Ser Arg Glu Pro Leu Gly Gly Leu Ser Ser Ser
   385                 390                 395                 400
   ctg gat act gaa gcc agc agg gcc tgc tca ggc cca ttc atg gag cag      1306
   Leu Asp Thr Glu Ala Ser Arg Ala Cys Ser Gly Pro Phe Met Glu Gln
                   405                 410                 415
   aga aga tcc aag ggc act aag aac ctg aag aaa ggt cca gtg ccc tgt      1354
   Arg Arg Ser Lys Gly Thr Lys Asn Leu Lys Lys Gly Pro Val Pro Cys
               420                 425                 430
   gcc caa gac cgg ggc aca gac aga agc tca gac aac tcc cac cag gac      1402
   Ala Gln Asp Arg Gly Thr Asp Arg Ser Ser Asp Asn Ser His Gln Asp
           435                 440                 445
   agg cca gag gaa ccc agc cca gga ggc tgc ccc aga ctg gag gaa gtg      1450
   Arg Pro Glu Glu Pro Ser Pro Gly Gly Cys Pro Arg Leu Glu Glu Val
       450                 455                 460
   aaa ata ccc cat gga gtg aag ctt gtg tgc tac ctg ggt tcc ggg cca      1498
   Lys Ile Pro His Gly Val Lys Leu Val Cys Tyr Leu Gly Ser Gly Pro
   465                 470                 475                 480
   gtg atc cag ctc ctg ggg gcc atc agc cac ggc cag gca ggg ggg cag      1546
   Val Ile Gln Leu Leu Gly Ala Ile Ser His Gly Gln Ala Gly Gly Gln
                   485                 490                 495
   ctg cca cca aag ctg gag gtt cta gag gac ttg atg gag gtc agc tca      1594
   Leu Pro Pro Lys Leu Glu Val Leu Glu Asp Leu Met Glu Val Ser Ser
               500                 505                 510
   ccc tca cct gcc cag agg ctc aga agg aag aaa agg ccc atg gtg cag      1642
   Pro Ser Pro Ala Gln Arg Leu Arg Arg Lys Lys Arg Pro Met Val Gln
           515                 520                 525
   ggc cct gct ggg tgc cag gtt ttc cag cct tct cct tca gga ggc aca      1690
   Gly Pro Ala Gly Cys Gln Val Phe Gln Pro Ser Pro Ser Gly Gly Thr
       530                 535                 540
   gca ggg gac cct ggt ggc ctc tct gac ccc ttc tac cct cca aga agc      1738
   Ala Gly Asp Pro Gly Gly Leu Ser Asp Pro Phe Tyr Pro Pro Arg Ser
   545                 550                 555                 560
   ggt tcc ctg gcc ctt ggc gac ccc agc tcg gac cct gca tgt tcc cag      1786
   Gly Ser Leu Ala Leu Gly Asp Pro Ser Ser Asp Pro Ala Cys Ser Gln
                   565                 570                 575
   agt ggc cca atg gag gct gaa gag gat tct ctt ccg gag cag cca gag      1834
   Ser Gly Pro Met Glu Ala Glu Glu Asp Ser Leu Pro Glu Gln Pro Glu
               580                 585                 590
   gac tca gct cag ctc caa cag gag aag cca tcc ctg tat att ggg gtg      1882
   Asp Ser Ala Gln Leu Gln Gln Glu Lys Pro Ser Leu Tyr Ile Gly Val
           595                 600                 605
   cgg ggc act gtt gtc cgt tcc atg cag gag gta cta tgg act cgc ctt      1930
   Arg Gly Thr Val Val Arg Ser Met Gln Glu Val Leu Trp Thr Arg Leu
       610                 615                 620
   cgg gag ctc cca gac cca gtg ctg agt gag gag gtg gtg gag ggc att      1978
   Arg Glu Leu Pro Asp Pro Val Leu Ser Glu Glu Val Val Glu Gly Ile
   625                 630                 635                 640
   gct gct ggc att gag gca gcc ctc tgg gac ctg aca caa ggc acc aat      2026
   Ala Ala Gly Ile Glu Ala Ala Leu Trp Asp Leu Thr Gln Gly Thr Asn
                   645                 650                 655
   ggc cgg tac aag acc aag tat cgc agc ctg ctg ttc aac ctg cgg gac      2074
   Gly Arg Tyr Lys Thr Lys Tyr Arg Ser Leu Leu Phe Asn Leu Arg Asp
               660                 665                 670
   ccc agg aac ctg gac ttg ttt ctc aaa gtg gtt cat gga gat gtc acc      2122
   Pro Arg Asn Leu Asp Leu Phe Leu Lys Val Val His Gly Asp Val Thr
           675                 680                 685
   ccc tac gac ctg gtg cgg atg agc tcg atg cag ctg gcc ccc cag gag      2170
   Pro Tyr Asp Leu Val Arg Met Ser Ser Met Gln Leu Ala Pro Gln Glu
       690                 695                 700
   ctg gcc cgc tgg cgg gac cag gag gag aaa agg ggc ctg aat atc att      2218
   Leu Ala Arg Trp Arg Asp Gln Glu Glu Lys Arg Gly Leu Asn Ile Ile
   705                 710                 715                 720
   gag cag caa cag aag gag ccg tgc aga ctt cca gcc tcc aaa atg acc      2266
   Glu Gln Gln Gln Lys Glu Pro Cys Arg Leu Pro Ala Ser Lys Met Thr
                   725                 730                 735
   cac aag ggc gaa gtg gag att cag cgg gac atg gac cag aca ctg acc      2314
   His Lys Gly Glu Val Glu Ile Gln Arg Asp Met Asp Gln Thr Leu Thr
               740                 745                 750
   ctg gag gat ctg gtg gga ccg cag atg ttc atg gac tgc agc cca cag      2362
   Leu Glu Asp Leu Val Gly Pro Gln Met Phe Met Asp Cys Ser Pro Gln
           755                 760                 765
   gcc ctg ccc atc gca tca gag gac acc acg ggg cag cat gac cac cac      2410
   Ala Leu Pro Ile Ala Ser Glu Asp Thr Thr Gly Gln His Asp His His
       770                 775                 780
   ttc tta gac ccc aac tgc cac atc tgc aag gac tgg gag ccc tcg aat      2458
   Phe Leu Asp Pro Asn Cys His Ile Cys Lys Asp Trp Glu Pro Ser Asn
   785                 790                 795                 800
   gag ctg cta ggc tcc ttc gaa gcc gcc aag agc tgc ggg gac aat atc      2506
   Glu Leu Leu Gly Ser Phe Glu Ala Ala Lys Ser Cys Gly Asp Asn Ile
                   805                 810                 815
   ttc cag aaa gcc cta agc caa act cct atg cct gct cca gag atg ccc      2554
   Phe Gln Lys Ala Leu Ser Gln Thr Pro Met Pro Ala Pro Glu Met Pro
               820                 825                 830
   aaa acc agg gag ttg tct ccc acg gaa cca cag gac agg gtc cct cca      2602
   Lys Thr Arg Glu Leu Ser Pro Thr Glu Pro Gln Asp Arg Val Pro Pro
           835                 840                 845
   tct ggg ctc cat gtg cct gct gca ccc aca aag gcc ctg ccc tgc ctg      2650
   Ser Gly Leu His Val Pro Ala Ala Pro Thr Lys Ala Leu Pro Cys Leu
       850                 855                 860
   cca ccc tgg gaa ggt gtt ctg gac atg ttc tcc atc aag cgg ttc cgg      2698
   Pro Pro Trp Glu Gly Val Leu Asp Met Phe Ser Ile Lys Arg Phe Arg
   865                 870                 875                 880
   gcc agg gcc cag ctg gtc tcg gga cac agc tgt cgg ctt gtc cag gct      2746
   Ala Arg Ala Gln Leu Val Ser Gly His Ser Cys Arg Leu Val Gln Ala
                   885                 890                 895
   ctg ccc acc gtg atc cgc tcg gca ggc tgc atc ccc tcc aac att gtc      2794
   Leu Pro Thr Val Ile Arg Ser Ala Gly Cys Ile Pro Ser Asn Ile Val
               900                 905                 910
   tgg gac ctt ctg gcc agc atc tgc cca gcc aag gcc aag gac gtc tgc      2842
   Trp Asp Leu Leu Ala Ser Ile Cys Pro Ala Lys Ala Lys Asp Val Cys
           915                 920                 925
   gtg gtc aga ctg tgc cca cat ggg gcc cgg gac acc cag aac tgc cgc      2890
   Val Val Arg Leu Cys Pro His Gly Ala Arg Asp Thr Gln Asn Cys Arg
       930                 935                 940
   ctg ctc tac tca tac ctc aat gat agg cag cgc cac ggg ctg gcc tct      2938
   Leu Leu Tyr Ser Tyr Leu Asn Asp Arg Gln Arg His Gly Leu Ala Ser
   945                 950                 955                 960
   gtg gag cac atg ggg atg gtc ctg ctg ccc ctg cct gcc ttc cag ccc      2986
   Val Glu His Met Gly Met Val Leu Leu Pro Leu Pro Ala Phe Gln Pro
                   965                 970                 975
   ctg ccc acc agg ctg cgc cct ttg ggg ggc cca ggc ctt tgg gct ctt      3034
   Leu Pro Thr Arg Leu Arg Pro Leu Gly Gly Pro Gly Leu Trp Ala Leu
               980                 985                 990
   cct gtc tcc cct ctc ctt tcc cca  ggt ctg gag gtc act  cac tca agt    3082
   Pro Val Ser Pro Leu Leu Ser Pro  Gly Leu Glu Val Thr  His Ser Ser
           995                 1000                1005
   ctg ttg  ctg gct gtg ctg ctc  ccc aag gaa ggg ctt  cca gac aca      3127
   Leu Leu  Leu Ala Val Leu Leu  Pro Lys Glu Gly Leu  Pro Asp Thr
       1010                1015                1020
   gca ggg  tcc agc ccc tgg ttg  ggg aag gtt caa aag  atg gtc tcc      3172
   Ala Gly  Ser Ser Pro Trp Leu  Gly Lys Val Gln Lys  Met Val Ser
       1025                1030                1035
   ttc aac  agt aag gtg gag aag  aga tac tat cag cca  gat gac agg      3217
   Phe Asn  Ser Lys Val Glu Lys  Arg Tyr Tyr Gln Pro  Asp Asp Arg
       1040                1045                1050
   agg ccg  aat gtg ccc ctg aag  ggc acc cct ccc cca  gga ggt gcc      3262
   Arg Pro  Asn Val Pro Leu Lys  Gly Thr Pro Pro Pro  Gly Gly Ala
       1055                1060                1065
   tgg cag  cag agc cag ggc agg  ggc agt ata gct cca  agg gga atc      3307
   Trp Gln  Gln Ser Gln Gly Arg  Gly Ser Ile Ala Pro  Arg Gly Ile
       1070                1075                1080
   tct gct  tgg cag agg ccc ccc  aga ggc agg ggg agg  ctc tgg cca      3352
   Ser Ala  Trp Gln Arg Pro Pro  Arg Gly Arg Gly Arg  Leu Trp Pro
       1085                1090                1095
   gag cct  gaa aac tgg cag cat  cct ggg cga ggg cag  tgg ccc cca      3397
   Glu Pro  Glu Asn Trp Gln His  Pro Gly Arg Gly Gln  Trp Pro Pro
       1100                1105                1110
   gag cca  ggc ttg cgc cag tcc  cag cat ccc tat tca  gta gca cca      3442
   Glu Pro  Gly Leu Arg Gln Ser  Gln His Pro Tyr Ser  Val Ala Pro
       1115                1120                1125
   gct ggt  cat ggc ttt ggc cgt  ggc cag cac ttc cac  agg gac tcc      3487
   Ala Gly  His Gly Phe Gly Arg  Gly Gln His Phe His  Arg Asp Ser
       1130                1135                1140
   tgt ccc  cac caa gcc ctg ctc  cgg cac ctc gaa tcc  ctg gcg acc      3532
   Cys Pro  His Gln Ala Leu Leu  Arg His Leu Glu Ser  Leu Ala Thr
       1145                1150                1155
   atg agt  cac cag ctc caa gcc  tta ctg tgc ccc cag  acc aag agc      3577
   Met Ser  His Gln Leu Gln Ala  Leu Leu Cys Pro Gln  Thr Lys Ser
       1160                1165                1170
   tcc atc  ccc cgc cct ctg cag  cgt ttg tct agc gcc  ctt gca gct      3622
   Ser Ile  Pro Arg Pro Leu Gln  Arg Leu Ser Ser Ala  Leu Ala Ala
       1175                 1180                 1185
   cca gag  ccc cct ggc cca gcc  cgt gac tcc tct ttg  ggg cct aca      3667
   Pro Glu  Pro Pro Gly Pro Ala  Arg Asp Ser Ser Leu  Gly Pro Thr
       1190                 1195                 1200
   gat gaa  gct ggc tct gag tgt  ccc ttc cct aga aag  gcc tga          3709
   Asp Glu  Ala Gly Ser Glu Cys  Pro Phe Pro Arg Lys  Ala
       1205                 1210                 1215
   ccctccttac ccaccagaac aggggttttg atgccctcac tagtgttgaa gcctgttcca   3769
   gagagaggtg ggactgcaag gagaggatgg tcagccctac ccacctgccc tgtttgagct   3829
   tcctgtttga caatgtttgc tgttgatttt ttgttcaata aagaatttgg taaaattgg    3888
<210> 78
   <211> 1216
   <212> PRT
   <213> Homo sapiens
<400> 78
   Met Ser Gln Ala Gly Asp Val Glu Gly Pro Ser Thr Gly Asp Pro Val
   1               5                   10                  15
   Leu Ser Pro Gln His Asn Cys Glu Leu Leu Gln Asn Met Glu Gly Ala
               20                  25                  30
   Ser Ser Met Pro Gly Leu Ser Pro Asp Gly Pro Gly Ala Ser Ser Gly
           35                  40                  45
   Pro Gly Val Arg Ala Gly Ser Arg Arg Lys Ile Pro Arg Lys Glu Ala
       50                  55                  60
   Leu Arg Gly Gly Ser Ser Arg Ala Ala Gly Ala Ala Glu Val Arg Pro
   65                  70                  75                  80
   Gly Val Leu Glu Leu Leu Ala Val Val Gln Ser Arg Gly Ser Met Leu
                   85                  90                  95
   Ala Pro Gly Leu His Met Gln Leu Pro Ser Val Pro Thr Gln Gly Arg
               100                 105                 110
   Ala Leu Thr Ser Lys Arg Leu Gln Val Ser Leu Cys Asp Ile Leu Asp
           115                 120                 125
   Asp Ser Cys Pro Arg Lys Leu Cys Ser Arg Ser Ala Gly Leu Pro Glu
       130                 135                 140
   Arg Ala Leu Ala Cys Arg Glu Arg Leu Ala Gly Val Glu Glu Val Ser
   145                 150                 155                 160
   Cys Leu Arg Pro Arg Glu Ala Arg Asp Gly Gly Met Ser Ser Pro Gly
                   165                 170                 175
   Cys Asp Arg Arg Ser Pro Thr Leu Ser Lys Glu Glu Pro Pro Gly Arg
               180                 185                 190
   Pro Leu Thr Ser Ser Pro Asp Pro Val Pro Val Arg Val Arg Lys Lys
           195                 200                 205
   Trp Arg Arg Gln Gly Ala His Ser Glu Cys Glu Glu Gly Ala Gly Asp
       210                 215                 220
   Phe Leu Trp Leu Asp Gln Ser Pro Arg Gly Asp Asn Leu Leu Ser Val
   225                 230                 235                 240
   Gly Asp Pro Pro Gln Val Ala Asp Leu Glu Ser Leu Gly Gly Pro Cys
                   245                 250                 255
   Arg Pro Pro Ser Pro Lys Asp Thr Gly Ser Gly Pro Gly Glu Pro Gly
               260                 265                 270
   Gly Ser Gly Ala Gly Cys Ala Ser Gly Thr Glu Lys Phe Gly Tyr Leu
           275                 280                 285
   Pro Ala Thr Gly Asp Gly Pro Gln Pro Gly Ser Pro Cys Gly Pro Val
       290                 295                 300
   Gly Phe Pro Val Pro Ser Gly Gly Glu Ser Leu Ser Ser Ala Ala Gln
   305                 310                 315                 320
   Ala Pro Pro Gln Ser Ala Ala Leu Cys Leu Gly Ala Ser Ala Gln Ala
                   325                 330                 335
   Ser Ala Glu Gln Gln Glu Ala Val Cys Val Val Arg Thr Gly Ser Asp
               340                 345                 350
   Glu Gly Gln Ala Pro Ala Gln Asp Gln Glu Glu Leu Glu Ala Lys Ala
           355                 360                 365
   Gln Pro Ala Ser Arg Gly Arg Leu Glu Gln Gly Leu Ala Ala Pro Ala
       370                 375                 380
   Asp Thr Cys Ala Ser Ser Arg Glu Pro Leu Gly Gly Leu Ser Ser Ser
   385                 390                 395                 400
   Leu Asp Thr Glu Ala Ser Arg Ala Cys Ser Gly Pro Phe Met Glu Gln
                   405                 410                 415
   Arg Arg Ser Lys Gly Thr Lys Asn Leu Lys Lys Gly Pro Val Pro Cys
               420                 425                 430
   Ala Gln Asp Arg Gly Thr Asp Arg Ser Ser Asp Asn Ser His Gln Asp
           435                 440                 445
   Arg Pro Glu Glu Pro Ser Pro Gly Gly Cys Pro Arg Leu Glu Glu Val
       450                 455                 460
   Lys Ile Pro His Gly Val Lys Leu Val Cys Tyr Leu Gly Ser Gly Pro
   465                 470                 475                 480
   Val Ile Gln Leu Leu Gly Ala Ile Ser His Gly Gln Ala Gly Gly Gln
                   485                 490                 495
   Leu Pro Pro Lys Leu Glu Val Leu Glu Asp Leu Met Glu Val Ser Ser
               500                 505                 510
   Pro Ser Pro Ala Gln Arg Leu Arg Arg Lys Lys Arg Pro Met Val Gln
           515                 520                 525
   Gly Pro Ala Gly Cys Gln Val Phe Gln Pro Ser Pro Ser Gly Gly Thr
       530                 535                 540
   Ala Gly Asp Pro Gly Gly Leu Ser Asp Pro Phe Tyr Pro Pro Arg Ser
   545                 550                 555                 560
   Gly Ser Leu Ala Leu Gly Asp Pro Ser Ser Asp Pro Ala Cys Ser Gln
                   565                 570                 575
   Ser Gly Pro Met Glu Ala Glu Glu Asp Ser Leu Pro Glu Gln Pro Glu
               580                 585                 590
   Asp Ser Ala Gln Leu Gln Gln Glu Lys Pro Ser Leu Tyr Ile Gly Val
           595                 600                 605
   Arg Gly Thr Val Val Arg Ser Met Gln Glu Val Leu Trp Thr Arg Leu
       610                 615                 620
   Arg Glu Leu Pro Asp Pro Val Leu Ser Glu Glu Val Val Glu Gly Ile
   625                 630                 635                 640
   Ala Ala Gly Ile Glu Ala Ala Leu Trp Asp Leu Thr Gln Gly Thr Asn
                   645                 650                 655
   Gly Arg Tyr Lys Thr Lys Tyr Arg Ser Leu Leu Phe Asn Leu Arg Asp
               660                 665                 670
   Pro Arg Asn Leu Asp Leu Phe Leu Lys Val Val His Gly Asp Val Thr
           675                 680                 685
   Pro Tyr Asp Leu Val Arg Met Ser Ser Met Gln Leu Ala Pro Gln Glu
       690                 695                 700
   Leu Ala Arg Trp Arg Asp Gln Glu Glu Lys Arg Gly Leu Asn Ile Ile
   705                 710                 715                 720
   Glu Gln Gln Gln Lys Glu Pro Cys Arg Leu Pro Ala Ser Lys Met Thr
                   725                 730                 735
   His Lys Gly Glu Val Glu Ile Gln Arg Asp Met Asp Gln Thr Leu Thr
               740                 745                 750
   Leu Glu Asp Leu Val Gly Pro Gln Met Phe Met Asp Cys Ser Pro Gln
           755                 760                 765
   Ala Leu Pro Ile Ala Ser Glu Asp Thr Thr Gly Gln His Asp His His
       770                 775                 780
   Phe Leu Asp Pro Asn Cys His Ile Cys Lys Asp Trp Glu Pro Ser Asn
   785                 790                 795                 800
   Glu Leu Leu Gly Ser Phe Glu Ala Ala Lys Ser Cys Gly Asp Asn Ile
                   805                 810                 815
   Phe Gln Lys Ala Leu Ser Gln Thr Pro Met Pro Ala Pro Glu Met Pro
               820                 825                 830
   Lys Thr Arg Glu Leu Ser Pro Thr Glu Pro Gln Asp Arg Val Pro Pro
           835                 840                 845
   Ser Gly Leu His Val Pro Ala Ala Pro Thr Lys Ala Leu Pro Cys Leu
       850                 855                 860
   Pro Pro Trp Glu Gly Val Leu Asp Met Phe Ser Ile Lys Arg Phe Arg
   865                 870                 875                 880
   Ala Arg Ala Gln Leu Val Ser Gly His Ser Cys Arg Leu Val Gln Ala
                   885                 890                 895
   Leu Pro Thr Val Ile Arg Ser Ala Gly Cys Ile Pro Ser Asn Ile Val
               900                 905                 910
   Trp Asp Leu Leu Ala Ser Ile Cys Pro Ala Lys Ala Lys Asp Val Cys
           915                 920                 925
   Val Val Arg Leu Cys Pro His Gly Ala Arg Asp Thr Gln Asn Cys Arg
       930                 935                 940
   Leu Leu Tyr Ser Tyr Leu Asn Asp Arg Gln Arg His Gly Leu Ala Ser
   945                 950                 955                 960
   Val Glu His Met Gly Met Val Leu Leu Pro Leu Pro Ala Phe Gln Pro
                   965                 970                 975
   Leu Pro Thr Arg Leu Arg Pro Leu Gly Gly Pro Gly Leu Trp Ala Leu
               980                 985                 990
   Pro Val Ser Pro Leu Leu Ser Pro  Gly Leu Glu Val Thr  His Ser Ser
           995                 1000                 1005
   Leu Leu  Leu Ala Val Leu Leu  Pro Lys Glu Gly Leu  Pro Asp Thr
       1010                 1015                 1020
   Ala Gly  Ser Ser Pro Trp Leu  Gly Lys Val Gln Lys  Met Val Ser
       1025                 1030                 1035
   Phe Asn  Ser Lys Val Glu Lys  Arg Tyr Tyr Gln Pro  Asp Asp Arg
       1040                 1045                 1050
   Arg Pro  Asn Val Pro Leu Lys  Gly Thr Pro Pro Pro  Gly Gly Ala
       1055                 1060                 1065
   Trp Gln  Gln Ser Gln Gly Arg  Gly Ser Ile Ala Pro  Arg Gly Ile
       1070                 1075                 1080
   Ser Ala  Trp Gln Arg Pro Pro  Arg Gly Arg Gly Arg  Leu Trp Pro
       1085                 1090                 1095
   Glu Pro  Glu Asn Trp Gln His  Pro Gly Arg Gly Gln  Trp Pro Pro
       1100                 1105                 1110
   Glu Pro  Gly Leu Arg Gln Ser  Gln His Pro Tyr Ser  Val Ala Pro
       1115                 1120                 1125
   Ala Gly  His Gly Phe Gly Arg  Gly Gln His Phe His  Arg Asp Ser
       1130                 1135                 1140
   Cys Pro  His Gln Ala Leu Leu  Arg His Leu Glu Ser  Leu Ala Thr
       1145                 1150                 1155
   Met Ser His Gln Leu Gln Ala Leu Leu Cys Pro Gln Thr Lys Ser
       1160                1165                1170
   Ser Ile Pro Arg Pro Leu Gln Arg Leu Ser Ser Ala Leu Ala Ala
       1175                1180                1185
   Pro Glu Pro Pro Gly Pro Ala Arg Asp Ser Ser Leu Gly Pro Thr
       1190                1195                1200
   Asp Glu Ala Gly Ser Glu Cys Pro Phe Pro Arg Lys Ala
       1205                1210                1215
<210> 79
   <211> 1903
   <212> DNA
   <213> Homo sapiens
<220>
   <221> CDS
   <222> (190)..(1401)
   <223>
<400> 79
   agcatgagtc agacagcctc tggctttctg gaagggcaag gactctatat atacagaggg     60
   agcttcctag ctgggatatt ggagcagcaa gaggctggga agccatcact taccttgcac    120
   tgagaaagaa gacaaaggca agttgaaaag cggagaaata gtggcccagt ggttgaaaaa    180
   ttgaagcaa atg cag gaa ttc ttt ggg ctg aaa gtg act ggg aaa cca gat    231
             Met Gln Glu Phe Phe Gly Leu Lys Val Thr Gly Lys Pro Asp
             1               5                   10
   gct gaa acc ctg aag gtg atg aag cag ccc aga tgt gga gtg cct gat      279
   Ala Glu Thr Leu Lys Val Met Lys Gln Pro Arg Cys Gly Val Pro Asp
   15                  20                  25                  30
   gtg gct cag ttt gtc ctc act gag ggg aac cct cgc tgg gag caa aca      327
   Val Ala Gln Phe Val Leu Thr Glu Gly Asn Pro Arg Trp Glu Gln Thr
                   35                  40                  45
   cat ctg acc tac agg att gaa aat tac acg cca gat ttg cca aga gca      375
   His Leu Thr Tyr Arg Ile Glu Asn Tyr Thr Pro Asp Leu Pro Arg Ala
               50                  55                  60
   gat gtg gac cat gcc att gag aaa gcc ttc caa ctc tgg agt aat gtc      423
   Asp Val Asp His Ala Ile Glu Lys Ala Phe Gln Leu Trp Ser Asn Val
           65                  70                  75
   aca cct ctg aca ttc acc aag gtc tct gag ggt caa gca gac atc atg      471
   Thr Pro Leu Thr Phe Thr Lys Val Ser Glu Gly Gln Ala Asp Ile Met
       80                  85                  90
   ata tct ttt gtc agg gga gat cat cgg gac aac tct cct ttt gat gga      519
   Ile Ser Phe Val Arg Gly Asp His Arg Asp Asn Ser Pro Phe Asp Gly
   95                  100                 105                 110
   cct gga gga aat ctt gct cat gct ttt caa cca ggc cca ggt att gga      567
   Pro Gly Gly Asn Leu Ala His Ala Phe Gln Pro Gly Pro Gly Ile Gly
                   115                 120                 125
   ggg gat gct cat ttt gat gaa gat gaa agg tgg acc aac aat ttc aga      615
   Gly Asp Ala His Phe Asp Glu Asp Glu Arg Trp Thr Asn Asn Phe Arg
               130                 135                 140
   gag tac aac tta cat cgt gtt gca gct cat gaa ctc ggc cat tct ctt      663
   Glu Tyr Asn Leu His Arg Val Ala Ala His Glu Leu Gly His Ser Leu
           145                 150                 155
   gga ctc tcc cat tct act gat atc ggg gct ttg atg tac cct agc tac      711
   Gly Leu Ser His Ser Thr Asp Ile Gly Ala Leu Met Tyr Pro Ser Tyr
       160                 165                 170
   acc ttc agt ggt gat gtt cag cta gct cag gat gac att gat ggc atc      759
   Thr Phe Ser Gly Asp Val Gln Leu Ala Gln Asp Asp Ile Asp Gly Ile
   175                 180                 185                 190
   caa gcc ata tat gga cgt tcc caa aat cct gtc cag ccc atc ggc cca      807
   Gln Ala Ile Tyr Gly Arg Ser Gln Asn Pro Val Gln Pro Ile Gly Pro
                   195                 200                 205
   caa acc cca aaa gcg tgt gac agt aag cta acc ttt gat gct ata act      855
   Gln Thr Pro Lys Ala Cys Asp Ser Lys Leu Thr Phe Asp Ala Ile Thr
               210                 215                 220
   acg att cgg gga gaa gtg atg ttc ttt aaa gac aga ttc tac atg cgc      903
   Thr Ile Arg Gly Glu Val Met Phe Phe Lys Asp Arg Phe Tyr Met Arg
           225                 230                 235
   aca aat ccc ttc tac ccg gaa gtt gag ctc aat ttc att tct gtt ttc      951
   Thr Asn Pro Phe Tyr Pro Glu Val Glu Leu Asn Phe Ile Ser Val Phe
       240                 245                 250
   tgg cca caa ctg cca aat ggg ctt gaa gct gct tac gaa ttt gcc gac      999
   Trp Pro Gln Leu Pro Asn Gly Leu Glu Ala Ala Tyr Glu Phe Ala Asp
   255                 260                 265                 270
   aga gat gaa gtc cgg ttt ttc aaa ggg aat aag tac tgg gct gtt cag     1047
   Arg Asp Glu Val Arg Phe Phe Lys Gly Asn Lys Tyr Trp Ala Val Gln
                   275                 280                 285
   gga cag aat gtg cta cac gga tac ccc aag gac atc tac agc tcc ttt     1095
   Gly Gln Asn Val Leu His Gly Tyr Pro Lys Asp Ile Tyr Ser Ser Phe
               290                 295                 300
   ggc ttc cct aga act gtg aag cat atc gat gct gct ctt tct gag gaa     1143
   Gly Phe Pro Arg Thr Val Lys His Ile Asp Ala Ala Leu Ser Glu Glu
           305                 310                 315
   aac act gga aaa acc tac ttc ttt gtt gct aac aaa tac tgg agg tat     1191
   Asn Thr Gly Lys Thr Tyr Phe Phe Val Ala Asn Lys Tyr Trp Arg Tyr
       320                 325                 330
   gat gaa tat aaa cga tct atg gat cca ggt tat ccc aaa atg ata gca     1239
   Asp Glu Tyr Lys Arg Ser Met Asp Pro Gly Tyr Pro Lys Met Ile Ala
   335                 340                 345                 350
   cat gac ttt cct gga att ggc cac aaa gtt gat gca gtt ttc atg aaa     1287
   His Asp Phe Pro Gly Ile Gly His Lys Val Asp Ala Val Phe Met Lys
                   355                 360                 365
   gat gga ttt ttc tat ttc ttt cat gga aca aga caa tac aaa ttt gat      1335
   Asp Gly Phe Phe Tyr Phe Phe His Gly Thr Arg Gln Tyr Lys Phe Asp
               370                 375                 380
   cct aaa acg aag aga att ttg act ctc cag aaa gct aat agc tgg ttc      1383
   Pro Lys Thr Lys Arg Ile Leu Thr Leu Gln Lys Ala Asn Ser Trp Phe
           385                 390                 395
   aac tgc agg aaa aat tga acattactaa tttgaatgga aaacacatgg             1431
   Asn Cys Arg Lys Asn
       400
   tgtgagtcca aagaaggtgt tttcctgaag aactgtctat tttctcagtc atttttaacc    1491
   tctagagtca ctgatacaca gaatataatc ttatttatac ctcagtttgc atattttttt    1551
   actatttaga atgtagccct ttttgtactg atataattta gttccacaaa tggtgggtac    1611
   aaaaagtcaa gtttgtggct tatggattca tataggccag agttgcaaag atcttttcca    1671
   gagtatgcaa ctctgacgtt gatcccagag agcagcttca gtgacaaaca tatcctttca    1731
   agacagaaag agacaggaga catgagtctt tgccggagga aaagcagctc aagaacacat    1791
   gtgcagtcac tggtgtcacc ctggataggc aagggataac tcttctaaca caaaataagt    1851
   gttttatgtt tggaataaag tcaaccttgt ttctactgtt ttatacactt tc            1903
<210> 80
   <211> 403
   <212> PRT
   <213> Homo sapiens
<400> 80
   Met Gln Glu Phe Phe Gly Leu Lys Val Thr Gly Lys Pro Asp Ala Glu
   1               5                   10                  15
   Thr Leu Lys Val Met Lys Gln Pro Arg Cys Gly Val Pro Asp Val Ala
               20                  25                  30
   Gln Phe Val Leu Thr Glu Gly Asn Pro Arg Trp Glu Gln Thr His Leu
           35                  40                  45
   Thr Tyr Arg Ile Glu Asn Tyr Thr Pro Asp Leu Pro Arg Ala Asp Val
       50                  55                  60
   Asp His Ala Ile Glu Lys Ala Phe Gln Leu Trp Ser Asn Val Thr Pro
   65                  70                  75                  80
   Leu Thr Phe Thr Lys Val Ser Glu Gly Gln Ala Asp Ile Met Ile Ser
                   85                  90                  95
   Phe Val Arg Gly Asp His Arg Asp Asn Ser Pro Phe Asp Gly Pro Gly
               100                 105                 110
   Gly Asn Leu Ala His Ala Phe Gln Pro Gly Pro Gly Ile Gly Gly Asp
           115                 120                 125
   Ala His Phe Asp Glu Asp Glu Arg Trp Thr Asn Asn Phe Arg Glu Tyr
       130                 135                 140
   Asn Leu His Arg Val Ala Ala His Glu Leu Gly His Ser Leu Gly Leu
   145                 150                 155                 160
   Ser His Ser Thr Asp Ile Gly Ala Leu Met Tyr Pro Ser Tyr Thr Phe
                   165                 170                 175
   Ser Gly Asp Val Gln Leu Ala Gln Asp Asp Ile Asp Gly Ile Gln Ala
               180                 185                 190
   Ile Tyr Gly Arg Ser Gln Asn Pro Val Gln Pro Ile Gly Pro Gln Thr
           195                 200                 205
   Pro Lys Ala Cys Asp Ser Lys Leu Thr Phe Asp Ala Ile Thr Thr Ile
       210                 215                 220
   Arg Gly Glu Val Met Phe Phe Lys Asp Arg Phe Tyr Met Arg Thr Asn
   225                 230                 235                 240
   Pro Phe Tyr Pro Glu Val Glu Leu Asn Phe Ile Ser Val Phe Trp Pro
                   245                 250                 255
   Gln Leu Pro Asn Gly Leu Glu Ala Ala Tyr Glu Phe Ala Asp Arg Asp
               260                 265                 270
   Glu Val Arg Phe Phe Lys Gly Asn Lys Tyr Trp Ala Val Gln Gly Gln
           275                 280                 285
   Asn Val Leu His Gly Tyr Pro Lys Asp Ile Tyr Ser Ser Phe Gly Phe
       290                 295                 300
   Pro Arg Thr Val Lys His Ile Asp Ala Ala Leu Ser Glu Glu Asn Thr
   305                 310                 315                 320
   Gly Lys Thr Tyr Phe Phe Val Ala Asn Lys Tyr Trp Arg Tyr Asp Glu
                   325                 330                 335
   Tyr Lys Arg Ser Met Asp Pro Gly Tyr Pro Lys Met Ile Ala His Asp
               340                 345                 350
   Phe Pro Gly Ile Gly His Lys Val Asp Ala Val Phe Met Lys Asp Gly
   355 360 365
   Phe Phe Tyr Phe Phe His Gly Thr Arg Gln Tyr Lys Phe Asp Pro Lys
   370 375 380
   Thr Lys Arg Ile Leu Thr Leu Gln Lys Ala Asn Ser Trp Phe Asn Cys
   385 390 395 400
   Arg Lys Asn
<210> 81
   <211> 1203
   <212> DNA
   <213> Homo sapiens
<220>
   <221> CDS
   <222> (76)..(783)
   <223>
<400> 81
   agaaagccgc gcacctcctc ccgccaggcg ctttctcgga cgccttgccc agcgggccgc      60
   ccgaccccct gcacc atg gac ccc gct cgc ccc ctg ggg ctg tcg att ctg     111
                    Met Asp Pro Ala Arg Pro Leu Gly Leu Ser Ile Leu
                    1               5                   10
   ctg ctt ttc ctg acg gag gct gca ctg ggc gat gct gct cag gag cca      159
   Leu Leu Phe Leu Thr Glu Ala Ala Leu Gly Asp Ala Ala Gln Glu Pro
           15                  20                  25
   aca gga aat aac gcg gag atc tgt ctc ctg ccc cta gac tac gga ccc      207
   Thr Gly Asn Asn Ala Glu Ile Cys Leu Leu Pro Leu Asp Tyr Gly Pro
       30                  35                  40
   tgc cgg gcc cta ctt ctc cgt tac tac tac gac agg tac acg cag agc      255
   Cys Arg Ala Leu Leu Leu Arg Tyr Tyr Tyr Asp Arg Tyr Thr Gln Ser
   45                  50                  55                  60
   tgc cgc cag ttc ctg tac ggg ggc tgc gag ggc aac gcc aac aat ttc      303
   Cys Arg Gln Phe Leu Tyr Gly Gly Cys Glu Gly Asn Ala Asn Asn Phe
                   65                  70                  75
   tac acc tgg gag gct tgc gac gat gct tgc tgg agg ata gaa aaa gtt      351
   Tyr Thr Trp Glu Ala Cys Asp Asp Ala Cys Trp Arg Ile Glu Lys Val
               80                  85                  90
   ccc aaa gtt tgc cgg ctg caa gtg agt gtg gac gac cag tgt gag ggg      399
   Pro Lys Val Cys Arg Leu Gln Val Ser Val Asp Asp Gln Cys Glu Gly
           95                  100                 105
   tcc aca gaa aag tat ttc ttt aat cta agt tcc atg aca tgt gaa aaa      447
   Ser Thr Glu Lys Tyr Phe Phe Asn Leu Ser Ser Met Thr Cys Glu Lys
       110                 115                 120
   ttc ttt tcc ggt ggg tgt cac cgg aac cgg att gag aac agg ttt cca      495
   Phe Phe Ser Gly Gly Cys His Arg Asn Arg Ile Glu Asn Arg Phe Pro
   125                 130                 135                 140
   gat gaa gct act tgt atg ggc ttc tgc gca cca aag aaa att cca tca      543
   Asp Glu Ala Thr Cys Met Gly Phe Cys Ala Pro Lys Lys Ile Pro Ser
                   145                 150                 155
   ttt tgc tac agt cca aaa gat gag gga ctg tgc tct gcc aat gtg act      591
   Phe Cys Tyr Ser Pro Lys Asp Glu Gly Leu Cys Ser Ala Asn Val Thr
               160                 165                 170
   cgc tat tat ttt aat cca aga tac aga acc tgt gat gct ttc acc tat      639
   Arg Tyr Tyr Phe Asn Pro Arg Tyr Arg Thr Cys Asp Ala Phe Thr Tyr
           175                 180                 185
   act ggc tgt gga ggg aat gac aat aac ttt gtt agc agg gag gat tgc      687
   Thr Gly Cys Gly Gly Asn Asp Asn Asn Phe Val Ser Arg Glu Asp Cys
       190                 195                 200
   aaa cgt gca tgt gca aaa gct ttg aaa aag aaa aag aag atg cca aag      735
   Lys Arg Ala Cys Ala Lys Ala Leu Lys Lys Lys Lys Lys Met Pro Lys
   205                 210                 215                 220
   ctt cgc ttt gcc agt aga atc cgg aaa att cgg aag aag caa ttt taa      783
   Leu Arg Phe Ala Ser Arg Ile Arg Lys Ile Arg Lys Lys Gln Phe
                   225                 230                 235
   acattcttaa tatgtcatct tgtttgtctt tatggcttat ttgcctttat ggttgtatct    843
   gaagaataat atgacagcat gaggaaacaa atcattggtg atttattcac cagtttttat    903
   taatacaagt cactttttca aaaatttgga tttttttata tataactagc tgctattcaa     963
   atgtgagtct accattttta atttatggtt caactgtttg tgagactgaa ttcttgcaat   1023
   gcataagata taaaagcaaa tatgactcac tcatttcttg gggtcgtatt cctgatttca   1083
   gaagaggatc ataactgaaa caacataaga caatataatc atgtgctttt aacatatttg   1143
   agaataaaaa ggactagcaa ataaaacaca aaaaaaaaaa aaaaaaaaaa aaaaaaaaaa   1203
<210> 82
   <211> 235
   <212> PRT
   <213> Homo sapiens
<400> 82
   Met Asp Pro Ala Arg Pro Leu Gly Leu Ser Ile Leu Leu Leu Phe Leu
   1               5                   10                  15
   Thr Glu Ala Ala Leu Gly Asp Ala Ala Gln Glu Pro Thr Gly Asn Asn
               20                  25                  30
   Ala Glu Ile Cys Leu Leu Pro Leu Asp Tyr Gly Pro Cys Arg Ala Leu
           35                  40                  45
   Leu Leu Arg Tyr Tyr Tyr Asp Arg Tyr Thr Gln Ser Cys Arg Gln Phe
       50                  55                  60
   Leu Tyr Gly Gly Cys Glu Gly Asn Ala Asn Asn Phe Tyr Thr Trp Glu
   65                  70                  75                  80
   Ala Cys Asp Asp Ala Cys Trp Arg Ile Glu Lys Val Pro Lys Val Cys
                   85                  90                  95
   Arg Leu Gln Val Ser Val Asp Asp Gln Cys Glu Gly Ser Thr Glu Lys
               100                 105                 110
   Tyr Phe Phe Asn Leu Ser Ser Met Thr Cys Glu Lys Phe Phe Ser Gly
           115                 120                 125
   Gly Cys His Arg Asn Arg Ile Glu Asn Arg Phe Pro Asp Glu Ala Thr
       130                 135                 140
   Cys Met Gly Phe Cys Ala Pro Lys Lys Ile Pro Ser Phe Cys Tyr Ser
   145                 150                 155                 160
   Pro Lys Asp Glu Gly Leu Cys Ser Ala Asn Val Thr Arg Tyr Tyr Phe
                   165                 170                 175
   Asn Pro Arg Tyr Arg Thr Cys Asp Ala Phe Thr Tyr Thr Gly Cys Gly
               180                 185                 190
   Gly Asn Asp Asn Asn Phe Val Ser Arg Glu Asp Cys Lys Arg Ala Cys
           195                 200                 205
   Ala Lys Ala Leu Lys Lys Lys Lys Lys Met Pro Lys Leu Arg Phe Ala
       210                 215                 220
   Ser Arg Ile Arg Lys Ile Arg Lys Lys Gln Phe
   225                 230                 235
<210> 83
   <211> 1903
   <212> DNA
   <213> Homo sapiens
<220>
   <221> CDS
   <222> (190)..(1401)
   <223>
<400> 83
   agcatgagtc agacagcctc tggctttctg gaagggcaag gactctatat atacagaggg     60
   agcttcctag ctgggatatt ggagcagcaa gaggctggga agccatcact taccttgcac    120
   tgagaaagaa gacaaaggca agttgaaaag cggagaaata gtggcccagt ggttgaaaaa    180
   ttgaagcaa atg cag gaa ttc ttt ggg ctg aaa gtg act ggg aaa cca gat    231
             Met Gln Glu Phe Phe Gly Leu Lys Val Thr Gly Lys Pro Asp
               1               5                   10
   gct gaa acc ctg aag gtg atg aag cag ccc aga tgt gga gtg cct gat      279
   Ala Glu Thr Leu Lys Val Met Lys Gln Pro Arg Cys Gly Val Pro Asp
   15                  20                  25                  30
   gtg gct cag ttt gtc ctc act gag ggg aac cct cgc tgg gag caa aca      327
   Val Ala Gln Phe Val Leu Thr Glu Gly Asn Pro Arg Trp Glu Gln Thr
                   35                  40                  45
   cat ctg acc tac agg att gaa aat tac acg cca gat ttg cca aga gca      375
   His Leu Thr Tyr Arg Ile Glu Asn Tyr Thr Pro Asp Leu Pro Arg Ala
               50                  55                  60
   gat gtg gac cat gcc att gag aaa gcc ttc caa ctc tgg agt aat gtc      423
   Asp Val Asp His Ala Ile Glu Lys Ala Phe Gln Leu Trp Ser Asn Val
           65                  70                  75
   aca cct ctg aca ttc acc aag gtc tct gag ggt caa gca gac atc atg      471
   Thr Pro Leu Thr Phe Thr Lys Val Ser Glu Gly Gln Ala Asp Ile Met
       80                  85                  90
   ata tct ttt gtc agg gga gat cat cgg gac aac tct cct ttt gat gga      519
   Ile Ser Phe Val Arg Gly Asp His Arg Asp Asn Ser Pro Phe Asp Gly
   95                  100                 105                 110
   cct gga gga aat ctt gct cat gct ttt caa cca ggc cca ggt att gga      567
   Pro Gly Gly Asn Leu Ala His Ala Phe Gln Pro Gly Pro Gly Ile Gly
                   115                 120                 125
   ggg gat gct cat ttt gat gaa gat gaa agg tgg acc aac aat ttc aga      615
   Gly Asp Ala His Phe Asp Glu Asp Glu Arg Trp Thr Asn Asn Phe Arg
               130                 135                 140
   gag tac aac tta cat cgt gtt gca gct cat gaa ctc ggc cat tct ctt      663
   Glu Tyr Asn Leu His Arg Val Ala Ala His Glu Leu Gly His Ser Leu
           145                 150                 155
   gga ctc tcc cat tct act gat atc ggg gct ttg atg tac cct agc tac      711
   Gly Leu Ser His Ser Thr Asp Ile Gly Ala Leu Met Tyr Pro Ser Tyr
       160                 165                 170
   acc ttc agt ggt gat gtt cag cta gct cag gat gac att gat ggc atc      759
   Thr Phe Ser Gly Asp Val Gln Leu Ala Gln Asp Asp Ile Asp Gly Ile
   175                 180                 185                 190
   caa gcc ata tat gga cgt tcc caa aat cct gtc cag ccc atc ggc cca      807
   Gln Ala Ile Tyr Gly Arg Ser Gln Asn Pro Val Gln Pro Ile Gly Pro
                   195                 200                 205
   caa acc cca aaa gcg tgt gac agt aag cta acc ttt gat gct ata act      855
   Gln Thr Pro Lys Ala Cys Asp Ser Lys Leu Thr Phe Asp Ala Ile Thr
               210                 215                 220
   acg att cgg gga gaa gtg atg ttc ttt aaa gac aga ttc tac atg cgc      903
   Thr Ile Arg Gly Glu Val Met Phe Phe Lys Asp Arg Phe Tyr Met Arg
           225                 230                 235
   aca aat ccc ttc tac ccg gaa gtt gag ctc aat ttc att tct gtt ttc      951
   Thr Asn Pro Phe Tyr Pro Glu Val Glu Leu Asn Phe Ile Ser Val Phe
       240                 245                 250
   tgg cca caa ctg cca aat ggg ctt gaa gct gct tac gaa ttt gcc gac      999
   Trp Pro Gln Leu Pro Asn Gly Leu Glu Ala Ala Tyr Glu Phe Ala Asp
   255                 260                 265                 270
   aga gat gaa gtc cgg ttt ttc aaa ggg aat aag tac tgg gct gtt cag      1047
   Arg Asp Glu Val Arg Phe Phe Lys Gly Asn Lys Tyr Trp Ala Val Gln
                   275                 280                 285
   gga cag aat gtg cta cac gga tac ccc aag gac atc tac agc tcc ttt      1095
   Gly Gln Asn Val Leu His Gly Tyr Pro Lys Asp Ile Tyr Ser Ser Phe
               290                 295                 300
   ggc ttc cct aga act gtg aag cat atc gat gct gct ctt tct gag gaa      1143
   Gly Phe Pro Arg Thr Val Lys His Ile Asp Ala Ala Leu Ser Glu Glu
           305                 310                 315
   aac act gga aaa acc tac ttc ttt gtt gct aac aaa tac tgg agg tat      1191
   Asn Thr Gly Lys Thr Tyr Phe Phe Val Ala Asn Lys Tyr Trp Arg Tyr
       320                 325                 330
   gat gaa tat aaa cga tct atg gat cca ggt tat ccc aaa atg ata gca      1239
   Asp Glu Tyr Lys Arg Ser Met Asp Pro Gly Tyr Pro Lys Met Ile Ala
   335                 340                 345                 350
   cat gac ttt cct gga att ggc cac aaa gtt gat gca gtt ttc atg aaa      1287
   His Asp Phe Pro Gly Ile Gly His Lys Val Asp Ala Val Phe Met Lys
                   355                 360                 365
   gat gga ttt ttc tat ttc ttt cat gga aca aga caa tac aaa ttt gat      1335
   Asp Gly Phe Phe Tyr Phe Phe His Gly Thr Arg Gln Tyr Lys Phe Asp
               370                 375                 380
   cct aaa acg aag aga att ttg act ctc cag aaa gct aat agc tgg ttc      1383
   Pro Lys Thr Lys Arg Ile Leu Thr Leu Gln Lys Ala Asn Ser Trp Phe
           385                 390                 395
   aac tgc agg aaa aat tga acattactaa tttgaatgga aaacacatgg            1431
   Asn Cys Arg Lys Asn
       400
   tgtgagtcca aagaaggtgt tttcctgaag aactgtctat tttctcagtc atttttaacc   1491
   tctagagtca ctgatacaca gaatataatc ttatttatac ctcagtttgc atattttttt   1551
   actatttaga atgtagccct ttttgtactg atataattta gttccacaaa tggtgggtac    1611
   aaaaagtcaa gtttgtggct tatggattca tataggccag agttgcaaag atcttttcca   1671
   gagtatgcaa ctctgacgtt gatcccagag agcagcttca gtgacaaaca tatcctttca   1731
   agacagaaag agacaggaga catgagtctt tgccggagga aaagcagctc aagaacacat   1791
   gtgcagtcac tggtgtcacc ctggataggc aagggataac tcttctaaca caaaataagt   1851
   gttttatgtt tggaataaag tcaaccttgt ttctactgtt ttatacactt tc           1903
<210> 84
   <211> 403
   <212> PRT
   <213> Homo sapiens
<400> 84
   Met Gln Glu Phe Phe Gly Leu Lys Val Thr Gly Lys Pro Asp Ala Glu
   1               5                   10                  15
   Thr Leu Lys Val Met Lys Gln Pro Arg Cys Gly Val Pro Asp Val Ala
               20                  25                  30
   Gln Phe Val Leu Thr Glu Gly Asn Pro Arg Trp Glu Gln Thr His Leu
           35                  40                  45
   Thr Tyr Arg Ile Glu Asn Tyr Thr Pro Asp Leu Pro Arg Ala Asp Val
       50                  55                  60
   Asp His Ala Ile Glu Lys Ala Phe Gln Leu Trp Ser Asn Val Thr Pro
   65                  70                  75                  80
   Leu Thr Phe Thr Lys Val Ser Glu Gly Gln Ala Asp Ile Met Ile Ser
                   85                  90                  95
   Phe Val Arg Gly Asp His Arg Asp Asn Ser Pro Phe Asp Gly Pro Gly
               100                 105                 110
   Gly Asn Leu Ala His Ala Phe Gln Pro Gly Pro Gly Ile Gly Gly Asp
           115                 120                 125
   Ala His Phe Asp Glu Asp Glu Arg Trp Thr Asn Asn Phe Arg Glu Tyr
       130                 135                 140
   Asn Leu His Arg Val Ala Ala His Glu Leu Gly His Ser Leu Gly Leu
   145                 150                 155                 160
   Ser His Ser Thr Asp Ile Gly Ala Leu Met Tyr Pro Ser Tyr Thr Phe
                   165                 170                 175
   Ser Gly Asp Val Gln Leu Ala Gln Asp Asp Ile Asp Gly Ile Gln Ala
               180                 185                 190
   Ile Tyr Gly Arg Ser Gln Asn Pro Val Gln Pro Ile Gly Pro Gln Thr
           195                 200                 205
   Pro Lys Ala Cys Asp Ser Lys Leu Thr Phe Asp Ala Ile Thr Thr Ile
       210                 215                 220
   Arg Gly Glu Val Met Phe Phe Lys Asp Arg Phe Tyr Met Arg Thr Asn
   225                 230                 235                 240
   Pro Phe Tyr Pro Glu Val Glu Leu Asn Phe Ile Ser Val Phe Trp Pro
                   245                 250                 255
   Gln Leu Pro Asn Gly Leu Glu Ala Ala Tyr Glu Phe Ala Asp Arg Asp
   260 265 270
   Glu Val Arg Phe Phe Lys Gly Asn Lys Tyr Trp Ala Val Gln Gly Gln
   275 280 285
   Asn Val Leu His Gly Tyr Pro Lys Asp Ile Tyr Ser Ser Phe Gly Phe
   290 295 300
   Pro Arg Thr Val Lys His Ile Asp Ala Ala Leu Ser Glu Glu Asn Thr
   305 310 315 320
   Gly Lys Thr Tyr Phe Phe Val Ala Asn Lys Tyr Trp Arg Tyr Asp Glu
   325 330 335
   Tyr Lys Arg Ser Met Asp Pro Gly Tyr Pro Lys Met Ile Ala His Asp
   340 345 350
   Phe Pro Gly Ile Gly His Lys Val Asp Ala Val Phe Met Lys Asp Gly
   355 360 365
   Phe Phe Tyr Phe Phe His Gly Thr Arg Gln Tyr Lys Phe Asp Pro Lys
   370 375 380
   Thr Lys Arg Ile Leu Thr Leu Gln Lys Ala Asn Ser Trp Phe Asn Cys
   385 390 395 400
   Arg Lys Asn
<210> 85
   <211> 938
   <212> DNA
   <213> Homo sapiens
<220>
   <221> CDS
   <222> (1)..(468)
   <223>
<400> 85
   atg gtc agc tcc tgt tgt ggc tcc gtg tgc tct gac cag ggc tgc agc       48
   Met Val Ser Ser Cys Cys Gly Ser Val Cys Ser Asp Gln Gly Cys Ser
   1               5                   10                  15
   caa gac ctc tgt cag gag acc tgc tgc cgc ccc agc tgc tgt cag acc       96
   Gln Asp Leu Cys Gln Glu Thr Cys Cys Arg Pro Ser Cys Cys Gln Thr
               20                  25                  30
   acc tgt tgc agg acc acc tgc tac cgc ccc agc tgt tgt gtg tcc agc      144
   Thr Cys Cys Arg Thr Thr Cys Tyr Arg Pro Ser Cys Cys Val Ser Ser
           35                  40                  45
   tgc tgc agg ccc cag tgc tgc cag tct gtg tgc tgc caa ccc acc tgc      192
   Cys Cys Arg Pro Gln Cys Cys Gln Ser Val Cys Cys Gln Pro Thr Cys
       50                  55                  60
   tgt cgc ccc acc tgc tgt gag acg acc tgc tgc cac cct agg tgc tgc      240
   Cys Arg Pro Thr Cys Cys Glu Thr Thr Cys Cys His Pro Arg Cys Cys
   65                  70                  75                  80
   atc tcc agc tgc tgc cgc ccc agc tgc tgt atg tcc agc tgc tgc aag      288
   Ile Ser Ser Cys Cys Arg Pro Ser Cys Cys Met Ser Ser Cys Cys Lys
                   85                  90                  95
   ccc cag tgc tgc cag tct gtg tgc tgc cag ccc acc tgc tgc cgc ccc      336
   Pro Gln Cys Cys Gln Ser Val Cys Cys Gln Pro Thr Cys Cys Arg Pro
               100                 105                 110
   agc tgc tgc cgc ccc tgc tgc tgc ctg cgt cca gtc tgt ggc cga gtc      384
   Ser Cys Cys Arg Pro Cys Cys Cys Leu Arg Pro Val Cys Gly Arg Val
           115                 120                 125
   tcc tgc cac acc act tgc tat cgc cca acc tgt gtc atc tcc acc tgt      432
   Ser Cys His Thr Thr Cys Tyr Arg Pro Thr Cys Val Ile Ser Thr Cys
       130                 135                 140
   ccc cgc ccc ttg tgc tgt gcc tcc tct tgc tgc tga gcccactgcc           478
   Pro Arg Pro Leu Cys Cys Ala Ser Ser Cys Cys
   145                 150                 155
   ctggctcacg tcccccttca ccactggccc acagatgtag acccttctac tgtgctgacc     538
   attaggatac atgaagtggg gttgatgtca ttcaatagga tggaccttat gcttccaaag    598
   agcccaccac catttcactg actctgtgag aacattctgg ttcattttaa actccctcct    658
   ttgctttctt tttcttctgg tggtggcacc aaatgtgaat taatttgtaa tacactagct    718
   aagaaattat tccaatcttc tgatttcctt attttcttta tcactttaag gtacagattc    778
   tccttctcag tgaggtagat attatctgca ggaccagttt tgtcactgat gttgcaccct    838
   cagatccagc cacccaattg tattctgtgt ttctcctagg gtgaatttct tatgctttgt    898
   tgtatctctg ctttctaata aacttttctg cacttaagaa                          938
<210> 86
   <211> 155
   <212> PRT
   <213> Homo sapiens
<400> 86
   Met Val Ser Ser Cys Cys Gly Ser Val Cys Ser Asp Gln Gly Cys Ser
   1               5                   10                  15
   Gln Asp Leu Cys Gln Glu Thr Cys Cys Arg Pro Ser Cys Cys Gln Thr
               20                  25                  30
   Thr Cys Cys Arg Thr Thr Cys Tyr Arg Pro Ser Cys Cys Val Ser Ser
           35                  40                  45
   Cys Cys Arg Pro Gln Cys Cys Gln Ser Val Cys Cys Gln Pro Thr Cys
       50                  55                  60
   Cys Arg Pro Thr Cys Cys Glu Thr Thr Cys Cys His Pro Arg Cys Cys
   65                  70                  75                  80
   Ile Ser Ser Cys Cys Arg Pro Ser Cys Cys Met Ser Ser Cys Cys Lys
                   85                  90                  95
   Pro Gln Cys Cys Gln Ser Val Cys Cys Gln Pro Thr Cys Cys Arg Pro
               100                 105                 110
   Ser Cys Cys Arg Pro Cys Cys Cys Leu Arg Pro Val Cys Gly Arg Val
           115                 120                 125
   Ser Cys His Thr Thr Cys Tyr Arg Pro Thr Cys Val Ile Ser Thr Cys
       130                 135                 140
   Pro Arg Pro Leu Cys Cys Ala Ser Ser Cys Cys
   145                 150                 155
<210> 87
   <211> 1143
   <212> DNA
   <213> Homo sapiens
<220>
   <221> CDS
   <222> (40)..(597)
   <223>
<400> 87
   ctcttggaaa cccaaataga tccttcaccc tctgacacc atg gtc aac tcc tgt        54
                                              Met Val Asn Ser Cys
                                              1               5
   tgt ggc tcc gtg tgc tct gac caa ggc tgt ggc caa gac ctc tgc cag      102
   Cys Gly Ser Val Cys Ser Asp Gln Gly Cys Gly Gln Asp Leu Cys Gln
                   10                  15                  20
   gag acc tgc tgc tgc ccc agc tgc tgt cag acc acc tgc tgc agg acc      150
   Glu Thr Cys Cys Cys Pro Ser Cys Cys Gln Thr Thr Cys Cys Arg Thr
               25                  30                  35
   acc tgc tac cgc ccc agc tac agt gtg tcc tgc tgc tgc aga ccc cag      198
   Thr Cys Tyr Arg Pro Ser Tyr Ser Val Ser Cys Cys Cys Arg Pro Gln
           40                  45                  50
   tgc tgc cag tct gtg tgc tgc cag ccc acc tgc tgt cgc ccc agc tgc      246
   Cys Cys Gln Ser Val Cys Cys Gln Pro Thr Cys Cys Arg Pro Ser Cys
       55                  60                  65
   tgt gtg tcc agc tgc tgc aag ccc cag tgc tgc cag tct gtg tgc tgc      294
   Cys Val Ser Ser Cys Cys Lys Pro Gln Cys Cys Gln Ser Val Cys Cys
   70                  75                  80                  85
   cag ccc acc tgc tgc cac cct agc tgc tgc atc tcc agc tgc tgc cgc      342
   Gln Pro Thr Cys Cys His Pro Ser Cys Cys Ile Ser Ser Cys Cys Arg
                   90                  95                  100
   ccc agc tgc tgt gtg tcc agc tgc tgc aag ccc cag tgc tgc cag tct      390
   Pro Ser Cys Cys Val Ser Ser Cys Cys Lys Pro Gln Cys Cys Gln Ser
               105                 110                 115
   gtg tgc tgc cag ccc aac tgc tgc cgc ccc agc tgc agc atc tcc agc      438
   Val Cys Cys Gln Pro Asn Cys Cys Arg Pro Ser Cys Ser Ile Ser Ser
           120                 125                 130
   tgc tgc cgc ccc tct tgc tgt gaa tcc agc tgc tgc cgc ccc tgc tgc      486
   Cys Cys Arg Pro Ser Cys Cys Glu Ser Ser Cys Cys Arg Pro Cys Cys
       135                 140                 145
   tgc ctg cgt cca gtc tgt ggc cga gtc tcc tgc cac acc act tgc tat      534
   Cys Leu Arg Pro Val Cys Gly Arg Val Ser Cys His Thr Thr Cys Tyr
   150                 155                 160                 165
   cgc cca gcc tgt gtc atc tcc acc tgc ccc cgc ccc gtg tgc tgc gcc      582
   Arg Pro Ala Cys Val Ile Ser Thr Cys Pro Arg Pro Val Cys Cys Ala
                   170                 175                 180
   tcc tct tgc tgc tga gcccactgcc ctggctcatc tctcccttca ccgctggtcc      637
   Ser Ser Cys Cys
               185
   acaaatgtag accattcttc tgtgctgaat attaggacac atggagtggg atttatgtca    697
   ttcagcaggg tggacctcat gtatccaatg agcccatcac catcccgctg actctgtgag    757
   aacattctgg ttcattttaa actccctccc ttgctttctt tttcttccag tcatggcacc    817
   aaatacgaat taatttgtaa tccactagct aagaaattat tccaatcctc taaattcctc    877
   attttttaaa atcattttga gcctacagaa tatccttccc aattaggtac acattatctc     937
   cctttcaaac atactatttg tctgtcagcc tttcagtcat tcttttcttt tggaaaggta    997
   ggaggctgcc cctcccgtgc tctcccgctt tctccctgct ctctctttct ctcactgttc   1057
   aagtttgtca gaatttttct attttattag ttctttatct ttattgtgtt cacaaaatat   1117
   attgtattaa acttttcatt tagaaa                                         1143
<210> 88
   <211> 185
   <212> PRT
   <213> Homo sapiens
<400> 88
   Met Val Asn Ser Cys Cys Gly Ser Val Cys Ser Asp Gln Gly Cys Gly
   1               5                   10                  15
   Gln Asp Leu Cys Gln Glu Thr Cys Cys Cys Pro Ser Cys Cys Gln Thr
               20                  25                  30
   Thr Cys Cys Arg Thr Thr Cys Tyr Arg Pro Ser Tyr Ser Val Ser Cys
           35                  40                  45
   Cys Cys Arg Pro Gln Cys Cys Gln Ser Val Cys Cys Gln Pro Thr Cys
       50                  55                  60
   Cys Arg Pro Ser Cys Cys Val Ser Ser Cys Cys Lys Pro Gln Cys Cys
   65                  70                  75                  80
   Gln Ser Val Cys Cys Gln Pro Thr Cys Cys His Pro Ser Cys Cys Ile
                   85                  90                  95
   Ser Ser Cys Cys Arg Pro Ser Cys Cys Val Ser Ser Cys Cys Lys Pro
               100                 105                 110
   Gln Cys Cys Gln Ser Val Cys Cys Gln Pro Asn Cys Cys Arg Pro Ser
           115                 120                 125
   Cys Ser Ile Ser Ser Cys Cys Arg Pro Ser Cys Cys Glu Ser Ser Cys
       130                 135                 140
   Cys Arg Pro Cys Cys Cys Leu Arg Pro Val Cys Gly Arg Val Ser Cys
   145                 150                 155                 160
   His Thr Thr Cys Tyr Arg Pro Ala Cys Val Ile Ser Thr Cys Pro Arg
                   165                 170                 175
   Pro Val Cys Cys Ala Ser Ser Cys Cys
               180                 185
<210> 89
   <211> 1100
   <212> DNA
   <213> Homo sapiens
<220>
   <221> CDS
   <222> (1)..(633)
   <223>
<400> 89
   atg gtc agc tcc tgt tgt ggc tcc gtg tgc tct gac cag ggc tgc ggc       48
   Met Val Ser Ser Cys Cys Gly Ser Val Cys Ser Asp Gln Gly Cys Gly
   1               5                   10                  15
   caa gac ctc tgt cag gag acc tgc tgc cgc ccc agc tgc tgt gag acc       96
   Gln Asp Leu Cys Gln Glu Thr Cys Cys Arg Pro Ser Cys Cys Glu Thr
               20                  25                  30
   acc tgc tgc agg acc acc tgc tgc cgc ccc agc tgt tgt gta tcc agc      144
   Thr Cys Cys Arg Thr Thr Cys Cys Arg Pro Ser Cys Cys Val Ser Ser
           35                  40                  45
   tgc tgc agg ccc cag tgc tgc cag tct gtg tgc tgc caa ccc act tgt      192
   Cys Cys Arg Pro Gln Cys Cys Gln Ser Val Cys Cys Gln Pro Thr Cys
       50                  55                  60
   tcc cgc ccc agc tgc tgt cag acc acc tgt tgc agg acc acc tgc tac      240
   Ser Arg Pro Ser Cys Cys Gln Thr Thr Cys Cys Arg Thr Thr Cys Tyr
   65                  70                  75                  80
   cgc ccc agc tgt tgt gtg tcc agc tgc tgc agg ccc cag tgc tgc cag      288
   Arg Pro Ser Cys Cys Val Ser Ser Cys Cys Arg Pro Gln Cys Cys Gln
                   85                  90                  95
   cct gcg tgc tgc caa ccc act tgc tgt cgc ccc agc tgc tgt gag acg      336
   Pro Ala Cys Cys Gln Pro Thr Cys Cys Arg Pro Ser Cys Cys Glu Thr
               100                 105                 110
   acc tgc tgc cac cct agg tgc tgc atc tcc agc tgc tgt cgc ccc agc      384
   Thr Cys Cys His Pro Arg Cys Cys Ile Ser Ser Cys Cys Arg Pro Ser
           115                 120                 125
   tgc tgt gtg tcc agc tgc tgc aag ccc cag tgc tgc cag tct gtg tgc      432
   Cys Cys Val Ser Ser Cys Cys Lys Pro Gln Cys Cys Gln Ser Val Cys
       130                 135                 140
   tgc cag ccc aac tgc tgc cgc ccc agc tgc agc atc tcc agc tgc tgc      480
   Cys Gln Pro Asn Cys Cys Arg Pro Ser Cys Ser Ile Ser Ser Cys Cys
   145                 150                 155                 160
   cgc ccc tct tgc tgt gaa tcc agc tgc tgc cgc ccc tgc tgc tgc gtg      528
   Arg Pro Ser Cys Cys Glu Ser Ser Cys Cys Arg Pro Cys Cys Cys Val
                   165                 170                 175
   cgt cca gtc tgt ggc cga gtc tcc tgc cac acc act tgc tat cgc cca      576
   Arg Pro Val Cys Gly Arg Val Ser Cys His Thr Thr Cys Tyr Arg Pro
               180                 185                 190
   acc tgt gtc atc tcc agc tgc ccc cgc ccc ttg tgc tgt gcc tcc tct      624
   Thr Cys Val Ile Ser Ser Cys Pro Arg Pro Leu Cys Cys Ala Ser Ser
           195                 200                 205
   tgc tgc tga gcccactgcc ctggcttatc tcccccttca ccactggccc              673
   Cys Cys
       210
   acagatgtag acccttctac tgtgctgacc attaggatac aggaagtggg gttgatgtca   733
   ttcaatagaa tggaactcat gtttccaatg agcccatcac catttcactg actctttgag   793
   aacattctga ttcattttaa actccctccc ttgctttctt tttcttccgg tggtggcacc   853
   aaatgtgaat taatttgtaa tccactagct agaaattatt ccaatcctct aatttcctca   913
   ttttctttat cactttgagg tacagatctt cttctcagtg aggcagacat tatctgcagg   973
   accagttttg tcactgaggt tgcaccctca gatccagccg cccagttata ttctgtgttt  1033
   ctcctagtgt gaatttctta tgctttgttg catctctgct ttttaataaa atttctgtac  1093
   ataagaa                                                            1100
<210> 90
   <211> 210
   <212> PRT
   <213> Homo sapiens
<400> 90
   Met Val Ser Ser Cys Cys Gly Ser Val Cys Ser Asp Gln Gly Cys Gly
   1               5                   10                  15
   Gln Asp Leu Cys Gln Glu Thr Cys Cys Arg Pro Ser Cys Cys Glu Thr
               20                  25                  30
   Thr Cys Cys Arg Thr Thr Cys Cys Arg Pro Ser Cys Cys Val Ser Ser
           35                  40                  45
   Cys Cys Arg Pro Gln Cys Cys Gln Ser Val Cys Cys Gln Pro Thr Cys
       50                  55                  60
   Ser Arg Pro Ser Cys Cys Gln Thr Thr Cys Cys Arg Thr Thr Cys Tyr
   65                  70                  75                  80
   Arg Pro Ser Cys Cys Val Ser Ser Cys Cys Arg Pro Gln Cys Cys Gln
                   85                  90                  95
   Pro Ala Cys Cys Gln Pro Thr Cys Cys Arg Pro Ser Cys Cys Glu Thr
               100                 105                 110
   Thr Cys Cys His Pro Arg Cys Cys Ile Ser Ser Cys Cys Arg Pro Ser
           115                 120                 125
   Cys Cys Val Ser Ser Cys Cys Lys Pro Gln Cys Cys Gln Ser Val Cys
       130                 135                 140
   Cys Gln Pro Asn Cys Cys Arg Pro Ser Cys Ser Ile Ser Ser Cys Cys
   145                 150                 155                 160
   Arg Pro Ser Cys Cys Glu Ser Ser Cys Cys Arg Pro Cys Cys Cys Val
                   165                 170                 175
   Arg Pro Val Cys Gly Arg Val Ser Cys His Thr Thr Cys Tyr Arg Pro
               180                 185                 190
   Thr Cys Val Ile Ser Ser Cys Pro Arg Pro Leu Cys Cys Ala Ser Ser
           195                 200                 205
   Cys Cys
       210
<210> 91
   <211> 5770
   <212> DNA
   <213> Homo sapiens
<220>
   <221> CDS
   <222> (233)..(4195)
   <223>
<400> 91
   gacaaatgct ctggctccgg tggtgacagg ttgtccagct tcttacagcc atcttcactg     60
   aaactaaggt taactcctca ctctctatgg acggctactc tctatttcca agggcgtgaa    120
   gaatttccct cttctcctgt gctttcatct aaaagttctc cctggataat tatcattgca    180
   gtggagtgcc tggattggac atcctcatct gggtcaacta aaaaaagaaa gc atg caa    238
                                                             Met Gln
                                                             1
   gac gac agc ata gaa gct tct act tcc ata tct cag ctt cta aga gag      286
   Asp Asp Ser Ile Glu Ala Ser Thr Ser Ile Ser Gln Leu Leu Arg Glu
           5                   10                  15
   agc tat tta gct gaa acc aga cat cgg gga aac aat gag agg agt cga      334
   Ser Tyr Leu Ala Glu Thr Arg His Arg Gly Asn Asn Glu Arg Ser Arg
       20                  25                  30
   gcg gag ccc tcc tcc aac cct tgc cat ttc ggc agt cct tct ggg gcc      382
   Ala Glu Pro Ser Ser Asn Pro Cys His Phe Gly Ser Pro Ser Gly Ala
   35                  40                  45                  50
   gct gaa gga ggc gga ggc caa gat gac ctt cca gat ctt tca gcc ttt      430
   Ala Glu Gly Gly Gly Gly Gln Asp Asp Leu Pro Asp Leu Ser Ala Phe
                   55                  60                  65
   ctg agc caa gaa gaa tta gac gaa agt gtc aat ttg gca aga ctg gcc      478
   Leu Ser Gln Glu Glu Leu Asp Glu Ser Val Asn Leu Ala Arg Leu Ala
               70                  75                  80
   atc aat tac gac cct ttg gag aag gca gat gaa act caa gct aga aaa      526
   Ile Asn Tyr Asp Pro Leu Glu Lys Ala Asp Glu Thr Gln Ala Arg Lys
           85                  90                  95
   cga ctt tct cct gat cag atg aaa cac tca cct aat tta agt ttt gag      574
   Arg Leu Ser Pro Asp Gln Met Lys His Ser Pro Asn Leu Ser Phe Glu
       100                 105                 110
   cct aac ttc tgc cag gat aac cct cga agt ccc acc agc tct aaa gaa      622
   Pro Asn Phe Cys Gln Asp Asn Pro Arg Ser Pro Thr Ser Ser Lys Glu
   115                 120                 125                 130
   agc ccc cag gag gca aaa agg cca cag tat tgt tct gaa acc cag tcc      670
   Ser Pro Gln Glu Ala Lys Arg Pro Gln Tyr Cys Ser Glu Thr Gln Ser
                   135                 140                 145
   aaa aaa gta ttt tta aat aag gct gcc gac ttc att gaa gag cta tcc      718
   Lys Lys Val Phe Leu Asn Lys Ala Ala Asp Phe Ile Glu Glu Leu Ser
               150                 155                 160
   tcc ctt ttc aaa tcc cac agc tcc aaa agg att aga cct cgt gcc tgc      766
   Ser Leu Phe Lys Ser His Ser Ser Lys Arg Ile Arg Pro Arg Ala Cys
           165                 170                 175
   aaa aac cac aag agt aaa ctg gaa tct caa aac aaa gtt atg cag gaa      814
   Lys Asn His Lys Ser Lys Leu Glu Ser Gln Asn Lys Val Met Gln Glu
       180                 185                 190
   aac agc tcc agt ttc tca gat ctg tca gaa aga cga gaa aga tct tct      862
   Asn Ser Ser Ser Phe Ser Asp Leu Ser Glu Arg Arg Glu Arg Ser Ser
   195                 200                 205                 210
   gtt ccc atc cct atc cct gcg gat acc agg gat aat gaa gtg aat cac      910
   Val Pro Ile Pro Ile Pro Ala Asp Thr Arg Asp Asn Glu Val Asn His
                   215                 220                 225
   gcc ctg gaa cag cag gaa gcc aag agg cgt gaa gcg gag cag gct gcc      958
   Ala Leu Glu Gln Gln Glu Ala Lys Arg Arg Glu Ala Glu Gln Ala Ala
               230                 235                 240
   agt gag gcg gct ggt gga gac act aca cca ggg tct tcc cct tca tct      1006
   Ser Glu Ala Ala Gly Gly Asp Thr Thr Pro Gly Ser Ser Pro Ser Ser
           245                 250                 255
   ctg tac tat gaa gaa cct ctg ggg caa cct ccc cgg ttc act caa aag      1054
   Leu Tyr Tyr Glu Glu Pro Leu Gly Gln Pro Pro Arg Phe Thr Gln Lys
       260                 265                 270
   tta cgg agc aga gaa gtt cca gaa gga act cga gta cag ttg gat tgc      1102
   Leu Arg Ser Arg Glu Val Pro Glu Gly Thr Arg Val Gln Leu Asp Cys
   275                 280                 285                 290
   ata gtg gta gga att cca cca cct caa gta agg tgg tac tgt gaa ggc      1150
   Ile Val Val Gly Ile Pro Pro Pro Gln Val Arg Trp Tyr Cys Glu Gly
                   295                 300                 305
   aag gag ctt gaa aat tcc cca gat att cac atc gtc cag gca gga aat      1198
   Lys Glu Leu Glu Asn Ser Pro Asp Ile His Ile Val Gln Ala Gly Asn
               310                 315                 320
   ctg cac tca ctg acc att gcg gaa gcc ttt gaa gag gac aca gga cgc      1246
   Leu His Ser Leu Thr Ile Ala Glu Ala Phe Glu Glu Asp Thr Gly Arg
           325                 330                 335
   tat tcc tgc ttt gct tct aac atc tat ggg aca gat tcg act tct gct      1294
   Tyr Ser Cys Phe Ala Ser Asn Ile Tyr Gly Thr Asp Ser Thr Ser Ala
       340                 345                 350
   gag att tat ata gaa ggg gtt tct tct tct gac tca gaa ggc gac cct      1342
   Glu Ile Tyr Ile Glu Gly Val Ser Ser Ser Asp Ser Glu Gly Asp Pro
   355                 360                 365                 370
   aac aag gaa gag atg aat cga atc cag aag cca aat gag gtg tca tct      1390
   Asn Lys Glu Glu Met Asn Arg Ile Gln Lys Pro Asn Glu Val Ser Ser
                   375                 380                 385
   cct ccc act acc tct gca gtc att cct cca gca gta ccc caa gcc cag      1438
   Pro Pro Thr Thr Ser Ala Val Ile Pro Pro Ala Val Pro Gln Ala Gln
               390                 395                 400
   cat ttg gtg gcc caa cct cgt gtg gca acc atc cag cag tgt cag agc      1486
   His Leu Val Ala Gln Pro Arg Val Ala Thr Ile Gln Gln Cys Gln Ser
           405                 410                 415
   ccc acc aat tac ttg cag gga ttg gat gga aaa cct atc att gca gct      1534
   Pro Thr Asn Tyr Leu Gln Gly Leu Asp Gly Lys Pro Ile Ile Ala Ala
       420                 425                 430
   cct gtg ttt aca aag atg cta caa aat ttg tca gct tct gag ggt cag      1582
   Pro Val Phe Thr Lys Met Leu Gln Asn Leu Ser Ala Ser Glu Gly Gln
   435                 440                 445                 450
   ctg gtt gtc ttt gaa tgc aga gta aaa gga gct cca tct cct aag gtt      1630
   Leu Val Val Phe Glu Cys Arg Val Lys Gly Ala Pro Ser Pro Lys Val
                   455                 460                 465
   gag tgg tat aga gaa ggg act tta ata gaa gat tct cca gat ttt agg      1678
   Glu Trp Tyr Arg Glu Gly Thr Leu Ile Glu Asp Ser Pro Asp Phe Arg
               470                 475                 480
   att tta cag aaa aaa cct cga tcc atg gca gag cca gag gag att tgc      1726
   Ile Leu Gln Lys Lys Pro Arg Ser Met Ala Glu Pro Glu Glu Ile Cys
           485                 490                 495
   acc ttg gtc att gct gag gtg ttt gca gaa gat tct ggg tgc ttc aca      1774
   Thr Leu Val Ile Ala Glu Val Phe Ala Glu Asp Ser Gly Cys Phe Thr
       500                 505                 510
   tgt act gca agc aac aaa tac ggc aca gtg tca agc att gca cag ctg      1822
   Cys Thr Ala Ser Asn Lys Tyr Gly Thr Val Ser Ser Ile Ala Gln Leu
   515                 520                 525                 530
   cac gtg aga gga aat gag gac ctc agc aac aac ggg tct ctt cac tca      1870
   His Val Arg Gly Asn Glu Asp Leu Ser Asn Asn Gly Ser Leu His Ser
                   535                 540                 545
   gcc aac tct acc acc aac ctg gca gct att gag cca cag ccc tcc cca      1918
   Ala Asn Ser Thr Thr Asn Leu Ala Ala Ile Glu Pro Gln Pro Ser Pro
               550                 555                 560
   ccc cac tca gag cct cca tct gtg gaa caa ccc ccc aaa ccc aaa ctc      1966
   Pro His Ser Glu Pro Pro Ser Val Glu Gln Pro Pro Lys Pro Lys Leu
           565                 570                 575
   gag ggg gtt ctg gtg aac cac aat gag ccc cgg tcc agc tcc agg att      2014
   Glu Gly Val Leu Val Asn His Asn Glu Pro Arg Ser Ser Ser Arg Ile
       580                 585                 590
   ggg ctt cgt gtg cac ttc aac ctg cct gaa gat gac aaa gga agt gaa      2062
   Gly Leu Arg Val His Phe Asn Leu Pro Glu Asp Asp Lys Gly Ser Glu
   595                 600                 605                 610
   gca tcc tcc gag gct ggt gtg gtg acc acc aga cag acc agg ccc gat      2110
   Ala Ser Ser Glu Ala Gly Val Val Thr Thr Arg Gln Thr Arg Pro Asp
                   615                 620                 625
   tct ttc cag gag agg ttc aac gga cag gca aca aaa acc cca gag cct      2158
   Ser Phe Gln Glu Arg Phe Asn Gly Gln Ala Thr Lys Thr Pro Glu Pro
               630                 635                 640
   tct tcc ccc gtg aaa gag ccc cct cca gtt ctg gcc aaa ccc aaa ctt      2206
   Ser Ser Pro Val Lys Glu Pro Pro Pro Val Leu Ala Lys Pro Lys Leu
           645                 650                 655
   gat tcc act cag tta caa cag ctt cat aac caa gtc tta ctg gaa caa      2254
   Asp Ser Thr Gln Leu Gln Gln Leu His Asn Gln Val Leu Leu Glu Gln
       660                 665                 670
   cac caa ttg caa aac cca cct cct tca tct cct aag gag ttt cct ttc      2302
   His Gln Leu Gln Asn Pro Pro Pro Ser Ser Pro Lys Glu Phe Pro Phe
   675                 680                 685                 690
   agc atg act gtt ttg aac tcc aat gct ccc cca gcg gtg aca aca tcc      2350
   Ser Met Thr Val Leu Asn Ser Asn Ala Pro Pro Ala Val Thr Thr Ser
                   695                 700                 705
   agt aag cag gtg aag gct cct tca tca cag acg ttc agc ttg gcc cgg      2398
   Ser Lys Gln Val Lys Ala Pro Ser Ser Gln Thr Phe Ser Leu Ala Arg
               710                 715                 720
   ccg aag tat ttc ttc ccc tcc acg aac acc acc gca gca act gtg gcc      2446
   Pro Lys Tyr Phe Phe Pro Ser Thr Asn Thr Thr Ala Ala Thr Val Ala
           725                 730                 735
   cct tcc agc tct ccg gtg ttc act ttg agc agc act cct caa act att      2494
   Pro Ser Ser Ser Pro Val Phe Thr Leu Ser Ser Thr Pro Gln Thr Ile
       740                 745                 750
   cag agg aca gtg agc aaa gaa agc ctc tta gtg tct cac ccc tct gtg      2542
   Gln Arg Thr Val Ser Lys Glu Ser Leu Leu Val Ser His Pro Ser Val
   755                 760                 765                 770
   caa acc aaa tct cca gga ggg ctt tcc atc caa aat gag cca ctc cca      2590
   Gln Thr Lys Ser Pro Gly Gly Leu Ser Ile Gln Asn Glu Pro Leu Pro
                   775                 780                 785
   cca ggc cca aca gaa cca aca cca cca cca ttc aca ttt tcc atc ccc      2638
   Pro Gly Pro Thr Glu Pro Thr Pro Pro Pro Phe Thr Phe Ser Ile Pro
               790                 795                 800
   agc gga aac cag ttt cag ccc cgc tgt gtg tcc cca att cct gtc tct      2686
   Ser Gly Asn Gln Phe Gln Pro Arg Cys Val Ser Pro Ile Pro Val Ser
           805                 810                 815
   cct acc agc cgg att cag aac cca gtg gct ttc ctc agc tct gtt ctg      2734
   Pro Thr Ser Arg Ile Gln Asn Pro Val Ala Phe Leu Ser Ser Val Leu
       820                 825                 830
   cct tct ctc cct gcc atc cca ccc aca aat gcc atg ggg ctg cct aga      2782
   Pro Ser Leu Pro Ala Ile Pro Pro Thr Asn Ala Met Gly Leu Pro Arg
   835                 840                 845                 850
   agt gca cca tcc atg cca tcc cag gga tta gcg aag aaa aat aca aag      2830
   Ser Ala Pro Ser Met Pro Ser Gln Gly Leu Ala Lys Lys Asn Thr Lys
                   855                 860                 865
   tct cct caa cca gtg aat gat gat aac att cgt gaa act aag aac gca      2878
   Ser Pro Gln Pro Val Asn Asp Asp Asn Ile Arg Glu Thr Lys Asn Ala
               870                 875                 880
   gtg att cga gac ttg ggg aaa aaa ata act ttc agt gat gtc aga cca      2926
   Val Ile Arg Asp Leu Gly Lys Lys Ile Thr Phe Ser Asp Val Arg Pro
           885                 890                 895
   aac cag cag gag tac aaa att tca agc ttt gag cag agg ctg atg aat      2974
   Asn Gln Gln Glu Tyr Lys Ile Ser Ser Phe Glu Gln Arg Leu Met Asn
       900                 905                 910
   gaa ata gag ttt cgc ttg gaa cgt act cct gtt gat gaa tca gat gat      3022
   Glu Ile Glu Phe Arg Leu Glu Arg Thr Pro Val Asp Glu Ser Asp Asp
   915                 920                 925                 930
   gaa att caa cat gat gag atc ccc acg ggc aag tgt att gct ccc atc     3070
   Glu Ile Gln His Asp Glu Ile Pro Thr Gly Lys Cys Ile Ala Pro Ile
                   935                     940                 945
   ttt gac aag aga ctc aag cac ttc cgg gtc aca gaa ggc tct cca gtc     3118
   Phe Asp Lys Arg Leu Lys His Phe Arg Val Thr Glu Gly Ser Pro Val
               950                 955                     960
   aca ttc acc tgc aaa att gtt ggg ata cct gtt cca aag gtt tac tgg     3166
   Thr Phe Thr Cys Lys Ile Val Gly Ile Pro Val Pro Lys Val Tyr Trp
           965                 970                     975
   ttc aaa gat ggg aag cag att tct aag aga aat gag cac tgc aaa atg     3214
   Phe Lys Asp Gly Lys Gln Ile Ser Lys Arg Asn Glu His Cys Lys Met
       980                     985                 990
   agg cga gaa gga gat ggg  aca tgc tct ctg cac  att gaa tcc act      3259
   Arg Arg Glu Gly Asp Gly  Thr Cys Ser Leu His  Ile Glu Ser Thr
   995                 1000                 1005
   acc  agt gat gac gat ggc  aac tac acc atc atg  gca gcc aac ccc     3304
   Thr  Ser Asp Asp Asp Gly  Asn Tyr Thr Ile Met  Ala Ala Asn Pro
   1010                 1015                 1020
   cag  ggg aga atc agc tgt  tct ggc cac ttg atg  gta caa agt ttg     3349
   Gln  Gly Arg Ile Ser Cys  Ser Gly His Leu Met  Val Gln Ser Leu
   1025                 1030                 1035
   ccc  att cgc agt cgg cta  acc tct gct ggt cag  tct cac agg gga     3394
   Pro  Ile Arg Ser Arg Leu  Thr Ser Ala Gly Gln  Ser His Arg Gly
   1040                 1045                 1050
   aga  tcc cga gtg caa gaa  aga gac aaa gag ccc  cta cag gaa cgc     3439
   Arg  Ser Arg Val Gln Glu  Arg Asp Lys Glu Pro  Leu Gln Glu Arg
   1055                 1060                 1065
   ttt  ttc cga cca cat ttc  ctg cag gct cct ggg  gat atg gta gct     3484
   Phe  Phe Arg Pro His Phe  Leu Gln Ala Pro Gly  Asp Met Val Ala
   1070                 1075                 1080
   cat  gag ggg cgc ctc tgt  cgg ctg gac tgt aag  gtg agt ggt tta     3529
   His  Glu Gly Arg Leu Cys  Arg Leu Asp Cys Lys  Val Ser Gly Leu
   1085                 1090                 1095
   ccg  ccc ccg gag ctg aca  tgg cta ctc aat ggc  caa cct gtg cta     3574
   Pro  Pro Pro Glu Leu Thr  Trp Leu Leu Asn Gly  Gln Pro Val Leu
   1100                 1105                 1110
   cca  gat gcc tcc cac aag  atg ctg gtc agg gag  acc gga gtc cac     3619
   Pro  Asp Ala Ser His Lys  Met Leu Val Arg Glu  Thr Gly Val His
   1115                 1120                 1125
   tct  ctg ctc att gac cca  ctc act cag cgc gac  gca ggg acc tat     3664
   Ser  Leu Leu Ile Asp Pro  Leu Thr Gln Arg Asp  Ala Gly Thr Tyr
   1130                 1135                 1140
   aag  tgc atc gct acc aac  aaa acc ggg cag aat  tct ttt agt ctg     3709
   Lys  Cys Ile Ala Thr Asn  Lys Thr Gly Gln Asn  Ser Phe Ser Leu
   1145                 1150                 1155
   gag  ctc tct gta gta gcc  aaa gag gtg aag aaa  gca cct gtg atc     3754
   Glu  Leu Ser Val Val Ala  Lys Glu Val Lys Lys  Ala Pro Val Ile
   1160                 1165                 1170
   ctg  gag aaa cta cag aac  tgc ggt gtt ccc gaa  ggc cac ccc gtg      3799
   Leu  Glu Lys Leu Gln Asn  Cys Gly Val Pro Glu  Gly His Pro Val
   1175                 1180                 1185
   aga  ctg gag tgc cgc gtg  ata ggc atg ccc cca  cct gtg ttc tac      3844
   Arg  Leu Glu Cys Arg Val  Ile Gly Met Pro Pro  Pro Val Phe Tyr
   1190                 1195                 1200
   tgg  aag aaa gac aat gag  acc atc cct tgc acc  aga gag agg atc      3889
   Trp  Lys Lys Asp Asn Glu  Thr Ile Pro Cys Thr  Arg Glu Arg Ile
   1205                 1210                 1215
   agt  atg cac cag gac aca  aca ggg tat gcc tgc  ctt ctc att cag      3934
   Ser  Met His Gln Asp Thr  Thr Gly Tyr Ala Cys  Leu Leu Ile Gln
   1220                 1225                 1230
   cca  gcc aag aaa tca gac  gct gga tgg tac acg  ttg tca gcc aag      3979
   Pro  Ala Lys Lys Ser Asp  Ala Gly Trp Tyr Thr  Leu Ser Ala Lys
   1235                 1240                 1245
   aat  gaa gcc ggc atc gtg  tcg tgc act gcc agg  ctg gat ata tac      4024
   Asn  Glu Ala Gly Ile Val  Ser Cys Thr Ala Arg  Leu Asp Ile Tyr
   1250                 1255                 1260
   gct  cag tgg cac cat cag  atc cca ccg ccc atg  tct gtc cgg ccc      4069
   Ala  Gln Trp His His Gln  Ile Pro Pro Pro Met  Ser Val Arg Pro
   1265                 1270                 1275
   agt  ggc agt cgc tac gga  tct ctc acc agt aaa  gga ctt gac ata      4114
   Ser  Gly Ser Arg Tyr Gly  Ser Leu Thr Ser Lys  Gly Leu Asp Ile
   1280                 1285                 1290
   ttt  tct gcc ttt tcc tcc  atg gaa agc acg atg  gtg tat tca tgc      4159
   Phe  Ser Ala Phe Ser Ser  Met Glu Ser Thr Met  Val Tyr Ser Cys
   1295                 1300                 1305
   tct  tct cgg agt gta gtg  gag agt gat gaa ctt  taa gaatgtctag       4205
   Ser  Ser Arg Ser Val Val  Glu Ser Asp Glu Leu
   1310                 1315                 1320
   gtacctgctg tgtaagagag cggactgtgg agggggaatg agaacaagcc agacttggtg   4265
   gtttccaagc aaccgaagtt gagtaagttc ccacactgct ggacctgtgg caaagagtgc   4325
   tttgaataag tcagctaggg attcttgcag tctcagctga gggagaaagg tagggctgtg   4385
   ccttctaaag attccaacag agatgtgaga agataataca gatgaaccaa agatgtcaca   4445
   cagttgccat ccactgcttt gggaaaaaac tagcatgctc ccctgctccc tgttgtgtta   4505
   gggatgttta ggtcatacta ggagcaatta ggagccatat gcctgggctg agaagagcta   4565
   ggcagtagtg accttaggat atgactaact caccaaacaa tgccaaggag aaaggcggac   4625
   aggtcaccat cacctttcat cgattacatg tatagcagta gttttggtga attcaccaaa   4685
   tcagcttcca cttagcatta ggaggtcatg ccatacaact cacgtatgcg ggcaaacact   4745
   tttgatttgc atatcctggg tgtactgagc cacataataa ggtgtcaaaa tgtgcttgag   4805
   attccaaaaa cttgctgaaa cacttgatat tgcacagtgc acttattttg tggccaaagc   4865
   atggccttac cagcttgtct gcactatttt cttttgggtg gtgactgttt ttttctgact   4925
   ttgcatatcc ctagacacta gaactaaagg attggtaata aaccaatcag aaagaaatcc   4985
   tctgtggggt cactttaaaa actactagct tcaactacca cttacaaaat gtgcaagagt   5045
   catcatttgc ccataaaata cacctcatgg gctcctaccc ctttccccaa aagtctgaaa   5105
   gtgtaggagg aaggaagaga tacaaacaag gagaggaaat gatgggagag tgttgttttt   5165
   gtcacttgcc ccagcagagc aggggttttg gaaggagagg tctttaatag ctttgaaatg   5225
   ctttgagatc attggtcaaa ttgcattttc tatgtagaga atattctgct aggtggaaaa   5285
   gttgggagaa aggggaatat gcatctttat tctaatcacc aattcaaacc ctgcctctta   5345
   aggaattttt agacttaagt tcactgtgat atccctagta cctagaagag tacctagcac   5405
   aaagtagaca ttcaataaat atttgctggt tgaatgaatc ttctgttatt atgtctatta   5465
   taagataacc taatcttata ttctgcagga atatgccacc cacacaccag tccaaacact   5525
   gccctgggaa tgctcatcac agcacagttt gctctaggct gtgcagggaa atgaattgca   5585
   ggtgtctgtt ttcaattccc tgtgctggaa tccctggtgc tgtctgtgcc aagcacactc   5645
   agctggcatt gtatttgtgt gaacagacag taactgctta gaaaggcttg aaactgtctt   5705
   cacttcaagg caaggatttc cagcataaaa ataaattcat tcactggaga aaaaaaaaaa   5765
   aaaaa                                                               5770
<210> 92
   <211> 1320
   <212> PRT
   <213> Homo sapiens
<400> 92
   Met Gln Asp Asp Ser Ile Glu Ala Ser Thr Ser Ile Ser Gln Leu Leu
   1               5                   10                  15
   Arg Glu Ser Tyr Leu Ala Glu Thr Arg His Arg Gly Asn Asn Glu Arg
               20                  25                  30
   Ser Arg Ala Glu Pro Ser Ser Asn Pro Cys His Phe Gly Ser Pro Ser
           35                  40                  45
   Gly Ala Ala Glu Gly Gly Gly Gly Gln Asp Asp Leu Pro Asp Leu Ser
       50                  55                  60
   Ala Phe Leu Ser Gln Glu Glu Leu Asp Glu Ser Val Asn Leu Ala Arg
   65                  70                  75                  80
   Leu Ala Ile Asn Tyr Asp Pro Leu Glu Lys Ala Asp Glu Thr Gln Ala
                   85                  90                  95
   Arg Lys Arg Leu Ser Pro Asp Gln Met Lys His Ser Pro Asn Leu Ser
               100                 105                 110
   Phe Glu Pro Asn Phe Cys Gln Asp Asn Pro Arg Ser Pro Thr Ser Ser
           115                 120                 125
   Lys Glu Ser Pro Gln Glu Ala Lys Arg Pro Gln Tyr Cys Ser Glu Thr
       130                 135                 140
   Gln Ser Lys Lys Val Phe Leu Asn Lys Ala Ala Asp Phe Ile Glu Glu
   145                 150                 155                 160
   Leu Ser Ser Leu Phe Lys Ser His Ser Ser Lys Arg Ile Arg Pro Arg
                   165                 170                 175
   Ala Cys Lys Asn His Lys Ser Lys Leu Glu Ser Gln Asn Lys Val Met
               180                 185                 190
   Gln Glu Asn Ser Ser Ser Phe Ser Asp Leu Ser Glu Arg Arg Glu Arg
           195                 200                 205
   Ser Ser Val Pro Ile Pro Ile Pro Ala Asp Thr Arg Asp Asn Glu Val
       210                 215                 220
   Asn His Ala Leu Glu Gln Gln Glu Ala Lys Arg Arg Glu Ala Glu Gln
   225                 230                 235                 240
   Ala Ala Ser Glu Ala Ala Gly Gly Asp Thr Thr Pro Gly Ser Ser Pro
                   245                 250                 255
   Ser Ser Leu Tyr Tyr Glu Glu Pro Leu Gly Gln Pro Pro Arg Phe Thr
               260                 265                 270
   Gln Lys Leu Arg Ser Arg Glu Val Pro Glu Gly Thr Arg Val Gln Leu
           275                 280                 285
   Asp Cys Ile Val Val Gly Ile Pro Pro Pro Gln Val Arg Trp Tyr Cys
       290                 295                 300
   Glu Gly Lys Glu Leu Glu Asn Ser Pro Asp Ile His Ile Val Gln Ala
   305                 310                 315                 320
   Gly Asn Leu His Ser Leu Thr Ile Ala Glu Ala Phe Glu Glu Asp Thr
                   325                 330                 335
   Gly Arg Tyr Ser Cys Phe Ala Ser Asn Ile Tyr Gly Thr Asp Ser Thr
               340                 345                 350
   Ser Ala Glu Ile Tyr Ile Glu Gly Val Ser Ser Ser Asp Ser Glu Gly
   355 360 365
   Asp Pro Asn Lys Glu Glu Met Asn Arg Ile Gln Lys Pro Asn Glu Val
   370 375 380
   Ser Ser Pro Pro Thr Thr Ser Ala Val Ile Pro Pro Ala Val Pro Gln
   385 390 395 400
   Ala Gln His Leu Val Ala Gln Pro Arg Val Ala Thr Ile Gln Gln Cys
   405 410 415
   Gln Ser Pro Thr Asn Tyr Leu Gln Gly Leu Asp Gly Lys Pro Ile Ile
   420 425 430
   Ala Ala Pro Val Phe Thr Lys Met Leu Gln Asn Leu Ser Ala Ser Glu
   435 440 445
   Gly Gln Leu Val Val Phe Glu Cys Arg Val Lys Gly Ala Pro Ser Pro
   450 455 460
   Lys Val Glu Trp Tyr Arg Glu Gly Thr Leu Ile Glu Asp Ser Pro Asp
   465 470 475 480
   Phe Arg Ile Leu Gln Lys Lys Pro Arg Ser Met Ala Glu Pro Glu Glu
   485 490 495
   Ile Cys Thr Leu Val Ile Ala Glu Val Phe Ala Glu Asp Ser Gly Cys
   500 505 510
   Phe Thr Cys Thr Ala Ser Asn Lys Tyr Gly Thr Val Ser Ser Ile Ala
   515 520 525
   Gln Leu His Val Arg Gly Asn Glu Asp Leu Ser Asn Asn Gly Ser Leu
   530 535 540
   His Ser Ala Asn Ser Thr Thr Asn Leu Ala Ala Ile Glu Pro Gln Pro
   545 550 555 560
   Ser Pro Pro His Ser Glu Pro Pro Ser Val Glu Gln Pro Pro Lys Pro
   565 570 575
   Lys Leu Glu Gly Val Leu Val Asn His Asn Glu Pro Arg Ser Ser Ser
   580 585 590
   Arg Ile Gly Leu Arg Val His Phe Asn Leu Pro Glu Asp Asp Lys Gly
   595 600 605
   Ser Glu Ala Ser Ser Glu Ala Gly Val Val Thr Thr Arg Gln Thr Arg
   610 615 620
   Pro Asp Ser Phe Gln Glu Arg Phe Asn Gly Gln Ala Thr Lys Thr Pro
   625 630 635 640
   Glu Pro Ser Ser Pro Val Lys Glu Pro Pro Pro Val Leu Ala Lys Pro
   645 650 655
   Lys Leu Asp Ser Thr Gln Leu Gln Gln Leu His Asn Gln Val Leu Leu
   660 665 670
   Glu Gln His Gln Leu Gln Asn Pro Pro Pro Ser Ser Pro Lys Glu Phe
   675 680 685
   Pro Phe Ser Met Thr Val Leu Asn Ser Asn Ala Pro Pro Ala Val Thr
   690 695 700
   Thr Ser Ser Lys Gln Val Lys Ala Pro Ser Ser Gln Thr Phe Ser Leu
   705 710 715 720
   Ala Arg Pro Lys Tyr Phe Phe Pro Ser Thr Asn Thr Thr Ala Ala Thr
   725 730 735
   Val Ala Pro Ser Ser Ser Pro Val Phe Thr Leu Ser Ser Thr Pro Gln
   740 745 750
   Thr Ile Gln Arg Thr Val Ser Lys Glu Ser Leu Leu Val Ser His Pro
   755 760 765
   Ser Val Gln Thr Lys Ser Pro Gly Gly Leu Ser Ile Gln Asn Glu Pro
   770 775 780
   Leu Pro Pro Gly Pro Thr Glu Pro Thr Pro Pro Pro Phe Thr Phe Ser
   785 790 795 800
   Ile Pro Ser Gly Asn Gln Phe Gln Pro Arg Cys Val Ser Pro Ile Pro
   805 810 815
   Val Ser Pro Thr Ser Arg Ile Gln Asn Pro Val Ala Phe Leu Ser Ser
   820 825 830
   Val Leu Pro Ser Leu Pro Ala Ile Pro Pro Thr Asn Ala Met Gly Leu
   835 840 845
   Pro Arg Ser Ala Pro Ser Met Pro Ser Gln Gly Leu Ala Lys Lys Asn
       850                 855                 860
   Thr Lys Ser Pro Gln Pro Val Asn Asp Asp Asn Ile Arg Glu Thr Lys
   865                 870                 875                 880
   Asn Ala Val Ile Arg Asp Leu Gly Lys Lys Ile Thr Phe Ser Asp Val
                   885                 890                 895
   Arg Pro Asn Gln Gln Glu Tyr Lys Ile Ser Ser Phe Glu Gln Arg Leu
               900                 905                 910
   Met Asn Glu Ile Glu Phe Arg Leu Glu Arg Thr Pro Val Asp Glu Ser
           915                 920                 925
   Asp Asp Glu Ile Gln His Asp Glu Ile Pro Thr Gly Lys Cys Ile Ala
       930                 935                 940
   Pro Ile Phe Asp Lys Arg Leu Lys His Phe Arg Val Thr Glu Gly Ser
   945                 950                 955                 960
   Pro Val Thr Phe Thr Cys Lys Ile Val Gly Ile Pro Val Pro Lys Val
                   965                 970                 975
   Tyr Trp Phe Lys Asp Gly Lys Gln Ile Ser Lys Arg Asn Glu His Cys
               980                 985                 990
   Lys Met Arg Arg Glu Gly Asp Gly  Thr Cys Ser Leu His  Ile Glu Ser
           995                 1000                 1005
   Thr Thr  Ser Asp Asp Asp Gly  Asn Tyr Thr Ile Met  Ala Ala Asn
       1010                 1015                 1020
   Pro Gln  Gly Arg Ile Ser Cys  Ser Gly His Leu Met  Val Gln Ser
       1025                 1030                 1035
   Leu Pro  Ile Arg Ser Arg Leu  Thr Ser Ala Gly Gln  Ser His Arg
       1040                 1045                 1050
   Gly Arg  Ser Arg Val Gln Glu  Arg Asp Lys Glu Pro  Leu Gln Glu
       1055                 1060                 1065
   Arg Phe  Phe Arg Pro His Phe  Leu Gln Ala Pro Gly  Asp Met Val
       1070                 1075                 1080
   Ala His  Glu Gly Arg Leu Cys  Arg Leu Asp Cys Lys  Val Ser Gly
       1085                 1090                 1095
   Leu Pro  Pro Pro Glu Leu Thr  Trp Leu Leu Asn Gly  Gln Pro Val
       1100                 1105                 1110
   Leu Pro  Asp Ala Ser His Lys  Met Leu Val Arg Glu  Thr Gly Val
       1115                 1120                 1125
   His Ser  Leu Leu Ile Asp Pro  Leu Thr Gln Arg Asp  Ala Gly Thr
       1130                 1135                 1140
   Tyr Lys  Cys Ile Ala Thr Asn  Lys Thr Gly Gln Asn  Ser Phe Ser
       1145                 1150                 1155
   Leu Glu  Leu Ser Val Val Ala  Lys Glu Val Lys Lys  Ala Pro Val
       1160                 1165                 1170
   Ile Leu  Glu Lys Leu Gln Asn  Cys Gly Val Pro Glu  Gly His Pro
       1175                 1180                 1185
   Val Arg  Leu Glu Cys Arg Val  Ile Gly Met Pro Pro  Pro Val Phe
       1190                 1195                 1200
   Tyr Trp  Lys Lys Asp Asn Glu  Thr Ile Pro Cys Thr  Arg Glu Arg
       1205                 1210                 1215
   Ile Ser  Met His Gln Asp Thr  Thr Gly Tyr Ala Cys  Leu Leu Ile
       1220                 1225                 1230
   Gln Pro  Ala Lys Lys Ser Asp  Ala Gly Trp Tyr Thr  Leu Ser Ala
       1235                 1240                 1245
   Lys Asn  Glu Ala Gly Ile Val  Ser Cys Thr Ala Arg  Leu Asp Ile
       1250                 1255                 1260
   Tyr Ala  Gln Trp His His Gln  Ile Pro Pro Pro Met  Ser Val Arg
       1265                 1270                 1275
   Pro Ser  Gly Ser Arg Tyr Gly  Ser Leu Thr Ser Lys  Gly Leu Asp
       1280                 1285                 1290
   Ile Phe  Ser Ala Phe Ser Ser  Met Glu Ser Thr Met  Val Tyr Ser
       1295                 1300                 1305
   Cys Ser  Ser Arg Ser Val Val  Glu Ser Asp Glu Leu
       1310                 1315                 1320
<210> 93
   <211> 341
   <212> DNA
   <213> Homo sapiens
<400> 93
   atagggcgga gggaagctca tcagtggggc cacgagctga gtgcgtcctg tcactccact      60
   cccatgtccc ttgggaaggt ctgagactag ggccagaggc ggccctaaca gggctctccc     120
   tgagcttcgg ggaggtgagt tcccagagaa cggggctccg cgcgaggtca gactgggcag     180
   gagatgccgt ggaccccgcc cttcggggag gggcccggcg gatgcctcct ttgccggagc     240
   ttggaacaga ctcacggcca gcgaagtgag ttcaatggct gaggtgaggt accccgcagg     300
   ggacctcata acccaattca gactactctc ctccgcccat t                         341
<210> 94
   <211> 3038
   <212> DNA
   <213> Homo sapiens
<220>
   <221> CDS
   <222> (410)..(1876)
   <223>
<400> 94
   ggagtgccca ggatggagtg cagtggctat tcacaggttt aatcatagct cactaccgtc     60
   ttgaatccct gggctcaagt gatcctcctg cctcagcccc tgagtagctg gtactaaagt    120
   taccttccac gctgtatgtc cgtgagaaat acaatgaaga cggtctgaat aatgaactgg    180
   taatcttcga taatatgaaa cgaatttaag agaacatatc aaagagaagg ctctcaaaat    240
   aggctgctgc aaaagctggt atattgtccg gttgctctct cagaatctcg cgtgtcagcc    300
   cttcaagaag attcccaaat ccttgtggaa ttcggtagtg ggtgttggag aatggaatcg    360
   acatcttctt ggtattggtt ttcgctttgc ttcatacatc aataatgat atg gtg ctg    418
                                                         Met Val Leu
                                                         1
   cag aag gag cct gct ggg gca gtg ata tgg ggc ttc ggt aca cct gga      466
   Gln Lys Glu Pro Ala Gly Ala Val Ile Trp Gly Phe Gly Thr Pro Gly
       5                   10                  15
   gcc aca gtg acc gtg acc ctg cgc caa ggt cag gaa acc atc atg aag      514
   Ala Thr Val Thr Val Thr Leu Arg Gln Gly Gln Glu Thr Ile Met Lys
   20                  25                  30                  35
   aaa gtg acc agt gtg aaa gct cac tct gat acg tgg atg gtg gta ctg      562
   Lys Val Thr Ser Val Lys Ala His Ser Asp Thr Trp Met Val Val Leu
                   40                  45                  50
   gat cct atg aag cct gga gga cct ttc gaa gtg atg gca caa cag act      610
   Asp Pro Met Lys Pro Gly Gly Pro Phe Glu Val Met Ala Gln Gln Thr
               55                  60                  65
   ttg gag aaa ata aac ttc acc ctg aga gtt cat gac gtc ctg ttt gga      658
   Leu Glu Lys Ile Asn Phe Thr Leu Arg Val His Asp Val Leu Phe Gly
           70                  75                  80
   gat gtc tgg ctc tgt agt ggg cag agt aac atg cag atg act gtg tta      706
   Asp Val Trp Leu Cys Ser Gly Gln Ser Asn Met Gln Met Thr Val Leu
       85                  90                  95
   cag ata ttt aat gct aca agg gag ttg tct aac act gcg gca tat cag      754
   Gln Ile Phe Asn Ala Thr Arg Glu Leu Ser Asn Thr Ala Ala Tyr Gln
   100                 105                 110                 115
   tct gtc cgc atc ctc tct gtc tct ccc att caa gca gag cag gag ctg      802
   Ser Val Arg Ile Leu Ser Val Ser Pro Ile Gln Ala Glu Gln Glu Leu
                   120                 125                 130
   gag gac ctt gtt gcg gtt gac ttg cag tgg tct aag ccc acc tca gaa      850
   Glu Asp Leu Val Ala Val Asp Leu Gln Trp Ser Lys Pro Thr Ser Glu
               135                 140                 145
   aac tta ggc cat gga tat ttc aag tac atg tca gca gtg tgc tgg ctc      898
   Asn Leu Gly His Gly Tyr Phe Lys Tyr Met Ser Ala Val Cys Trp Leu
           150                 155                 160
   ttt gga cgt cac ctt tat gac act ctg cag tat ccc atc ggg ctg atc      946
   Phe Gly Arg His Leu Tyr Asp Thr Leu Gln Tyr Pro Ile Gly Leu Ile
       165                 170                 175
   gcc tcc agc tgg ggc ggg aca ccc att gaa gcc tgg tca tct gga cgg      994
   Ala Ser Ser Trp Gly Gly Thr Pro Ile Glu Ala Trp Ser Ser Gly Arg
   180                 185                 190                 195
   tca ctg aaa gcc tgt ggg gtc cct aaa caa ggg tcc att cca tac gat     1042
   Ser Leu Lys Ala Cys Gly Val Pro Lys Gln Gly Ser Ile Pro Tyr Asp
                   200                 205                 210
   tct gta act ggt ccc agt aag cac tct gtt ctc tgg aat gcc atg atc     1090
   Ser Val Thr Gly Pro Ser Lys His Ser Val Leu Trp Asn Ala Met Ile
               215                 220                 225
   cat cca ctg tgc aat atg act ctg aaa ggg gta gta tgg tac cag ggg     1138
   His Pro Leu Cys Asn Met Thr Leu Lys Gly Val Val Trp Tyr Gln Gly
           230                 235                 240
   gag tcc aat ata aat tat aac acg gat ctg tac aat tgc aca ttc cct     1186
   Glu Ser Asn Ile Asn Tyr Asn Thr Asp Leu Tyr Asn Cys Thr Phe Pro
       245                 250                 255
   gca ctc atc gaa gac tgg cgt gaa acc ttc cac cgt ggt tcc cag ggg     1234
   Ala Leu Ile Glu Asp Trp Arg Glu Thr Phe His Arg Gly Ser Gln Gly
   260                 265                 270                 275
   cag acg gag cgt ttc ttc cca ttt gga ctt gtc cag tta tct tca gat     1282
   Gln Thr Glu Arg Phe Phe Pro Phe Gly Leu Val Gln Leu Ser Ser Asp
                   280                 285                 290
   ttg tct aag aag agc tca gac gat gga ttt ccc cag atc cgt tgg cat     1330
   Leu Ser Lys Lys Ser Ser Asp Asp Gly Phe Pro Gln Ile Arg Trp His
               295                 300                 305
   caa aca gca gac ttc ggc tat gtc ccc aac cca aag atg ccc aat act     1378
   Gln Thr Ala Asp Phe Gly Tyr Val Pro Asn Pro Lys Met Pro Asn Thr
           310                 315                 320
   ttc atg gct gta gct atg gat ctc tgt gat aga gac tcg cct ttt ggc     1426
   Phe Met Ala Val Ala Met Asp Leu Cys Asp Arg Asp Ser Pro Phe Gly
       325                 330                 335
   agc atc cac cct cga gat aaa cag act gtg gct tat cgg ctg cat ttg      1474
   Ser Ile His Pro Arg Asp Lys Gln Thr Val Ala Tyr Arg Leu His Leu
   340                 345                 350                 355
   ggg gcc cgt gct ctg gct tat ggt gag aag aat ttg acc ttt gaa gga      1522
   Gly Ala Arg Ala Leu Ala Tyr Gly Glu Lys Asn Leu Thr Phe Glu Gly
                   360                 365                 370
   cca ctg cct gag aag ata gaa ctc ttg gct cac aag ggg ctg ctc aat      1570
   Pro Leu Pro Glu Lys Ile Glu Leu Leu Ala His Lys Gly Leu Leu Asn
               375                 380                 385
   ctc aca tat tac cag caa atc cag gtg cag aaa aag gac aac aag ata      1618
   Leu Thr Tyr Tyr Gln Gln Ile Gln Val Gln Lys Lys Asp Asn Lys Ile
           390                 395                 400
   ttt gag atc tcc tgt tgc agt gac cat cga tgc aag tgg ctt cca gct      1666
   Phe Glu Ile Ser Cys Cys Ser Asp His Arg Cys Lys Trp Leu Pro Ala
       405                 410                 415
   tct atg aac acc gtc tcc acc cag tcc ctg acc ctg gcg atc gat tct      1714
   Ser Met Asn Thr Val Ser Thr Gln Ser Leu Thr Leu Ala Ile Asp Ser
   420                 425                 430                 435
   tgt cat ggc act gtg gtt gct ctc cgc tat gct tgg acc acg tgg cct      1762
   Cys His Gly Thr Val Val Ala Leu Arg Tyr Ala Trp Thr Thr Trp Pro
                   440                 445                 450
   tgt gaa tat aag cag tgt ccc cta tac cac ccc agt agt gcc ctg cca      1810
   Cys Glu Tyr Lys Gln Cys Pro Leu Tyr His Pro Ser Ser Ala Leu Pro
               455                 460                 465
   gcc cct ccc ttc att gct ttc att aca gac cag ggt cct gga cat cag      1858
   Ala Pro Pro Phe Ile Ala Phe Ile Thr Asp Gln Gly Pro Gly His Gln
           470                 475                 480
   agc aat gtt gct aaa tga ctgtttcagt atgatcagaa cttagatata             1906
   Ser Asn Val Ala Lys
       485
   aggatgggtc cttcagattt tagcatttag gagtttcaat aataaccatt gcttttaaag   1966
   gaaattaata gaaagcctca ttgaatggct ttcagctagc acatggctgt ttctatattc   2026
   tgatgagccc aggcttatag gtaacttgaa atgcttgctt tttgttccct agttggtcta   2086
   agggtctgta ttggactaat tctgaactac agacaaattg gacctcaatg tcatttactt   2146
   ccctcatatt aatgggagtg aaatgtctaa tacttttgcc cctttttatc cagagttgtg   2206
   ggaatctcag gattggaaga gattttaaag gccacatagg ccagctagtg ttcatgtgtt   2266
   ctttataaaa tttctcccat ccaagtacta accaggcccg accctgctta gcttccgaga   2326
   tcagatgaga tcaggcgcgt tcagggtgat atggccgtag acgtctttac aaaattcctg   2386
   acaggtggtt actgaatctc tctatgaact ttccattcaa aactttccaa gtttttcctt   2446
   atgtggaacc gaaatctttc tttctcccgt gaaactttac tactatcaga taattgaaga   2506
   cagatctctt tgtattctct tcaagcccaa accaattctg ttccttcaat ctaaatagtg   2566
   gtaatatgaa tgtttaagaa atgaaataag aaacatgtgc aggcactttg gaaggtgcta 2626
   agtgactgcc ctaaggaatg aaaagcaagg gccaggtggg agtagcccag cgaaggcact 2686
   tgggctgcca ggaacaggag gcgtgggaaa ctctggctta ggaaaacatg aacacagggg 2746
   caacagaggc aaactgttgt tcgagttaaa tataaatctc aggctcttta aaggtaaaag 2806
   gtttaaggat aatccatttg gaagaagaaa agagtgaggc tgaaagtaaa gccacatgac 2866
   aagcatataa aaaaaaatgc agatgataca aatatgaaag aggccttcag tgtttgttta 2926
   ttaagaatct taatgcagtt tactgatgga ttaaaaacag ctaacattgt ctgaaaatta 2986
   tgttacctat aagaagttgg aaataaataa aagcataatc actagtgatg tg 3038
<210> 95
   <211> 488
   <212> PRT
   <213> Homo sapiens
<400> 95
   Met Val Leu Gln Lys Glu Pro Ala Gly Ala Val Ile Trp Gly Phe Gly
   1               5                   10                  15
   Thr Pro Gly Ala Thr Val Thr Val Thr Leu Arg Gln Gly Gln Glu Thr
               20                  25                  30
   Ile Met Lys Lys Val Thr Ser Val Lys Ala His Ser Asp Thr Trp Met
           35                  40                  45
   Val Val Leu Asp Pro Met Lys Pro Gly Gly Pro Phe Glu Val Met Ala
       50                  55                  60
   Gln Gln Thr Leu Glu Lys Ile Asn Phe Thr Leu Arg Val His Asp Val
   65                  70                  75                  80
   Leu Phe Gly Asp Val Trp Leu Cys Ser Gly Gln Ser Asn Met Gln Met
                   85                  90                  95
   Thr Val Leu Gln Ile Phe Asn Ala Thr Arg Glu Leu Ser Asn Thr Ala
               100                 105                 110
   Ala Tyr Gln Ser Val Arg Ile Leu Ser Val Ser Pro Ile Gln Ala Glu
           115                 120                 125
   Gln Glu Leu Glu Asp Leu Val Ala Val Asp Leu Gln Trp Ser Lys Pro
       130                 135                 140
   Thr Ser Glu Asn Leu Gly His Gly Tyr Phe Lys Tyr Met Ser Ala Val
   145                 150                 155                 160
   Cys Trp Leu Phe Gly Arg His Leu Tyr Asp Thr Leu Gln Tyr Pro Ile
   165 170 175
   Gly Leu Ile Ala Ser Ser Trp Gly Gly Thr Pro Ile Glu Ala Trp Ser
   180 185 190
   Ser Gly Arg Ser Leu Lys Ala Cys Gly Val Pro Lys Gln Gly Ser Ile
   195 200 205
   Pro Tyr Asp Ser Val Thr Gly Pro Ser Lys His Ser Val Leu Trp Asn
   210 215 220
   Ala Met Ile His Pro Leu Cys Asn Met Thr Leu Lys Gly Val Val Trp
   225 230 235 240
   Tyr Gln Gly Glu Ser Asn Ile Asn Tyr Asn Thr Asp Leu Tyr Asn Cys
   245 250 255
   Thr Phe Pro Ala Leu Ile Glu Asp Trp Arg Glu Thr Phe His Arg Gly
   260 265 270
   Ser Gln Gly Gln Thr Glu Arg Phe Phe Pro Phe Gly Leu Val Gln Leu
   275 280 285
   Ser Ser Asp Leu Ser Lys Lys Ser Ser Asp Asp Gly Phe Pro Gln Ile
   290 295 300
   Arg Trp His Gln Thr Ala Asp Phe Gly Tyr Val Pro Asn Pro Lys Met
   305 310 315 320
   Pro Asn Thr Phe Met Ala Val Ala Met Asp Leu Cys Asp Arg Asp Ser
   325 330 335
   Pro Phe Gly Ser Ile His Pro Arg Asp Lys Gln Thr Val Ala Tyr Arg
   340 345 350
   Leu His Leu Gly Ala Arg Ala Leu Ala Tyr Gly Glu Lys Asn Leu Thr
   355 360 365
   Phe Glu Gly Pro Leu Pro Glu Lys Ile Glu Leu Leu Ala His Lys Gly
   370 375 380
   Leu Leu Asn Leu Thr Tyr Tyr Gln Gln Ile Gln Val Gln Lys Lys Asp
   385 390 395 400
   Asn Lys Ile Phe Glu Ile Ser Cys Cys Ser Asp His Arg Cys Lys Trp
   405 410 415
   Leu Pro Ala Ser Met Asn Thr Val Ser Thr Gln Ser Leu Thr Leu Ala
   420 425 430
   Ile Asp Ser Cys His Gly Thr Val Val Ala Leu Arg Tyr Ala Trp Thr
   435 440 445
   Thr Trp Pro Cys Glu Tyr Lys Gln Cys Pro Leu Tyr His Pro Ser Ser
   450 455 460
   Ala Leu Pro Ala Pro Pro Phe Ile Ala Phe Ile Thr Asp Gln Gly Pro
   465 470 475 480
   Gly His Gln Ser Asn Val Ala Lys
   485
<210> 96
   <211> 20
   <212> DNA
   <213> Artificial
<220>
   <223> PCR primer
<400> 96
   aaaagcaggt tggtcactgg 20
<210> 97
   <211> 20
   <212> DNA
   <213> Artificial
<220>
   <223> PCR primer
<400> 97
   cgacttctgc ccattctctc 20
<210> 98
   <211> 20
   <212> DNA
   <213> Artificial
<220>
   <223> PCR primer
<400> 98
   gccaccagct gtcatcacta 20
<210> 99
   <211> 20
   <212> DNA
   <213> Artificial
<220>
   <223> PCR primer
<400> 99
   ccaacaccac aaggaggagt 20
<210> 100
   <211> 20
   <212> DNA
   <213> Artificial
<220>
   <223> PCR primer
<400> 100
   actgtcccgg agacaatgac 20
<210> 101
   <211> 20
   <212> DNA
   <213> Artificial
<220>
   <223> PCR primer
<400> 101
   tgctcctaaa gccacacctt 20
<210> 102
   <211> 20
   <212> DNA
   <213> Artificial
<220>
   <223> PCR primer
<400> 102
   ccagtctgct ctgcctatcc 20
<210> 103
   <211> 20
   <212> DNA
   <213> Artificial
<220>
   <223> PCR primer
<400> 103
   aacgtcagga actccacacc 20
<210> 104
   <211> 20
   <212> DNA
   <213> Artificial
<220>
   <223> PCR primer
<400> 104
   ccagtccatt ctgggtccta 20
<210> 105
   <211> 20
   <212> DNA
   <213> Artificial
<220>
   <223> PCR primer
<400> 105
   tggcatgctg ttgtatgctt 20
<210> 106
   <211> 20
   <212> DNA
   <213> Artificial
<220>
   <223> PCR primer
<400> 106
   ctgggagcaa acacatctga 20
<210> 107
   <211> 20
   <212> DNA
   <213> Artificial
<220>
   <223> PCR primer
<400> 107
   agttcatgag ctgcaacacg 20

## Description

### Technical Field

The present invention relates to a method of examining polycystic kidney disease and a disease marker.

### Background Art

Polycystic kidney disease is classified into autosomal dominant polycystic kidney disease (ADPKD) and autosomal recessive polycystic kidney disease (ARPKD). ADPKD is developed by about one in 4000 people in Japan. The assumed number of ADPKD patients is said to range from 20,000 to 50,000. ADPKD is causative disease of end-stage chronic renal failure that results in dialysis introduction, and it is the fourth most common disease following diabetic nephropathy, primary glomerular nephritis, and hypertensive nephrosclerosis.

This disease is an autosomal dominant disease due to a genetic mutation of PKD1 or PKD2 (JP Patent Publication (Kohyo) No. 2001-520502 A, JP Patent Publication (Kohyo) No. 2004-504038 A, and JP Patent Publication (Kokai) No. 2009-065988 A). Also, such an autosomal dominant polycystic kidney disease causes a decrease in GLIS3 gene expression level. Hence, it has been reported that the phenomenon has been used for diagnosis of the disease (JP Patent Publication (Kokai) No. 2006-288265 A).

Polycysts are regarded as constituting major kidney pathology. As forms of extrarenal pathology, cyst formation in the liver, pancreas, spleen, generative organ, arachnoid membrane, and the like, cerebral aneurysm and aortic aneurysm, valvular disease of the heart, diverticula of the colon, hernia, and the like have been confirmed. The typical age at onset is middle age, and the age span widely ranges from neonate to 80-year-old.

Although a causative gene of the autosomal dominant polycystic kidney disease has been specified, no effective therapeutic method has been established.

Nakamura et al. observed elevation of serum levels of metalloproteinase-1, tissue inhibitor of metalloproteinase-1 and type IV collagen, and plasma levels of metalloproteinase-9 in polycystic kidney disease (American Journal of Nephrology, vol. 20, no. 1, 1 January 2000, pages 32-36).

Kistler et al. identified a unique urinary biomarker profile in patients with autosomal dominant polycystic kidney disease (Kidney International, vol. 76, no. 1, 1 July 2009, pages 89-96).

Meijer et al. performed a cross-sectional analysis of the association of urinary biomarkers with disease severity in patients with autosomal dominant polycystic kidney disease (American Journal of Kidney Diseases, vol. 56, no. 5, 1 November 2010, pages 883-895).

### Summary of Invention

An object of the present invention is to provide a method of examining (or detecting or diagnosing) polycystic kidney disease or a complication that accompanies polycystic kidney disease through comparison of disease markers using samples from subjects, and disease markers of the disease.

Furthermore, described herein is a method of screening for a drug useful for preventing or treating polycystic kidney disease or a complication that accompanies polycystic kidney disease using the disease markers, and a drug or a medicine useful for treating the disease.
As used herein, the "polycystic kidney disease" includes autosomal dominant polycystic kidney disease (ADPKD) and autosomal recessive polycystic kidney disease (ARPKD), preferably ADPKD.
[1] Described herein is a method of examining whether or not a subject has polycystic kidney disease or a risk of developing the disease, comprising the following steps of:
   (a) measuring the expression levels of 1 or more genes selected from or all genes of the group consisting of NTNG1, POSTN, TNC, KALI, BST1, ACAT2, INSIG1, SCD, HSD3B1, KRT7, USP40, andSULT1E1 in a sample from a subject; and
   (b) determining that the subject has polycystic kidney disease or a risk of developing the disease when the expression levels of 1 or more genes selected from or all genes of the group consisting of the NTNG1, POSTN, TNC, KALI, BST1, HSD3B1, KRT7, USP40, and SULT1E1 are lower than those in a control sample, or, determining that the subject has polycystic kidney disease or a risk of developing the disease when the expression levels of ACAT2, INSIG1, or SCD or the three genes are higher than those in the control sample.
[2] Described herein is the method according to [1], wherein the sample is at least one type of sample selected from the group consisting of blood, serum, blood plasma, cell extracts, urine, lymph fluids, tissue fluids, ascites, spinal fluids, and other body fluids, or tissues and cells.
[3] Described herein is the method according to [1], wherein the sample is a vascular endothelial cell obtained via differentiation induction from an iPS cell formed from an isolated somatic cell of a subject, and the gene in step (a) is selected from the group consisting of NTNG1, POSTN, TNC, KALI, BST1, ACAT2, INSIG1, and SCD.
[4] Described herein is the method according to [1], wherein the sample is a vascular mural cell obtained via differentiation induction from an iPS cell formed from an isolated somatic cell of a subject, and the gene in step (a) is selected from the group consisting of HSD3B1, KRT7, USP40, and SULT1E1.
[5] Described herein is the method according to any of [1] to [4], wherein the step of measuring the expression level of the gene is a step of measuring the amount of mRNA, cRNA, or cDNA of the gene or the amount of a protein encoded by the gene in the sample.
[6] The invention provides a method of examining whether or not a subject has a complication of polycystic kidney disease or a risk of developing the complication, comprising the following steps of:
   (a) measuring the expression level of MMP1 gene and measuring the expression levels of 1 or more genes selected from or all genes of the group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE in a sample from the subject; and
   (b) determining that the subject has a complication of polycystic kidney disease or a risk of developing the complication when the expression level of MMP1 is higher than that of a control sample; and when the expression levels of 1 or more genes selected from or all genes of the group consisting of NTNG1, POSTN, TNC, KALI, BST1, HSD3B1, KRT7, USP40, and SULT1E1 are lower than those in a control sample, or when the expression levels of 1 or more genes selected from or all genes of the group consisting of ACAT2,INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE are higher than those in the control sample; wherein the complication of polycystic kidney disease is selected from the group consisting of: vascular lesions; valvular disease of heart; diverticula of colon; hernia; ductal lesions; and symptoms of cyst formation in the liver, pancreas, spleen, and generative organ.
[7] The invention provides the method according to [6], further comprising:
   (a2) measuring the expression levels of all genes of the group consisting of CD274, CTGF, MMP10, and NRCAM in the sample from the subject; and
   (b2) determining that the subject has the complication of polycystic kidney disease or the risk of developing the complication when the expression levels of the CD274, CTGF, MMP10, and NRCAM genes are higher than those in the control sample.
[8] The invention provides the method according to [6] or [7], wherein the complication is cerebral aneurysm.
[9] The invention provides the method according to any of [6] to [8], wherein the sample is selected from the group consisting of blood, serum, blood plasma, cell extracts, urine, lymph fluids, tissue fluids, ascites, spinal fluids, and other body fluids, or tissues and cells.
[10] The invention provides the method according to any of [6] to [8], wherein the sample is a vascular endothelial cell obtained via differentiation induction from an iPS cell formed from an isolated somatic cell of a subject with polycystic kidney disease.
[11] The invention provides the method according to [10] wherein the genes in step (a) further comprise 1 or more genes selected from or all genes of the group consisting of NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2.
[12] The invention provides the method according to any of [6] to [8], wherein the sample is a vascular mural cell obtained via differentiation induction from an iPS cell formed from an isolated somatic cell of a subject with polycystic kidney disease.
[13] The invention provides the method according to [12] wherein the gene in step (a) is 1 or more genes selected from or all genes of the group consisting of HSD3B1, KRT7, USP40, SULT1E1, TFPI2, HMGA2, KRTAP4-7, KRTAP4-8, KRTAP4-9, MYPN, RPPH1, and SIAE.
[14] The invention provides the method according to any of [6] to [13], wherein the step of measuring the expression level of the gene is a step of measuring the amount of the mRNA, cRNA, or cDNA of the gene or the amount of the protein encoded by the gene.
[15] Described herein is a disease marker for examining polycystic kidney disease or a complication of polycystic kidney disease, comprising:
   a polynucleotide(s) and/or a polynucleotide(s) complementary thereto, which has/have at least 15 continuous nucleotides in the nucleotide sequence(s) of 1 or more genes selected from or all genes of the group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE; and/or an antibody (or antibodies) or a fragment(s) thereof, which recognizes a protein(s) encoded by 1 or more genes selected from or all genes of the group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE.
[16] Described herein is the disease marker according to [15], wherein the complication is cerebral aneurysm.
[17] Described herein is the disease marker according to [16], comprising:
   a polynucleotide(s) and/or a polynucleotide(s) complementary thereto, which has/have at least 15 continuous nucleotides in the nucleotide sequence(s) of 1 or more genes selected from or all genes of the group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE; and/or, an antibody (or antibodies) or a fragment(s) thereof, which recognizes a protein(s) encoded by 1 or more genes selected from or all genes of the group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE.
[18] Described herein is a kit for examining polycystic kidney disease or a complication of polycystic kidney disease, comprising:
   a polynucleotide(s) and/or a polynucleotide(s) complementary thereto, which has/have at least 15 continuous nucleotides in the nucleotide sequence(s) of 1 or more genes selected from or all genes of the group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE; and/or
   an antibody (or antibodies) or a fragment(s) thereof, which recognizes a protein(s) encoded by 1 or more genes selected from or all genes of the group consisting of
      NTNG1, POSTN, TNC, KALI, BST1, ACAT2, INSIG1, SCD, HSD3B1, KRT7, USP40, SULT1E1, BMP6, CD274, CTGF, E2F7, EDN1,FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE.
[19] Described herein is the kit according to [18], wherein the complication is cerebral aneurysm.
[20] Described herein is the kit according to [19], comprising:
   a polynucleotide(s) and/or a polynucleotide(s) complementary thereto, which has/have at least 15 continuous nucleotides in the nucleotide sequence(s) of 1 or more genes selected from or all genes of the group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE; and/or
   an antibody (or antibodies) or a fragment(s) thereof, which recognizes a protein(s) encoded by 1 or more genes selected from or all genes of the group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE.
[21] Described herein is a method of screening for a therapeutic agent or a preventive agent for polycystic kidney disease, comprising the following steps of:
   (a) bringing each of candidate substances into contact with a vascular endothelial cell obtained via differentiation induction from an iPS cell formed from an isolated somatic cell of a subject with polycystic kidney disease;
   (b) measuring the expression levels or the transcriptional activity of 1 or more genes selected from or all genes of the group consisting of NTNG1, POSTN, TNC, KALI, BST1, ACAT2, INSIG1, and SCD; and
   (c) selecting a candidate substance as a therapeutic agent or a preventive agent for polycystic kidney disease, when the expression levels or the transcriptional activity of 1 or more genes selected from or all genes of the group consisting of NTNG1, POSTN, TNC, KALI, and BST1 exhibit an increase compared with a case in which the candidate substance is not brought into contact, or,
   when the expression levels or the transcriptional activity of 1 or more genes selected from or all genes of the group consisting of ACAT2, INSIG1, and SCD exhibit a decrease compared with a case in which the candidate substance is not brought into contact.
[22] Described herein is a method of screening for a therapeutic agent or a preventive agent for polycystic kidney disease, comprising the following steps of:
   (a) bringing each of candidate substances into contact with a vascular mural cell obtained via differentiation induction from an iPS cell formed from an isolated somatic cell of a subject with polycystic kidney disease;
   (b) measuring the expression levels or the transcriptional activity of 1 or more genes selected from or all genes of the group consisting of HSD3B1, KRT7, USP40, and SULT1E1; and
   (c) selecting a candidate substance as a therapeutic agent or a preventive agent for polycystic kidney disease, when the expression levels or the transcriptional activity exhibit an increase compared with a case in which the candidate substance is not brought into contact.
[23] Described herein is the screening method according to [21] or [22], wherein the step of measuring the expression level of the gene is a step of measuring the amount of mRNA, cRNA, or cDNA of the gene.
[24] Described herein is a method of screening for a therapeutic agent or a preventive agent for a complication of polycystic kidney disease, comprising the following steps of:
   (a) bringing each of candidate substances into contact with a vascular endothelial cell obtained via differentiation induction from an iPS cell formed from an isolated somatic cell of a subject with a complication of polycystic kidney disease;
   (b) measuring the expression levels or the transcriptional activity of 1 or more genes selected from or all genes of the group consisting of NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2; and
   (c) selecting a candidate substance as a therapeutic agent or a preventive agent for a complication of polycystic kidney disease, when the expression levels or the transcriptional activity of 1 or more genes selected from or all genes of the group consisting of NTNG1, POSTN, TNC, KALI, and BST1 exhibit an increase compared with a case in which the candidate substance is not brought into contact, or
   when the expression levels or the transcriptional activity of 1 or more genes selected from or all genes of the group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 exhibit a decrease compared with a case in which the candidate substance is not brought into contact.
[25] Described herein is a method of screening for a therapeutic agent or a preventive agent for a complication of polycystic kidney disease, comprising the following steps of:
   (a) bringing each of candidate substances into contact with a vascular mural cell obtained via differentiation induction from an iPS cell formed from an isolated somatic cell of a patient with a complication of polycystic kidney disease;
   (b) measuring the expression levels or the transcriptional activity of 1 or more genes selected from or all genes of the group consisting of HSD3B1, KRT7, USP40, SULT1E1, MMP1, TFPI2, HMGA2, KRTAP4-7, KRTAP4-8, KRTAP4-9, MYPN, RPPH1, and SIAE; and
   (c) selecting a candidate substance as a therapeutic agent or a preventive agent for a complication of polycystic kidney disease, when the expression levels or the transcriptional activity of 1 or more genes selected from or all genes of the group consisting of HSD3B1, KRT7, USP40, and SULT1E1 exhibit an increase compared with a case in which the candidate substance is not brought into contact, or,
   when the expression levels or the transcriptional activity of 1 or more genes selected from or all genes of the group consisting of MMP1, TFPI2, HMGA2, KRTAP4-7, KRTAP4-8, KRTAP4-9, MYPN, RPPH1, and SIAE exhibit a decrease compared with a case in which the candidate substance is not brought into contact.
[26] Described herein is the screening method according to [24] and [25], wherein the complication is cerebral aneurysm.
[27] Described herein is the screening method according to [24] and [25], wherein the step of measuring the expression level of the gene is a step of measuring the amount of mRNA, cRNA, or cDNA of the gene.
[28] Described herein is a composition for use in treating or preventing a complication of polycystic kidney disease, comprising an antisense oligonucleotide, or siRNA or shRNA against a MMP1 gene and one or more antisense oligonucleotide, or siRNA or shRNA against nucleic acids selected from the group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE genes or transcription products thereof ; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.
[29] Described herein is the composition for use according to [28], wherein the complication is cerebral aneurysm.
[30] Described herein is the composition for use according to [29], comprising an antisense oligonucleotide, or siRNA or shRNA against nucleic acids selected from the group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE genes or transcription products thereof.
[31] Described herein is a vascular endothelial cell obtained via differentiation induction from an iPS cell formed from an isolated somatic cell of a subject suffered from polycystic kidney disease and having cerebral aneurysm as a complication, wherein the expression levels of NTNG1, POSTN, TNC, KALI, and BST1 are lower and the expression levels of ACAT2, INSIG1, SCD, BMP6, CD274, CTGF, E2F7, EDN1, FAM43A, FRMD3,
   MMP10, MYEOV, NR2F1, NRCAM, PCSK1, PLXNA2, SLC30A3, SNAI1, SPOCD1, MMP1, and TFPI2 are higher than those in a vascular endothelial cell obtained via differentiation induction from an iPS cell formed from an isolated somatic cell of a healthy subject.
[32] Described herein is a vascular endothelial cell obtained via differentiation induction from an iPS cell formed from an isolated somatic cell of a subject suffered from polycystic kidney disease and having cerebral aneurysm as a complication, wherein the expression levels of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 are high.
[33] Described herein is a vascular mural cell obtained via differentiation induction from an iPS cell formed from an isolated somatic cell of a subject suffered from polycystic kidney disease and having cerebral aneurysm as a complication, wherein the expression levels of HSD3B1, KRT7, USP40, and SULT1E1 are lower and the expression levels of MMP1, TFPI2, HMGA2, KRTAP4-7, KRTAP4-8, KRTAP4-9, MYPN, RPPH1, and SIAE are higher than those in a vascular mural cell obtained via differentiation induction from an iPS cell formed from an isolated somatic cell of a healthy subject.
[34] Described herein is a vascular mural cell obtained via differentiation induction from an iPS cell formed from an isolated somatic cell of a subject suffered from polycystic kidney disease and having cerebral aneurysm as a complication, wherein the expression levels of MMP1, TFPI2, HMGA2, KRTAP4-7, KRTAP4-8, KRTAP4-9, MYPN, RPPH1, and SIAE are high.
According to the method described herein, examination of polycystic kidney disease or a complication that accompanies polycystic kidney disease (hereinafter, simply referred to as "a complication of polycystic kidney disease" or "a polycystic kidney disease complication"), and preparation of a disease marker for the disease, screening for a drug useful for preventing or treating the disease, and preparation of a drug useful for treating the diseases can be carried out.

### Brief Description of Drawings

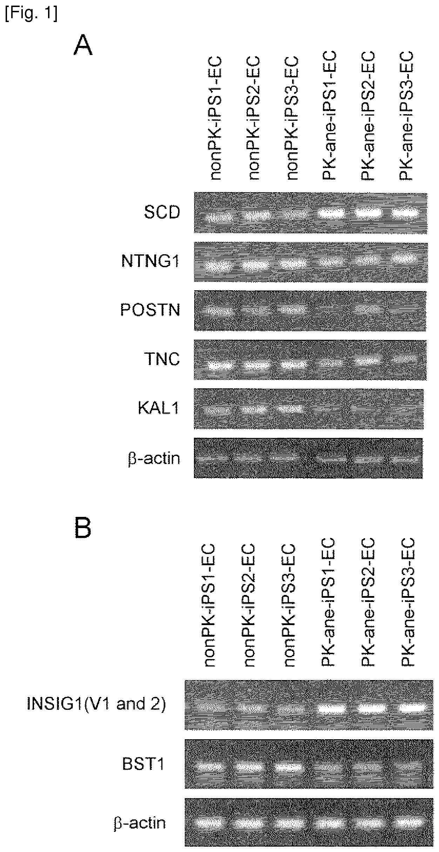

Figure 1 shows the results of RT-PCR for the indicated genes (Fig. 1A: SCD, NTNG1, POSTN, TNC, KALI; Fig. 1B: INSIG1 (VI and 2), BST1) in vascular endothelial cells obtained via differentiation induction from iPS cells formed from fibroblast cells of Japanese persons (nonPK) not developing autosomal dominant polycystic kidney disease (PK) and of autosomal dominant polycystic kidney disease (PK) patients with cerebral aneurysm.

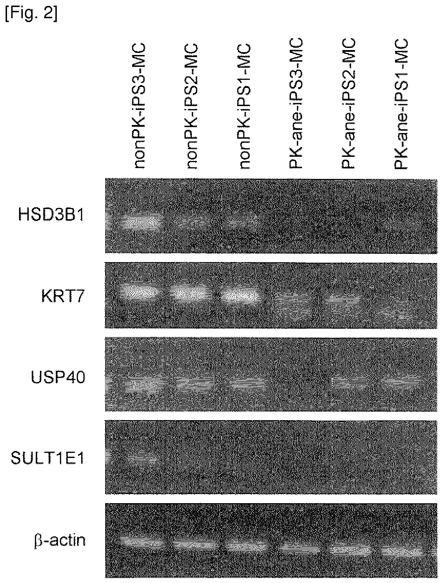

Figure 2 shows the results of RT-PCR for the indicated genes (i.e., HSD3B1, KRT7, USP40, SULT1E1) in vascular mural cells obtained via differentiation induction from iPS cells formed from fibroblast cells of nonPK persons and PK patients.

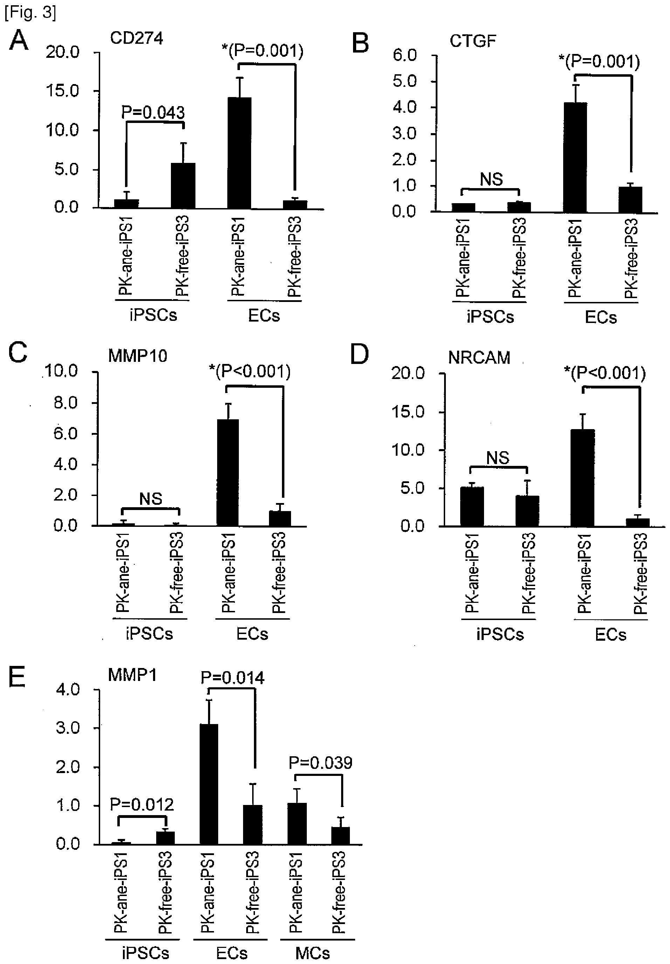

Figure 3 shows the results of quantitative PCR (i.e., expression levels) for the indicated genes (i.e., A, CD274; B, CTGF; C, MMP10; D, NRCAM; E, MMP1) in iPS cells or iPS cell-derived vascular endothelial cells or iPS cell-derived vascular mural cells, which cells were directly or indirectly prepared from fibroblast cells of autosomal dominant polycystic kidney disease patients with cerebral aneurysm (PK-ane-iPS1) or without cerebral aneurysm (PK-free-iPS3).

### Description of Embodiments

### <Polynucleotides as disease markers>

The present invention is based on the following finding. The presence or the absence or the degree of an increase (rise) and/or a decrease (fall) in the expression level of at least one of genes listed in Table 1 compared with control samples is measured (qualitative and/or quantitative measurement), so that the onset of polycystic kidney disease or a complication of polycystic kidney disease or the risk of developing the disease can be specifically detected and thus the disease can be examined precisely.

Specifically, described herein is a disease marker useful as a tool with which the presence or the absence of the onset of or the degree of polycystic kidney disease or a complication of polycystic kidney disease can be examined through measurement of the presence or the absence of an increase and/or a decrease in or the degree of the expression of the above gene in a subject.

As used herein, the term of "polycystic kidney disease" means both of autosomal dominant polycystic kidney disease and autosomal recessive polycystic kidney disease unless otherwise mentioned.

As used herein, the term "subject" refers to a mammalian animal, which includes, but is not limited to, primate, rodent, ungulate or the like, preferably human. The subject may be used exchangeably with another term "patient."

In the present invention, examples of a complication of polycystic kidney disease include vascular lesions such as aortic aneurysm, cerebral aneurysm, or subarachnoid hemorrhage, valvular disease of heart, diverticula of colon, hernia, and ductal lesions such as choledochal dilatation, as well as symptoms such as cyst formation in the liver, pancreas, spleen, and generative organs. An example of such a complication that should be particularly preferably detected is cerebral aneurysm.

### [Table 1]

**Table 1**

| Gene Name | GenBank Accession No. | SEQ ID NO: | |
|---|---|---|---|
| | | Gene | Protein |
| NTNG1 | NM_001113226 | 1 | 2 |
| POSTN | NM_001135934 | 3 | 4 |
| TNC | NM_002160 | 5 | 6 |
| KAL1 | NM_000216 | 7 | 8 |
| BST1 | NM_004334 | 9 | 10 |
| INSIG1 | NM_005542 | 11 | 12 |
| SCD | NM_005063 | 13 | 14 |
| HSD3B1 | NM_000862 | 15 | 16 |
| KRT7 | NM_005556 | 17 | 18 |
| USP40 | NM_018218 | 19 | 20 |
| SULT1E1 | NM_005420 | 21 | 22 |
| ACAT2 | NM_005891 | 45 | 46 |
| BMP6 | NM_001718 | 47 | 48 |
| CD274 | NM_014143 | 49 | 50 |
| CTGF | NM_001901 | 51 | 52 |
| E2F7 | NM_203394 | 53 | 54 |
| EDN1 | NM_001955 | 55 | 56 |
| FAM43A | NM_153690 | 57 | 58 |
| FRMD3 | NM_174938 | 59 | 60 |
| MMP10 | NM_002425 | 61 | 62 |
| MYEOV | NM_138768 | 63 | 64 |
| NR2F1 | NM_005654 | 65 | 66 |
| NRCAM | NM_001037132 | 67 | 68 |
| PCSK1 | NM_000439 | 69 | 70 |
| PLXNA2 | NM_025179 | 71 | 72 |
| SLC30A3 | NM_003459 | 73 | 74 |
| SNAI1 | NM_005985 | 75 | 76 |
| SPOCD1 | NM_144569 | 77 | 78 |
| MMP1 | NM_001145938 | 79 | 80 |
| TFPI2 | NM_006528 | 81 | 82 |
| HMGA2 | NM_001145938 | 83 | 84 |
| KRTAP4-7 | NM_033061 | 85 | 86 |
| KRTAP4-8 | NM_031960 | 87 | 88 |
| KRTAP4-9 | NM_001146041 | 89 | 90 |
| MYPN | NM_032578 | 91 | 92 |
| RPPH1 | NR_002312 | 93 | - |
| SIAE | NM_001199922 | 94 | 95 |

The disease marker is characterized by comprising a polynucleotide and/or a polynucleotide complementary thereto, which has at least 15 continuous nucleotides in an open reading frame (ORF) sequence in the nucleotide sequences of the genes listed in Table 1. Persons skilled in the art can easily understand that the following sequences are contained as transcriptional mutants or splicing mutants in the genes listed in Table 1 and examples are not particularly limited thereto. Specifically, in Table 1, NTNG1 comprises the sequence according to accession No. NM_001113228 or NM_014917, POSTN comprises the sequence according to accession No. NM_001135935, NM_001135936, or NM_006475, INSIG1 comprises the sequence according to accession No. NM_198336 or NM_198337, EDN1 comprises the sequence according to accession No. NM_001168319, NRCAM comprises the sequence according to accession No. NM_001193582, NM_001193583, NM_001193584, or NM_005010, PCSK1 comprises the sequence according to accession No. NM_001177875 or NM_001177876, MMP1 comprises the sequence according to accession No. NM_002421, HMGA2 comprises the sequence according to accession No. NM_002421, and SIAE comprises the sequence according to accession No. NM_170601.

The term "polynucleotide complementary thereto (complementary strand or reverse strand) as used herein refers to a polynucleotide that is in a basically complementary relationship (on the basis of a base pair relationship such as A:T and G:C) with: an ORF sequence of the nucleotide sequence shown in SEQ ID NO: 1, 3, 5, 7, 9, 11, 13, 15, 17, 19, 21, 45, 47, 49, 51, 53, 55, 57, 59, 61, 63, 65, 67, 69, 71, 73, 75, 77, 79, 81, 83, 85, 87, 89, 91, 93, or 94; or a sequence (partial sequence) (for the sake of convenience, these ORF sequence and partial sequence are also referred to as "forward strands") having an at least continuous 15-nucleotide-long nucleotide sequence in the ORF sequence. However, such a complementary strand may not only be a complete complementary sequence to the nucleotide sequence of a target forward strand, but also be a sequence having a complementary relationship with the other to a degree such that it can hybridize to a target forward strand under stringent conditions. In addition, stringent conditions can be determined based on the melting temperature (Tm) of a nucleic acid to which a complex or a probe is bound, as taught by Berger and Kimmel (1987, Guide to Molecular Cloning Techniques Methods in Enzymology, Vol. 152, Academic Press, San Diego CA). Examples of washing conditions after hybridization generally include conditions of about 1 x SSC, 0.1% SDS, and 37 degrees C. A complementary strand to be used herein is preferably capable of maintaining its state of hybridizing to a target forward strand even when washed under such conditions. Examples of more stringent hybridization conditions include, but are not particularly limited to, conditions that allow a forward strand and a complementary strand to be able to maintain the hybridization state even when they are washed under washing conditions of about 0.5 x SSC, 0.1% SDS, and 42 degrees C or more stringent washing conditions of 0.1 x SSC, 0.1% SDS, and 65 degrees C. Specific examples of such a complementary strand include a strand comprising a nucleotide sequence that is in a completely complementary relationship with the nucleotide sequence of a target forward strand, and a strand comprising a nucleotide sequence having at least 90%, and preferably at least 95%, 96%, 97%, 98%, or 99% sequence identity with the strand.

Also, examples of a polynucleotide on the forward strand side include not only an ORF sequence of the nucleotide sequence according to SEQ ID NO: 1, 3, 5, 7, 9, 11, 13, 15, 17, 19, 21,45, 47, 49, 51, 53, 55, 57, 59, 61, 63, 65, 67, 69, 71, 73, 75, 77, 79, 81, 83, 85, 87, 89, 91, 93, or 94, or a partial sequence thereof, but also a strand comprising a nucleotide sequence that is in a further complementary relationship with the nucleotide sequence of the above forward strand.

The above forward-strand polynucleotide and complementary-strand (reverse-strand) polynucleotide may be separately used as disease markers in a single-strand form or a double-strand form.

As described above, the disease marker for polycystic kidney disease or a complication of polycystic kidney disease may be a polynucleotide comprising a partial sequence of the above ORF sequence or a sequence complementary thereto as long as it selectively (or specifically) recognizes a gene(s) listed in Table 1 or a polynucleotide from the gene. In this case, examples of such a polynucleotide include polynucleotides having a length of at least 15, at least 18, at least 19, at least 20, at least 30, at least 40, at least50, at least 60, at least 70, or at least 100 continuous nucleotides, which is arbitrarily selected from the nucleotide sequence of the above ORF sequence or a sequence complementary thereto.

In addition, as used herein, the term "selectively (or specifically) recognizes" means, but is not limited to: that when a Northern blot method or a Southern blot method is employed for example, genes listed in Table 1 or polynucleotides from the genes can be specifically detected; or that when an RT-PCR method is employed, the genes listed in Table 1 or polynucleotides formed from the genes are specifically amplified and generated, as long as persons skilled in the art can determine that detected products in the Northern blot method or the Southern blot method or products in the above RT-PCR method are derived from the genes listed in Table 1 or polynucleotides originating therefrom.

Such a disease marker can be designed based on the nucleotide sequence of the gene shown in SEQ ID NO: 1, 3, 5, 7, 9, 11, 13, 15, 17, 19, 21, 45, 47, 49, 51, 53, 55, 57, 59, 61, 63, 65, 67, 69, 71, 73, 75, 77, 79, 81, 83, 85, 87, 89, 91, 93, or 94, using primer 3 (http//www.genome.wi.mit.edu/cgi-bin/primer/primer3.cgi) or vector NTI (Infomax), for example. Specifically, candidate sequences of primers or probes, which are obtained by subjecting the nucleotide sequences of the genes listed in Table 1 to primer 3 or vector NTI software, or sequences containing at least the sequence as a portion can be used as primers or probes.

A disease marker may have a length of at least 15 continuous nucleotides, at least 18 continuous nucleotides, at least 19 continuous nucleotides, at least 20 continuous nucleotides, at least 30 continuous nucleotides, at least 40 continuous nucleotides, or at least 50 continuous nucleotides, at least 60 continuous nucleotides, at least 70 continuous nucleotides, or at least 100 continuous nucleotides, as described above. Specifically, the length can be appropriately selected and determined depending on the applications of the marker.

The disease marker can be used as a primer for specifically recognizing and amplifying RNA resulting from the expression and/or transcription of the gene or a polynucleotide (e.g., cDNA) from the RNA, or can be used as a probe for specifically detecting the RNA or a polynucleotide (e.g., cDNA) from the RNA.

When the above disease marker is used as a primer for examining or detecting polycystic kidney disease or a complication of polycystic kidney disease, an example thereof is a disease marker with a nucleotide length generally ranging from 15 bp to 100 bp, preferably ranging from 15 bp to 50 bp, or more preferably ranging from 20 bp to 35 bp. Also, when the disease marker is used as a detection probe, an example thereof is a disease marker with a nucleotide length ranging from generally 15 bp to the full-length sequence, preferably ranging from 15 bp to 1 kb, or more preferably ranging from 50 bp to 500 bp.

When the disease marker is used as a primer, preferable primer sets are exemplified in Table 2.

### [Table 2]

**Table 2**

| Primer Name ^{*)} | Sequence | SEQ ID NO: |
|---|---|---|
| NTNG1-primer-F | CCCCTGAGCTGATGTTTGAT | 23 |
| NTNG1-primer-R | GACGCCCAGATTCAAAGGTA | 24 |
| POSTN-primer-F | GCAGACACACCTGTTGGAAA | 25 |
| POSTN-primer-R | GTCACGGGGATTTCTTTGAA | 26 |
| TNC-primer-F | GTCACCGTGTCAACCTGATG | 27 |
| TNC-primer-R | GCCTGCCTTCAAGATTTCTG | 28 |
| KALI-primer-F | GGCTGAGGTCACTACGGAAA | 29 |
| KALI-primer-R | GGTCTCAGATTGGGCACTGT | 30 |
| BST1-primer-F | GTGCTGCCCTCAGACTATGACC | 31 |
| BST1-primer-R | CTGCCATACAGAACATCGCTCAG | 32 |
| INSIG1-primer-F | GGTGGACATTTGATCGTTCC | 33 |
| INSIG1-primer-R | TGACGCCTCCTGAGAAAAAT | 34 |
| SCD-primer-F | TGGTTTCACTTGGAGCTGTG | 35 |
| SCD-primer-R | GCCATGCAATCAATGAAGAA | 36 |
| HSD3B1-primer-F | AGGACGTCTCGGTCATCATC | 37 |
| HSD3B1-primer-R | CTGGCACACTAGCTTGGACA | 38 |
| KRT7 -primer-F | CAGGAACTCATGAGCGTGAA | 39 |
| KRT7 -primer-R | GATATTCACGGCTCCCACTC | 40 |
| USP40-primer-F | GGGGCACCGTATTACTTGAA | 41 |
| USP40-primer-R | GGCTTCTTGGCTTTTCCTTC | 42 |
| SULT1E1-primer-F | CAGAAATTGTCGCCCTTCAT | 43 |
| SULT1E1-primer-R | GATTCCTTCATTTGCTGCTCA | 44 |
| ACAT2-primer-F | AAAAGCAGGTTGGTCACTGG | 96 |
| ACAT2-primer-R | CGACTTCTGCCCATTCTCTC | 97 |
| CD274-primer-F | GCCACCAGCTGTCATCACTA | 98 |
| CD274-primer-R | CCAACACCACAAGGAGGAGT | 99 |
| CTGF -primer-F | ACTGTCCCGGAGACAATGAC | 100 |
| CTGF -primer-R | TGCTCCTAAAGCCACACCTT | 101 |
| MMP10-primer-F | CCAGTCTGCTCTGCCTATCC | 102 |
| MMP10-primer-R | AACGTCAGGAACTCCACACC | 103 |
| NRCAM -primer-F | CCAGTCCATTCTGGGTCCTA | 104 |
| NRCAM -primer-R | TGGCATGCTGTTGTATGCTT | 105 |
| MMP1-primer-F | CTGGGAGCAAACACATCTGA | 106 |
| MMP1-primer-R | AGTTCATGAGCTGCAACACG | 107 |

| | | |
|---|---|---|
| ^{*)} F indicates a forward primer. R indicates a reverse primer. | | |

When the disease marker is used as a probe, the probe may be labeled with a radioisotope (e.g., ³²P or ³³P), a fluorescent substance (fluorescamine, rhodamine,Texas Red, Dansyl, or a derivative thereof), a chemiluminescence substance, an enzyme, or the like. Such a labeled disease marker can be appropriately used as a probe (or a detection marker).
The disease marker can be used as a primer or a probe according to conventional methods including known methods for specifically recognizing or detecting a specific gene, mRNA, and cDNA, such as a Northern blot method, a Southern blot method, RT-PCR, and in situ hybridization.

In the present invention, examination of a complication of polycystic kidney disease (which may also be referred to as "detection" or "diagnosis") can be performed by measuring the presence or the absence of an increase or a decrease in or the level (the degree of increase or decrease in expression) of the expression of a gene(s) (listed in Table 1) in a sample obtained from at least 1 type of sample selected from the group consisting of subject-derived blood, serum, blood plasma, cell extracts, urine, lymph fluids, tissue fluids, ascites, spinal fluids, and other body fluids, or tissues and cells (e.g., renal tissues or renal cells, and somatic cells obtained via differentiation induction from iPS cells), and evaluating the thus obtained results.

In an aspect of the present invention, the present invention provides a method of examining whether or not a subject has a complication of polycystic kidney disease or a risk of developing the complication, comprising the following steps of:
(a) measuring the expression level of MMP1 gene and measuring the expression levels of 1 or more genes selected from or all genes of a group consisting of NTNG1, POSTN, TNC, KALI, BST1, ACAT2, INSIG1, SCD , HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2,SLC30A3, SNAI1, SPOCD1, TFPI2, HMGA2, KRTAP4-7, KRTAP4-8, KRTAP4-9, MYPN, RPPH1, and SIAE in a sample from a subject; and
(b) determining that the subject has the complication of polycystic kidney disease or a risk of developing the complication when the expression level of MMP1 is higher than that of a control sample;
and when the expression levels of 1 or more genes selected from or all genes of a group consisting of NTNG1, POSTN, TNC, KALI, BST1, HSD3B1, KRT7, USP40, and SULT1E1 are lower than those in a control sample, or when the expression level of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE are higher than those in the control sample; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.

In an embodiment of the present invention, vascular endothelial cells obtained via differentiation induction in vitro from iPS cells formed from somatic cells isolated from a subject may be used as samples in the method of examining (detecting or diagnosing) a complication of polycystic kidney disease according to the present invention. In this case, the method can be performed by measuring the presence or the absence of a decrease in or the level (the degree of decrease in expression) of the expression of 1 or more genes selected from or all genes of a group consisting of NTNG1, POSTN, TNC, KALI, and BST1 in Table 1 and then evaluating the thus obtained results, and/or the method can be performed by measuring the presence or the absence of increase in or the level (degree of increase in expression) of the expression of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 in Table 1, and then evaluating the thus obtained results.
Specifically, the method comprises the steps of: inducing iPS cells from somatic cells isolated from a subject to cause differentiation induction of vascular endothelial cells from the iPS cells; measuring the expression level of MMP1 gene and measuring the expression levels or the transcriptional activity of 1 or more genes selected from or all genes of a group consisting of NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 in vascular endothelial cells; and determining that the subject has a complication of polycystic kidney disease or a risk of developing the complication when the expression level of MMP1 is higher than that of a control sample;
and when the expression levels or the transcriptional activity of 1 or more genes selected from or all genes of a group consisting of NTNG1, POSTN, TNC, KALI, and BST1 exhibit a decrease (or reduction) compared with a control sample (that is, vascular endothelial cells obtained via differentiation induction from iPS cells formed from somatic cells of a healthy subject), or when the expression levels or the transcriptional activity of 1 or more genes selected from or all genes of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 exhibit an increase (or enhancement) compared with the control sample; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.
In another embodiment, vascular mural cells obtained via differentiation induction from iPS cells formed from somatic cells of a subject may also be used as samples for the examination (detection or diagnosis) of a complication of polycystic kidney disease according to the present invention. In this case, the method can be performed by measuring the presence or the absence of decrease in or the level (the degree of decrease in expression) of the expression of 1 or more genes selected from or all genes of a group consisting of HSD3B1, KRT7, USP40, and SULT1E1 in Table 1, and then evaluating the thus obtained results, and/or measuring
the presence or the absence of increase in or the level (the degree of increase in expression) of the expression of 1 or more genes selected from or all genes of the group consisting of TFPI2, HMGA2, KRTAP4-7, KRTAP4-8, KRTAP4-9, MYPN, RPPH1, and SIAE in Table 1, and then evaluating the thus obtained results.
Specifically, the method comprises the steps of: inducing iPS cells from somatic cells of a subject to cause differentiation induction of vascular mural cells from the iPS cells; measuring the expression level of MMP1 gene and measuring the expression levels or the transcriptional activity of 1 or more genes selected from or all genes of a group consisting of HSD3B1, KRT7, USP40, and SULT1E1 in the vascular mural cells; and determining that the subject has a complication of polycystic kidney disease or a risk of developing the complication when the expression level of MMP1 is higher than that of a control sample; and when the expression levels or the transcriptional activity of 1 or more genes selected from or all genes of a group consisting of HSD3B1, KRT7, USP40, and SULT1E1 exhibit a decrease (or reduction) compared with a control sample (that is, vascular mural cells obtained via differentiation induction from iPS cells formed from somatic cells of a healthy subject), or when the expression levels or the transcriptional activity of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 exhibit an increase (or enhancement) compared with the control sample; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.

### <Antibodies as disease markers>

Described herein is an antibody capable of specifically recognizing the expression product (protein) of a gene(s) listed in Table 1 as a disease marker for polycystic kidney disease or a complication of polycystic kidney disease.

An example of a protein encoded by a gene(s) listed in Table 1 is a protein encoded by the polynucleotide shown in SEQ ID NO: 1, 3, 5, 7, 9, 11, 13, 15, 17, 19, 21, 23, 25, 27, 29, 31,33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, or 94. Described herein is a protein having the amino acid sequence shown in SEQ ID NO: 2, 4, 6, 8, 10, 12, 14, 16, 18, 20, 22, 46, 48, 50, 52, 54, 56, 58, 60, 62, 64, 66, 68, 70, 72, 74, 76, 78, 80, 82, 84, 86, 88, 90, 92, or 95.

The form of the antibody is not particularly limited and may be a polyclonal or monoclonal antibody, which can be prepared using a protein(s) listed in Table 1 as an immunizing antigen, a chimeric antibody (e.g., a human/mouse chimeric antibody), a humanized antibody, a human antibody, or the like, or, a fragment (e.g., Fab, Fab', F(ab')₂, Fc, Fv, and scFv) of such an antibody. Moreover, an antibody having an antigen binding ability for a polypeptide comprising at least 8 continuous amino acids (e.g., 10 to 20 amino acids) in the amino acid sequence of the protein is also described herein.

Methods for producing such antibodies are known. The antibody can also be produced according to these conventional methods (Current protocols in Molecular Biology edit. Ausubel et al. (1987) Publish. John Wiley and Sons. Section 11. 12-11. 13).

Specifically, when the antibody is a polyclonal antibody, a non-human animal such as a rabbit is immunized with the protein encoded by a gene listed in Table 1, expressed in Escherichia coli or the like, and then purified according to conventional methods or an oligo peptide synthesized having a partial amino acid sequence of the protein. Thus, the polyclonal antibody can be obtained from the serum of the immunized animal according to conventional methods.

Meanwhile, when the antibody is a monoclonal antibody, it can be obtained from hybridoma cells prepared by fusing the thus obtained spleen cells and myeloma cells (obtained from the above-immunized non-human animal) through HAT selection and affinity assay with a target polypeptide, for example (Current protocols in Molecular Biology edit. Ausubel et al., (1987) Publish. John Wiley and Sons. Section 11. 4-11. 11).

The protein to be used for preparation of an antibody can be obtained by DNA cloning, construction of each plasmid, transfection into a host, culture of a transformant, and subsequent collection of the protein from a culture product based on the gene sequence information provided by the present invention. These procedures can be carried out according to methods known by persons skilled in the art or methods described in documents (Molecular Cloning, T. Maniatis et al., CSH Laboratory (1983), DNA Cloning, DM. Glover, IRL PRESS (1985)), for example. Specifically, a recombinant DNA (or an expression vector) that enables expression of a gene in desired host cells is prepared, the DNA is introduced into host cells for transformation, the transformant is cultured, and then a target protein is collected from the thus obtained culture product, so that the protein can be obtained. Also, the protein can also be produced by general chemical synthesis methods (peptide synthesis) according to the amino acid sequence information provided herein, as another technique.

In addition, examples of the proteins encoded by genes listed in Table 1 include not only a protein having the amino acid sequence shown in SEQ ID NO: 2, 4, 6,8, 10, 12, 14, 16, 18, 20, 22, 46, 48, 50, 52, 54, 56, 58, 60, 62, 64, 66, 68, 70, 72, 74, 76, 78, 80, 82, 84, 86, 88, 90, 92, or 95, but also a portion homologous thereto. An example of such a homologous portion is a protein comprising an amino acid sequence that has deletion, substitution, or addition of 1 or a plurality of amino acids, preferably 1 or several amino acids, with respect to each amino acid sequence shown in SEQ ID NO: above, or, an amino acid sequence having at least 90%, preferably at least 95%, 96%, or 97%, further preferably at least 98%, and most preferably at least 99% sequence identity with each amino acid sequence shown in SEQ ID NO: above, and, having biological functions equivalent to and/or immunological activity equivalent to that of the protein having each amino acid sequence shown in SEQ ID NO: above. Such a homologous portion contains a mutant on the basis of racial polymorphism, mutation, splice mutation, and the like.

In addition, an example of a protein having biological functions equivalent to the known functions of a protein having the amino acid sequence shown in SEQ ID NO: 2, 4, 6, 8, 10, 12, 14, 16, 18, 20, 22, 46, 48, 50, 52, 54, 56, 58, 60, 62, 64, 66, 68, 70, 72, 74, 76, 78, 80, 82, 84, 86, 88, 90, 92, or 95 is a protein having biochemical or pharmacological functions equivalent to the same. Also, an example of such a protein having immunological activity equivalent to that of the above protein is a protein capable of specifically binding to an antibody against the above protein.

The term "sequence identity" as used herein can be defined as a percentage (%) of the number of identical amino acid residues or identical nucleotides of total number of amino acid residues or nucleotides (both cases include the number of gaps) when two amino acid sequences or two nucleotide sequences are aligned to achieve the maximum match of amino acids or nucleotides with or without introduction of gaps.

The sequence identity can be determined using BLAST (Altschul SF, et al, (1997) Nucleic Acids Res. 25 (17): 3389-402 or (1990) J Mol Biol. 215(3): 403-10) that can be found from the NCBI server, ncbi. nlm. nih. gov/BLAST/.

Furthermore, the number of amino acid mutations or mutation sites in proteins is not limited, as long as their biological functions and/or immunological activity is retained.

An indicator for determining the way of substituting, inserting, or deleting amino acid residues and the number thereof without losing biological functions or immunological activity can be found using a computer program known to persons skilled in the art, such as DNA Star software. For example, the number of mutations typically accounts for 10% or less of all amino acids, preferably 5% or less of all amino acids, and further preferably accounts for 1% or less of all amino acids. Also, amino acids to be substituted are not particularly limited, as long as proteins obtained after substitution with the relevant amino acids have biological functions and/or immunological activity equivalent to that of the original protein. Preferably, in view of the retention of protein configuration, amino acids to be substituted have properties such as electrical properties and structural properties (e.g., polarity, electric charge, solubility, hydrophobicity, hydrophilicity, and amphiphilicity of amino acid residues) analogous to those of unsubstituted amino acids. For example, Ala, Val, Leu, Ile, Pro, Met, Phe, and Trp are amino acids that are classified as nonpolar amino acids from each other. Gly, Ser, Thr, Cys, Tyr, Asn, and Gln are amino acids that are classified as uncharged amino acids. Asp and Glu are amino acids that are classified as acidic amino acids. Further, Lys, Arg, and His are amino acids that are classified as basic amino acids. Therefore, amino acids to be substituted can be appropriately selected from among amino acids belonging to the same group using these amino acid properties as indicators.

Also, the antibody may be prepared using a polypeptide (intended to include an oligo peptide) having a partial amino acid sequence of a protein encoded by a gene(s) listed in Table 1 (see above). A polypeptide to be used for inducing such an antibody is not required to have functional bioactivity, but desirably has immunogenicity analogous to that of the protein. A preferable example of a polypeptide to be used herein has such immunogenicity and comprises at least 8 continuous amino acid residues (e.g., 10 to 20 amino acid residues) in the amino acid sequence of SEQ ID NO: 2, 4, 6, 8, 10, 12, 14, 16, 18, 20, 22, 46, 48, 50, 52, 54, 56, 58, 60, 62, 64, 66, 68, 70, 72, 74, 76, 78, 80, 82, 84, 86, 88, 90, 92, or 95.

An antibody against such a polypeptide can also be prepared by enhancing immunological responses with the use of various adjuvants depending on host animals. Examples of such an adjuvant include, but are not limited to: Freund's adjuvant; and mineral gel such as aluminum hydroxide; as well as surface active substances such as lysolecithin, pluronic polyols, polyanions, peptides, oil emulsions, keyhole limpet hemocyanin, and dinitrophenol; and human adjuvants such as BCG (Bacillus Caluuette Guecin) and Corynebacterium-parvum.

The antibody has a property of specifically binding to a protein encoded by a gene(s) listed in Table 1.

Hence, through the use of such an antibody, the above protein contained in a sample from a subject can be specifically detected and quantitatively determined. Specifically, the antibody of the present invention is useful for examining (or detecting or diagnosing) polycystic kidney disease or a complication of polycystic kidney disease.

### <Method of examining polycystic kidney disease or a complication of polycystic kidney disease>

Described herein is a method comprising the following steps, (a-1) and (b-1), as a method of examining polycystic kidney disease:
(a-1) measuring the expression levels of a protein encoded by 1 or more genes selected from or all genes of a group consisting of NTNG1, POSTN, TNC, KALI, BST1, ACAT2, INSIG1, SCD, HSD3B1, KRT7, USP40, and SULT1E1 in a sample from a subject; and
(b-1) determining that the subject has polycystic kidney disease or a risk of developing the disease when the expression levels of the proteins encoded by 1 or more genes selected from or all genes of a group consisting of NTNG1, POSTN, TNC, KALI, BST1, HSD3B 1, KRT7, USP40, and SULT1E1 are lower than those in a healthy subject-derived control sample, or determining that the subject has polycystic kidney disease or a risk of developing the disease when the expression level of 1 or more genes selected from or all genes of a group consisting of ACAT2, INSIG1, and SCD are higher than those in a healthy subject-derived control sample.

Similarly, a method comprising the following steps, (a-2) and (b-2), is provided as the method of examining a complication of polycystic kidney disease:
(a-2) measuring the expression level of MMP1 gene and measuring the expression levels of 1 or more genes selected from or all genes of a group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE in a sample from a subject with polycystic kidney disease; and
(b-2) determining that the subject has a complication of polycystic kidney disease or a risk of developing the complication when the expression level of MMP1 is higher than that of a control sample;
and when the expression levels of 1 or more genes selected from or all genes of a group consisting of NTNG1, POSTN, TNC, KALI, BST1, HSD3B1, KRT7, USP40, andSULTlEl are lower than those in a healthy subject-derived control sample, or determining that the subject has a complication of the same or a risk of developing the complication when the expression levels of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE are higher than those in a healthy subject-derived control sample; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.

Similarly, a method comprising the following steps, (a-3) and (b-3), is provided as the method of examining cerebral aneurysm which is a complication:
(a-3) measuring the expression level of MMP1 gene and measuring the expression levels of 1 or more genes selected from or all genes of a group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE in a sample from a subject with polycystic kidney disease; and
(b-3) determining that the subject has cerebral aneurysm or a risk of developing the disease when the expression level of MMP1 is higher than that of a control sample;
and when the expression levels of 1 or more genes selected from or all genes of a group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE are higher than those in a healthy subject-derived control sample.

Here, as samples from a subject or control samples from a healthy subject, blood, serum, blood plasma, cell extracts, urine, lymph fluids, tissue fluids, ascites, spinal fluids, or other body fluids, or tissues or cells (e.g., renal tissues or renal cells or somatic cells obtained via differentiation induction from iPS cells) can be used. As such somatic cells obtained via differentiation induction from the iPS cells formed from somatic cells of a subject, tubular cells, collecting tubule cells, bile duct cells, hepatocytes, pancreatic ductal cells, pancreatic cells, bowel cells, germ cells, vascular endothelial cells or vascular mural cells, and preferably, vascular endothelial cells or vascular mural cells can be used, for example. Control samples to be used herein are corresponding somatic cells obtained via differentiation induction from iPS cells formed from somatic cells of a healthy subject. The healthy subject-derived somatic cells for induction of iPS cells may differ from or may be of the same type as subject-derived somatic cells. Examples of such somatic cells are as described later in the section of "method of producing iPS cells." Also, in this connection, "corresponding somatic cells" means that somatic cells obtained via differentiation induction from iPS cells formed from healthy subject-derived somatic cells are cells of the same type as that of somatic cells obtained via differentiation induction from iPS cells formed from subject-derived somatic cells.

When vascular endothelial cells obtained via in vitro differentiation induction from iPS cells formed from isolated subject-derived somatic cells is used as a sample from a subject, the present disclosure further provides a method comprising the following steps, (a-4) and (b-4), as the method of examining polycystic kidney disease:
(a-4) measuring the expression levels of 1 or more genes selected from or all genes of a group consisting of NTNG1, POSTN, TNC, KALI, BST1, ACAT2, INSIG1, SCD, BMP6, CD274, CTGF, E2F7,EDN1, FAM43A, FRMD3, MMP10, MYEOV, NR2F1, NRCAM, PCSK1, PLXNA2, SLC30A3, SNAI1, SPOCD1, MMP1, and TFPI2 or the expression levels of a protein(s) encoded by the gene(s) in vascular endothelial cells obtained via differentiation induction from iPS cells formed from subject-derived somatic cells; and
(b-4) determining that the subject has polycystic kidney disease or a risk of developing the disease when the expression levels of 1 or more genes selected from or all genes of a group consisting of NTNG1, POSTN, TNC, KALI, and BST1 or the expression levels of the protein(s) encoded by the gene(s) are lower than those in a healthy subject-derived control sample, or, determining that the subject has polycystic kidney disease or a risk of developing the disease when the expression levels of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 are higher than those in a healthy subject-derived control sample.

In another aspect, the present disclosure further provides a method comprising the following steps, (a-5) and (b-5), as the method of examining polycystic kidney disease, when vascular mural cells obtained via in vitro differentiation induction from iPS cells formed from isolated subject-derived somatic cells are used as a sample from a subject:
(a-5) measuring the expression levels of 1 or more genes selected from or all genes of a group consisting of HSD3B1, KRT7, USP40, SULT1E1, MMP1, TFPI2, HMGA2, KRTAP4-7, KRTAP4-8, KRTAP4-9, MYPN, RPPH1, and SIAE or the expression levels of a protein(s) encoded by the gene(s) in vascular mural cells obtained via differentiation induction from iPS cells formed from subject-derived somatic cells; and
(b-5) determining that the subject has polycystic kidney disease or a risk of developing the disease when the expression levels of 1 or more genes selected from or all genes of a group consisting of HSD3B1, KRT7, USP40, and SULT1E1 or the expression level(s) of the protein(s) encoded by the gene(s) are lower than those in vascular mural cells (control sample) obtained via differentiation induction from iPS cells formed from a healthy subject-derived somatic cells, or determining that the subject has polycystic kidney disease or a risk of developing the disease when the expression levels of 1 or more genes selected from or all genes of a group consisting of MMP1, TFPI2, HMGA2, KRTAP4-7, KRTAP4-8, KRTAP4-9, MYPN, RPPH1, and SIAE are higher than those in a healthy subject-derived control sample.

In another aspect, the present invention further provides a method comprising the following steps, (a-6) and (b-6), as the method of examining a complication of polycystic kidney disease when vascular endothelial cells obtained via differentiation induction from iPS cells formed from subject-derived somatic cells are used as a sample from a subject:
(a-6) measuring the expression level of MMP1 gene and measuring the expression levels of 1 or more genes selected from or all genes of a group consisting of NTNG1, POSTN, TNC, KALI, BST1, ACAT2, INSIG1, SCD, BMP6, CD274, CTGF, E2F7,EDN1, FAM43A, FRMD3, MMP10, MYEOV, NR2F1, NRCAM, PCSK1, PLXNA2, SLC30A3, SNAI1, SPOCD1, and TFPI2 or the expression levels of the protein(s) encoded by the gene(s) in vascular endothelial cells obtained via differentiation induction from iPS cells formed from somatic cells of a subject with polycystic kidney disease; and
(b-6) determining that the subject has a complication of polycystic kidney disease or a risk of developing the complication when the expression level of MMP1 is higher than that of a control sample;
and when the expression levels of 1 or more genes selected from or all genes of a group consisting of NTNG1, POSTN, TNC, KALI, and BST1 or the expression levels of the protein(s) encoded by the gene(s) are lower than those in vascular endothelial cells (control sample) obtained via differentiation induction from iPS cells formed from healthy subject-derived somatic cells, or determining that the subject has a complication of polycystic kidney disease or a risk of developing the complication when the expression levels of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 are higher than those in the control sample; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.

A method comprising the following steps, (a-7) and (b-7), is provided as the method of examining aneurysm when vascular endothelial cells obtained via differentiation induction from iPS cells formed from somatic cells of a subject with polycystic kidney disease are used:
(a-7) measuring the expression level of MMP1 gene and measuring the expression levels of 1 or more genes selected from or all genes of a group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 or the expression levels of a protein(s) encoded by the gene(s) in vascular endothelial cells obtained via differentiation induction from iPS cells formed from somatic cells of a subject with polycystic kidney disease; and
(b-7) determining that the subject has cerebral aneurysm or a risk of developing the disease when the expression level of MMP1 is higher than that of a control sample;
and when the expression levels of 1 or more genes selected from or all genes of a group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2 are higher than those in the control sample.

In another aspect, the present invention further provides a method comprising the following steps, (a-8) and (b-8), as the method of examining a complication of polycystic kidney disease when vascular mural cells obtained via differentiation induction from iPS cells formed from somatic cells from a subject are used as a sample from a subject:
(a-8) measuring the expression level of MMP1 gene and measuring the expression levels of 1 or more genes selected from or all genes of a group consisting of HSD3B1, KRT7, USP40, SULT1E1, TFPI2, HMGA2, KRTAP4-7, KRTAP4-8, KRTAP4-9, MYPN, RPPH1, and SIAE or the expression levels of a protein(s) encoded by the gene(s) in vascular mural cells obtained via differentiation induction from iPS cells formed from somatic cells of a subject with polycystic kidney disease; and
(b-8) determining that the subject has a complication of polycystic kidney disease when the expression level of MMP1 is higher than that of a control sample;
and when the expression levels of 1 or more genes selected from or all genes of a group consisting of HSD3B1, KRT7, USP40, and SULT1E1 or the expression levels of the protein(s) encoded by the gene(s) are lower than those in vascular mural cells (control sample) obtained via differentiation induction from iPS cells formed from somatic cells of a healthy subject, or when the expression levels of 1 or more genes selected from or all genes of a group consisting of TFPI2, HMGA2, KRTAP4-7, KRTAP4-8, KRTAP4-9, MYPN, RPPH1, and SIAE are higher than those in the control sample; wherein the complication of polycystic kidney disease is selected from the group consisting of vascular lesions; valvular disease of heart; diverticula of colon; hernia; ductal lesions; and symptoms of cyst formation in the liver, pancreas, spleen, and generative organ.

A method comprising the following steps, (a-9) and (b-9), is provided as a more preferable method of examining a complication of cerebral aneurysm when vascular mural cells obtained via differentiation induction from iPS cells formed from somatic cells of a subject with polycystic kidney disease are used:
(a-9) measuring the expression level of MMP1 gene and measuring the expression levels of 1 or more genes selected from or all genes of a group consisting of TFPI2, HMGA2, KRTAP4-7, KRTAP4-8, KRTAP4-9, MYPN, RPPH1, and SIAE or the expression levels of a protein(s) encoded by the gene(s) in vascular mural cells obtained via differentiation induction from iPS cells formed from somatic cells of a subject with polycystic kidney disease; and
(b-9) determining that the subject has cerebral aneurysm or a risk of developing the disease when the expression level of MMP1 is higher than that of a control sample;
and when the expression levels of 1 or more genes selected from or all genes of a group consisting of TFPI2, HMGA2, KRTAP4-7, KRTAP4-8, KRTAP4-9, MYPN, RPPH1, and SIAE are higher than those in the control sample.

The term "control" as used herein preferably refers to, unless otherwise specified, a measurement result obtained by a step similar to the above from a healthy subject not affected by polycystic kidney disease.

Here, blood, serum, blood plasma, cell extracts, urine, lymph fluids, tissue fluids, ascites, spinal fluids, and other body fluids, or, somatic cells obtained via differentiation induction from iPS cells formed from somatic cells of a subject (e.g., tubular cells, collecting tubule cells, bile duct cells, hepatocytes, pancreatic ductal cells, pancreatic cells, bowel cells, germ cells, vascular endothelial cells, and vascular mural cells) may be directly used. More preferable examples thereof include subject-derived blood, serum, blood plasma, cell extracts, urine, lymph fluids, tissue fluids, ascites, spinal fluids, and other body fluids, or, mRNA prepared by a conventional method from the above cells obtained via differentiation induction from subject-derived iPS cells or a polynucleotide further prepared from such mRNA (e.g., cDNA and cRNA), proteins, or extracts containing protein-containing fractions. Here, methods for producing tubular cells, collecting tubule cells, bile duct cells, hepatocytes, pancreatic ductal cells, pancreatic cells, bowel cells, germ cells, vascular endothelial cells, or vascular mural cells from iPS cells are not particularly limited. These cells can be obtained by appropriately extracting from the embryoid bodies or formed teratomas (e.g., JP Patent Publication (Kokai) No. 2006-239169 A). Hepatocytes can be prepared by a method described in WO2006/082890, JP Patent Publication (Kokai) No. 2010-75631 A or Hay DC, et al, Proc Natl Acad Sci U.S.A., 105, 12301-6 2008, but the examples thereof are not particularly limited thereto. Similarly, pancreatic cells can be prepared using the method described in WO2007/103282. iPS cells as well as vascular endothelial cells or vascular mural cells can be produced by methods described later.

The examination method of the present invention can be specifically performed as described below depending on the types of biological samples to be subjected to measurement.

When mRNA or a polynucleotide prepared therefrom (e.g., cDNA or cRNA) is used as a subject to be measured, the following steps can be used as the above steps (i) and (ii):
(i) binding a mRNA prepared from a sample from a subject or a complementary polynucleotide transcribed from the mRNA, to the above disease marker; and
(ii) measuring RNA from a biological sample or a complementary polynucleotide (cDNA) transcribed from the RNA, which has bound to the disease marker, using the existing amount of the above disease marker as an indicator.

When mRNA or a polynucleotide prepared therefrom (e.g., cDNA or cRNA) (hereinafter, referred to as "mRNA" or the like) is used as a sample to be used for detection, the examination method of the present invention is carried out by detecting and measuring the degree of expression or the expression level of a gene(s) listed in Table 1 such as the mRNA. Specifically, the measurement of mRNA can be carried out by a known method, such as the Northern blot method, the Southern blot method, RT-PCR, quantitative PCR, DNA chip analysis, or in situ hybridization analysis, using the disease markers comprising the above polynucleotides of the present invention as primers or probes.

When the Northern blot method or the Southern blot method is used, the expression level of a target gene in mRNA or the like can be detected or measured using the above disease marker of the present invention as a probe. A specific example thereof comprises labeling the disease marker of the present invention (or complementary strand in the case of RNA) with a radioisotope (RI, e.g., ³²P or ³³P, a fluorescent substance, or the like, performing hybridization of the labeled disease marker to mRNA or the like from a living tissue of a subject, which has been transferred to a nylon membrane or the like according to conventional methods, and then detecting or measuring the thus formed double strand of the disease marker and mRNA or the like through detection of signals from the label (e.g., RI or a fluorescent substance) for the disease marker using a radiation detector (Typhoon FLA 9000, GE HEALTHCARE) or fluorescence detector. Alternatively, a method that can be used herein comprises labeling a disease marker according to protocols attached to AlkPhos Direct Labeling and Detection System (Amersham Pharmacia Biotech), performing hybridization of the labeled disease marker to mRNA or the like from a living tissue of a subject, and then detecting or measuring signals from the label of the disease marker using a MultiBio Imager STORM 860 (Amersham Pharmacia Biotech), for example.

When the RT-PCR method is used, the expression levels of the genes listed in Table 1 in RNA or the like can be detected or measured using the above disease markers of the present invention as primers.

A specific example of the method comprises preparing cDNA according to conventional methods from RNA from a living tissue of a subject, performing PCR according to a conventional method using the resulting cDNA as a template and the disease markers of the present invention as primers, so that a target gene region can be amplified, and detecting the thus obtained amplified double-stranded DNA.

PCR comprises performing approximately 20-40 cycles, each consisting of a denaturation step, an annealing step, and an extension step, for example. The denaturation step is to denature and dissociate double-stranded DNA into single-stranded DNAs at generally about 94-98 degrees C for about 10 sec to about 2 min). The annealing step is to perform annealing a sense primer or an antisense primer to each single-stranded DNA as a template at generally about 50-68 degrees C for about 10 sec to 1 min. The extension step is to extend primers along the template DNA, which is generally performed at about 72 degrees C for about 20 sec to 10 min, for example. Before the above cycles are performed, pretreatment may be performed for double-stranded DNA under conditions similar to the conditions for denaturation. Also, after completion of the above cycles, posttreatment may be performed under conditions similar to the conditions for extension. For PCR, PCR buffer and heat stable DNA polymerase are used. Amplification products can be confirmed by electrophoresis, for example. PCR can be performed using a commercial PCR apparatus such as a thermal cycler.

Furthermore, when DNA chip analysis is employed, an example thereof is a method that comprises preparing a DNA chip in which the disease marker of the present invention as a DNA probe (either single-stranded or double-stranded polynucleotide) attached to the surface, causing cRNA (prepared by a conventional method from RNA derived from a living tissue of a subject) to hybridize to the DNA chip, binding the formed double strand of DNA and cRNA to the disease marker of the present invention as a labeled probe (separately labeled with RI, a fluorescent substance, or the like), and then performing detection. An example of such a DNA chip with which the expression level of a gene can be detected or measured as described above is a Gene Chip (Affymetrix).

When a protein is used as a subject to be measured, an example of a method to be employed herein comprises bringing a protein into contact with an antibody against the disease marker described herein, and then detecting or quantitatively determining the protein or a partial peptide thereof binding to the antibody by a known detection method such as the Western blot method or ELISA using the disease marker as an indicator.

The Western blot method can be carried out by: after the use of the above antibody against the disease marker as a primary antibody, labeling a complex of a protein or a partial peptide thereof and the disease marker (primary antibody) with the use of a secondary antibody (the antibody labeled with a radioisotope such as ¹²⁵I, an enzyme such as horseradish peroxidase (HRP), or a fluorescent substance, which is capable of binding to the primary antibody), and then detecting and measuring signals from the radioisotope or the fluorescent substance using a radiation meter (Typhoon FLA 9000, GE HEALTHCARE) or fluorescence detector. Alternatively, after an antibody against the above disease marker is used as a primary antibody, detection can be performed according to protocols attached to the ECL Plus Western Blotting Detection System (Amersham Pharmacia Biotech) and then measurement can be performed using Multi Bio Imager STORM860 (Amersham Pharmacia Biotech).

When subject-derived blood, serum, blood plasma, cell extracts, urine, lymph fluids, tissue fluids, ascites, spinal fluids, or other body fluids exemplified above, or tissues or cells are used as samples, immunoassay can be performed. Examples of such a method include radioimmunoassay, enzyme immunoassay, fluorescence immunoassay, luminescent immunoassay, immunoprecipitation, immunonephelometry, Western blot, and immunodiffusion. A preferable example thereof is enzyme immunoassay and a particularly preferable example thereof is enzyme-linked immunosorbent assay (ELISA) (e.g., sandwich ELISA). The above immunological methods such as ELISA can be performed by methods known by persons skilled in the art. Specifically, ELISA is performed as follows. A solution containing an antibody against the disease marker is added as a primary antibody to a support such as a plate, so as to immobilize the antibody. After washing of the plate, blocking is performed using BSA or the like in order to prevent non-specific binding of proteins. The plate is washed again, and then a sample is added to the plate. After incubation, washing is performed and then a labeled antibody such as a biotin-labeled antibody is added as a secondary antibody. After appropriate incubation, the plate is washed and then avidin bound to an enzyme such as alkaline phosphatase or peroxidase is added. After incubation, the plate is washed, a substrate corresponding to the enzyme binding to avidin is added, and then the amount of a desired protein is detected using an enzymatic change or the like of the substrate as an indicator.

Autosomal dominant polycystic kidney disease or a complication of polycystic kidney disease can be examined (or detected or diagnosed) by, preferably:
comparing the expression level of a gene listed in Table 1 (above) or a protein encoded by the gene in a sample from a subject (the expression level is reflected by the amount of binding of a polynucleotide as the disease marker with a probe for examination, or the amount of binding of a protein or a partial peptide as the disease marker with an antibody for examination or a fragment thereof, as measured above) with the expression level of the gene or the protein in a sample (as a control to be similarly measured) from a healthy subject who is not affected by polycystic kidney disease;
and performing examination based on differences between the two. In this case, if the expression level of a target gene or protein in a subject is 1.5 or more times, 2 or more times, 3 or more times, preferably 5 or more times, and further preferably 10 or more times lower or higher than that of a healthy subject, whether or not the subject has the disease can be confirmed with even higher reliability.

In addition, comparison between a sample from a subject and a control sample from a healthy subject for the expression level of a target gene or the protein can be performed by performing measurements in parallel. If the measurements are not performed in parallel, an average or median of the expression levels obtained by measuring a plurality of (at least 2, preferably 3 or more, and more preferably 5 or more) controls under substantially the same measurement conditions can be used for comparison as a basic level.

### <Method of producing iPS cells>

iPS cells to be used in the present invention can be prepared by introducing a specific nuclear reprogramming factor (also, referred to as "reprogramming factor") in the form of DNA or protein into somatic cells or increasing the expression of the endogenous mRNA or protein of the nuclear reprogramming factor using a drug (K. Takahashi and S. Yamanaka (2006) Cell, 126: 663-676, K. Takahashi et al. (2007) Cell, 131: 861-872, J. Yu et al. (2007) Science, 318: 1917-1920, M. Nakagawa et al. (2008) Nat. Biotechnol., 26: 101-106, International Publication WO 2007/069666, and International Publication WO 2010/068955). A nuclear reprogramming factor may be a gene specifically expressed in ES cells, a gene playing an important role in maintenance of undifferentiation of ES cells, or a gene product thereof. Examples of such nuclear reprogramming factor include, but are not particularly limited to, Oct3/4, Klf4, Klf1,Klf2, Klf5, Sox2, Sox1, Sox3, Sox15, Sox17, Sox18, c-Myc, L-Myc, N-Myc, TERT, SV40 Large T antigen, HPV16 E6, HPV16 E7, Bmil, Lin28, Lin28b, Nanog, Esrrb, Esrrg, and Glis1. These reprogramming factors may be used in combination upon establishment of iPS cells. Such combination may contain at least one, two, or three reprogramming factors above and preferably contains 3 or 4 reprogramming factors above.

The nucleotide sequence information of the mouse and human cDNAs of each of the above nuclear reprogramming factors and the amino acid sequence information of proteins encoded by the cDNAs can be obtained by referring to NCBI accession numbers described in WO 2007/069666. Also, the mouse and human cDNA sequence and amino acid sequence information of L-Myc, Lin28, Lin28b, Esrrb, Esrrg, and Glis1 can be each obtained by referring to the following NCBI accession numbers. Persons skilled in the art can prepare desired nuclear reprogramming factors by a conventional technique based on the cDNA sequence or amino acid sequence information.
Gene name Mouse Human
L-Myc NM_008506 NM_001033081
Lin28 NM_145833 NM_024674
Lin28b NM_001031772 NM_001004317
Esrrb NM_011934 NM_004452
Esrrg NM_011935 NM_001438
Glis1 NM_147221 NM_147193
These nuclear reprogramming factors may be introduced in the form of protein into somatic cells by a technique such as lipofection, binding with a cell membrane-permeable peptide, or microinjection. Alternatively, they can also be introduced in the form of DNA into somatic cells by a technique such as a technique using a vector (e.g., a virus, a plasmid, or an artificial chromosome), lipofection, a technique using a liposome, or microinjection.

Examples of the viral vector include retrovirus vector, lentivirus vector (see Cell, 126, pp.663-676, 2006; Cell, 131, pp.861-872, 2007; and Science, 318, pp. 1917-1920, 2007), adenovirus vector (see Science, 322, 945-949, 2008), adeno-associated virus vector, and Sendai virus vector (see Proc Jpn Acad Ser B Phys Biol Sci. 85, 348-62, 2009).

Also, examples of the artificial chromosome vector include human artificial chromosome (HAC), yeast artificial chromosome (YAC), and bacterial artificial chromosome (BAC and PAC).

As the plasmid, a plasmid for mammalian cells can be used (see Science, 322: 949-953, 2008).

The above vector can contain regulatory sequences such as a promoter, an enhancer, a ribosome binding sequence, a terminator, and a polyadenylation site, so that a nuclear reprogramming factor can be expressed. Examples of the promoter to be used herein include EF1 alpha promoter, CAG promoter, SR alpha promoter, SV40 promoter, LTR promoter, CMV (cytomegalovirus) promoter, RSV (Rous sarcoma virus) promoter, MoMuLV (Moloney mouse leukemia virus) LTR, and HSV-TK (herpes simplex virus thymidine kinase) promoter. Particularly, examples thereof include EF1 alpha promoter, CAG promoter, MoMuLV LTR, CMV promoter, and SR alpha promoter.

The vector may further contain, if necessary, a selection marker sequence such as a drug resistance gene (e.g., a kanamycin resistance gene, an ampicillin resistance gene, and a puromycin resistance gene), a thymidine kinase gene, and a diphtheria toxin gene, and a reporter gene sequence such as a green fluorescent protein (GFP), beta glucuronidase (GUS), and FLAG.

Also, in order to cleave both a gene encoding a nuclear reprogramming factor or a promoter and a gene encoding a nuclear reprogramming factor binding thereto after introduction into somatic cells, the above vector may have LoxP sequences located before and after the relevant portions. In another preferred embodiment, a method comprising incorporating a transgene into a chromosome using transposon, causing transferase to act on cells using a plasmid vector or an adenovirus vector, and then completely removing the transgene from the chromosome can be employed. An example of preferable transposon is piggyBac or the like that is lepidopteran insect-derived transposon (Kaji, K. et al., (2009), Nature, 458: 771-775, Woltjen et al., (2009), Nature, 458: 766-770, WO 2010/012077).

Furthermore, the above vector may also contain sequences of replication origins of lymphotrophic herpes virus, BK virus, and Bovine papilloma virus and sequences relating to the replication thereof so that they can be episomally present as a result of replication even if they are not incorporated into a chromosome. For example, the vector contains EBNA-1 and oriP or Large T and SV40ori sequences (WO 2009/115295, WO 2009/157201, and WO 2009/149233).

Furthermore, for simultaneous introduction of a plurality of nuclear reprogramming factors, an expression vector for polycistronic expression may also be used. For polycistronic expression, sequences of genes may be connected to each other by intervening IRES or a foot and mouth disease virus (FMDV) 2A coding region between them (Science, 322: 949-953, 2008; WO 2009/092042; and WO 2009/152529).

Upon nuclear reprogramming, to improve the efficiency for induction of iPS cells, in addition to the above factors or elements, histone deacetylase (HDAC) inhibitors [e.g., low-molecular weight inhibitors such as valproic acid (VPA) (Nat. Biotechnol., 26(7): 795-797 (2008)), trichostatin A, sodium butyrate, MC 1293, and M344, and nucleic acid expression inhibitors such as siRNA and shRNA against HDAC (e.g., HDAC1 siRNA Smartpool (Registered Trademark) (Millipore) and HuSH 29mer shRNA Constructs against HDAC1 (OriGene))], DNA methyltransferase inhibitors (e.g., 5'-azacytidine) (Nat. Biotechnol., 26 (7): 795-797 (2008)), G9a histone methyltransferase inhibitors [e.g., low-molecular-weight inhibitors such as BIX-01294 (Cell Stem Cell, 2: 525-528 (2008)) and nucleic acid expression inhibitors such as siRNA and shRNA against G9a (e.g., G9a siRNA (human) (Santa Cruz Biotechnology))], L-channel calcium agonists (e.g., Bayk8644) (Cell Stem Cell, 3, 568-574 (2008)), p53 inhibitors (e.g., siRNA and shRNA against p53) (Cell Stem Cell, 3, 475-479 (2008)), Wnt Signalling activator (e.g., soluble Wnt3a) (Cell Stem Cell, 3, 132-135 (2008)), growth factors such as LIF or bFGF, ALK5 inhibitors (e.g., SB431542) (Nat Methods, 6: 805-8 (2009)), mitogen-activated protein kinase signaling inhibitors, glycogen synthase kinase-3 inhibitors (PloS Biology, 6(10), 2237-2247 (2008)), miRNA such as miR-291-3p, miR-294, and miR-295 (R. L. Judson et al., Nat. Biotechnol., 27: 459-461 (2009)), for example, can be used.

Examples of a drug to be used in a method for increasing the expression of an endogenous protein of a nuclear reprogramming factor include 6-bromoindirubin-3'-oxime, indirubin-5-nitro-3'-oxime, valproic acid, 2-(3-(6-methylpyridin-2-yl)-1H-pyrazol-4-yl)-1,5-naphthyridine, 1-(4-methylphenyl)-2-(4,5,6,7-tetrahydro-2-imino-3(2H)-benzothiazolyl)ethanone HBr(pifithrin-alpha), prostaglandin J2, and prostaglandin E2 (WO 2010/068955).

Examples of a culture medium for inducing iPS cells include (1) a DMEM, DMEM/ F12, or DME medium containing 10-15% FBS (these media may further appropriately contain LIF, penicillin/streptomycin, puromycin, L-glutamine, nonessential amino acids, Beta-mercaptoethanol, and the like), (2) a medium for ES cell culture containing bFGF or SCF, such as a medium for mouse ES cell culture (e.g., TX-WES medium (Thromb-X)), and a medium for primate ES cell culture (e.g., a medium for primate (human & monkey) ES cells, ReproCELL, Kyoto, Japan (mTeSR-1)).

An example of culture methods is as follows. Somatic cells are brought into contact with nuclear reprogramming factors (nucleic acids or proteins) in a DMEM or DMEM/ F12 medium containing 10% FBS at 37 degrees C in the presence of 5% CO₂ and are cultured for about 4 to 7 days. Subsequently, the cells are reseeded on feeder cells (e.g., mitomycin C-treated STO cells or SNL cells). About 10 days after contact between the somatic cells and the nuclear reprogramming factors, cells are cultured in a bFGF-containing medium for primate ES cell culture. About 30-45 days or more after the contact, ES cell-like colonies can be formed. Cells may also be cultured under conditions in which the oxygen concentration is as low as 5-10% in order to increase the efficiency for induction of iPS cells.

Alternatively, cells may be cultured using DMEM containing 10-% FBS (which may further appropriately contain LIF, penicillin/streptomycin, puromycin, L-glutamine, nonessential amino acids, beta-mercaptoethanol, and the like) on feeder cells (e.g., mitomycin C-treated STO cells or SNL cells). After about 25-30 days or more, ES cell-like colonies can be formed.

During the above culture, medium exchange with a fresh medium is performed once a day from day 2 after the start of culture. In addition, the number of somatic cells to be used for nuclear reprogramming is not limited, but ranges from approximately 5 x 10³ to approximately 5 x 10⁶ cells per culture dish (100 cm²).

When a gene containing a drug resistance gene is used as a marker gene, cells expressing the marker gene can be selected by culturing the cells in a medium (i.e., a selective medium) containing the relevant drug. Also, cells expressing the marker gene can be detected when the marker gene is a fluorescent protein gene, through observation with a fluorescence microscope, by adding a luminescent substrate in the case of a luminescent enzyme gene, or adding a chromogenic substrate in the case of a chromogenic enzyme gene.

As used herein, the term "somatic cell" refers to any cell (including matured cell, somatic stem cell or tissue stem cell, and precursor cell) excluding germline cells and ES cells. Examples of "somatic cells" for induction of iPS cells, as used herein, include, but are not limited to, keratinizing epithelial cells (e.g., keratinizing epidermal cells), mucosal epithelial cells (e.g., epithelial cells of the surface layer of tongue), exocrine epithelial cells (e.g., mammary glandular cells), hormone-secreting cells (e.g., adrenal medullary cells), cells for metabolism and storage (e.g., hepatocytes), boundary-forming luminal epithelial cells (e.g., type I alveolar cells), luminal epithelial cells of internal tubules (e.g., vascular endothelial cells), ciliated cells having carrying capacity (e.g., airway epithelial cells), cells for secretion to extracellular matrix (e.g., fibroblasts), contractile cells (e.g., smooth muscle cells), cells of blood and immune system (e.g., T lymphocytes), cells involved in sensation (e.g., rod cells), autonomic nervous system neurons (e.g., cholinergic neurons), sense organ and peripheral neuron supporting cells (e.g., satellite cells), nerve cells and glial cells of the central nervous system (e.g., astroglial cells), chromocytes (e.g., retinal pigment epithelial cells), and precursor cells thereof (tissue precursor cells). Without particular limitation concerning the degree of cell differentiation, both undifferentiated precursor cells (also including somatic stem cells) and terminally-differentiated mature cells can be similarly used as origins for somatic cells in the present invention. Examples of undifferentiated precursor cells include tissue stem cells (somatic stem cells) such as neural stem cells, hematopoietic stem cells, mesenchymal stem cells, and dental pulp stem cells.

### <Method of inducing differentiation of vascular endothelial cells>

An induction differentiation method that can be used for producing vascular endothelial cells from iPS cells obtained as described above comprises the following steps of:
(1) performing adhesion culture using a medium for primate ES/iPS cells on a coated culture dish;
(2) adding various additives to the medium and culturing cells;
(3) adding a growth factor to a serum free medium and culturing cells;
(4) separating VEGFR2-positive, TRA1-negative and VE-cadherin-positive cells; and
(5) performing adhesion culture using a growth medium for vascular endothelial cells on a coated culture dish.

Vascular endothelial cells in the present invention preferably express a vascular endothelial cell marker such as VE-cadherin, CD31, CD34, or eNOS and have a cob-blestoned appearance.

Prior to the above step (1), iPS cells can be dissociated therefrom by an arbitrary method. For a method for dissociation, a mechanical dissociation or a dissociation solution having both protease activity and collagenase activity (e.g., Accutase (trademark) (Invitrogen) or Accumax (trademark) (Accumax)) or having collagenase activity alone can be used.

Also, examples of a coating agent to be used in steps (1) and (5) include Matrigel (BD), type-I collagen, type-IV collagen, gelatin, laminin, heparan sulfate proteoglycan, and entactin, and combinations thereof. A preferable example of the same in step (1) is type-I collagen. A preferable example of the same in step (5) is type-IV collagen.

A medium for producing vascular endothelial cells can be prepared using a medium used for culturing animal cells, as a basal medium. Examples of the basal medium include IMDM, Medium 199, Eagle's Minimum Essential Medium (EMEM), alpha MEM, Doulbecco's modified Eagle's Medium (DMEM), Ham's F12 medium, RPMI 1640 medium, Fischer's medium, and mixtures thereof. Furthermore, such a medium may contain serum or may be serum free. If necessary, for example, a medium may contain one or more serum substitutes, such as albumin, transferrin, Knockout Serum Replacement (KSR) (substitute for FBS upon culture of ES cells), fatty acids, insulin, a collagen progenitor, trace elements, 2-mercaptoethanol, 3'-thiolglycerol, as well as, one or more substances such as lipids, amino acids, L-glutamine, Glutamax (Invitrogen), nonessential amino acids, vitamins, antibiotics, antioxidants, pyruvic acid, buffering agents, inorganic salts, N2 supplement (Invitrogen), B27 supplement (Invitrogen), GSK-3 alpha/beta inhibitor, and growth factors such as VEGF. Examples of the medium that contains these additives in advance include a medium for primate ES/iPS cells (ReproCELL, Japan), Stem Pro (trademark) (Invitrogen), and a growth medium for vascular endothelial cells (Lonza). Examples of preferable media in the present invention include a medium for primate ES/iPS cells in step (1), a medium for primate ES/iPS cells supplemented with the N2 supplement, the B27 supplement, and the GSK-3 alpha/beta inhibitor in step (2), Stem Pro (trademark) containing VEGF in step (3), and a growth medium for vascular endothelial cells in step (5).

Examples of the GSK-3 alpha/beta inhibitor include SB216763, SB415286, FRAT1/FRAT2, Lithium, Kempaullone, Alsterpaullone, Indiubin-3'-oxime, BIO, TDZD-8, and Ro31-8220.

The temperature for culture ranges from about 30 degrees C to 40 degrees C and is preferably about 37 degrees C, but the examples are not limited thereto. Culture is carried out under atmosphere containing CO₂. CO₂ concentration preferably ranges from about 2 to 5%. The time for culture is not particularly limited and ranges from 1 to 2 days and more preferably 1 day in step (1), ranges from 2 to 5 days and more preferably 3 days in step (2), ranges from 3 to 7 days and more preferably 5 days in step (3), and ranges from 3 or more days in step (5), for example.

VEGFR2-postive, TRA1-negative, and VE-cadherin-positive cells can be separated by a method known by persons skilled in the art from cells stained with anti-VEGFR2, anti-TRA1, and anti-VE-cadherin antibodies using a flow cytometer or the like.

Here, in the case of vascular endothelial cells prepared as described above from iPS cells formed from subjects suffered from polycystic kidney disease and having cerebral aneurysm as a complication, the expression levels of NTNG1, POSTN, TNC, KALI, and BST1 are lower and the expression levels of ACAT2, INSIG1, and SCD are higher than those in vascular endothelial cells prepared from iPS cells derived from a healthy subject not affected by polycystic kidney disease.

Moreover, in the case of vascular endothelial cells prepared as described above from iPS cells formed from subjects suffered from polycystic kidney disease and having cerebral aneurysm as a complication, the expression levels of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 are higher than those in vascular endothelial cells prepared from iPS cells formed from subjects suffered from polycystic kidney disease but not affected by cerebral aneurysm.

### <Method of inducing differentiation of vascular mural cells>

For production of vascular mural cells, an induction differentiation method that can be used herein comprises the steps analogous to the above steps (1) to (3) for preparation of vascular endothelial cells, followed by steps (4') and (5'):
(1) performing adhesion culture using a medium for primate ES/iPS cells on a coated culture dish;
(2) adding various additives to the medium and culturing cells;
(3) adding a growth factor to a serum free medium and culturing cells;
(4') separating VEGFR2-positive, TRA1-negative, and VE-cadherin-negative cells; and
(5') performing adhesion culture using a medium containing a growth factor on a coated culture dish.

In the present invention the vascular mural cells are involved of pericytes or smooth muscle cells. Vascular mural cells in the present invention are preferably spindle-shaped cells expressing vascular mural cell markers such as alpha smooth muscle actin and calponin.

A medium used in step (5') can be prepared using a medium used for culturing animal cells asa basal medium. Examples of the basal medium include IMDM, Medium 199, Eagle's Minimum Essential Medium (EMEM or MEM), alpha MEM, Doulbecco's modified Eagle's Medium (DMEM), Ham's F12medium, an RPMI 1640 medium, Fischer's medium, and mixtures thereof. Furthermore, such a medium may contain serum or may be serum free. If necessary, for example, a medium may contain one or more serum substitutes, such as albumin, transferrin, Knockout Serum Replacement (KSR) (i.e., a substitute for FBS upon culture of ES cells), fatty acids, insulin, a collagen progenitor, trace elements, 2-mercaptoethanol, 3'-thiolglycerol, as well as, one or more substances such as lipids, amino acids, L-glutamine, Glutamax (Invitrogen), nonessential amino acids, vitamins, antibiotics, antioxidants, pyruvic acid, buffering agents, inorganic salts, N2 supplement (Invitrogen), B27 supplement (Invitrogen), a GSK-3 alpha/beta inhibitor, and growth factors such as PDGF-BB. An example of preferable medium is MEM containing 2-% FCS and PDGF-BB.

The temperature for culture ranges from about 30 degrees C to 40 degrees C and is preferably about 37 degrees C, but the examples are not limited thereto. Culture is carried out under atmosphere containing CO₂. CO₂ concentration preferably ranges from about 2 to 5%. The time for culture is not particularly limited and is 3 days or more in the step (5'), for example.

VEGFR2-positive, TRA1-negative, and VE-cadherin-negative cells can be separated by a method known by persons skilled in the art from cells stained with anti-VEGFR2, anti-TRAl, and anti-VE-cadherin antibodies using a flow cytometer or the like.

Here, in the case of vascular mural cells produced as described above from iPS cells formed from somatic cells of subjects suffered from polycystic kidney disease and having cerebral aneurysm as a complication, the expression levels of HSD3B1, KRT7, USP40, and SULT1E1 are lower than those in iPS cells formed from a healthy subject not affected by polycystic kidney disease.

Moreover, in the case of vascular mural cells prepared as described above from iPS cells formed from somatic cells of subjects suffered from polycystic kidney disease and having cerebral aneurysm as a complication, the expression levels of MMP1, TFPI2, HMGA2, KRTAP4-7, KRTAP4-8, KRTAP4-9, MYPN, RPPH1, and SIAE are higher than those in vascular mural cells prepared from iPS cells formed from somatic cells of subjects suffered from polycystic kidney disease but not affected by cerebral aneurysm.

### <Screening method>

Described herein is a method of screening for a candidate drug that is useful for treating or preventing polycystic kidney disease or a complication of polycystic kidney disease. Through the use of such a screening method using the expression levels of the genes listed in Table 1 as indicators, the therapeutic agent(s) or the preventive agent(s) can be identified. The expression levels of the genes listed in Table 1 (above) can be measured using the above disease markers.

The method of screening for a therapeutic agent or a preventive agent for polycystic kidney disease can comprise the following steps of:
(A-1) bringing each of candidate substances into contact with a somatic cell obtained by differentiation induction from iPS cells formed from a somatic cell of a subject suffered from polycystic kidney disease;
(B-1) measuring the expression levels or the transcriptional activity of 1 or more genes selected from or all genes of a group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE; and
(C-1) selecting a candidate substance as a therapeutic agent or a preventive agent for polycystic kidney disease when the expression levels or transcriptional activity of 1 or more genes selected from or all genes of a group consisting of NTNG1, POSTN, TNC, KALI, BST1, HSD3B1, KRT7, USP40, and SULT1E1 exhibit an increase compared with a case in which the candidate substance is not brought into contact, or, selecting a candidate substance as a therapeutic agent or a preventive agent for polycystic kidney disease when the expression levels or transcriptional activity of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM,PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE exhibit a decrease compared with a case in which the candidate substance is not brought into contact.

Here, examples of somatic cells obtained via differentiation induction from iPS cells include tubular cells, collecting tubule cells, bile duct cells, hepatocytes, pancreatic ductal cells, pancreatic cells, bowel cells, germ cells, vascular endothelial cells, and vascular mural cells. Preferable examples thereof include vascular endothelial cells and vascular mural cells. Methods for producing tubular cells, collecting tubule cells, bile duct cells, hepatocytes, pancreatic ductal cells, pancreatic cells, bowel cells, germ cells, vascular endothelial cells, or vascular mural cells from iPS cells are not particularly limited. Somatic cells as used herein can be obtained by appropriately extracting from embryoid bodies or formed teratomas (e.g., JP Patent Publication (Kokai) No. 2006-239169 A). Hepatocytes are not particularly limited and can be prepared by the method according to WO2006/082890, JP Patent Publication (Kokai) No. 2010-75631

A or Hay DC, et al, Proc Natl Acad Sci U.S.A., 105, 12301-6, 2008. Similarly, pancreatic cells can be prepared using the method according to WO2007/103282. iPS cells and vascular endothelial cells or vascular mural cells can be produced by the above methods.

Preferably, the method of screening for a therapeutic agent or a preventive agent for polycystic kidney disease uses vascular endothelial cells and can comprise the following steps of:
(A-2) bringing each of candidate substances into contact with a vascular endothelial cell obtained via differentiation induction from iPS cells formed from a subject suffered from polycystic kidney disease;
(B-2) measuring the expression levels or the transcriptional activity of a gene(s) selected from among NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2; and
(C-2) selecting a candidate substance as a therapeutic agent or a preventive agent for polycystic kidney disease when the expression levels or the transcriptional activity of 1 or more genes selected from or all genes of a group consisting of NTNG1, POSTN, TNC, KALI, and BST1 exhibit an increase compared with a case in which the candidate substance is not brought into contact, or, selecting a candidate substance as a therapeutic agent or a preventive agent for polycystic kidney disease when the expression levels or the transcriptional activity of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 exhibits a decrease compared with a case in which the candidate substance is not brought into contact.

Alternatively, the method using vascular mural cells can comprise the following steps of:
(A-3) bringing each of candidate substances into contact with vascular mural cells obtained via differentiation induction from iPS cells formed from a somatic cell of a subject suffered from polycystic kidney disease;
(B-3) measuring the expression levels or the transcriptional activity of 1 or more genes selected from or all genes of a group consisting of HSD3B1, KRT7, USP40, SULT1E1, MMP1, TFPI2, HMGA2, KRTAP4-7, KRTAP4-8, KRTAP4-9, MYPN, RPPH1, and SIAE; and
(C-3) selecting a candidate substance as a therapeutic agent or a preventive agent for polycystic kidney disease when the expression levels or the transcriptional activity of 1 or more genes selected from or all genes of a group consisting of HSD3B 1, KRT7, USP40, and SULT1E1 exhibit an increase compared with a case in which the candidate substance is not brought into contact, or, selecting a candidate substance as a therapeutic agent or a preventive agent for polycystic kidney disease when the expression levels or the transcriptional activity of 1 or more genes selected from or all genes of a group consisting of MMP1, TFPI2, HMGA2, KRTAP4-7, KRTAP4-8, KRTAP4-9, MYPN, RPPH1, and SIAE exhibit a decrease compared with a case in which the candidate substance is not brought into contact.

The method of screening for a therapeutic agent or a preventive agent for a complication of polycystic kidney disease can comprise the following steps of:
(A-4) bringing each of candidate substances into contact with a somatic cell obtained via differentiation induction from iPS cells formed from a somatic cell of a patient with a complication of polycystic kidney disease;
(B-4) measuring the expression levels or the transcriptional activity of 1 or more genes selected from or all genes of a group consisting of NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE; and
(C-4) selecting a candidate substance as a therapeutic agent or a preventive agent for a complication of polycystic kidney disease when the expression levels or the transcriptional activity of 1 or more genes selected from or all genes of a group consisting of NTNG1, POSTN, TNC, KALI, BST1, HSD3B1, KRT7, USP40, and SULT1E1 exhibit an increase compared with a case in which the candidate substance is not brought into contact, or, selecting a candidate substance as a therapeutic agent or a preventive agent for a complication of polycystic kidney disease when the expression levels or the transcriptional activity of 1 or more genes selected from or all genes of a group consisting of ACAT2, INSIG1, SCD, BMP6, CD274, CTGF, E2F7, EDN1,FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE exhibit a decrease compared with a case in which the candidate substance is not brought into contact.

In the present invention, examples of a complication of polycystic kidney disease include vascular lesions such as aortic aneurysm, cerebral aneurysm, or subarachnoid hemorrhage, valvular disease of heart, diverticula of colon, hernia, ductal lesions such as choledochal dilatation, as well as cyst formation in the liver, the pancreas, the spleen and generative organs. An example of a complication to be particularly detected is cerebral aneurysm.

Preferably, the method of screening for a therapeutic agent or a preventive agent for a complication of polycystic kidney disease uses vascular endothelial cells and comprises the following steps of:
(A-5) bringing each of candidate substances into contact with a vascular endothelial cell obtained via differentiation induction from iPS cells formed from a somatic cell of a patient with a complication of polycystic kidney disease;
(B-5) measuring the expression levels or the transcriptional activity of 1 or more genes selected from or all genes of a group consisting of NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2; and
(C-5) selecting a candidate substance as a therapeutic agent or a preventive agent for a complication that accompanies polycystic kidney disease when the expression levels or the transcriptional activity of 1 or more genes selected from or all genes of a group consisting of NTNG1, POSTN, TNC, KALI, and BST1 exhibit an increase compared with a case in which the candidate substance is not brought into contact, or, selecting a candidate substance as a therapeutic agent or a preventive agent for a complication that accompanies polycystic kidney disease when the expression levels or the transcriptional activity of 1 or more genes selected from or all genes of a group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 exhibit a decrease compared with a case in which the candidate substance is not brought into contact.

More preferably, the method of screening for a therapeutic agent or a preventive agent for cerebral aneurysm that accompanies polycystic kidney disease uses vascular endothelial cells and comprises the following steps of:
(A-6) bringing candidate substances into contact with vascular endothelial cells obtained via differentiation induction from iPS cells formed from a patient with a complication of polycystic kidney disease,
(B-6) measuring the expression levels or the transcriptional activity of 1 or more genes selected from or all genes of a group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2, and
(C-6) selecting a candidate substance as a therapeutic agent or a preventive agent for cerebral aneurysm that accompanies polycystic kidney disease when the expression levels or the transcriptional activity of 1 or more genes selected from or all genes of a group consisting of BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2 exhibits a decrease compared with a case in which the candidate substance is not brought into contact.

Alternatively, the relevant method uses vascular mural cells and can comprise the following steps of:
(A-7) bringing each of candidate substances into contact with vascular mural cells obtained via differentiation induction from iPS cells formed from a somatic cell of a subject with a complication of polycystic kidney disease;
(B-7) measuring the expression levels or the transcriptional activity of 1 or more genes selected from or all genes of a group consisting of HSD3B1, KRT7, USP40, SULT1E1, MMP1, TFPI2, HMGA2, KRTAP4-7, KRTAP4-8, KRTAP4-9, MYPN, RPPH1, and SIAE; and
(C-7) selecting a candidate substance as a therapeutic agent or a preventive agent for a complication that accompanies polycystic kidney disease when the expression levels or the transcriptional activity of 1 or more genes selected from or all genes of a group consisting of HSD3B1, KRT7, USP40, and SULT1E1 exhibit an increase compared with a case in which the candidate substance is not brought into contact, or, selecting a candidate substance as a therapeutic agent or a preventive agent for a complication that accompanies polycystic kidney disease when the expression levels or the transcriptional activity of 1 or more genes selected from or all genes of a group consisting of MMP1, TFPI2, HMGA2, KRTAP4-7, KRTAP4-8, KRTAP4-9, MYPN, RPPH1, and SIAE exhibit a decrease compared with a case in which the candidate substance is not brought into contact.

Preferably, the method of screening for a therapeutic agent or a preventive agent for cerebral aneurysm that accompanies polycystic kidney disease uses vascular mural cells and can comprise the following steps of:
(A-8) bringing each of candidate substances into contact with vascular mural cells obtained via differentiation induction of iPS cells formed from a somatic cell of a subject with a complication of polycystic kidney disease;
(B-8) measuring the expression levels or the transcriptional activity of 1 or more genes selected from or all genes of a group consisting of MMP1, TFPI2, HMGA2, KRTAP4-7, KRTAP4-8, KRTAP4-9, MYPN, RPPH1, and SIAE; and
(C-8) selecting a candidate substance as a therapeutic agent or a preventive agent for a complication that accompanies polycystic kidney disease when the expression levels or the transcriptional activity of 1 or more genes selected from or all genes of a group consisting of MMP1, TFPI2, HMGA2, KRTAP4-7, KRTAP4-8, KRTAP4-9, MYPN, RPPH1, and SIAE exhibit a decrease compared with a case in which the candidate substance is not brought into contact.

Here, the above expression levels can be detected using the above disease markers.

The above transcriptional activity can be detected using a reporter gene that is controlled by the transcriptional regulatory region thereof. Specifically, the method can comprise the following steps of:
(A-9) bringing each of candidate substances into contact with a somatic cell obtained via differentiation induction from iPS cells formed from a somatic cell of a subject suffered from polycystic kidney disease, wherein the somatic cell contain a reporter gene that is controlled by the transcriptional regulatory region of 1 or a plurality of genes selected from among NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE;
(B-9) measuring the expression or the activity of the reporter gene; and
(C-9) selecting a candidate substance that increases the expression level or the activity level of the reporter gene compared with the expression level in the absence of the candidate substance, when the selected gene(s) is NTNG1, POSTN, TNC, KALI, BST1, HSD3B1, KRT7, USP40 and/or SULT1E1, or, selecting a candidate substance that decreases the expression level of the reporter gene when the selected gene(s) is ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1 and/or SIAE compared with the expression level detected in the absence of the candidate substance.

Preferably, the relevant method uses vascular endothelial cells as somatic cells obtained via differentiation induction from iPS cells and can comprise the following steps of:
(A-10) bringing each of candidate substances into contact with a vascular endothelial cell obtained via differentiation induction from iPS cells formed from a somatic cell of a subject suffered from polycystic kidney disease, wherein the vascular endothelial cell contains a reporter gene that is controlled by the transcriptional regulatory region of 1 or a plurality of genes selected from among NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2;
(B-10) measuring the expression or the activity of the reporter gene; and
(C-10) selecting a candidate substance that increases the expression level or the activity level of the reporter gene compared with the expression level in the absence of the candidate substance when the selected gene(s) is NTNG1, POSTN, TNC, KALI and/or BST1, or selecting a candidate substance that decreases the expression level of the reporter gene compared with the expression level detected in the absence of the candidate substance, when the selected gene(s) is ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1 and/or TFPI2.

Alternatively, the method uses vascular mural cells and can comprise the following steps of:
(A-11) bringing each of candidate substances into contact with vascular mural cells obtained via differentiation induction from iPS cells of a subject suffered from polycystic kidney disease, wherein the vascular mural cells contain a reporter gene that is controlled by the transcriptional regulatory region of 1 or a plurality of genes selected from among HSD3B1, KRT7, USP40, SULT1E1, MMP1, TFPI2, HMGA2, KRTAP4-7, KRTAP4-8, KRTAP4-9, MYPN, RPPH1 and/or SIAE;
(B-11) measuring the expression or the activity of the reporter gene; and
(C-11) selecting a candidate substance that increases the expression level or the activity level of the reporter gene compared with the expression level detected in the absence of the candidate substance when the selected gene(s) is HSD3B1, KRT7, USP40 and/or SULT1E1, or selecting a candidate substance that decreases the expression level of the reporter gene compared with the expression level detected in the absence of the candidate substance when the gene is MMP1, TFPI2, HMGA2, KRTAP4-7, KRTAP4-8, KRTAP4-9, MYPN, RPPH1 and/or SIAE.

The method of screening for a therapeutic agent or a preventive agent fora complication of polycystic kidney disease can comprise the following steps of:
(A-12) bringing each of candidate substances into contact with somatic cells obtained via differentiation induction from iPS cells formed from a somatic cell of a subject with a complication of polycystic kidney disease, wherein the somatic cells contain a reporter gene that is controlled by the transcriptional regulatory region of 1 or a plurality of genes selected from among NTNG1, POSTN, TNC, KALI, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1, and SIAE;
(B-12) measuring the expression or the activity of the reporter gene; and
(C-12) selecting a candidate substance that increases the expression level or the activity level of the reporter gene compared with the expression level in the absence of the candidate substance when the selected gene(s) is NTNG1, POSTN, TNC, KALI, BST1, HSD3B1, KRT7, USP40 and/or SULT1E1, or selecting a candidate substance that decreases the expression level of the reporter gene compared with the expression level detected in the absence of the candidate substance when the selected gene(s) is ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, TFPI2, HMGA2, KRTAP4-7, KRTAP4-8, KRTAP4-9, MYPN, RPPH1 and/or SIAE.

Preferably, the method uses vascular endothelial cells as somatic cells obtained via differentiation induction from iPS cells and can comprise the following steps of:
(A-13) bringing each of candidate substances into contact with vascular endothelial cells obtained via differentiation induction from iPS cells formed from a somatic cell of a subject with a complication of polycystic kidney disease, wherein the endothelial cells contain a reporter gene that is controlled by the transcriptional regulatory region of 1 or a plurality of genes selected from among NTNG1, POSTN, TNC, KALI, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2;
(B-13) measuring the expression or the activity of the reporter gene; and
(C-13) selecting a candidate substance that increases the expression level or the activity level of the reporter gene compared with the expression level in the absence of the candidate substance when the selected gene(s) is NTNG1, POSTN, TNC, KALI and/or BST1, or selecting a candidate substance that decreases the expression level of the reporter gene compared with the expression level detected in the absence of the candidate substance when the selected gene(s) is ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1 and/or TFPI2.

More preferably, the method uses vascular endothelial cells as somatic cells obtained via differentiation induction from iPS cells and comprises the following steps of:
(A-14) bringing each of candidate substances into contact with a vascular endothelial cell obtained via differentiation induction from iPS cells formed from a somatic cell of a subject suffered from polycystic kidney disease who also develops cerebral aneurysm as a complication, wherein the endothelial cells contain a reporter gene that is controlled by the transcriptional regulatory region of 1 or a plurality of genes selected from among BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and TFPI2;
(B-14) measuring the expression or the activity of the reporter gene; and
(C-14) selecting a candidate substance that decreases the expression level of the reporter gene compared with the expression level detected in the absence of the candidate substance when the selected gene(s) is BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, MMP1, and/or TFPI2.

Alternatively, the method uses vascular mural cells and can comprise the following steps of:
(A-15) bringing each of candidate substances into contact with vascular mural cells obtainedvia differentiation induction from iPS cells formed from a somatic cell of a subject with a complication of polycystic kidney disease, wherein the vascular mural cells contain a reporter gene that is controlled by the transcriptional regulatory region of 1 or a plurality of genes selected from among HSD3B1, KRT7, USP40, SULT1E1, MMP1, TFPI2, HMGA2, KRTAP4-7, KRTAP4-8, KRTAP4-9, MYPN, RPPH1, and SIAE;
(B-15) measuring the expression or the activity of the reporter gene; and
(C-15) selecting a candidate substance that increases the expression level or the activity level of the reporter gene compared with the expression level in the absence of the candidate substance when the selected gene(s) is HSD3B1, KRT7, USP40 and/or SULT1E1, or selecting a candidate substance that decreases the expression level of the reporter gene compared with the expression level detected in the absence of the candidate substance when the selected gene(s) is MMP1, TFPI2, HMGA2, KRTAP4-7, KRTAP4-8, KRTAP4-9, MYPN, RPPH1 and/or SIAE.

Preferably, the method uses vascular mural cells as somatic cells obtained via differentiation induction from iPS cells and can comprise the following steps of:
(A-16) bringing each of candidate substances into contact with vascular mural cells obtained via differentiation induction from iPS cells formed from a somatic cell of a subject suffered from polycystic kidney disease who also develops cerebral aneurysm as a complication, wherein the vascular mural cells contain a reporter gene that is controlled by the transcriptional regulatory region of 1 or a plurality of genes selected from among MMP1, TFPI2, HMGA2, KRTAP4-7, KRTAP4-8, KRTAP4-9, MYPN, RPPH1, and SIAE;
(B-16) measuring the expression or the activity of the reporter gene; and
(C-16) selecting a candidate substance that decreases the expression level of the reporter gene compared with the expression level detected in the absence of the candidate substance when the selected gene(s) is MMP1, TFPI2, HMGA2, KRTAP4-7, KRTAP4-8, KRTAP4-9, MYPN, RPPH1 and/or SIAE.

The transcriptional regulatory regions of the genes listed in Table 1 (see above) can be isolated from the genomic libraries based on the nucleotide sequence information of the genes. Cells containing a reporter gene that is controlled by the transcriptional regulatory region of the gene can be prepared by introducing a vector containing the reporter gene sequence operably linked to the transcriptional regulatory region sequence into cells. With the use of the homologous recombination method, a reporter gene sequence may be inserted downstream of the transcriptional regulatory region by a method known by persons skilled in the art, so that it is operably linked thereto.

The above vector introduction and homologous recombination method may be performed in any of cells including somatic cells, iPS cells, vascular endothelial cells, and vascular mural cells. The homologous recombination method is desirably performed using iPS cells.

Reporter genes known in the art can be used as appropriate reporter genes, and include, but are not particularly limited, green fluorescent protein (GFP), yellow fluorescent protein (YFP), red fluorescent protein (RFP), luciferase, beta glucuronidase (GUS), beta-galactosidase, HRP, chloramphenicol acetyltransferase, or the like.

In the screening method described herein, arbitrary candidate substances can be used. Examples of candidate substances include, but are not limited to, cell extracts, cell culture supernatants, microbial fermentation products, marine organism-derived extracts, plant extracts, purified proteins or crude proteins, peptides, non-peptide compounds, synthetic low-molecular-weight compounds, and natural compounds.

Candidate substances can also be obtained using any one of many approaches in combinatorial library methods known in the art including (1) a biological library method, (2) a synthetic library method using deconvolution, (3) a "one-bead one-compound" library method, and (4) a synthetic library method using affinity chromatography selection. An example of the biological library method using affinity chromatography selection is limited to a peptide library method, however, the other 4 approaches can be applied to peptides, non-peptide oligomers, or low-molecular-weight compound libraries (Lam (1997) Anticancer Drug Des. 12: 145-67). Examples of a method for synthesizing a molecular library can be found in the art (DeWitt et al. (1993) Proc. Natl. Acad. Sci. U.S.A. 90: 6909-13; Erb et al. (1994) Proc. Natl. Acad. Sci. U.S.A. 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994)J. Med. Chem. 37: 1233-51). Compound libraries can be constructed in the form of solutions (see Houghten (1992) Bio/Techniques 13: 412-21) or beads (Lam (1991) Nature 354: 82-4), chips (Fodor (1993) Nature 364: 555-6), bacteria (U.S. Patent No. 5,223,409), spores (U.S. Patent No. 5,571,698, U.S. Patent No. 5,403,484, and U.S. Patent No. 5,223,409), plasmids (Cull et al. (1992) Proc. Natl. Acad. Sci. U.S.A. 89: 1865-9), or phages (Scott and Smith (1990) Science 249: 386-90; Devlin (1990) Science 249: 404-6; Cwirla et al. (1990) Proc. Natl. Acad. Sci. U.S.A. 87: 6378-82; Felici (1991) J. Mol. Biol. 222: 301-10; U.S. Patent Application No. 2002103360).

### <Antisense oligonucleotide, or siRNA or shRNA, and therapeutic agent produced using them>

An example of an antisense oligonucleotide in the present disclosure is an oligonucleotide hybridizing to a site within the nucleotide sequence of ACAT2, INSIG1, SCD, BMP6, CD274, CTGF,E2F7, EDN1, FAM43A, FRMD3, MMP10, MYEOV, NR2F1, NRCAM, PCSK1, PLXNA2, SLC30A3, SNAI1, SPOCD1, MMP1, TFPI2 HMGA2, KRTAP4-7, KRTAP4-8, KRTAP4-9, MYPN, RPPH1, or SIAE. Such an antisense oligonucleotide has a nucleotide sequence complementary to the nucleotide sequence comprising at least 15 continuous nucleotides (e.g., 20 to 200 continuous nucleotides) in the nucleotide sequence of preferably SEQ ID NO: 11, 13, 45, 47, 49, 51, 53, 55, 57, 59, 61, 63, 65, 67, 69, 71, 73, 75, 77, 79, 81, 83, 85, 87, 89, 91, 93, or 94. A further preferable antisense oligonucleotide contains an initiation codon in the above at least 15 continuous nucleotides.

A derivative or a modification product of the above antisense oligonucleotide can be used as an antisense oligonucleotide. Examples of such modification include a methyl-phosphonate-type or an ethyl-phosphonate-type lower alkyl phosphonate modification, phosphorothioate modification, and phosphoramidite modification.

In the present disclosure, the nucleotide sequence of siRNA or shRNA can be designed using an siRNA design computer program that is available from www.ambion.com/techlib/ misc/siRNA_finder.html. The computer program is used for selecting a nucleotide sequence for siRNA or shRNA synthesis based on the following protocols.

An siRNA or shRNA target site can be selected as follows.
(1) A downstream portion from the AUG initiation codon as a starting point of ACAT2 (SEQ ID NO: 45), INSIG1 (SEQ ID NO: 11), SCD (SEQ ID NO: 13), BMP6 (SEQ ID NO: 47), CD274 (SEQ ID NO: 49), CTGF (SEQ ID NO: 51), E2F7 (SEQ ID NO: 53), EDN1(SEQ ID NO: 55), FAM43A (SEQ ID NO: 57), FRMD3 (SEQ ID NO: :59), MMP10 (SEQ ID NO: 61), MYEOV (SEQ ID NO: 63), NR2F1(SEQ ID NO: 65), NRCAM(SEQ ID NO: 67), PCSK1 (SEQ ID NO: 69), PLXNA2 (SEQ ID NO: 71), SLC30A3 (SEQ ID NO:73), SNAI1 (SEQ ID NO: 75), SPOCD1 (SEQ ID NO: 77), MMP1 (SEQ ID NO: 79), TFPI2 (SEQ ID NO: 81), HMGA2 (SEQ ID NO: 83), KRTAP4-7 (SEQ ID NO: 85), KRTAP4-8 (SEQ ID NO: 87), KRTAP4-9 (SEQ ID NO: 89), MYPN (SEQ ID NO: 91), RPPH1 (SEQ ID NO: 93), or SIAE (SEQ ID NO: 94) is scanned for an AA dinucleotide sequence. As a useful siRNA or shRNA target site, 19 nucleotides adjacent to the 3' side, or a site in which each AA appears, is used as a candidate sequence. According to Tuschl, et al. (1999) Genes Dev 13: 3191-7, the design of siRNA or shRNA for the 5'-untranslated region (UTR) and the 3'-UTR and a region (within 75 nucleotides) near the initiation codon is not recommended since an UTR binding protein and/or a translation initiation complex interferes with the binding of an siRNA endonuclease complex.
(2) A target site and a human genome data base are compared, so as to exclude all target sequences having significant sequence identity with other coding sequences. Sequence identity can be examined using BLAST (Altschul SF, et al, (1997) Nucleic Acids Res. 25 (17): 3389-402or (1990) J Mol Biol. 215 (3): 403-10) found on the NCBI server, ncbi.nlm.nih.gov/BLAST/.
(3) A target sequence appropriate for synthesis is selected.

A sense strand comprising the thus selected siRNA target site may form double stranded RNA through hybridization with a corresponding antisense strand. Single-stranded RNA in which the sense strand and the antisense strand corresponding thereto are linked via a loop sequence, i.e. shRNA, can form a hair-pin loop structure. Such double stranded RNA may contain mismatch of 1 (or less) nucleotide per 10 nucleotides. Preferably, strands of a double stranded complex are completely complementary to each other, containing no mismatch. The present disclosure also describes a vector containing 1 or a plurality of nucleic acids of the above sense strand and cells containing the vector.

As an siRNA composition, a vector comprising a sequence (shRNA) ligated to comprise a hair-pin loop structure comprising a sense strand, an antisense strand, or both strands, so as to be transcribed by an RNA polymerase III transcriptional unit (e.g., an intranuclear low molecular weight RNA (snRNA) U6 promoter, or a human HI RNA promoter) can be used. Alternatively, double stranded RNA may be directly used.

Double stranded RNA may be chemically stabilized in order to prevent in vivo degradation by nuclease. A method of preparing chemically stabilized RNA molecules is known in the art.

Typically, such a molecule contains a backbone modified to be able to avoid the action of ribonuclease and nucleotides. Another example of modification is cholesterol conjugated siRNA (Song et al, (2003) Nature Med. 9: 347-51). An siRNA composition or an shRNA composition may further contain liposomes (e.g., cationic liposomes or anionic liposomes), nanoparticles, or a viral vector including retrovirus, adenovirus, or adeno-associated virus. Moreover, an siRNA composition or an shRNA composition may contain a pharmaceutically acceptable carrier such as physiological saline.

The above siRNA composition or shRNA composition can be used for treatment of polycystic kidney disease or a complication of polycystic kidney disease through parenteral administration such as, an intravenous, subcutaneous, intramuscular, or intraperitoneal administration route. The above siRNA composition or shRNA composition may be directly injected to an affected portion.

The dose of an antisense oligonucleotide, siRNA, or shRNA depends on many factors including body weight, age, gender, administration time, and the route of administration, symptoms, and other medicaments to be administered simultaneously. The dose of the above drug for intravenous administration desirably ranges from about 10⁶ to 10²² copies.

### Examples

The present invention will hereafter be described in more detail with reference to the following examples, although the technical scope of the present invention is not limited thereto.

### Example 1

### <Establishment of iPS cell lines>

### <Fibroblast cells>

Fibroblasts were established by culturing skin samples obtained via biopsies from four autosomal dominant polycystic kidney disease patients with onset of cerebral aneurysm as a complication and from three autosomal dominant polycystic kidney disease patients not developing cerebral aneurysm under agreement. The resultants were each designated PK-ane fibroblasts and PK-free fibroblasts, respectively, and then used in this Example. Meanwhile, a fibroblast cell line of three healthy persons not developing autosomal dominant polycystic kidney disease and cerebral aneurysm is designated nonPK fibroblast and then used in this Example.

### <iPS cell induction>

Human cDNAs corresponding to Oct3/4, Sox2, Klf4, and c-Myc were introduced into the above fibroblasts using retrovirus according to the method described by Takahashi, K. et al. (Cell, 131(5), 861, 2007). Similarly, human cDNAs corresponding to Oct3/4, Sox2, and Klf4 were introduced into the above fibroblasts using a retrovirus according to the method as described by Nakagawa, M. et al. (Nat Biotechnol 26 (1), 101, 2008). After gene introduction, fibroblasts were transferred onto SNL feeder cells, followed by, on the next day, medium exchange with culture solutions (for primate ES cells) supplemented with 4 ng/ml bFGF (Wako). Colonies that had appeared were picked up, so that one iPS cell line was selected per fibroblast cell line. The thus selected PK-ane fibroblast-derived iPS cell lines were designated PK-ane-iPS1, PK-ane-iPS2, PK-ane-iPS3, and PK-ane-iPS4; PK-free fibroblast-derived iPS cell lines were designated PK-free-iPS1, PK-free-iPS2, and PK-free-iPS3; as well as nonPK fibroblast-derived iPS cell lines were designated nonPK-iPS1, nonPK-iPS2, and nonPK-iPS3. Here, PK-ane-iPS1, PK-ane-iPS2, PK-ane-iPS4, PK-free-iPS2, PK-free-iPS3, nonPK-iPS2, and nonPK-iPS3 were prepared by introduction of four factors (Oct3/4, Sox2, Klf4, and c-Myc). PK-ane-iPS3, PK-free-iPS1, and nonPK-iPS1 were prepared by introduction of three factors (Oct3/4, Sox2, and Klf4).

### Example 2

### <Induction of differentiation into vascular endothelial cells>

Each iPS cell line colony prepared as described in Example 1 was separated into pieces with an appropriate size, sprayed over a type-I collagen coating dish (Becton Dickinson), followed by 1 day of culture in a medium for primate ES/iPS cells (ReproCELL) to adhere the cells to the dish surface. On day 2, a GSK-3 alpha/beta inhibitor (Sigma), N2 supplement, and B27 supplement (both, Invitrogen) were added and then cells were cultured for further 3 days. Then the medium was exchanged with a serum free medium for human hematopoietic stem cells (Invitrogen), and then 50 ng/ ml VEGF (Peprotec Inc) was added. After further 5 days of culture, cells were dissociated, and then VEGFR2-positive, TRA1-negative, and VE-cadherin-positive cells were separated by FACS. Subsequently, the separated cells were sprayed over type-IV collagen coating dishes (Becton Dickinson), and then cultured in a growth medium for vascular endothelial cells (Lonza). At the stage where vascular endothelial cell markers such as VE-cadherin, CD31, CD34, and eNOS were expressed and vascular endothelial cell sheets having a cobblestoned appearance were constructed, cells were collected as vascular endothelial cells (EC). EC cells prepared from iPS cell lines were designated PK-ane-iPS1-EC, PK-ane-iPS2-EC, PK-ane-iPS3-EC, PK-ane-iPS4-EC, PK-free-iPS1-EC, PK-free-iPS2-EC, PK-free-iPS3-EC, nonPK-iPSl-EC, nonPK-iPS2-EC, and nonPK-iPS3-EC, respectively.

### Example 3

### <Induction of differentiation into vascular mural cells>

Each of the above prepared iPS cell line colonies was separated into pieces with an appropriate size, sprayed over a type-I collagen coating dish (Becton Dickinson), followed by 1 day of culture with a medium for primate ES/iPS cells (ReproCELL) to adhere the cells to the dish surface. On day 2, a GSK-3 alpha/beta inhibitor (Sigma), N2 supplement, and B27 supplement (both, Invitrogen) were added, and then cells were cultured for further 3 days. Then the medium was exchanged with a serum free medium for human hematopoietic stem cells (Invitrogen). After 5 days of culture, cells were dissociated, and then VEGFR2-positive, TRA1-negative, and VE-cadherin-negative cells were separated by FACS. Subsequently, the thus separated cells were sprayed over a type-IV collagen coating dish (Becton Dickinson) and further cultured in MEM containing 2-% FCS and 20 ng/ml PDGF-BB (Peprotech Inc). Thus, cells were induced to differentiate into vascular mural cells (MC) expressing vascular mural cell markers such as alpha smooth muscle actin and calponin and presenting spindle shapes and then collected. MC cells prepared from iPS cell lines were designated PK-ane-iPS1-MC, PK-ane-iPS2-MC, PK-ane-iPS3-MC, PK-ane-iPS4- MC, PK-free-iPS1-MC, PK-free-iPS2-MC, PK-free-iPS3-MC, nonPK-iPSl-MC, nonPK-iPS2-MC, and nonPK-iPS3-MC.

### Example 4

### <Confirmation of each gene expression>

The expression levels of NTNG1, POSTN, TNC, KALI, BST1, INSIG1, SCD, and ACAT2 in PK-ane-iPS1-EC, PK-ane-iPS2-EC, PK-ane-iPS3-EC, nonPK-iPSl-EC, nonPK-iPS2-EC, and nonPK-iPS3-EC obtained in Example 2 were measured by RT-PCR using primers listed in Table 2. The expression level of each gene in PK-iPS cell-derived EC was compared with that in nonPK-iPS cell-derived EC. Thus, it was confirmed that PK-ane-iPS cell-derived EC expressed NTNG1, POSTN, TNC, KALI, and BST1 at low levels but expressed INSIG1, SCD, and ACAT2 at high levels (Fig. 1). Similarly, the expression levels of HSD3B1, KRT7, USP40, and SULT1E1 in PK-ane-iPS1-MC, PK-ane-iPS2-MC, PK-ane-iPS3-MC, nonPK-iPS1-MC, nonPK-iPS2-MC, and nonPK-iPS3-MC obtained in Example 3 were measured by RT-PCR using primers listed in Table 2. The expression level of each gene in PK-iPS cell-derived MC was compared with that in nonPK-iPS cell-derived MC. It was confirmed that PK-iPS cell-derived MC expressed HSD3B1, KRT7, USP40, and SULT1E1 at low levels (Fig. 2).

Subsequently, RNA extracted from PK-ane-iPS1-EC, PK-ane-iPS2-EC, PK-ane-iPS3-EC, PK-ane-iPS4-EC, PK-free-iPS1-EC, PK-free-iPS2-EC, and PK-free-iPS3-EC obtained in Example 2 was subjected to measurement of gene expression intensity using a microarray (Affymetrix). Groups with cerebral aneurysm as a complication and groups without cerebral aneurysm as a complication were compared for all 12 combinations of clones. Gene groups expressed at high levels in all groups with cerebral aneurysm as a complication are shown in Table 3. Also, the ratio of the expression level in PK-ane-iPS1-EC to that in PK-free-iPS1-EC in one case out of the above 12 combinations is shown in Table 3.

### [Table 3]

**Table 3**

| Gene group expressed at high levels in vascular endothelial cells obtained via differentiation induction from iPS cells formed from fibroblast cells of PKD subjects with cerebral aneurysm | |
|---|---|
| Gene Name | Fold Change (PK-ane-iPS1-EC/PK-free-iPS1-EC) |
| SPOCD1 | 2.4045105 |
| PLXNA2 | 2.0494196 |
| MYEOV | 2.5981545 |
| MMP10 | 1.7859154 |
| MMP1 | 8.141003 |
| E2F7 | 3.2456584 |
| SLC30A3 | 2.6372058 |
| SNAI1 | 2.5232387 |
| FAM43A | 1.5843235 |
| NR2F1 | 1.7354009 |
| PCSK1 | 3.1585443 |
| BMP6 | 2.5192003 |
| EDN1 | 3.805765 |
| CTGF | 2.8271823 |
| TFPI2 | 1.6620069 |
| NRCAM | 3.4262772 |
| CD274 | 1.5687742 |
| FRMD3 | 3.1186438 |

Similarly, RNAs extracted from PK-ane-iPS1-MC, PK-ane-iPS2-MC, PK-ane-iPS3-MC, PK-ane-iPS4-MC, PK-free-iPS1-MC, PK-free-iPS2-MC, and PK-free-iPS3-MC obtained in Example 3 were subjected to measurement of gene expression intensity using a microarray (Affymetrix). Groups with cerebral aneurysm as a complication and groups without cerebral aneurysm as a complication were compared for all the 12 combinations of clones. Gene groups expressed at high levels in all groups with cerebral aneurysm as a complication are shown in Table 4. Also, the ratio of the expression level in PK-ane-iPS1-MC to that in PK-free-iPS2-MC in one case out of the above 12 combinations is shown in Table 4.

### [Table 4]

**Table 4**

| Gene group expressed at high levels in vascular mural cells obtained via differentiation induction from iPS cells formed from fibroblast cells of PKD subjects with cerebral aneurysm | |
|---|---|
| Gene Name | Fold Change (PK-ane-iPS1-MC/PK-free-iPS2-MC) |
| MYPN | 1.3425403 |
| SIAE | 2.1190434 |
| MMP1 | 4.1157513 |
| HMGA2 | 1.9827896 |
| RPPH1 | 3.5703707 |
| KRTAP4-7 | 2.1406236 |
| KRTAP4-9 | |
| KRTAP4-8 | |
| TFPI2 | 13.641734 |

Additionally, the expression levels of CD274, CTGF, MMP10, NRCAM, and MMP1 in endothelial cells (ECs) or mural cells (MCs) derived from PK-ane-iPS1 and PK-free-iPS3 obtained in Example 2 or 3 were measured by quantitative PCR using primers listed in Table 2. As the result, the expression levels of the indicated genes in the EC or MC induced from iPS cells with cerebral aneurysm were significantly higher than that without cerebral aneurysm, although there was no difference between the expression levels in undifferentiated cells with and without cerebral aneurysm (Fig. 3). These results were corresponding to above.

### Industrial Applicability

The present invention makes it possible to perform a method of examining autosomal dominant polycystic kidney disease or a complication of autosomal dominant polycystic kidney disease, as well as screening for a remedy for the same. Thus, the present invention is medically very useful.

## Claims

1. A method of examining whether or not a subject has a complication of polycystic kidney disease or a risk of developing the complication, comprising the following steps of:
(a) measuring the expression level of MMP1 gene and measuring the expression levels of 1 or more genes selected from or all genes of the group consisting of NTNG1, POSTN, TNC, KAL1, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE genes in a sample from the subject; and
(b) determining that the subject has the complication of polycystic kidney disease or the risk of developing the complication when the expression level of MMP1 is higher than that in a control sample; and
when the expression levels of 1 or more genes selected from or all genes of the group consisting of ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1, and SIAE genes are higher than those in the control sample, or when the expression levels of 1 or more genes selected from or all genes of the group consisting of NTNG1, POSTN, TNC, KAL1, BST1, HSD3B1, KRT7, USP40, and SULT1E1 genes are lower than those in a control sample;
wherein the complication of polycystic kidney disease is selected from the group consisting of: vascular lesions; valvular disease of heart; diverticula of colon; hernia; ductal lesions; and symptoms of cyst formation in the liver, pancreas, spleen, and generative organ.

2. The method according to claim 1, further comprising:
(a2) measuring the expression levels of all genes of the group consisting of CD274, CTGF, MMP10, and NRCAM in the sample from the subject; and
(b2) determining that the subject has the complication of polycystic kidney disease or the risk of developing the complication when the expression levels of the CD274, CTGF, MMP10, and NRCAM genes are higher than those in the control sample.

3. The method according to any one of claims 1 or 2, wherein the complication is cerebral aneurysm.

4. The method according to any one of claims 1 to 3, wherein the sample is selected from the group consisting of blood, serum, blood plasma, cell extracts, urine, lymph fluids, tissue fluids, ascites, spinal fluids, and other body fluids, or tissues and cells.

5. The method according to any one of claims 1 to 3, wherein the sample is a vascular endothelial cell obtained via differentiation induction from an iPS cell formed from an isolated somatic cell of a subject with polycystic kidney disease.

6. The method according to claim 5 wherein the genes in step (a) further comprise 1 or more genes selected from or all genes of the group consisting of NTNG1, POSTN, TNC, KAL1, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, and TFPI2.

7. The method according to any one of claims 1 to 3, wherein the sample is a vascular mural cell obtained via differentiation induction from an iPS cell formed from an isolated somatic cell of a subject with polycystic kidney disease.

8. The method according to claim 7 wherein the gene in step (a) is 1 or more genes selected from or all genes of the group consisting of HSD3B1, KRT7, USP40, SULT1E1, TFPI2, HMGA2, KRTAP4-7, KRTAP4-8, KRTAP4-9, MYPN, RPPH1, and SIAE.

9. The method according to any of claim 1 to 8, wherein the step of measuring the expression level of the gene is a step of measuring the amount of the mRNA, cRNA, or cDNA of the gene or the amount of the protein encoded by the gene.

## Patentansprüche

1. Verfahren zum Untersuchen, ob ein Individuum eine Komplikation einer polyzystischen Nierenkrankheit hat oder nicht oder einem Risiko für die Entwicklung der Krankheit unterliegt oder nicht, umfassend die folgenden Schritte:
(a) Messen des Expressionsniveaus des MMP1-Gens und Messen der Expressionsniveaus von 1 oder mehr Genen, ausgewählt aus oder von allen Genen der Gruppe, bestehend aus den NTNG1-, POSTN-, TNC-, KAL1-, BST1-, ACAT2-, INSIG1-, SCD-, HSD3B1-, KRT7-, USP40-, SULT1E1-, BMP6-, CD274-, CTGF-, E2F7-, EDN1-, FAM43A-, FRMD3-, MMP10-, MYEOV-, NR2F1-, NRCAM-, PCSK1-, PLXNA2-, SLC30A3-, SNAI1-, SPOCD1-, TFPI2-, HMGA2-, KRTAP4-7-, KRTAP4-8-, KRTAP4-9-, MYPN-, RPPH1- und SIAE-Genen in einer Probe aus dem Individuum; und
(b) Bestimmen, dass das Individuum die Komplikation einer polyzystischen Nierenerkrankung aufweist oder dem Risiko zur Entwicklung der Erkrankung unterliegt, wenn das Expressionsniveau von MMP1 höher ist als in einer Kontrollprobe; und
wenn die Expressionsniveaus von 1 oder mehr Genen ausgewählt aus oder von allen Genen der Gruppe, bestehend aus ACAT2-, INSIG1-, SCD-, BMP6-, CD274-, CTGF-, E2F7-, EDN1-, FAM43A-, FRMD3-, MMP10-, MYEOV-, NR2F1-, NRCAM-, PCSK1-, PLXNA2-, SLC30A3-, SNAI1-, SPOCD1-, TFPI2-, HMGA2-, KRTAP4-7-, KRTAP4-8-, KRTAP4-9-, MYPN-, RPPH1- und SIAE-Genen höher sind als die in der Kontrollprobe, oder wenn die Expressionsniveaus von 1 oder mehr Genen ausgewählt aus oder von allen Genen der Gruppe, bestehend aus NTNG1-, POSTN-, TNC-, KAL1-, BST1-, HSD3B1-, KRT7-, USP40- und SULT1E1-Genen niedriger sind als die in einer Kontrollprobe;
wobei die Komplikation der polyzystischen Nierenkrankheit ausgewählt ist aus der Gruppe, bestehend aus vaskulären Läsionen; Herzklappenerkrankung; Kolondivertikel; Hernie; Ductusläsionen; und Symptomen der Zystenbildung in Leber, Pankreas, Milz und Fortpflanzungsorgan.

2. Verfahren nach Anspruch 1, zudem umfassend:
(a2) Messen der Expressionsniveaus aller Gene aus der Gruppe, bestehend aus CD274, CTGF, MMP10 und NRCAM in der Probe aus dem Individuum; und
(b2) Bestimmen, dass das Individuum die Komplikation der polyzystischen Nierenkrankheit hat oder dem Risiko zur Entwicklung der Komplikation unterliegt, wenn die Expressionsniveaus der CD274-, CTGF-, MMP10- und NRCAM-Gene höher als die in der Kontrollprobe sind.

3. Verfahren nach einem der Ansprüche 1 oder 2, wobei die Komplikation ein zerebrales Aneurysma ist.

4. Verfahren nach einem der Ansprüche 1 bis 3, wobei die Probe ausgewählt ist aus der Gruppe bestehend aus Blut, Serum, Blutplasma, Zellextrakten, Urin, Lymphflüssigkeiten, Gewebeflüssigkeiten, Aszites, Wirbelsäulenflüssigkeiten und anderen Körperflüssigkeiten oder Geweben und Zellen.

5. Verfahren nach einem der Ansprüche 1 bis 3, wobei die Probe eine vaskuläre Endothelzelle ist, die durch Differenzierungsinduktion aus einer iPS-Zelle erhalten wird, die aus einer isolierten somatischen Zelle eines Individuums mit polyzystischer Nierenkrankheit gebildet wird.

6. Verfahren nach Anspruch 5, wobei die Gene in Schritt (a) zudem 1 oder mehr Gene umfassen, ausgewählt aus oder von allen Genen der Gruppe, bestehend aus NTNG1, POSTN, TNC, KAL1, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1 und TFPI2.

7. Verfahren nach einem der Ansprüche 1 bis 3, wobei die Probe eine vaskuläre Wandzelle ist, die durch Differenzierungsinduktion aus einer iPS-Zelle erhalten wird, die aus einer isolierten somatischen Zelle eines Individuums mit polyzystischer Nierenkrankheit gebildet wird.

8. Verfahren nach Anspruch 7, wobei das Gen in Schritt (a) 1 oder mehr Gene ist, ausgewählt aus oder von allen Genen der Gruppe bestehend aus HSD3B1, KRT7, USP40, SULT1E1, TFPI2, HMGA2, KRTAP4-7, KRTAP4-8, KRTAP4-9, MYPN, RPPH1 und SIAE.

9. Verfahren nach einem der Ansprüche 1 bis 8, wobei der Schritt des Messens des Expressionsniveaus des Gens ein Schritt des Messens der Menge an mRNA, cRNA oder cDNA des Gens oder der Menge des Proteins ist, das durch das Gen kodiert wird.

## Revendications

1. Procédé consistant à examiner si un sujet a ou non une complication de la polykystose rénale ou un risque de développer la complication, comprenant les étapes suivantes consistant à :
(a) mesurer le niveau d'expression du gène MMP1 et mesurer les niveaux d'expression de 1 ou plusieurs gène(s) choisi(s) parmi des ou tous les gènes du groupe constitué par les gènes NTNG1, POSTN, TNC, KAL1, BST1, ACAT2, INSIG1, SCD, HSD3B1, KRT7, USP40, SULT1E1, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1 et SIAE dans un échantillon provenant du sujet ; et
(b) déterminer que le sujet a la complication de la polykystose rénale ou le risque de développer la complication quand le niveau d'expression du MMP1 est supérieur à celui dans un échantillon témoin ; et
quand les niveaux d'expression de 1 ou plusieurs gène(s) choisi(s) parmi des ou tous les gènes du groupe constitué par les gènes ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1, TFPI2, HMGA2, KRTAP4-7, KRTAP4-8, KRTAP4-9, MYPN, RPPH1 et SIAE sont supérieurs à ceux dans l'échantillon témoin ou quand les niveaux d'expression de 1 ou plusieurs gène(s) choisi(s) parmi des ou tous les gènes du groupe constitué par les gènes NTNG1, POSTN, TNC, KAL1, BST1, HSD3B1, KRT7, USP40 et SULT1E1 sont inférieurs à ceux dans un échantillon témoin ;
dans lequel la complication de la polykystose rénale est choisie dans le groupe constitué par : des lésions vasculaires ; une cardiopathie valvulaire ; des diverticules du côlon ; une hernie ; des lésions canalaires ; et des symptômes d'une formation de kystes dans le foie, le pancréas, la rate et un organe génital.

2. Procédé, selon la revendication 1, comprenant en outre les étapes consistant à :
(a2) mesurer les niveaux d'expression de tous les gènes du groupe constitué par les CD274, CTGF, MMP10 et NRCAM dans l'échantillon provenant du sujet ; et
(b2) déterminer que le sujet a la complication de la polykystose rénale ou le risque de développer la complication quand les niveaux d'expression des gènes CD274, CTGF, MMP10 et NRCAM sont supérieurs à ceux dans l'échantillon témoin.

3. Procédé selon l'une quelconque des revendications 1 ou 2, dans lequel la complication est un anévrisme cérébral.

4. Procédé selon l'une quelconque des revendications 1 à 3, dans lequel l'échantillon est choisi dans le groupe constitué par le sang, le sérum, le plasma sanguin, des extraits cellulaires, l'urine, des liquides lymphatiques, des liquides tissulaires, des ascites, des liquides céphalorachidiens et d'autres liquides corporels ou tissus et cellules.

5. Procédé selon l'une quelconque des revendications 1 à 3, dans lequel l'échantillon est une cellule endothéliale vasculaire obtenue via une induction de différentiation à partir d'une cellule iPS formée à partir d'une cellule somatique isolée d'un sujet étant atteint d'une polykystose rénale.

6. Procédé selon la revendication 5, dans lequel les gènes dans l'étape (a) comprennent en outre 1 ou plusieurs gène(s) choisi(s) parmi des ou tous les gènes du groupe constitué par les NTNG1, POSTN, TNC, KAL1, BST1, ACAT2, INSIG1, SCD, BMP6, CD274, CTGF, E2F7, EDN1, FAM43A, FRMD3, MMP10, MYEOV, NR2F1, NRCAM, PCSK1, PLXNA2, SLC30A3, SNAI1, SPOCD1 et TFPI2.

7. Procédé selon l'une quelconque des revendications 1 à 3, dans lequel l'échantillon est une cellule murale vasculaire obtenue via une induction de différentiation à partir d'une cellule iPS formée à partir d'une cellule somatique isolée d'un sujet étant atteint d'une polykystose rénale.

8. Procédé selon la revendication 7, dans lequel le gène dans l'étape (a) est 1 ou plusieurs gène(s) choisi(s) parmi des ou tous les gènes du groupe constitué par les HSD3B1, KRT7, USP40, SULT1E1, TFPI2, HMGA2, KRTAP4-7, KRTAP4-8, KRTAP4-9, MYPN, RPPH1 et SIAE.

9. Procédé selon l'une quelconque des revendications 1 à 8, dans lequel l'étape de mesure du niveau d'expression du gène est une étape de mesure de la quantité d'ARNm, d'ARNc ou d'ADNc du gène ou de la quantité de protéine codée par le gène.
